# EUROPEAN PATENT APPLICATION

(11) **EP 3 369 822 A1**
(43) Date of publication of application: **05.09.2018**
(21) Application number: 18162793.6
(22) Date of filing: 02.05.2012
(51) Int. Cl.: C12N 15/82, C07K 14/435, A01H 5/00, A01H 5/10

(54) **DOWNREGULATION OF GENE EXPRESSION IN PLANT PATHOGENS**

(30) Priority: 18.07.2011 US 201161508826 P; 20.04.2012 WO PCT/EP2012/057333
(62) Divisional of application: 12721445.0
(71) Applicant: Devgen NV, 9052 Gent (BE)
(72) Inventor: BEGHYN, Myriam, 9052 Gent (BE); RAEMAEKERS, Romaan Jan Maria, 8052 Gent (BE); BOGAERT, Thierry, 9052 Gent (BE); FELDMANN, Pascale, 9052 Gent (BE)
(74) Representative: Syngenta International AG

(57) **Abstract**

The present invention relates to genetic control of infestation by insect pest species, particularly prevention and/or control of pest infestation of plants, using interfering ribonucleic acid (RNA) molecules. The invention provides transgenic plants that (i) express or are capable of expressing interfering RNAs of the invention and (ii) are resistant to infestation by insect pest species.

## Description

### Field of the invention

The present invention relates generally to genetic control of infestation by insect pest species, particularly prevention and/or control of pest infestation of plants. More specifically, the invention relates to down-regulation of expression of target genes in insect pest species by interfering ribonucleic acid (RNA) molecules. Also provided are transgenic plants that (i) express or are capable of expressing interfering RNAs of the invention and (ii) are resistant to infestation by insect pest species.

### Background to the invention

There exists an abundance of insect pest species that can infect or infest a wide variety of environments and host organisms. Insect pests include a variety of species from the insect Orders *Hemiptera* (true bugs), *Coleoptera* (beetles), *Siphonaptera* (fleas), *Dichyoptera* (cockroaches and mantids), *Lepidoptera* (moths and butterflies), *Orthoptera* (e.g. grasshoppers) and *Diptera* (true flies). Pest infestation can lead to significant damage. Insect pests that infest plant species are particularly problematic in agriculture as they can cause serious damage to crops and significantly reduce plant yields. A wide variety of different types of plant are susceptible to pest infestation including commercial crops such as rice, cotton, soybean, potato and corn.

Traditionally, infestation with insect pests has been prevented or controlled through the use of chemical pesticides. However, these chemicals are not always suitable for use in the treatment of crops as they can be toxic to other species and can cause significant environmental damage. Over more recent decades, researchers have developed more environmentally-friendly methods of controlling pest infestation. For example, microorganisms such as *Bacillus thuringiensis* bacteria that naturally express proteins toxic to insect pests have been used. Scientists have also isolated the genes encoding these insecticidal proteins and used them to generate transgenic crops resistant to insect pests *e.g.* corn and cotton plants genetically engineered to produce proteins of the Cry family. Although bacterial toxins have been highly successful in controlling certain types of pest, they are not effective against all pest species. Researchers have therefore looked for other more targeted approaches to pest control and in particular to RNA interference or 'gene silencing' as a means to control pests at the genetic level.

RNA interference or 'RNAi' is a process whereby the expression of genes in the context of a cell or whole organism is down-regulated in a sequence-specific manner. RNAi is now a well-established technique in the art for inhibiting or down-regulating gene expression in a wide variety of organisms including pest organisms such as fungi, nematodes and insects. Furthermore, previous studies have shown that down-regulation of target genes in insect pest species can be used as a means to control pest infestation.

WO2007/074405 describes methods of inhibiting expression of target genes in invertebrate pests including Colorado potato beetle. Furthermore, WO2009/091864 describes compositions and methods for the suppression of target genes from insect pest species including pests from the *Lygus* genus.

Although the use of RNAi for down-regulating gene expression in pest species is known in the art, the success of this technique for use as a pest control measure depends on selection of the most appropriate target genes, namely those wherein loss of function results in significant disruption of an essential biological process and/or death of the organism. The present invention is thus directed towards the down-regulation of particular target genes in insect pests as a means to achieve more effective prevention and/or control of insect pest infestation, particularly of plants.

### Summary of the invention

The current inventors sought to identify improved means for preventing and/or controlling insect pest infestation using genetic approaches. In particular, they investigated the use of RNAi to down-regulate genes in such a way as to impair the ability of the insect pest to survive, grow, progress through different stages of the insect's life cycle (for instance through metamorphosis from pupae to adult), colonize specific environments and/or infest host organisms and thus limit the damage caused by the pest.

Therefore, in accordance with one aspect of the invention, there is provided a transgenic plant, or reproductive or propagation material for a transgenic plant or a cultured transgenic plant cell, which expresses or is capable of expressing at least one an interfering ribonucleic acid (RNA or double stranded RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 or the complement thereof, or having a nucleotide sequence that, when the two sequences are optimally aligned and compared, is at least 75%, preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 or the complement thereof, or
(ii) is selected from the group of genes having a nucleotide sequence consisting of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(iii) is selected from the group of genes having a nucleotide sequence comprising a fragment of at least 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or having a nucleotide sequence that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(iv) is selected from the group of genes having a nucleotide sequence comprising a fragment of at least 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence of said fragment is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(v) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or
(vi) is selected from the group of genes having a nucleotide sequence encoding an amino acid sequence that, when the two amino acid sequences are optimally aligned and compared, is at least 70% preferably at least 75%, 80%, 85%, 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389.

In a particular aspect of the invention, interfering RNA molecules expressed by the plants of the current invention comprise at least one double-stranded region, typically the silencing element of the interfering RNA, comprising a sense RNA strand annealed by complementary basepairing to an antisense RNA strand wherein the sense strand of the dsRNA molecule comprises a sequence of nucleotides complementary to a sequence of nucleotides located within the RNA transcript of the target gene.

In one embodiment, the present invention relates to a transgenic plant, or reproductive or propagation material for a transgenic plant or a cultured transgenic plant cell which expresses or is capable of expressing an interfering RNA molecule which comprises at least one double-stranded region, typically the silencing element of the interfering RNA molecule, comprising a sense RNA strand annealed by complementary basepairing to an antisense RNA strand wherein the sense strand of the dsRNA molecule comprises a sequence of at least 17, 18, 19, 20, 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides, that is at least 75% preferably at least 80%, 85%, 90%, 95%, 98%, 99% or 100% complementary to a sequence of nucleotides located within the RNA transcript of a target gene from the troponin/myofilament complex.

In one embodiment, the target gene encodes an insect wings up A (troponin I) protein (e.g. an insect orthologue of the CG7178 Dm protein), said target gene being represented by SEQ ID NOs 1, 2, 174, 404, 175, 180, 181, 188 and 189. In a preferred embodiment, the insect orthologue has at least 85%, 90%, 92%, 94%, 96%, 98%, 99% or 100% amino acid sequence identity to one or more of SEQ ID NOs. 79, 349, 405, 352 or 356.

In one embodiment, the target gene encodes an upheld protein (e.g. an insect orthologue of the CG7107 Dm protein), said target gene being represented by SEQ ID NOs 121, 130, 142, 143, 176, 177, 182 and 183. In a preferred embodiment, the insect orthologue has at least 85%, 90%, 92%, 94%, 96%, 98%, 99% or 100% amino acid sequence identity to one or more of SEQ ID NOs. 330, 350 or 353.

In one embodiment, the target gene encodes the tropomyosin 1 protein (e.g. an insect orthologue of the CG4898 Dm protein), or the tropomyosin 2 protein (e.g. an insect orthologue of the CG4843 Dm protein), said target gene being represented by SEQ ID NOs 123 and 132. In a preferred embodiment, the insect orthologue has at least 85%, 90%, 92%, 94%, 96%, 98%, 99% or 100% amino acid sequence identity to SEQ ID NO. 332.

In one embodiment, the target gene encodes the myosin heavy chain (e.g. an insect orthologue of the CG17927 Dm protein), said target gene being represented by SEQ ID NOs 122, 131, 144, 145, 178 and 179. In a preferred embodiment, the insect orthologue has at least 85%, 90%, 92%, 94%, 96%, 98%, 99% or 100% amino acid sequence identity to one or more of SEQ ID NOs. 331 or 351.

In one embodiment, the target gene encodes the myosin light chain cytoplasmic protein (e.g. an insect orthologue of the CG3201 Dm protein), said target gene being represented by SEQ ID NOs 124 and 133. In a preferred embodiment, the insect orthologue has at least 85%, 90%, 92%, 94%, 96%, 98%, 99% or 100% amino acid sequence identity to SEQ ID NO. 333.

In one embodiment, the target gene encodes the spaghetti squash protein (e.g. an insect orthologue of the CG3595 Dm protein), said target gene being represented by SEQ ID NOs 125 and 134. In a preferred embodiment, the insect orthologue has at least 85%, 90%, 92%, 94%, 96%, 98%, 99% or 100% identity to SEQ ID NO. 334.

In one embodiment, the target gene encodes the zipper protein (e.g. an insect orthologue of the CG15792 Dm protein), said target gene being represented by SEQ ID NOs 126 and 135. In a preferred embodiment, the insect orthologue has at least 85%, 90%, 92%, 94%, 96%, 98%, 99% or 100% identity to SEQ ID NO. 335.

In one embodiment, the target gene encodes the troponin C (e.g. an insect orthologue of the CG2981, CG7930, CG9073, CG6514, CG12408, CG9073, CG7930, CG2981, CG12408 or CG6514 Dm protein), said target gene being represented by SEQ ID NOs 127 and 136, or 128 and 137, or 184 and 185. In a preferred embodiment, the insect orthologue has at least 85%, 90%, 92%, 94%, 96%, 98%, 99% or 100% amino acid sequence identity to one or more of SEQ ID NOs. 336, 337 and 354.

According to another aspect the present invention relates to a transgenic plant, or reproductive or propagation material for a transgenic plant or a cultured transgenic plant cell which expresses or is capable of expressing an interfering RNA molecule which comprises at least one double-stranded region, typically the silencing element of the interfering RNA molecule, comprising a sense RNA strand annealed by complementary basepairing to an antisense RNA strand wherein the sense strand of the dsRNA molecule comprises a sequence of at least 17, 18, 19, 20, 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides, that is at least 75% preferably at least 80%, 85%, 90%, 95%, 98%, 99% or 100% complementary to a sequence of nucleotides located within the RNA transcript of a target gene that encodes an insect ribosomal protein.

In one embodiment, the target gene encodes ribosomal protein S3A (e.g. an insect orthologue of the CG2168 Dm protein), said target gene being represented by SEQ ID NOs 11, 12 and 141. In a preferred embodiment, the insect orthologue has at least 85%, 90%, 92%, 94%, 96%, 98%, 99% or 100% amino acid sequence identity to one or both of SEQ ID NO. 84 or 328.

In one embodiment, the target gene encodes the ribosomal protein LP1 (e.g. an insect orthologue of the CG4087 Dm protein), said target gene being represented by SEQ ID NO 3 and 4. In a preferred embodiment, the insect orthologue has at least 85%, 90%, 92%, 94%, 96%, 98%, 99% or 100% amino acid sequence identity to SEQ ID NO.80.

In one embodiment, the target gene encodes the ribosomal protein S3 (e.g. an insect orthologue of the CG6779 Dm protein), said target gene being represented by SEQ ID NOs 7 and 8. In a preferred embodiment, the insect orthologue has at least 85%, 90%, 92%, 94%, 96%, 98%, 99% or 100% amino acid sequence identity to SEQ ID NO.82.

In one embodiment, the target gene encodes the ribosomal protein L10Ab (e.g. an insect orthologue of the CG7283 Dm protein) represented by SEQ ID NOs 9 and 10. In a preferred embodiment, the insect orthologue has at least 85%, 90%, 92%, 94%, 96%, 98%, 99% or 100% amino acid sequence identity to SEQ ID NO. 83.

In one embodiment, the target gene encodes the ribosomal protein S18 (e.g. an insect orthologue of the CG8900 Dm protein), said target gene being represented by SEQ ID NO 13 and 14. In a preferred embodiment, the insect orthologue has at least 85%, 90%, 92%, 94%, 96%, 98%, 99% or 100% amino acid sequence identity to SEQ ID NO.85.

In one embodiment, the target gene encodes the ribosomal protein L4 (e.g. an insect orthologue of the CG5502 Dm protein), said target gene represented by SEQ ID NO 5 and 6. In a preferred embodiment, the insect orthologue has at least 85%, 90%, 92%, 94%, 96%, 98%, 99% or 100% amino acid sequence identity to SEQ ID NO.81.

In one embodiment, the target gene encodes the ribosomal protein S27 (e.g. an insect orthologue of the CG10423 Dm protein), said target gene being represented by SEQ ID NO 15 and 16, 204 and 205. In a preferred embodiment, the insect orthologue has at least 85%, 90%, 92%, 94%, 96%, 98%, 99% or 100% amino acid sequence identity to one or both of SEQ ID NOs.86 and 359.

In one embodiment, the target gene encodes the ribosomal protein L6 (e.g. an insect orthologue of the CG11522 Dm protein), said target gene being represented by SEQ ID NO 17 and 18. In a preferred embodiment, the insect orthologue has at least 85%, 90%, 92%, 94%, 96%, 98%, 99% or 100% amino acid sequence identity to SEQ ID NO. 87.

In one embodiment, the target gene encodes the ribosomal protein S13 (e.g. an insect orthologue of the CG13389 Dm protein), said target gene being represented by SEQ ID NO 19 and 20. In a preferred embodiment, the insect orthologue has at least 85%, 90%, 92%, 94%, 96%, 98%, 99% or 100% amino acid sequence identity to SEQ ID NO. 88.

In one embodiment, the target gene encodes the ribosomal protein L12 (e.g. an insect orthologue of the CG3195 Dm protein), said target gene being represented by SEQ ID NOs 21 and 22. In a preferred embodiment, the insect orthologue has at least 85%, 90%, 92%, 94%, 96%, 98%, 99% or 100% amino acid sequence identity to SEQ ID NO.89.

In one embodiment, the target gene encodes the ribosomal protein L26 (e.g. an insect orthologue of the CG6846 Dm protein), said target gene being represented by SEQ ID NOs 158 and 159. In a preferred embodiment, the insect orthologue has at least 85%, 90%, 92%, 94%, 96%, 98%, 99% or 100% amino acid sequence identity to SEQ ID NO. 343.

In one embodiment, the target gene encodes the ribosomal protein L21 (e.g. an insect orthologue of the CG12775 Dm protein), said target gene being represented by SEQ ID NO 165, 166 and 167. In a preferred embodiment, the insect orthologue has at least 85%, 90%, 92%, 94%, 96%, 98%, 99% or 100% amino acid sequence identity to SEQ ID NOs 347 and 348.

In one embodiment, the target gene encodes the ribosomal protein S12 (e.g. an insect orthologue of the CG11271 Dm protein), said target gene being represented by SEQ ID NOs 156 and 157. In a preferred embodiment, the insect orthologue has at least 85%, 90%, 92%, 94%, 96%, 98%, 99% or 100% amino acid sequence identity to SEQ ID NO. 342.

In one embodiment, the target gene encodes the ribosomal protein S28b (e.g. an insect orthologue of the CG2998 Dm protein), said target gene being represented by SEQ ID NOs 160 and 161. In a preferred embodiment, the insect orthologue has at least 85%, 90%, 92%, 94%, 96%, 98%, 99% or 100% amino acid sequence identity to SEQ ID NO. 344.

In one embodiment, the target gene encodes the ribosomal protein L13 (e.g. an insect orthologue of the CG4651 Dm protein), said target gene being represented by SEQ ID NOs. 154 and 155. In a preferred embodiment, the insect orthologue has at least 85%, 90%, 92%, 94%, 96%, 98%, 99% or 100% amino acid sequence identity to SEQ ID NO. 341.

In one embodiment, the target gene encodes the ribosomal protein L10 (e.g. an insect orthologue of the CG17521 Dm protein), said target gene being represented by SEQ ID NOs. 163 and 164. In a preferred embodiment, the insect orthologue has at least 85%, 90%, 92%, 94%, 96%, 98%, 99% or 100% amino acid sequence identity to SEQ ID NO. 345.

In one embodiment, the target gene encodes the ribosomal protein L5 (e.g. an insect orthologue of the CG17489 Dm protein), said target gene being represented by SEQ ID NOs. 152 and 153. In a preferred embodiment, the insect orthologue has at least 85%, 90%, 92%, 94%, 96%, 98%, 99% or 100% amino acid sequence identity to SEQ ID NO. 340.

In one embodiment, the target gene encodes the ribosomal protein S15Aa (e.g. an insect orthologue of the CG2033 Dm protein), said target gene being represented by SEQ ID NOs. 150 and 151. In a preferred embodiment, the insect orthologue has at least 85%, 90%, 92%, 94%, 96%, 98%, 99% or 100% amino acid sequence identity to SEQ ID NO. 339.

In one embodiment, the target gene encodes the ribosomal protein L19 (e.g. an insect orthologue of the CG2746 Dm protein), said target gene being represented by SEQ ID NOs. 200 and 201. In a preferred embodiment, the insect orthologue has at least 85%, 90%, 92%, 94%, 96%, 98%, 99% or 100% amino acid sequence identity to SEQ ID NO.357.

In one embodiment, the target gene encodes the ribosomal protein L27 (e.g. an insect orthologue of the CG4759 Dm protein), said target gene being represented by SEQ ID NO. 386. In a preferred embodiment, the insect orthologue has at least 85%, 90%, 92%, 94%, 96%, 98%, 99% or 100% amino acid sequence identity to SEQ ID NO.390.

In one embodiment, the target gene encodes the mitochondrial cytochrome c oxidase subunit II protein (e.g. an insect orthologue of the CG34069 Dm protein), said target gene being represented by SEQ ID NO 25 and 26. In a preferred embodiment, the insect orthologue has at least 85%, 90%, 92%, 94%, 96%, 98%, 99% or 100% amino acid sequence identity to SEQ ID NO. 91.

In one embodiment, the target gene encodes the ATP synthase-γ chain (e.g. an insect orthologue of the CG7610 Dm protein), said target gene being represented by SEQ ID NOs 129 and 138. In a preferred embodiment, the insect orthologue has at least 85%, 90%, 92%, 94%, 96%, 98%, 99% or 100% amino acid sequence identity to SEQ ID NO. 338.

In one embodiment, the target gene encodes the ubiquitin-5E (e.g. an insect orthologue of the CG32744 Dm protein) said target gene being represented by SEQ ID NOs. 186 and 187, 202 and 203. In a preferred embodiment, the insect orthologue has at least 85%, 90%, 92%, 94%, 96%, 98%, 99% or 100% amino acid sequence identity to one or both of SEQ ID NOs.355 and 358.

In one embodiment, the target gene encodes the proteasome beta-type subunit (e.g. an insect orthologue of the CG17331 Dm protein) said target gene being represented by SEQ ID NO. 387. In a preferred embodiment, the insect orthologue has at least 85%, 90%, 92%, 94%, 96%, 98%, 99% or 100% amino acid sequence identity to SEQ ID NO.391.

In one embodiment, the target gene encodes the protein which is an insect orthologue of the CG13704 Dm protein, said target gene being represented by SEQ ID NO.388. In a preferred embodiment, the insect orthologue has at least 85%, 90%, 92%, 94%, 96%, 98%, 99% or 100% amino acid sequence identity to SEQ ID NO.392.

In one embodiment, the target gene encodes the Rpn12 protein (e.g. an insect orthologue of the CG4157 Dm protein) said target gene being represented by SEQ ID NO. 389. In a preferred embodiment, the insect orthologue has at least 85%, 90%, 92%, 94%, 96%, 98%, 99% or 100% amino acid sequence identity to SEQ ID NO.393.

In accordance with a further aspect of the invention, there is provided an isolated polynucleotide selected from the group consisting of:
(i) a polynucleotide which comprises at least 21, preferably at least 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 or the complement thereof, or
(ii) a polynucleotide which consists of at least 21, preferably at least 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(iii) a polynucleotide which comprises at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, that, when the two sequences are optimally aligned and compared, said polynucleotide is at least 75 % preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(iv) a polynucleotide which comprises a fragment of at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide as represented in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein said fragment or said complement has a nucleotide sequence that, when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(v) a polynucleotide which consists of a fragment of at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide as represented in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein said fragment or said complement has a nucleotide sequence that, when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(vi) a polynucleotide encoding an amino acid sequence that, when the two amino acid sequences are optimally aligned and compared, is at least 70 % preferably at least 75%, 80%, 85%, 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 and
wherein said polynucleotide is no longer than 10000, 9000, 8000, 7000, 6000, 5000, 4000, 3000, 2000 or 1500 nucleotides.

The amino acid sequences encoded by the target genes of the invention are represented by SEQ ID NOs 79, 349, 405, 352, 356, 80, 326, 81, 327, 82, 83, 328, 84, 329, 85, 86, 359, 87 to 91, 330, 350, 353, 331, 351, 332 to 336, 337, 354, 338 to 344, 346, 345, 347, 348, 357, 355, 358, 390 to 393.

In a particular aspect of the invention, the isolated polynucleotide is part of an interfering RNA molecule, typically part of the silencing element, comprising at least one double-stranded region comprising a sense RNA strand annealed by complementary basepairing to an antisense RNA strand wherein the sense strand of the dsRNA molecule comprises a sequence of nucleotides complementary to a sequence of nucleotides located within the RNA transcript of the target gene. More particularly, the isolated polynucleotide is cloned in a DNA construct in a sense and antisense orientation so that the upon transcription of the sense and antisense polynucleotide a dsRNA molecule is formed, which functions upon uptake by a pest to inhibit or down-regulate the expression of a target gene within said pest.

In certain aspects the present invention relates to isolated polynucleotides that are cloned in a DNA construct in a sense and antisense orientation so that the upon transcription of the sense and antisense polynucleotide a dsRNA molecule is formed, which functions upon uptake by an insect to inhibit or down-regulate the expression of a target gene within the troponin/myofilament complex.

In one embodiment, the target gene encodes an insect wings up A (troponin I) protein (e.g. an insect orthologue of the CG7178 Dm protein), said target gene being represented by SEQ ID NOs 1, 2, 174, 404, 175, 180, 181, 188 and 189. In a preferred embodiment, the insect orthologue has at least 85%, 90%, 92%, 94%, 96%, 98%, 99% or 100% amino acid sequence identity to one or more of SEQ ID NOs. 79, 349, 405, 352 or 356.

In one embodiment, the target gene encodes an upheld protein (e.g. an insect orthologue of the CG7107 Dm protein), said target gene being represented by SEQ ID NOs 121, 130, 142, 143, 176, 177, 182 and 183. In a preferred embodiment, the insect orthologue has at least 85%, 90%, 92%, 94%, 96%, 98%, 99% or 100% amino acid sequence identity to one or more of SEQ ID NOs. 330, 350 or 353.

In one embodiment, the target gene encodes the tropomyosin 1 protein (e.g. an insect orthologue of the CG4898 Dm protein), or the tropomyosin 2 protein (e.g. an insect orthologue of the CG4843 Dm protein), said target gene being represented by SEQ ID NOs 123 and 132. In a preferred embodiment, the insect orthologue has at least 85%, 90%, 92%, 94%, 96%, 98%, 99% or 100% amino acid sequence identity to SEQ ID NO. 332.

In one embodiment, the target gene encodes the myosin heavy chain (e.g. an insect orthologue of the CG17927 Dm protein), said target gene being represented by SEQ ID NOs 122, 131, 144, 145, 178 and 179. In a preferred embodiment, the insect orthologue has at least 85%, 90%, 92%, 94%, 96%, 98%, 99% or 100% amino acid sequence identity to one or more of SEQ ID NOs. 331 or 351.

In one embodiment, the target gene encodes the myosin light chain cytoplasmic protein (e.g. an insect orthologue of the CG3201 Dm protein), said target gene being represented by SEQ ID NOs 124 and 133. In a preferred embodiment, the insect orthologue has at least 85%, 90%, 92%, 94%, 96%, 98%, 99% or 100% amino acid sequence identity to SEQ ID NO. 333.

In one embodiment, the target gene encodes the spaghetti squash protein (e.g. an insect orthologue of the CG3595 Dm protein), said target gene being represented by SEQ ID NOs 125 and 134. In a preferred embodiment, the insect orthologue has at least 85%, 90%, 92%, 94%, 96%, 98%, 99% or 100% identity to SEQ ID NO. 334.

In one embodiment, the target gene encodes the zipper protein (e.g. an insect orthologue of the CG15792 Dm protein), said target gene being represented by SEQ ID NOs 126 and 135. In a preferred embodiment, the insect orthologue has at least 85%, 90%, 92%, 94%, 96%, 98%, 99% or 100% identity to SEQ ID NO. 335.

In one embodiment, the target gene encodes the troponin C (e.g. an insect orthologue of the CG2981, CG7930, CG9073, CG6514, CG12408, CG9073, CG7930, CG2981, CG12408 or CG6514 Dm protein), said target gene being represented by SEQ ID NOs 127 and 136, or 128 and 137, or 184 and 185. In a preferred embodiment, the insect orthologue has at least 85%, 90%, 92%, 94%, 96%, 98%, 99% or 100% amino acid sequence identity to one or more of SEQ ID NOs. 336, 337 and 354.

According to other embodiments, the present invention relates to an isolated polynucleotide that is cloned in a DNA construct in a sense and antisense orientation so that the upon transcription of the sense and antisense polynucleotide a dsRNA molecule is formed, which functions upon uptake by an insect to inhibit or down-regulate the expression of a target gene that encodes an insect ribosomal protein.

In one embodiment, the target gene encodes ribosomal protein S3A (e.g. an insect orthologue of the CG2168 Dm protein), said target gene being represented by SEQ ID NOs 11, 12 and 141. In a preferred embodiment, the insect orthologue has at least 85%, 90%, 92%, 94%, 96%, 98%, 99% or 100% amino acid sequence identity to one or both of SEQ ID NO. 84 or 328.

In one embodiment, the target gene encodes the ribosomal protein LP1 (e.g. an insect orthologue of the CG4087 Dm protein), said target gene being represented by SEQ ID NO 3 and 4. In a preferred embodiment, the insect orthologue has at least 85%, 90%, 92%, 94%, 96%, 98%, 99% or 100% amino acid sequence identity to SEQ ID NO.80.

In one embodiment, the target gene encodes the ribosomal protein S3 (e.g. an insect orthologue of the CG6779 Dm protein), said target gene being represented by SEQ ID NOs 7 and 8. In a preferred embodiment, the insect orthologue has at least 85%, 90%, 92%, 94%, 96%, 98%, 99% or 100% amino acid sequence identity to SEQ ID NO.82.

In one embodiment, the target gene encodes the ribosomal protein L10Ab (e.g. an insect orthologue of the CG7283 Dm protein) represented by SEQ ID NOs 9 and 10. In a preferred embodiment, the insect orthologue has at least 85%, 90%, 92%, 94%, 96%, 98%, 99% or 100% amino acid sequence identity to SEQ ID NO. 83.

In one embodiment, the target gene encodes the ribosomal protein S18 (e.g. an insect orthologue of the CG8900 Dm protein), said target gene being represented by SEQ ID NO 13 and 14. In a preferred embodiment, the insect orthologue has at least 85%, 90%, 92%, 94%, 96%, 98%, 99% or 100% amino acid sequence identity to SEQ ID NO.85.

In one embodiment, the target gene encodes the ribosomal protein L4 (e.g. an insect orthologue of the CG5502 Dm protein), said target gene represented by SEQ ID NO 5 and 6. In a preferred embodiment, the insect orthologue has at least 85%, 90%, 92%, 94%, 96%, 98%, 99% or 100% amino acid sequence identity to SEQ ID NO.81.

In one embodiment, the target gene encodes the ribosomal protein S27 (e.g. an insect orthologue of the CG10423 Dm protein), said target gene being represented by SEQ ID NO 15 and 16, 204 and 205. In a preferred embodiment, the insect orthologue has at least 85%, 90%, 92%, 94%, 96%, 98%, 99% or 100% amino acid sequence identity to one or both of SEQ ID NOs.86 and 359.

In one embodiment, the target gene encodes the ribosomal protein L6 (e.g. an insect orthologue of the CG11522 Dm protein), said target gene being represented by SEQ ID NO 17 and 18. In a preferred embodiment, the insect orthologue has at least 85%, 90%, 92%, 94%, 96%, 98%, 99% or 100% amino acid sequence identity to SEQ ID NO. 87.

In one embodiment, the target gene encodes the ribosomal protein S13 (e.g. an insect orthologue of the CG13389 Dm protein), said target gene being represented by SEQ ID NO 19 and 20. In a preferred embodiment, the insect orthologue has at least 85%, 90%, 92%, 94%, 96%, 98%, 99% or 100% amino acid sequence identity to SEQ ID NO. 88.

In one embodiment, the target gene encodes the ribosomal protein L12 (e.g. an insect orthologue of the CG3195 Dm protein), said target gene being represented by SEQ ID NOs 21 and 22. In a preferred embodiment, the insect orthologue has at least 85%, 90%, 92%, 94%, 96%, 98%, 99% or 100% amino acid sequence identity to SEQ ID NO.89.

In one embodiment, the target gene encodes the ribosomal protein L26 (e.g. an insect orthologue of the CG6846 Dm protein), said target gene being represented by SEQ ID NOs 158 and 159. In a preferred embodiment, the insect orthologue has at least 85%, 90%, 92%, 94%, 96%, 98%, 99% or 100% amino acid sequence identity to SEQ ID NO. 343.

In one embodiment, the target gene encodes the ribosomal protein L21 (e.g. an insect orthologue of the CG12775 Dm protein), said target gene being represented by SEQ ID NO 165, 166 and 167. In a preferred embodiment, the insect orthologue has at least 85%, 90%, 92%, 94%, 96%, 98%, 99% or 100% amino acid sequence identity to SEQ ID NOs 347 and 348.

In one embodiment, the target gene encodes the ribosomal protein S12 (e.g. an insect orthologue of the CG11271 Dm protein), said target gene being represented by SEQ ID NOs 156 and 157. In a preferred embodiment, the insect orthologue has at least 85%, 90%, 92%, 94%, 96%, 98%, 99% or 100% amino acid sequence identity to SEQ ID NO. 342.

In one embodiment, the target gene encodes the ribosomal protein S28b (e.g. an insect orthologue of the CG2998 Dm protein), said target gene being represented by SEQ ID NOs 160 and 161. In a preferred embodiment, the insect orthologue has at least 85%, 90%, 92%, 94%, 96%, 98%, 99% or 100% amino acid sequence identity to SEQ ID NO. 344.

In one embodiment, the target gene encodes the ribosomal protein L13 (e.g. an insect orthologue of the CG4651 Dm protein), said target gene being represented by SEQ ID NOs. 154 and 155. In a preferred embodiment, the insect orthologue has at least 85%, 90%, 92%, 94%, 96%, 98%, 99% or 100% amino acid sequence identity to SEQ ID NO. 341.

In one embodiment, the target gene encodes the ribosomal protein L10 (e.g. an insect orthologue of the CG17521 Dm protein), said target gene being represented by SEQ ID NOs. 163 and 164. In a preferred embodiment, the insect orthologue has at least 85%, 90%, 92%, 94%, 96%, 98%, 99% or 100% amino acid sequence identity to SEQ ID NO. 345.

In one embodiment, the target gene encodes the ribosomal protein L5 (e.g. an insect orthologue of the CG17489 Dm protein), said target gene being represented by SEQ ID NOs. 152 and 153. In a preferred embodiment, the insect orthologue has at least 85%, 90%, 92%, 94%, 96%, 98%, 99% or 100% amino acid sequence identity to SEQ ID NO. 340.

In one embodiment, the target gene encodes the ribosomal protein S15Aa (e.g. an insect orthologue of the CG2033 Dm protein), said target gene being represented by SEQ ID NOs. 150 and 151. In a preferred embodiment, the insect orthologue has at least 85%, 90%, 92%, 94%, 96%, 98%, 99% or 100% amino acid sequence identity to SEQ ID NO. 339.

In one embodiment, the target gene encodes the ribosomal protein L19 (e.g. an insect orthologue of the CG2746 Dm protein), said target gene being represented by SEQ ID NOs. 200 and 201. In a preferred embodiment, the insect orthologue has at least 85%, 90%, 92%, 94%, 96%, 98%, 99% or 100% amino acid sequence identity to SEQ ID NO.357.

In one embodiment, the target gene encodes the ribosomal protein L27 (e.g. an insect orthologue of the CG4759 Dm protein), said target gene being represented by SEQ ID NO. 386. In a preferred embodiment, the insect orthologue has at least 85%, 90%, 92%, 94%, 96%, 98%, 99% or 100% amino acid sequence identity to SEQ ID NO.390.

Preferably, the methods of the invention find practical application in the prevention and/or control of insect pest infestation, in particular, control of pest infestation of crop plants such as but not limited to cotton, potato, rice, strawberries, alfalfa, soy, tomato, canola, sunflower, sorghum, pearl millet, corn, eggplant, pepper and tobacco. In addition, the interfering RNA of the invention may be introduced into the plants to be protected by routine genetic engineering techniques.

Therefore, in accordance with another aspect of the invention, there is provided a method for generating a transgenic plant resistant to infestation by an insect pest species comprising:
(a) transforming a plant cell with a DNA construct comprising a polynucleotide sequence encoding an interfering ribonucleic acid (RNA) that functions upon uptake by an insect pest species to down-regulate expression of a target gene in said insect pest species, wherein the target gene
   (i) is selected from the group of genes having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or having a nucleotide sequence that, when the two sequences are optimally aligned and compared, is at least 75%, preferably at least 80%, 85%, 90%, 95%, 98% or 99% identical any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
   (ii) is selected from the group of genes having a nucleotide sequence consisting of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
   (iii) is selected from the group of genes having a nucleotide sequence comprising a fragment of at least 20, 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or having a nucleotide sequence that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
   (iv) is selected from the group of genes having a nucleotide sequence comprising a fragment of at least 20, 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence of said fragment is at least 75% preferably at least 80%, 85%, 90%, 95%, 98% or 99% identical to said corresponding fragment of any of SEQ ID NOs. 1-26, 121-205, 386-389, 394, 400, or the complement thereof, or
   (v) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or
   (vi) is selected from the group of genes having a nucleotide sequence encoding an amino acid sequence that, when the two amino acid sequences are optimally aligned and compared, is at least 70% preferably at least 75%, 80%, 85%, 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389;
(b) regenerating a plant from the transformed plant cell; and
(c) growing the transformed plant under conditions suitable for the expression of the interfering RNA from the recombinant DNA construct, said plant thus being resistant to said pest as compared with an untransformed plant.

In a further aspect, provided herein is a method for preventing and/or controlling insect pest infestation in a field of crop plants, said method comprising expressing in said plants an effective amount of an interfering ribonucleic acid (RNA) that functions upon uptake by an insect pest species to down-regulate expression of a target gene in said insect pest species, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or having a nucleotide sequence so that, when the two sequences are optimally aligned and compared, is at least 75% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(ii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence is at least 75% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(iii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence of said fragment is at least 75% identical to said corresponding fragment of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or
(iv) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, wherein the two orthologous genes are similar in sequence to such a degree that when the two genes are optimally aligned and compared, the orthologue has a sequence that is at least 75% identical to any of the sequences represented by SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or
(v) is selected from the group of genes having a nucleotide sequence encoding an amino acid sequence that, when the two amino acid sequences are optimally aligned and compared, is at least 85% identical to the amino acid sequence encoded by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389.

In all aspects of the invention, in preferred embodiments, the target gene
(i) is selected from the group of genes having a nucleotide sequence comprising any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233 or the complement thereof, or having a nucleotide sequence so that, when the two sequences are optimally aligned and compared, is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or
(ii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or
(iii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233 said nucleotide sequence of said fragment is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or
(iv) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233.

In a preferred embodiment, the target gene encodes an insect protein chosen from the troponin/myofilament complex chosen from the group comprising the troponin I protein (e.g. an insect orthologue of the CG7178 Dm protein), the upheld protein (e.g. an insect orthologue of the CG7107 Dm protein), the tropomyosin 1 protein (e.g. an insect orthologue of the CG4898 Dm protein), the tropomyosin 2 protein (e.g. an insect orthologue of the CG4843 Dm protein), the myosin heavy chain (e.g. an insect orthologue of the CG17927 Dm protein), the myosin light chain cytoplasmic protein (e.g. an insect orthologue of the CG3201 Dm protein), the spaghetti squash protein (e.g. an insect orthologue of the CG3595 Dm protein), the zipper protein (e.g. an insect orthologue of the CG15792 Dm protein), and the troponin C protein (e.g. an insect orthologue of the CG2981, CG7930, CG9073, CG6514, CG12408, CG9073, CG7930, CG2981, CG12408 or CG6514 Dm protein).

In all aspects of the invention, in preferred embodiments, the target gene
(i) is selected from the group of genes having a nucleotide sequence comprising any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or having a nucleotide sequence so that, when the two sequences are optimally aligned and compared, is at least 75% preferably at least 80%, 85%, 90%, 95%, 98% or 99% identical to any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or
(ii) is selected from the group of genes having a nucleotide sequence comprising a fragment of at least 21 contiguous nucleotides of any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or having a nucleotide sequence so that, when said gene comprising said fragment is optimally aligned and compared with any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, said nucleotide sequence is at least 75% preferably at least 80%, 85%, 90%, 95%, 98% or 99% identical to any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or
(iii) is selected from the group of genes having a nucleotide sequence comprising a fragment of at least 21 contiguous nucleotides of any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, and wherein when said fragment is optimally aligned and compared with the corresponding fragment in any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, said nucleotide sequence of said fragment is at least 75% preferably at least 80%, 85%, 90%, 95%, 98% or 99% identical to said corresponding fragment of any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or
(iv) is an insect pest orthologue of a gene having a nucleotide sequence comprising any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273.

In a preferred embodiment, the target gene encodes an insect ribosomal protein chosen from the group comprising the ribosomal protein S3A (e.g. an insect orthologue of the CG2168 Dm protein), the ribosomal protein LP1 (e.g. an insect orthologue of the CG4087 Dm protein), the ribosomal protein S3 (e.g. an insect orthologue of the CG6779 Dm protein), the ribosomal protein L10Ab (e.g. an insect orthologue of the CG7283 Dm protein), the ribosomal protein S18 (e.g. an insect orthologue of the CG8900 Dm protein), the ribosomal protein L4 (e.g. an insect orthologue of the CG5502 Dm protein), the ribosomal protein S27 (e.g. an insect orthologue of the CG10423 Dm protein), the ribosomal protein L6 (e.g. an insect orthologue of the CG11522 Dm protein), the ribosomal protein S13 (e.g. an insect orthologue of the CG13389 Dm protein), and the ribosomal protein L12 (e.g. an insect orthologue of the CG3195 Dm protein), the ribosomal protein L26 (e.g. an insect orthologue of the CG6846 Dm protein), the ribosomal protein L21 (e.g. an insect orthologue of the CG12775 Dm protein), the ribosomal protein S12 (e.g. an insect orthologue of the CG11271 Dm protein), the ribosomal protein S28b (e.g. an insect orthologue of the CG2998 Dm protein), the ribosomal protein L13 (e.g. an insect orthologue of the CG4651 Dm protein), the ribosomal protein L10 (e.g. an insect orthologue of the CG17521 Dm protein), the ribosomal protein L5 (e.g. an insect orthologue of the CG17489 Dm protein), the ribosomal protein S15Aa (e.g. an insect orthologue of the CG2033 Dm protein), the ribosomal protein L19 (e.g. an insect orthologue of the CG2746 Dm protein), and the ribosomal protein L27 (e.g. an insect orthologue of the CG4759 Dm protein)

In all aspects of the invention, in preferred embodiments, the target gene
(i) is selected from the group of genes having a nucleotide sequence comprising any of SEQ ID NOs 1, 174, 404, 180, 188, 2, 175, 181, 189, 27 to 30, 282 to 285, 294 to 297, 310 to 313, 401, or the complement thereof, or having a nucleotide sequence so that, when the two sequences are optimally aligned and compared, is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, or the complement thereof, or
(ii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, or the complement thereof, or
(iii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, said nucleotide sequence of said fragment is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, or the complement thereof, or
(iv) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401.

In preferred embodiments, the target gene may encode an insect the troponin I protein (e.g. an insect orthologue of the CG7178 Dm protein). The insect troponin I protein may have an amino acid sequence which is at least 85%, 90%, 92%, 94%, 96%, 98%, 99% identical to the amino acid sequence as presented in any of SEQ ID NOs 79, 349, 405, 352 or 356 (when said encoded proteins are optimally aligned).

### Brief description of the Tables and Figures

**Table 1** *Lygus hesperus* novel targets identified from first screen.
**Table 1B** *Lygus hesperus* novel targets in Lh594 pathway.
**Table 1C** *Lygus hesperus* novel targets identified from second round screen.
**Table 2** Polynucleotide sequences of target genes identified in *Lygus hesperus.*
**Table 3** Amino acid sequences of target genes identified in *Lygus hesperus.*
**Table 4** dsRNAs (sense strand represented by equivalent DNA sequence) corresponding to *Lygus hesperus* target genes and primers for producing the dsRNAs.
**Table 5** *Lygus hesperus* targets ranking according to dose response curves (DRCs) and compared to bench mark targets Lh423 & Lh105.
**Table 6** *Lygus hesperus* targets from second round screen-ranking according to DRCs and compared to bench mark targets Lh423 & Lh594.
**Table 7** Overview of the testing of transgenic potato carrying *Lygus hesperus* hairpins.
**Table 8** Sequence of amplicons for target gene and the two house-keeping genes for qRT-PCR.
**Table 9** Polynucleotide sequences of target genes identified in Colorado potato beetle (CPB).
**Table 10** Amino acid sequences of target genes identified in CPB.
**Table 11** dsRNAs (sense strand represented by equivalent DNA sequence) corresponding to CPB target genes and primers for producing the dsRNAs.
**Table 12** Polynucleotide sequences of target genes identified in brown plant hopper (BPH).
**Table 13** Amino acid sequences of target genes identified in BPH.
**Table 14** dsRNAs (sense strand represented by equivalent DNA sequence) corresponding to BPH target genes and primers for producing the dsRNAs.
**Table 15** Primers used for amplification of aphid cDNAs, based on pea aphid genomic sequence.
**Table 16** Polynucleotide sequences of target genes identified in aphids.
**Table 17** Amino acid sequences of target genes identified in aphids.
**Table 18** dsRNAs (sense strand represented by equivalent DNA sequence) corresponding to aphid target genes and primers for producing the dsRNAs.
**Table 19** Degenerate primers used for amplification of CPB Ld594 cDNA
**Table 20** Degenerate primers used for amplification of BPH cDNAs
**Table 21:** *Leptinotarsa decemlineata* novel targets from the screen.
**Table 22:** *Nilaparvata lugens* novel identified target.
**Table 23:** *Acyrthosiphon pisum* novel identified targets.

**Figure 1****:** Plates Lh001_009 second confirmation assay. Dark bars: mortality at day 3 to 6, light bars: mortality at day 6 to 8. Candidate clones are named using the "Lygxxx" screening codes and the "Lhxxx" target nomenclature codes.
**Figure 2****:** Plates Lh010_020 second confirmation assay. Dark bars: mortality at day 3 to 6, light bars: mortality at day 6 to 8. Candidate clones are named using the "Lygxxx" screening codes and the "Lhxxx" target nomenclature codes.
**Figure 3****:** Mortality analysis of *Lygus* novel targets from plates Lh001 to Lh009, expressed as % mortality over a 10 day period. Controls are indicated in dotted lines. Positive control: Lh423 dsRNA (RpL19). Negative controls: GFP dsRNA and diet only (Control).
**Figure 4****:** Mortality analysis of *Lygus* novel targets from plates Lh010 to Lh020, expressed as % mortality over a 10 day period. Controls are indicated in dotted lines. Positive control: Lh423 (RpL19). Negative controls: GFP and diet only (Control).
**Figure 5** Schematic representation of the plant expression vector harbouring the *Lygus hesperus* hpRNA cassette. RB: right border; LB: left border; P35S: Cauliflower Mosaic Virus 35S promoter; T35S: Cauliflower Mosaic Virus 35S terminator; TNOS: nopaline synthase terminator; GFP: green fluorescent reporter gene; NPT II: coding sequence of neomycin phosphotransferase II gene; KmR: Kanamycin resistance gene; pBR322 ori: pBR322 origin of replication; pBR322 bom: pBR322 mobilization; pVS1 rep: pVS1 replicon; pVS1 sta: pVS1 stability element.
**Figure 6** Potato-*Lygus in planta* assay set up. White arrows indicate insect damage.
**Figures 7 to 11** *Lygus hesperus* novel targets - dose response curves at concentrations of purified synthetic dsRNA ranging from 0.4 to 0.025 µg/µl (in the figure, the unit "µg/µl" is not displayed). GFP dsRNA and milliQ water were used negative controls. dsRNA of targets were produced using the primers as described in the example section 1.1.
**Figure 12** Lh594 dose response curve, at dsRNA concentrations ranging from 0.05 to 0.001 µg/µl. GFP dsRNA and milliQ water were used negative controls.
**Figure 13** **A** dsRNA activity in *Lygus hesperus* bioassay in absence of tRNA. Lh594 (5µg/µl); positive control: Lh423 (5µg/µl); negative controls: GFP dsRNA (5µg/µl) and milliQ water; **B** Identification of Lh594 limit of activity using decreasing concentration of dsRNA (from 5 µg to 0.25 µg). Negative controls: GFP dsRNA (5µg/µl) and milliQ water.
**Figure 14** Plates Lh010 to Lh020 second confirmation assay of second screen targets. Dark bars: mortality at day 4 to 8, light bars: mortality at day 4 to 6. Candidate clones are named using the "Lygxxx" screening codes and the "Lhxxx" target nomenclature codes.
**Figure 15** Assay results for *Lygus* troponin pathway targets, tested at 0.5 µg/µl fixed.
**Figures 16A-B** *Lygus hesperus* novel targets from troponin pathway - dose response curves at concentrations of purified synthetic dsRNA ranging from 0.4 to 0.025 µg/µl (in the figure, the unit "µg/µl" is not always displayed). GFP dsRNA and milliQ water were used as negative controls.
**Figures 17 A-D** *Lygus hesperus* novel targets of second screen targets - dose response curves at concentrations of purified synthetic dsRNA ranging from 0.5 to 0.05 µg/µl. GFP dsRNA and milliQ water were used as negative controls.
**Figures 18A-B** Testing and selection of GUS transgenic events. Eight independent events of GUS hairpin transgenic line (P001) were tested in the *Lygus hesperus* single pot assay and compared to WT plantlets. All plantlets underwent the same treatment. One day old *Lygus hesperus* nymphs were added to each pot and the survival was checked over 9 days.
**Figure 19** Testing of Lh423 transgenic events: 28 independent transgenic events (P006 line) were tested in *the Lygus hesperus* single pot assay. Lh423 transgenic plantlets were compared to WT plantlets and to GUS transgenic events (P001 line). Single one day old *Lygus hesperus* nymphs were added to each pot and the survival was checked over 9 days.
**Figure 20** Testing of Lh423 transgenic events: 6 independent transgenic events (P006) leading to > 60% survival are shown. Lh423 transgenic plantlets were compared to WT plantlets and to GUS transgenic lines (P001). Single one day old *Lygus hesperus* nymphs were added to each pot and the survival was checked over 9 days.
**Figure 21** Testing of Lh594 transgenic events: 25 independent transgenic events (P007) were tested in *the Lygus hesperus* single pot assay. Lh594 transgenic plantlets were compared to WT plantlets and to GUS transgenic events (P001). Single one day old *Lygus hesperus* nymphs were added to each pot and the survival was checked over 11 days.
**Figure 22** Testing of Lh594 transgenic events: 6 independent transgenic events (P007) leading to 60% survival are shown. Lh594 transgenic plantlets were compared to WT plantlets and to GUS transgenic lines (P001). Single one day old *Lygus hesperus* nymphs were added to each pot and the survival was checked over 11 days.
**Figure 23** Relative value of Lh423 mRNA levels in the insects after feeding for 5 days on transgenic plants containing a GUS hairpin or an Lh423 hairpin. Samples were analysed with primers amplifying Lh423. Data was normalized using GeNorm, with 2 house-keeping genes, Lh425 and Lh427.
**Figure 24** Survival analysis of CPB larvae treated with 1 µg dsRNA Ld594, Ld619 and Ld620. Positive controls included 1 µg dsRNA of bench mark targets Ld513 and Ld049. Negative controls included milliQ water and FP.
**Figure 25** Effects of Ld594, Ld619 and Ld620 dsRNAs on pupation of CPB 4^{th} instar larvae, compared to untreated control (**UTC**). Bugs were fed 1 µg dsRNA dispensed in potato leaf disks, then were allowed to feed on untreated potato leaves (**A**) for 4 days before being placed on vermiculite. To assess the effect of the dsRNA, dead insects were excavated from the vermiculite (because of the strong effects induced by Ld594 dsRNA, no pupae could be recovered from the vermiculite and therefore, no image is available for this target dsRNA) (**B**).
**Figure 26** Effect of CPB Ld594, 619 & 620 dsRNAs on survival and fitness of CPB adults. Assessments were performed on days 4, 6, 7, 8, 11 and 13. Control MQ: milliQ water.
**Figure 27** Activity of dsRNA from Nl594 pathway in brown plant hopper. DsRNAs were tested at 0.5 µg/µl in presence of 0.1 % CHAPSO. Positive control: Nl537 dsRNA (0.5 µg/µl), negative controls: GFP dsRNA (0.5 µg/µl) and diet alone.
**Figure 28** Activity of dsRNA from Ap594, Ap423, Ap537 and Ap560 on *A. pisum.* DsRNAs were tested at 0.5 µg/µl in presence of 5 µg/µl tRNA. Negative control: GFP dsRNA (0.5 µg/µl).
**Figure 29** Mortality percentages of *L. decemlineata* larvae on artificial diet treated with dsRNA. Ld583, Ld584, Ld586 & Ld588 represent target clones. Positive control: Ld513; negative control: FP.

### Detailed description of the invention

The present inventors have discovered that down-regulating the expression of particular target genes in insect pest species by RNAi can be used to effectively prevent and/or control infestation by said insect pest. The use of RNAi to down-regulate the expression of target genes in insect pest species is applied herein to the generation of plants resistant to infestation by insect pests.

Therefore, in a first aspect, the present invention provides transgenic plants resistant to infestation by insect pest species. In particular, provided herein are transgenic plants which express or are capable of expressing at least one interfering ribonucleic acid (RNA) that functions upon uptake by an insect pest species to down-regulate the expression of a target gene as described elsewhere herein within said pest. The interfering RNA may be any of those as disclosed herein below. Preferably, the interfering RNA comprises or consists of at least one silencing element and said silencing element is a region of double-stranded RNA comprising annealed complementary strands, one strand of which (the sense strand) comprises a sequence of nucleotides which is at least partially complementary to a target nucleotide sequence within a target gene. Down-regulation of a pest target gene can be used to disrupt an essential biological process or function in the pest, wherein 'essential' refers to the fact that the process or function is required for initiation or maintenance of pest infestation.

As used herein, the term 'plant' may include any reproductive or propagation material for a plant. Reference to a plant may also include plant cells, plant protoplasts, plant tissue cultures, plant calli, plant clumps and plant cells that are intact in plants or parts of plants such as embryos, pollen, ovules, seeds, leaves, flowers, branches, fruit, kernels, ears, cobs, husks, stalks, roots, root tips and the like. Progeny, variants and mutants of any of the transgenic plants described herein are within the scope of the current invention. Also included is seed produced from any of said transgenic plants. As used herein, the term "control" of pest infestation refers to any effect on a pest that serves to limit and/or reduce either the numbers of pest organisms and/or the damage caused by the pest. Preferred target genes are therefore essential genes that control or regulate one or more essential biological functions within the insect pest, for example, cell division, reproduction, energy metabolism, digestion, neurological function and the like. Down-regulation of these essential genes by RNAi techniques can lead to death of the insect, or otherwise significantly retard growth and development or impair the ability of the pest to colonize an environment or infest host organisms.

The present inventors have now identified superior target genes of insect pest species belonging to the *Lygus, Leptinotarsa, Nilaparvata* and *Acyrthosiphum* genus, which targets are envisaged for use singly or in combination as an effective means for RNAi-mediated control of insect infestation of agronomically important crops. Orthologues of these newly identified target genes can be used in other insect species to control pest infestation of the corresponding relevant crops.

More specifically, the present inventors describe here that genes encoding for proteins of the troponin/myofilament complex form excellent target genes for suppression by the RNA inhibition machinery. One of these target genes encoded the insect troponin I protein (wings up A) which is an orthologue of the Drosophila CG7178 protein. This protein is involved in muscular contraction and belongs to a physiological pathway that was not yet fully explored for (insect) pest control through RNA inhibition. Moreover, since this protein complex is animal specific, no plant gene homologues or orthologues are known, reducing the risk of off-type plant phenotypes when expressing target dsRNA in plants. In addition, in Drosophila, troponin I is described as a haplo-insufficient gene, displaying a mutant phenotype in the heterozygote state. Such genes are particularly susceptible to reduced mRNA expression levels and as such can be considered as ideal RNAi targets.

Further interesting target genes in this troponin/myofilament complex are listed below and are being investigated further for RNAi control in *Lygus hesperus* and other insect pest species:

| **Annotation ID** | **Cytology** | **Dm identifier** |
|---|---|---|
| up | upheld | CG7107 |
| Tm1 | tropomyosin 1 | CG4898 |
| Tm2 | tropomyosin 2 | CG4843 |
| Mhc | myosin heavy chain | CG17927 |
| Mlc-c | myosin light chain cytoplasmic | CG3201 |
| sqh | spaghetti squash | CG3595 |
| zip | zipper | CG15792 |

In one aspect, the invention provides a transgenic plant, reproductive or propagative material derived therefrom or a cultured plant cell which expresses or is capable of expressing an interfering ribonucleic acid (RNA) that functions upon uptake by an insect pest species to down-regulate expression of a target gene in said insect pest.

In one embodiment the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233,or the complement thereof, or having a nucleotide sequence so that, when the two sequences are optimally aligned and compared, is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or
(ii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or
(iii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, said nucleotide sequence of said fragment is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233,or the complement thereof, or
(iv) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233,or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233.

In a preferred embodiment, the target gene encodes an insect protein chosen from the troponin/myofilament complex chosen from the group comprising the troponin I (e.g. an insect orthologue of the CG7178 Dm protein), the upheld protein (e.g. an insect orthologue of the CG7107 Dm protein), the tropomyosin 1 protein (e.g. an insect orthologue of the CG4898 Dm protein), the tropomyosin 2 protein (e.g. an insect orthologue of the CG4843 Dm protein), the myosin heavy chain (e.g. an insect orthologue of the CG17927 Dm protein), the myosin light chain cytoplasmic protein (e.g. an insect orthologue of the CG3201 Dm protein), the spaghetti squash protein (e.g. an insect orthologue of the CG3595 Dm protein), the zipper protein (e.g. an insect orthologue of the CG15792 Dm protein), the troponin C (e.g. an insect orthologue of the CG2981, CG7930, CG9073, CG6514, CG12408, CG9073, CG7930, CG2981, CG12408 or CG6514 Dm protein)

In other embodiments, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273 or the complement thereof, or having a nucleotide sequence so that, when the two sequences are optimally aligned and compared, is at least 75% preferably at least 80%, 85%, 90%, 95%, 98% or 99% identical to any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or
(ii) is selected from the group of genes having a nucleotide sequence comprising a fragment of at least 21 contiguous nucleotides of any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or having a nucleotide sequence so that, when said gene comprising said fragment is optimally aligned and compared with any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, said nucleotide sequence is at least 75% preferably at least 80%, 85%, 90%, 95%, 98% or 99% identical to any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or
(iii) is selected from the group of genes having a nucleotide sequence comprising a fragment of at least 21 contiguous nucleotides of any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, and wherein when said fragment is optimally aligned and compared with the corresponding fragment in any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, said nucleotide sequence of said fragment is at least 75% preferably at least 80%, 85%, 90%, 95%, 98% or 99% identical to said corresponding fragment of any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or
(iv) is an insect pest orthologue of a gene having a nucleotide sequence comprising any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or
(v) is selected from the group of genes having a nucleotide sequence encoding an amino acid sequence that, when the two amino acid sequences are optimally aligned and compared, is at least 85% preferably at least 90%, 95%, 98% or 99%identical to the amino acid sequence encoded by any of SEQ ID NOs 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273.

In a preferred embodiment, the target gene encodes an insect ribosomal protein chosen from the group comprising the ribosomal protein S3A (e.g. an insect orthologue of the CG2168 Dm protein), the ribosomal protein LP1 (e.g. an insect orthologue of the CG4087 Dm protein), the ribosomal protein S3 (e.g. an insect orthologue of the CG6779 Dm protein), the ribosomal protein L10Ab (e.g. an insect orthologue of the CG7283 Dm protein), the ribosomal protein S18 (e.g. an insect orthologue of the CG8900 Dm protein), the ribosomal protein L4 (e.g. an insect orthologue of the CG5502 Dm protein), the ribosomal protein S27 (e.g. an insect orthologue of the CG10423 Dm protein), the ribosomal protein L6 (e.g. an insect orthologue of the CG11522 Dm protein), the ribosomal protein S13 (e.g. an insect orthologue of the CG13389 Dm protein), and the ribosomal protein L12 (e.g. an insect orthologue of the CG3195 Dm protein), the ribosomal protein L26 (e.g. an insect orthologue of the CG6846 Dm protein), the ribosomal protein L21 (e.g. an insect orthologue of the CG12775 Dm protein), the ribosomal protein S12 (e.g. an insect orthologue of the CG11271 Dm protein), the ribosomal protein S28b (e.g. an insect orthologue of the CG2998 Dm protein), the ribosomal protein L13 (e.g. an insect orthologue of the CG4651 Dm protein), the ribosomal protein L10 (e.g. an insect orthologue of the CG17521 Dm protein), the ribosomal protein L5 (e.g. an insect orthologue of the CG17489 Dm protein), the ribosomal protein S15Aa (e.g. an insect orthologue of the CG2033 Dm protein), the ribosomal protein L19 (e.g. an insect orthologue of the CG2746 Dm protein), the ribosomal protein L27 (e.g. an insect orthologue of the CG4759 Dm protein)

In one embodiment, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 1, 174, 404, 180, 188, 2, 175, 181, 189, 27 to 30, 282 to 285, 294 to 297, 310 to 313, 401, or the complement thereof, or having a nucleotide sequence so that, when the two sequences are optimally aligned and compared, is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, or the complement thereof, or
(ii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, or the complement thereof, or
(iii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, said nucleotide sequence of said fragment is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, or the complement thereof, or
(iv) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401.

In preferred embodiments, the target gene may encode an insect the troponin I protein (e.g. an insect orthologue of the CG7178 Dm protein). The insect troponin I protein may have an amino acid sequence which is at least 85%, 90%, 92%, 94%, 96%, 98%, 99% identical to the amino acid sequence as presented in any of SEQ ID NOs 79, 349, 405, 352 or 356 (when said encoded proteins are optimally aligned).

In one embodiment, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 141, 11 12, or the complement thereof, or having a nucleotide sequence so that, when the two sequences are optimally aligned and compared, is at least 75% preferably at least 80%, 85%, 90%, 95%, 98% or 99% identical to any of SEQ ID NOs 141, 11, 12, or the complement thereof, or
(ii) is selected from the group of genes having a nucleotide sequence comprising a fragment of at least 21 contiguous nucleotides of any of SEQ ID NOs 141, 11, 12, or the complement thereof, or having a nucleotide sequence so that, when said gene comprising said fragment is optimally aligned and compared with any of SEQ ID NOs 141, 11, 12, said nucleotide sequence is at least 75% preferably at least 80%, 85%, 90%, 95%, 98% or 99% identical to any of SEQ ID NOs 141, 11, 12, or the complement thereof, or
(iii) is selected from the group of genes having a nucleotide sequence comprising a fragment of at least 21 contiguous nucleotides of any of SEQ ID NOs 141, 11, 12, or the complement thereof, and wherein when said fragment is optimally aligned and compared with the corresponding fragment in any of SEQ ID NOs 141, 11, 12, said nucleotide sequence of said fragment is at least 75% preferably at least 80%, 85%, 90%, 95%, 98% or 99% identical to said corresponding fragment of any of SEQ ID NOs 141, 11, 12, or the complement thereof, or
(iv) is an insect pest orthologue of a gene having a nucleotide sequence comprising any of SEQ ID NOs 141, 11, 12, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 141, 11, 12, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 141, 11, 12.

In one embodiment, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 17, 18, or the complement thereof, or having a nucleotide sequence so that, when the two sequences are optimally aligned and compared, is at least 75% preferably at least 80%, 85%, 90%, 95%, 98% or 99% identical to any of SEQ ID NOs 17, 18, or the complement thereof, or
(ii) is selected from the group of genes having a nucleotide sequence comprising a fragment of at least 21 contiguous nucleotides of any of SEQ ID NOs 17, 18, or the complement thereof, or having a nucleotide sequence so that, when said gene comprising said fragment is optimally aligned and compared with any of SEQ ID NOs 17, 18, said nucleotide sequence is at least 75% preferably at least 80%, 85%, 90%, 95%, 98% or 99% identical to any of SEQ ID NOs 17, 18, or the complement thereof, or
(iii) is selected from the group of genes having a nucleotide sequence comprising a fragment of at least 21 contiguous nucleotides of any of SEQ ID NOs 17, 18, or the complement thereof, and wherein when said fragment is optimally aligned and compared with the corresponding fragment in any of SEQ ID NOs 17, 18, said nucleotide sequence of said fragment is at least 75% preferably at least 80%, 85%, 90%, 95%, 98% or 99% identical to said corresponding fragment of any of SEQ ID NOs 17, 18, or the complement thereof, or
(iv) is an insect pest orthologue of a gene having a nucleotide sequence comprising any of SEQ ID NOs 17, 18, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 17, 18, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 17, 18.

In one embodiment, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 19, 20, or the complement thereof, or having a nucleotide sequence so that, when the two sequences are optimally aligned and compared, is at least 75% preferably at least 80%, 85%, 90%, 95%, 98% or 99% identical to any of SEQ ID NOs 19, 20, or the complement thereof, or
(ii) is selected from the group of genes having a nucleotide sequence comprising a fragment of at least 21 contiguous nucleotides of any of SEQ ID NOs 19, 20, or the complement thereof, or having a nucleotide sequence so that, when said gene comprising said fragment is optimally aligned and compared with any of SEQ ID NOs 19, 20, said nucleotide sequence is at least 75% preferably at least 80%, 85%, 90%, 95%, 98% or 99% identical to any of SEQ ID NOs 19, 20, or the complement thereof, or
(iii) is selected from the group of genes having a nucleotide sequence comprising a fragment of at least 21 contiguous nucleotides of any of SEQ ID NOs 19, 20, or the complement thereof, and wherein when said fragment is optimally aligned and compared with the corresponding fragment in any of SEQ ID NOs 19, 20, said nucleotide sequence of said fragment is at least 75% preferably at least 80%, 85%, 90%, 95%, 98% or 99% identical to said corresponding fragment of any of SEQ ID NOs 19, 20, or the complement thereof, or
(iv) is an insect pest orthologue of a gene having a nucleotide sequence comprising any of SEQ ID NOs 19, 20, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 19, 20, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 19, 20.

In one embodiment, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 165, 166, 167, or the complement thereof, or having a nucleotide sequence so that, when the two sequences are optimally aligned and compared, is at least 75% preferably at least 80%, 85%, 90%, 95%, 98% or 99% identical to any of SEQ ID NOs 165, 166, 167, or the complement thereof, or
(ii) is selected from the group of genes having a nucleotide sequence comprising a fragment of at least 21 contiguous nucleotides of any of SEQ ID NOs 165, 166, 167, or the complement thereof, or having a nucleotide sequence so that, when said gene comprising said fragment is optimally aligned and compared with any of SEQ ID NOs 165, 166, 167, said nucleotide sequence is at least 75% preferably at least 80%, 85%, 90%, 95%, 98% or 99% identical to any of SEQ ID NOs 165, 166, 167, or the complement thereof, or
(iii) is selected from the group of genes having a nucleotide sequence comprising a fragment of at least 21 contiguous nucleotides of any of SEQ ID NOs 165, 166, 167, or the complement thereof, and wherein when said fragment is optimally aligned and compared with the corresponding fragment in any of SEQ ID NOs 17, 18, said nucleotide sequence of said fragment is at least 75% preferably at least 80%, 85%, 90%, 95%, 98% or 99% identical to said corresponding fragment of any of SEQ ID NOs 165, 166, 167, or the complement thereof, or
(iv) is an insect pest orthologue of a gene having a nucleotide sequence comprising any of SEQ ID NOs 165, 166, 167, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 165, 166, 167, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 165, 166, 167.

In one embodiment, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 143, 121, 142, 176, 182, 130, 177, 183, or the complement thereof, or having a nucleotide sequence so that, when the two sequences are optimally aligned and compared, is at least 75% preferably at least 80%, 85%, 90%, 95%, 98% or 99% identical to any of SEQ ID NOs 143, 121, 142, 176, 182, 130, 177, 183, or the complement thereof, or
(ii) is selected from the group of genes having a nucleotide sequence comprising a fragment of at least 21 contiguous nucleotides of any of SEQ ID NOs 143, 121, 142, 176, 182, 130, 177, 183, or the complement thereof, or having a nucleotide sequence so that, when said gene comprising said fragment is optimally aligned and compared with any of SEQ ID NOs 143, 121, 142, 176, 182, 130, 177, 183, said nucleotide sequence is at least 75% preferably at least 80%, 85%, 90%, 95%, 98% or 99% identical to any of SEQ ID NOs 143, 121, 142, 176, 182, 130, 177, 183, or the complement thereof, or
(iii) is selected from the group of genes having a nucleotide sequence comprising a fragment of at least 21 contiguous nucleotides of any of SEQ ID NOs 143, 121, 142, 176, 182, 130, 177, 183, or the complement thereof, and wherein when said fragment is optimally aligned and compared with the corresponding fragment in any of SEQ ID NOs 143, 121, 142, 176, 182, 130, 177, 183, said nucleotide sequence of said fragment is at least 75% preferably at least 80%, 85%, 90%, 95%, 98% or 99% identical to said corresponding fragment of any of SEQ ID NOs 143, 121, 142, 176, 182, 130, 177, 183, or the complement thereof, or
(iv) is an insect pest orthologue of a gene having a nucleotide sequence comprising any of SEQ ID NOs 143, 121, 142, 176, 182, 130, 177, 183, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 143, 121, 142, 176, 182, 130, 177, 183, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 143, 121, 142, 176, 182, 130, 177, 183.

In one embodiment, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 145, 122, 144, 178, 131, 179 or the complement thereof, or having a nucleotide sequence so that, when the two sequences are optimally aligned and compared, is at least 75% preferably at least 80%, 85%, 90%, 95%, 98% or 99% identical to any of SEQ ID NOs 145, 122, 144, 178, 131, 179, or the complement thereof, or
(ii) is selected from the group of genes having a nucleotide sequence comprising a fragment of at least 21 contiguous nucleotides of any of SEQ ID NOs 145, 122, 144, 178, 131, 179, or the complement thereof, or having a nucleotide sequence so that, when said gene comprising said fragment is optimally aligned and compared with any of SEQ ID NOs 145, 122, 144, 178, 131, 179, said nucleotide sequence is at least 75% preferably at least 80%, 85%, 90%, 95%, 98% or 99% identical to any of SEQ ID NOs 145, 122, 144, 178, 131, 179, or the complement thereof, or
(iii) is selected from the group of genes having a nucleotide sequence comprising a fragment of at least 21 contiguous nucleotides of any of SEQ ID NOs 145, 122, 144, 178, 131, 179, or the complement thereof, and wherein when said fragment is optimally aligned and compared with the corresponding fragment in any of SEQ ID NOs 145, 122, 144, 178, 131, 179, said nucleotide sequence of said fragment is at least 75% preferably at least 80%, 85%, 90%, 95%, 98% or 99% identical to said corresponding fragment of any of SEQ ID NOs 145, 122, 144, 178, 131, 179, or the complement thereof, or
(iv) is an insect pest orthologue of a gene having a nucleotide sequence comprising any of SEQ ID NOs 145, 122, 144, 178, 131, 179, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 145, 122, 144, 178, 131, 179, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 145, 122, 144, 178, 131, 179.

In one embodiment, the present invention relates to a plant or reproductive or propagation material for a transgenic plant or a cultured transgenic plant cell which expresses or is capable of expressing an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene, and wherein the target gene
(i) is selected from the group of genes having a nucleotide sequence comprising any of SEQ ID NOs 128, 149, 184, 137, or the complement thereof, or having a nucleotide sequence so that, when the two sequences are optimally aligned and compared, is at least 75% preferably at least 80%, 85%, 90%, 95%, 98% or 99% identical to any of SEQ ID NOs 128, 149, 184, 137, or the complement thereof, or
(ii) is selected from the group of genes having a nucleotide sequence comprising a fragment of at least 21 contiguous nucleotides of any of SEQ ID NOs 128, 149, 184, 137, or the complement thereof, or having a nucleotide sequence so that, when said gene comprising said fragment is optimally aligned and compared with any of SEQ ID NOs 128, 149, 184, 137, said nucleotide sequence is at least 75% preferably at least 80%, 85%, 90%, 95%, 98% or 99% identical to any of SEQ ID NOs 128, 149, 184, 137, or the complement thereof, or
(iii) is selected from the group of genes having a nucleotide sequence comprising a fragment of at least 21 contiguous nucleotides of any of SEQ ID NOs 128, 149, 184, 137, or the complement thereof, and wherein when said fragment is optimally aligned and compared with the corresponding fragment in any of SEQ ID NOs 128, 149, 184, 137, said nucleotide sequence of said fragment is at least 75% preferably at least 80%, 85%, 90%, 95%, 98% or 99% identical to said corresponding fragment of any of SEQ ID NOs 128, 149, 184, 137, or the complement thereof, or
(iv) is an insect pest orthologue of a gene having a nucleotide sequence comprising any of SEQ ID NOs 128, 149, 184, 137, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 128, 149, 184, 137, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 128, 149, 184, 137.

As used herein, a "target gene" comprises any gene in the insect pest which one intends to down-regulate. In a preferred embodiment, the target gene is down-regulated so as to control pest infestation, for example by disrupting an essential biological process occurring in the pest, or by decreasing the pathogenicity of the pest. Preferred target genes therefore include but are not limited to those that play key roles in regulating feeding, survival, growth, development, reproduction, infestation and infectivity. According to one embodiment, the target gene is such that when its expression is down-regulated or inhibited, the insect pest is killed. According to another embodiment, the target gene is such that when its expression is down-regulated or inhibited, growth of the pest is prevented or retarded or stunted or delayed or impeded, pest reproduction is prevented, or transition through the life cycles of the pest is prevented. According to yet another embodiment of the invention, the target gene is such that when its expression is down-regulated or inhibited, the damage caused by the pest and/or the ability of the pest to infect or infest environments, surfaces and/or plant or crop species is reduced; or the pest stops feeding from its natural food resources such as plants and plant products. The terms "infest" and "infect" or "infestation" and "infection" are generally used interchangeably throughout.

The target genes may be expressed in all or some of the cells of the insect pest. Furthermore, the target genes may only be expressed by the insect pest at a particular stage of its life-cycle, for example, the mature adult phase, immature nymph or larval phase or egg phase.

As used herein "pest" species are preferably insect species that cause infection or infestation, preferably of plants.

Preferred plant pathogenic insects according to the invention are plant pest are selected from the group consisting of *Leptinotarsa* spp. (e.g. *L. decemlineata* (Colorado potato beetle), *L. juncta* (false potato beetle), or *L. texana* (Texan false potato beetle)); *Nilaparvata* spp. (e.g. *N. lugens (brown planthopper)); Laodelphax* spp. (e.g. *L. striatellus* (small brown planthopper)); *Nephotettix* spp. (e.g. *N. virescens* or *N. cincticeps* (green leafhopper), or *N.nigropictus* (rice leafhopper)); *Sogatella* spp. (e.g. *S*. *furcifera* (white-backed planthopper)); *Chilo* spp. (e.g. *C*. *suppressalis* (rice striped stem borer), *C*. *auricilius* (gold-fringed stem borer), or *C*. *polychrysus* (dark-headed stem borer)); *Sesamia* spp. (e.g. *S*. *inferens* (pink rice borer)); *Tryporyza* spp. (e.g. *T. innotata* (white rice borer), or *T. incertulas* (yellow rice borer)); *Anthonomus* spp. (e.g. *A. grandis* (boll weevil)); *Phaedon* spp. (e.g. *P*. *cochleariae* (mustard leaf beetle)); *Epilachna* spp. (e.g. *E. varivetis* (mexican bean beetle)); *Tribolium* spp. (e.g. *T. castaneum* (red floor beetle)); *Diabrotica* spp. (e.g. *D*. *virgifera virgifera* (western corn rootworm), *D. barberi* (northern corn rootworm), *D*. *undecimpunctata* howardi (southern corn rootworm), *D*. *virgifera* zeae (Mexican corn rootworm); *Ostrinia* spp. (e.g. *O*. *nubilalis* (European corn borer)); *Anaphothrips* spp. (e.g. *A. obscrurus* (grass thrips)); *Pectinophora* spp. (e.g. *P. gossypiella* (pink bollworm)); *Heliothis* spp. (e.g. *H. virescens* (tobacco budworm)); *Trialeurodes* spp. (e.g. *T. abutiloneus* (banded-winged whitefly) *T. vaporariorum* (greenhouse whitefly)); *Bemisia* spp. (e.g. *B*. *argentifoli*i (silverleaf whitefly)); *Aphis* spp. (e.g. *A. gossypii* (cotton aphid)); *Lygus* spp. (e.g. *L*. *lineolaris* (tarnished plant bug) or *L. hesperus* (western tarnished plant bug)); *Euschistus* spp. (e.g. *E*. *conspersus* (consperse stink bug)); *Chlorochroa* spp. (e.g. *C*. *sayi* (Say stinkbug)); *Nezara* spp. (e.g. *N. viridula* (southern green stinkbug)); *Thrips* spp. (e.g. *T. tabaci* (onion thrips)); *Frankliniella* spp. (e.g. *F. fusca* (tobacco thrips), or *F. occidentalis* (western flower thrips)); *Acheta* spp. (e.g. *A. domesticus* (house cricket)); *Myzus* spp. (e.g. *M. persicae* (green peach aphid)); *Macrosiphum spp. (e.g. M. euphorbiae* (potato aphid)); *Blissus* spp. (e.g. *B. leucopterus leucopterus* (chinch bug)); *Acrosternum* spp. (e.g. *A. hilare* (green stink bug)); *Chilotraea* spp. (e.g. *C*. *polychrysa* (rice stalk borer)); *Lissorhoptrus* spp. (e.g. *L. oryzophilus* (rice water weevil)); *Rhopalosiphum* spp. (e.g. *R*. *maidis* (corn leaf aphid)); and *Anuraphis* spp. (e.g. *A. maidiradicis* (corn root aphid)).

According to more specific embodiments, the invention is applicable to species belonging to the family of Chrysomelidae or leaf beatles. Chrysomelid beetles such Colorado potato Beetles, Flea Beetles, Corn Rootworms and Curculionids such as Alfalfa Weevils are particularly important pests. Specific Leptinotarsa species to control according to the invention include Colorado Potato Beetle (*Leptinotarsa decemlineata* (Say) and False Potato Beetle (*Leptinotarsa juncta* (Say). CPB is a (serious) pest on our domestic potato, other cultivated and wild tuber bearing and non-tuber bearing potato species and other Solanaceous (nightshades) plant species incuding the crop species tomato, eggplant, peppers, tobacco (Nicotiana species including ornamentals), ground cherry, rice, corn or cotton; and the weed/herb species, horse nettle, common nightshade, thorn apple, henbane and buffalo burr. Corn rootworms include species found in the genus Diabrotica (e.g., *D*. *undecimpunctata undecimpunctata, D. undecimpunctata howardii, D. longicornis, D. virgifera* and *D*. *balteata*). Corn rootworms cause extensive damage to corn and curcubits.

According to a more specific embodiment, the invention is applicable to species belonging to the order of Hemipterans (family of Aphidoidea), such as *Myzus persicae* (green peach aphid, *Aphis fabae* (Black Bean Aphid), *Acyrthosiphum pisum* (Pea Aphid), *Brevicoryne brassicae* (Cabbage Aphid), *Sitobion avenae* (Grain Aphid), *Cavariella aegopodii* (Carrot Aphid), *Aphis craccivora* (Groundnut Aphid), *Aphis gossypii* (Cotton Aphid), *Toxoptera aurantii* (Black Citrus Aphid), *Cavariella spp* (Willow Aphid), *Chaitophorus spp* (Willow Leaf Aphids), *Cinara spp.* (Black Pine Aphids), *Drepanosiphum platanoides* (Sycamore Aphid) *Elatobium spp* (Spruce Aphids) which cause damage to plants such as Prunus trees, particularly peach, apricot and plum; trees that are mainly cultured for wood production such as willows and poplars, to row crops such as corn, cotton, soy, wheat and rice, to vegetable crops of the families Solanaceae, Chenopodiaceae, Compositae, Cruciferae, and Cucurbitaceae, including but not limited to, artichoke, asparagus, bean, beets, broccoli, Brussels sprouts, cabbage, carrot, cauliflower, cantaloupe, celery, corn, cucumber, fennel, kale, kohlrabi, turnip, eggplant, lettuce, mustard, okra, parsley, parsnip, pea, pepper, potato, radish, spinach, squash, tomato, turnip, watercress, and watermelon; or field crops such as, but not limited to, tobacco, sugar beet, and sunflower; a flower crop or other ornamental plant such as pine trees and conifers . Other Hemipterans belong to *Nilaparvata ssp* (eg. *N. lugens, Sogatella furcifera*) and cause damage to rice plants. Other Hemipterans belong to *Lygus ssp* (eg. *Lygus hesperus, Lygus rugulipennis, Lygus lineolaris, Lygus sully*) and other species of plant-feeding insects in the family of the Miridae, and cause damage to cotton, potato plants, strawberries, cotton, alfalfa, canola, peach, plums, grape, lettuce, eggplant, onion, green beans. As well as several Mediterranean trees and several ornamental trees such as elm tree (*Ulmus spp*.) pine nut (*Pinus Pinea*) London plane tree (*Platanus Acerifolia*), white redbud (*Malus alba*). Other Hemipterans belong to the family of the Pentatomoidea, they are commonly referred to as shield bugs, chust bugs, and stink bugs (eg; the brown marmorated stink bug (*Halyomorpha halys*), the Consperse stink bug (*Euschistus conspersus*), southern green stink bug (*Nezara viridula*), forest bug (*Pentatoma rufipes*), harlequin bug (*Murgantia histrionica*), rice stink bug (*Oebalus pugnax*)) and cause damage to fruits including apples, peaches, figs, mulberries, citrus fruits and persimmons, blackberry, and vegetables including sweetcorn, tomatoes, soy beans, lima beans and green peppers, cabbage, cauliflower, turnips, horseradish, collards, mustard, Brussels sprouts, potato, egg plant, okra, beans, asparagus, beets, weeds, fruit trees and field crops such as field corn and soy bean. Stink bugs are also a pest of grasses, sorghum and rice.

A plant to be used in the methods of the invention, or a transgenic plant according to the invention encompasses any plant, but is preferably a plant that is susceptible to infestation by a plant pathogenic insect.

Accordingly, the present invention extends to plants and to methods as described herein wherein the plant is chosen from the following group of plants (or crops): alfalfa, apple, apricot, artichoke, asparagus, avocado, banana, barley, beans, beet, blackberry, blueberry, broccoli, Brussels sprouts, cabbage, canola, carrot, cassava, cauliflower, a cereal, celery, cherry, citrus, clementine, coffee, corn, cotton, cucumber, eggplant, endive, eucalyptus, figs, grape, grapefruit, groundnuts, ground cherry, kiwifruit, lettuce, leek, lemon, lime, pine, maize, mango, melon, millet, mushroom, nut oat, okra, onion, orange, an ornamental plant or flower or tree, papaya, parsley, pea, peach, peanut, peat, pepper, persimmon, pineapple, plantain, plum, pomegranate, potato, pumpkin, radicchio, radish, rapeseed, raspberry, rice, rye, sorghum, soy, soybean, spinach, strawberry, sugar beet, sugarcane, sunflower, sweet potato, tangerine, tea, tobacco, tomato, a vine, watermelon, wheat, yams and zucchini.

In specific embodiments, the present invention provides target genes which encode proteins involved in the function of a wings up A (troponin I), a mitochondrial cytochrome c oxidase subunit II protein, or one of the ribosomal proteins as specified in Table 1.

In preferred embodiments, the present invention provides target genes selected from the group of genes (i) having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or having a nucleotide sequence so that, when the two sequences are optimally aligned and compared, is at least 75%, preferably at least 80%, 85%, 90%, 95%, 98% or 99% identical any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (ii) having a nucleotide sequence consisting of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (iii) having a nucleotide sequence comprising a fragment of at least 20, 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000, or 3000 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (iv) having a nucleotide sequence comprising a fragment of at least 20, 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000, or 3000 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence of said fragment is at least 75% preferably at least 80%, 85%, 90%, 95%, 98% or 99% identical to said corresponding fragment of any of SEQ ID NOs. 1-26, 121-205, 386-389, 394, 400, or the complement thereof, or (v) having a nucleotide sequence encoding an amino acid sequence that, when the two amino acid sequences are optimally aligned and compared, is at least 70% preferably at least 75%, 80%, 85%, 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 or (vi) which gene is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389;
and wherein the nucleotide sequence of said gene is no longer than 10000, 9000, 8000, 7000, 6000, 5000, 4000, 3000, 2000 or 1500 nucleotides.

The amino acid sequences encoded by the target genes of the present invention are represented by SEQ ID NOs 79, 349, 405, 352, 356, 80, 326, 81, 327, 82, 83, 328, 84, 329, 85, 86, 359, 87 to 91, 330, 350, 353, 331, 351, 332 to 336, 337, 354, 338 to 344, 346, 345, 347, 348, 357, 355, 358, 390 to 393.

As used herein, the term "having" has the same meaning as "comprising".

As used herein, the term "sequence identity" is used to describe the sequence relationship between two or more nucleotide or amino acid sequences. The percentage of "sequence identity" between two sequences is determined by comparing two optimally aligned sequences over a comparison window (a defined number of positions), wherein the portion of the sequence in the comparison window may comprise additions or deletions (i.e. gaps) as compared to the reference sequence in order to achieve optimal alignment. The percentage sequence identity is calculated by determining the number of positions at which the identical nucleotide base or amino acid residue occurs in both sequences to yield the number of 'matched' positions, dividing the number of matched positions by the total number of positions in the comparison window and multiplying the result by 100. Methods and software for determining sequence identity are available in the art and include the Blast software and GAP analysis. For nucleic acids, the percent identity is calculated preferably by the BlastN alignment tool whereby the percent identity is calculated over the entire length of the query nucleotide sequence.

A person skilled in the art will recognise that homologues or orthologues (homologues existing in different species) of the target genes represented by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 can be identified. These pest homologues and/or orthologues are also within the scope of the current invention. Preferred homologues and/or orthologues are genes similar in nucleotide sequence to such a degree that when the two genes are optimally aligned and compared, the homologue and/or orthologue has a sequence that is at least 75%, preferably at least 80% or 85%, more preferably at least 90% or 95%, and most preferably at least about 99% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof. Similarly, also preferred homologues and/or orthologues are proteins that are similar in amino acid sequence to such a degree that when the two amino acid sequences are optimally aligned and compared, the homologue and/or orthologue has a sequence that is at least 75%, preferably at least 80% or 85%, more preferably at least 90% or 95%, and most preferably at least about 99% identical to any of SEQ ID NOs 79 to 91, 326-359, 390-395.

Other homologues are genes which are alleles of a gene comprising a sequence as represented by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389. Further preferred homologues are genes comprising at least one single nucleotide polymorphism (SNP) compared to a gene comprising a sequence as represented by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389.

The 'interfering ribonucleic acid (RNA)' of the current invention encompasses any type of RNA molecule capable of down-regulating or 'silencing' expression of a target gene, including but not limited to sense RNA, antisense RNA, short interfering RNA (siRNA), microRNA (miRNA), double-stranded RNA (dsRNA), hairpin RNA (RNA) and the like. Methods to assay for functional interfering RNA molecules are well known in the art and are disclosed elsewhere herein.

The interfering RNA molecules of the current invention effect sequence-specific down-regulation of expression of a target gene by binding to a target nucleotide sequence within the target gene. Binding occurs as a result of base pairing between complementary regions of the interfering RNA and the target nucleotide sequence. As used herein, the term 'silencing element' refers to the portion or region of the interfering RNA comprising or consisting of a sequence of nucleotides which is complementary, or at least partially complementary, to a target nucleotide sequence within the target gene, and which functions as the active portion of the interfering RNA to direct down-regulation of expression of said target gene. In one embodiment of the invention, the silencing element comprises or consists of a sequence of at least 17 contiguous nucleotides, preferably at least 18 or 19 contiguous nucleotides, more preferably at least 21 contiguous nucleotides, even more preferably at least 22, 23, 24 or 25 contiguous nucleotides complementary to a target nucleotide sequence within the target gene.

As used herein, "expression of a target gene" refers to the transcription and accumulation of the RNA transcript encoded by a target gene and/or translation of the mRNA into protein. The term 'down-regulate' is intended to refer to any of the methods known in the art by which interfering RNA molecules reduce the level of primary RNA transcripts, mRNA or protein produced from a target gene. In certain embodiments, down-regulation refers to a situation whereby the level of RNA or protein produced from a gene is reduced by at least 10%, preferably by at least 33%, more preferably by at least 50%, yet more preferably by at least 80%. In particularly preferred embodiments, down-regulation refers to a reduction in the level of RNA or protein produced from a gene by at least 80%, preferably by at least 90%, more preferably by at least 95%, and most preferably by at least 99% within cells of the insect pest as compared with an appropriate control insect pest which has for example, not been exposed to an interfering RNA or has been exposed to a control interfering RNA molecule. Methods for detecting reductions in RNA or protein levels are well known in the art and include RNA solution hybridization, Northern hybridization, reverse transcription (e.g. quantitative RT-PCR analysis), microarray analysis, antibody binding, enzyme-linked immunosorbent assay (ELISA) and Western blotting. In another embodiment of the invention, down-regulation refers to a reduction in RNA or protein levels sufficient to result in a detectable change in a phenotype of the pest as compared with an appropriate pest control, for example, cell death, cessation of growth, or the like. Down-regulation can thus be measured by phenotypic analysis of the insect pest using techniques routine in the art.

In a preferred embodiment of the invention, the interfering RNA down-regulates gene expression by RNA interference or RNAi. RNAi is a process of sequence-specific gene regulation typically mediated by double-stranded RNA molecules such as short interfering RNAs (siRNAs). siRNAs comprise a sense RNA strand annealed by complementary basepairing to an antisense RNA strand. The sense strand or 'guide strand' of the siRNA molecule comprises a sequence of nucleotides complementary to a sequence of nucleotides located within the RNA transcript of the target gene. The sense strand of the siRNA is therefore able to anneal to the RNA transcript via Watson-Crick-type basepairing and target the RNA for degradation within a cellular complex known as the RNAi-induced silencing complex or RISC. Thus, in the context of preferred interfering RNA molecules of the current invention, the silencing element as referred to herein may be a double-stranded region comprising annealed complementary strands, at least one strand of which comprises or consists of a sequence of nucleotides which is complementary or at least partially complementary to a target nucleotide sequence within a target gene. In one embodiment the double-stranded region has a length of at least 21, 22, 23, 24, 25, 30, 35, 40, 50, 55, 60, 70, 80, 90, 100, 125, 150, 175, 200, 300, 400, 500, 600, 700, 800, 900, 1000, 1100, 1200, 1300, 1400, 1500, 2000 or 3000 base pairs.

Longer double-stranded RNA (dsRNA) molecules comprising one or more functional double-stranded silencing elements as described elsewhere herein, and capable of RNAi-mediated gene silencing are also contemplated within the scope of the current invention. Such longer dsRNA molecules comprise at least 80, 200, 300, 350, 400, 450, 500, 550, 600, 700, 800, 900, 1000, 1100, 1200, 1300, 1400, 1500, 2000 or 3000 base pairs. These dsRNA molecules may serve as precursors for the active siRNA molecules that direct the RNA transcript to the RISC complex for subsequent degradation. dsRNA molecules present in the environment surrounding an organism or the cells thereof may be taken up by the organism and processed by an enzyme called Dicer to yield siRNA molecules. Alternatively, the dsRNA may be produced *in vivo i.e.* transcribed from a polynucleotide or polynucleotides encoding the same present within a cell, for instance a bacterial cell or a plant cell, and subsequently processed by Dicer either within the host cell or preferably within the insect pest cells following uptake of the longer precursor dsRNA. The dsRNA may be formed from two separate (sense and antisense) RNA strands that anneal by virtue of complementary basepairing.

Alternatively, the dsRNA may be a single strand that is capable of folding back on itself to form a hairpin RNA (RNA) or stem-loop structure. In the case of a RNA, the double-stranded region or 'stem' is formed from two regions or segments of the RNA that are essentially inverted repeats of one another and possess sufficient complementarity to allow the formation of a double-stranded region. One or more functional double-stranded silencing elements may be present in this 'stem region' of the molecule. The inverted repeat regions are typically separated by a region or segment of the RNA known as the 'loop' region. This region can comprise any nucleotide sequence conferring enough flexibility to allow self-pairing to occur between the flanking complementary regions of the RNA. In general, the loop region is substantially single-stranded and acts as a spacer element between the inverted repeats.

All the interfering RNA molecules of the invention effect sequence-specific down-regulation of expression of a target gene by binding to a target nucleotide sequence within the target gene. Binding occurs as a result of complementary base pairing between the silencing element of the interfering RNA and the target nucleotide sequence. The interfering RNA molecules of the invention comprise at least one or at least two silencing elements. In one embodiment of the current invention, the target nucleotide sequence comprises a sequence of nucleotides as represented by the RNA transcript of the target gene, or a fragment thereof wherein the fragment is preferably at least 17 nucleotides, more preferably at least 18, 19 or 20 nucleotides, or most preferably at least 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 nucleotides. In a preferred embodiment of the current invention, the target nucleotide sequence comprises a sequence of nucleotides equivalent to the RNA transcript encoded by any of the polynucleotides selected from the group consisting of (i) a polynucleotide which comprises at least 21, preferably at least 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100 or 1115 contiguous nucleotides of a nucleotide sequence as represented by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (ii) a polynucleotide which consists of at least 21, preferably at least 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof,
or (iii) a polynucleotide which comprises at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, so that, when the two sequences are optimally aligned and compared, said polynucleotide is at least 75 % preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (iv) a polynucleotide which comprises a fragment of at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide as represented in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein said fragment or said complement has a nucleotide sequence so that, when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 or the complement thereof,
or (v) a polynucleotide which consists of a fragment of at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide as represented in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein said fragment or said complement has a nucleotide sequence that, when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 or the complement thereof, or (vi) a polynucleotide encoding an amino acid sequence that, when the two amino acid sequences are optimally aligned and compared, is at least 70 % preferably at least 75%, 80%, 85%, 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389. In a more preferred embodiment of the above, said polynucleotide is no longer than 10000, 9000, 8000, 7000, 6000, 5000, 4000, 3000, 2000 or 1500 nucleotides.

Preferably, the interfering RNA molecules of the current invention comprise at least one double-stranded region, typically the silencing element of the interfering RNA, comprising a sense RNA strand annealed by complementary basepairing to an antisense RNA strand wherein the sense strand of the dsRNA molecule comprises a sequence of nucleotides complementary to a sequence of nucleotides located within the RNA transcript of the target gene.

The silencing element, or at least one strand thereof wherein the silencing element is double-stranded, may be fully complementary or partially complementary to the target nucleotide sequence of the target gene. As used herein, the term "fully complementary" means that all the bases of the nucleotide sequence of the silencing element are complementary to or 'match' the bases of the target nucleotide sequence. The term "at least partially complementary" means that there is less than a 100% match between the bases of the silencing element and the bases of the target nucleotide sequence. The skilled person will understand that the silencing element need only be at least partially complementary to the target nucleotide sequence in order to mediate down-regulation of expression of the target gene. It is known in the art that RNA sequences with insertions, deletions and mismatches relative to the target sequence can still be effective at RNAi. According to the current invention, it is preferred that the silencing element and the target nucleotide sequence of the target gene share at least 80% or 85% sequence identity, preferably at least 90% or 95% sequence identity, or more preferably at least 97% or 98% sequence identity and still more preferably at least 99% sequence identity. Alternatively, the silencing element may comprise 1, 2 or 3 mismatches as compared with the target nucleotide sequence over every length of 24 partially complementary nucleotides.

It will be appreciated by the person skilled in the art that the degree of complementarity shared between the silencing element and the target nucleotide sequence may vary depending on the target gene to be down-regulated or depending on the insect pest species in which gene expression is to be controlled.

In another embodiment of the current invention, the silencing element comprises a sequence of nucleotides that is the RNA equivalent of any of the polynucleotides selected from the group consisting of a polynucleotide which comprises at least 21, preferably at least 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100 or 1115 contiguous nucleotides of a nucleotide sequence as represented by any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389or the complement thereof, or (ii) a polynucleotide which comprises at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented in any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, so that, when the two sequences are optimally aligned and compared, said polynucleotide is at least 75 % preferably at least 80%, 85%, 90%, 95%, 98% or 99% identical to any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (iii) a polynucleotide which comprises a fragment of at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide as represented in any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein said fragment or said complement has a nucleotide sequence so that, when said fragment is optimally aligned and compared with the corresponding fragment in any of SEQ ID NOs. 1-26, 121-205, 386-389, 394, 400, said nucleotide sequence is at least 75% preferably at least 80%, 85%, 90%, 95%, 98% or 99% identical to said corresponding fragment of any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 or the complement thereof, wherein said polynucleotide is no longer than 10000, 9000, 8000, 7000, 6000, 5000, 4000, 3000, 2000 or 1500 nucleotides. It will be appreciated that in such embodiments the silencing element may comprise or consist of a region of double-stranded RNA comprising annealed complementary strands, one strand of which, the sense strand, comprises a sequence of nucleotides at least partially complementary to a target nucleotide sequence within a target gene.

The target nucleotide sequence may be selected from any suitable region or nucleotide sequence of the target gene or RNA transcript thereof. For example, the target nucleotide sequence may be located within the 5'UTR or 3'UTR of the target gene or RNA transcript or within exonic or intronic regions of the gene.

The skilled person will be aware of methods of identifying the most suitable target nucleotide sequences within the context of the full-length target gene. For example, multiple silencing elements targeting different regions of the target gene can be synthesised and tested. Alternatively, digestion of the RNA transcript with enzymes such as RNAse H can be used to determine sites on the RNA that are in a conformation susceptible to gene silencing. Target sites may also be identified using *in silico* approaches, for example, the use of computer algorithms designed to predict the efficacy of gene silencing based on targeting different sites within the full-length gene.

The interfering RNAs of the current invention may comprise one silencing element or multiple silencing elements, wherein each silencing element comprises or consists of a sequence of nucleotides which is at least partially complementary to a target nucleotide sequence within a target gene and that functions upon uptake by an insect pest species to down-regulate expression of said target gene. Concatemeric RNA constructs of this type are described in WO2006/046148 as incorporated herein by reference. In the context of the present invention, the term 'multiple' means at least two, at least three, at least four, etc and up to at least 10, 15, 20 or at least 30. In one embodiment, the interfering RNA comprises multiple copies of a single silencing element i.e. repeats of a silencing element that binds to a particular target nucleotide sequence within a specific target gene. In another embodiment, the silencing elements within the interfering RNA comprise or consist of different sequences of nucleotides complementary to different target nucleotide sequences. It should be clear that combinations of multiple copies of the same silencing element combined with silencing elements binding to different target nucleotide sequences are within the scope of the current invention.

The different target nucleotide sequences may originate from a single target gene in an insect pest species in order to achieve improved down-regulation of a specific target gene in an insect pest species. In this case, the silencing elements may be combined in the interfering RNA in the original order in which the target nucleotide sequences occur in the target gene, or the silencing elements may be scrambled and combined randomly in any rank order in the context of the interfering RNA as compared with the order of the target nucleotide sequences in the target gene.

Alternatively, the different target nucleotide sequences are representing a single target gene but originating from different insect pest species.

Alternatively, the different target nucleotide sequences may originate from different target genes. If the interfering RNA is for use in preventing and/or controlling pest infestation, it is preferred that the different target genes are chosen from the group of genes regulating essential biological functions of insect pest species, including but not limited to survival, growth, development, reproduction and pathogenicity. The target genes may regulate the same or different biological pathways or processes. In one embodiment, at least one of the silencing elements comprises or consists of a sequence of nucleotides which is at least partially complementary to a target nucleotide sequence within a target gene wherein the target gene is selected from the group of genes as described earlier.

In a further embodiment of the invention, the different genes targeted by the different silencing elements originate from the same insect pest species. This approach is designed to achieve enhanced attack against a single insect pest species. In particular, the different target genes may be expressed differentially in the different stages of the insect's life cycle, for example, the mature adult, immature larval and egg stages. The interfering RNA of the invention may thus be used to prevent and/or control insect pest infestation at more than one stage of the insect's life cycle.

In an alternative embodiment of the invention, the different genes targeted by the different silencing elements originate from different insect pest species. The interfering RNA of the invention can thus be used to prevent and/or control infestation by more than one insect pest species simultaneously. The silencing elements may be arranged as one contiguous region of the interfering RNA or may be separated by the presence of linker sequences. The linker sequence may comprise a short random nucleotide sequence that is not complementary to any target nucleotide sequences or target genes. In one embodiment, the linker is a conditionally self-cleaving RNA sequence, preferably a pH-sensitive linker or a hydrophobic-sensitive linker. In one embodiment, the linker comprises a sequence of nucleotides equivalent to an intronic sequence. Linker sequences of the current invention may range in length from about 1 base pair to about 10000 base pairs, provided that the linker does not impair the ability of the interfering RNA to down-regulate the expression of target gene(s).

In addition to the silencing element(s) and any linker sequences, the interfering RNA of the invention may comprise at least one additional polynucleotide sequence. In different embodiments of the invention, the additional sequence is chosen from (i) a sequence capable of protecting the interfering RNA against RNA processing, (ii) a sequence affecting the stability of the interfering RNA, (iii) a sequence allowing protein binding, for example to facilitate uptake of the interfering RNA by cells of the insect pest species, (iv) a sequence facilitating large-scale production of the interfering RNA, (v) a sequence which is an aptamer that binds to a receptor or to a molecule on the surface of the insect pest cells to facilitate uptake, or (v) a sequence that catalyses processing of the interfering RNA within the insect pest cells and thereby enhances the efficacy of the interfering RNA. Structures for enhancing the stability of RNA molecules are well known in the art and are described further in WO2006/046148 as incorporated herein by reference.

The length of the interfering RNA of the invention needs to be sufficient for uptake by the cells of an insect pest species and down-regulation of target genes within the pest as described elsewhere herein. However, the upper limit on length may be dependent on (i) the requirement for the interfering RNA to be taken up by cells of the pest and (ii) the requirement for the interfering RNA to be processed in the cells of the pest to mediate gene silencing via the RNAi pathway. The length may also be dictated by the method of production and the formulation for delivery of the interfering RNA to cells. Preferably, the interfering RNA of the current invention will be between 21 and 10000 nucleotides in length, preferably between 50 and 5000 nucleotides or between 100 and 2500 nucleotides, more preferably between 80 and 2000 nucleotides in length.

The interfering RNA may contain DNA bases, non-natural bases or non-natural backbone linkages or modifications of the sugar-phosphate backbone, for example to enhance stability during storage or enhance resistance to degradation by nucleases. Furthermore, the interfering RNA may be produced chemically or enzymatically by one skilled in the art through manual or automated reactions. Alternatively, the interfering RNA may be transcribed from a polynucleotide encoding the same. Thus, provided herein is an isolated polynucleotide encoding any of the interfering RNAs of the current invention.

Also provided herein is an isolated polynucleotide selected from the group consisting of (i) a polynucleotide which comprises at least 21, preferably at least 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented by any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (ii) a polynucleotide which consists of at least 21, preferably at least 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100 or 1115 contiguous nucleotides of a nucleotide sequence as represented by any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (iii) a polynucleotide which comprises at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented in any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, so that, when the two sequences are optimally aligned and compared, said polynucleotide is at least 75 % preferably at least 80%, 85%, 90%, 95%, 98% or 99% identical to any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or (iv) a polynucleotide which comprises a fragment of at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide as represented in any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein said fragment or said complement has a nucleotide sequence so that, when said fragment is optimally aligned and compared with the corresponding fragment in any of SEQ ID NOs. 1-26, 121-205, 386-389, 394, 400, said nucleotide sequence is at least 75% preferably at least 80%, 85%, 90%, 95%, 98% or 99% identical to said corresponding fragment of any of SEQ ID NOs. 1-26, 121-205, 386-389, 394, 400 or the complement thereof, or (v) a polynucleotide which consists of a fragment of at least 21, preferably at least 22, 23 or 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide as represented in any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein said fragment or said complement has a nucleotide sequence so that, when said fragment is optimally aligned and compared with the corresponding fragment in any of SEQ ID NOs. 1-26, 121-205, 386-389, 394, 400, said nucleotide sequence is at least 75% preferably at least 80%, 85%, 90%, 95%, 98% or 99 %identical to said corresponding fragment of any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389 or the complement thereof, or (vi) a polynucleotide encoding an amino acid sequence that, when the two amino acid sequences are optimally aligned and compared, is at least 70 % preferably at least 75%, 80%, 85%, 90%, 95%, 98% or 99% identical to the amino acid sequence encoded by any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, and wherein said polynucleotide is no longer than 10000, 9000, 8000, 7000, 6000, 5000, 4000, 3000, 2000 or 1500 nucleotides.

In preferred embodiments, the isolated polynucleotide is part of an interfering RNA molecule, typically part of the silencing element, comprising at least one double-stranded region comprising a sense RNA strand annealed by complementary basepairing to an antisense RNA strand wherein the sense strand of the dsRNA molecule comprises a sequence of nucleotides complementary to a sequence of nucleotides located within the RNA transcript of the target gene. The sense strand of the dsRNA is therefore able to anneal to the RNA transcript and target the RNA for degradation within the RNAi-induced silencing complex or RISC.

The polynucleotides of the invention may be inserted via routine molecular cloning techniques into DNA constructs or vectors known in the art. Therefore, according to one embodiment, a DNA construct comprising any of the polynucleotides of the current invention is provided. Preferably, provided herein is a DNA construct comprising a polynucleotide encoding at least one of the interfering RNAs of the current invention. The DNA construct may be a recombinant DNA vector, for example a bacterial or yeast vector or plant vector. In a preferred embodiment of the invention, the DNA construct is an expression construct and the polynucleotide is operably linked to at least one regulatory sequence capable of driving expression of the polynucleotide sequence. The term 'regulatory sequence' is to be taken in a broad context and is intended to refer to any nucleotide sequence capable of effecting expression of polynucleotides to which it is operably linked including but not limited to promoters, enhancers and other naturally-occurring or synthetic transcriptional activator elements. The regulatory sequence may be located at the 5' or 3' end of the polynucleotide sequence. The term 'operably linked' refers to a functional linkage between the regulatory sequence and the polynucleotide sequence such that the regulatory sequence drives expression of the polynucleotide. Operably linked elements may be contiguous or non-contiguous.

Preferably, the regulatory sequence is a promoter selected from the group comprising but not limited to constitutive promoters, inducible promoters, tissue-specific promoters and growth/developmental stage-specific promoters. In one embodiment, the polynucleotide is placed under the control of a strong constitutive promoter such as any selected from the group comprising the CaMV35S promoter, doubled CaMV35S promoter, ubiquitin promoter, actin promoter, rubisco promoter, GOS2 promoter, Figwort mosaic virus 34S promoter. In another embodiment, the regulatory sequence is a plant promoter for use in regulating expression of the polynucleotide in plants. Plant promoters, in particular, tissue-specific plant promoters encompassed within the scope of the current invention are described in more detail elsewhere herein.

Optionally, one or more transcription termination sequences may be incorporated in the expression construct of the invention. The term 'transcription termination sequence' encompasses a control sequence at the end of a transcriptional unit, which signals termination of transcription, 3' processing and poly-adenylation of a primary transcript. Additional regulatory sequences including but not limited to transcriptional or translational enhancers may be incorporated in the expression construct, for instance as with the double enhanced CaMV35S promoter.

The present invention also encompasses a method for generating any of the interfering RNAs of the invention comprising the steps of (i) contacting a polynucleotide encoding said interfering RNA or a DNA construct comprising the same with cell-free components; or (ii) introducing (e.g. by transformation, transfection or injection) a polynucleotide encoding said interfering RNA or a DNA construct comprising the same into a cell.

The invention thus also relates to any double stranded ribonucleotide produced from the expression of a polynucleotide described herein.

Accordingly, also provided herein is a host cell transformed with any of the polynucleotides described herein. Further encompassed by the present host cells comprising any of the interfering RNA's of the current invention, any of the polynucleotides of the current invention or a DNA construct comprising the same. The host cell may be a prokaryotic cell including but not limited to gram-positive and gram-negative bacterial cells, or an eukaryotic cell including but not limited to yeast cells or plant cells. Preferably, said host cell is a bacterial cell or a plant cell. The bacterial cell can be chosen from the group comprising, but not limited to, Gram positive and Gram negative cells comprising *Escherichia* spp. (e.g. *E. coli*), *Bacillus* spp. (e.g. *B. thuringiensis*), *Rhizobium* spp., *Lactobacillus* spp., *Lactococcus* spp., *Pseudomonas* spp. and *Agrobacterium* spp.. The polynucleotide or DNA construct of the invention may exist or be maintained in the host cell as an extra-chromosomal element or may be stably incorporated into the genome of the host cell. Characteristics of particular interest in selecting a host cell for the purposes of the current invention include the ease with which the polynucleotide or DNA construct encoding the interfering RNA can be introduced into the host, the availability of compatible expression systems, the efficiency of expression, and the stability of the interfering RNA in the host.

Preferably, the interfering RNAs of the invention are expressed in a plant host cells. Preferred plants of interest include but are not limited to cotton, potato, rice, tomato, canola, soy, sunflower, sorghum, pearl millet, corn, alfalfa, strawberries, eggplant, pepper and tobacco.

In situations wherein the interfering RNA is expressed within a host cell and/or is used to prevent and/or control pest infestation of a host organism, it is preferred that the interfering RNA does not exhibit significant 'off-target' effects i.e. the interfering RNA does not affect expression of genes within the host. Preferably, the silencing element does not exhibit significant complementarity with nucleotide sequences other than the intended target nucleotide sequence of the target gene. In one embodiment of the invention, the silencing element shows less than 30%, more preferably less than 20%, more preferably less than 10% and even more preferably less than 5% sequence identity with any gene of the host cell or organism. If genomic sequence data is available for the host organism, one can cross-check identity with the silencing element using standard bioinformatics tools. In one embodiment, there is no sequence identity between the silencing element and a gene from the host cell or host organism over a region of 17, more preferably over a region of 18 or 19 and most preferably over a region of 20 or 21 contiguous nucleotides.

In the practical application of the invention, the interfering RNAs of the invention may be used for the prevention and/or control of any insect pest belonging to the Orders *Coleoptera, Lepidoptera, Diptera, Dichyoptera, Orthoptera, Hemiptera and Siphonaptera.*

Also provided herein is a method for preventing and/or controlling pest infestation, comprising contacting an insect pest species with an effective amount of at least one interfering RNA wherein the RNA functions upon uptake by said pest to down-regulate expression of an essential pest target gene. The essential target gene may be any pest gene involved in the regulation of an essential biological process required by the pest to initiate or maintain infestation including but not limited to survival, growth, development, reproduction and pathogenicity. In particular, the target gene may be any of the pest genes as described elsewhere herein.

Furthermore, there is provided herein a method for preventing and/or controlling insect pest infestation in a field of crop plants, said method comprising expressing in said plants an effective amount of an interfering RNA as described herein.

Wherein the method is for the control of pest infestation, the phrase 'effective amount' extends to the quantity or concentration of interfering RNA required to produce a phenotypic effect on the pest such that the numbers of pest organisms infesting a host organism are reduced and/or the amount of damage caused by the pest is reduced. In one embodiment, the phenotypic effect is death of the pest and the interfering RNA is used to achieve at least 20%, 30%, 40%, preferably at least 50%, 60%, 70%, more preferably at least 80% or 90% pest mortality as compared to control insect pests. In a further embodiment, the phenotypic effects include but are not limited to stunting of pest growth, cessation of feeding and reduced egg-laying. The total numbers of pest organisms infesting a host organism may thus be reduced by at least 20%, 30%, 40%, preferably at least 50%, 60%, 70%, more preferably at least 80% or 90% as compared with control pests. Alternatively, the damage caused by the insect pest may be reduced by at least 20%, 30%, 40%, preferably at least 50%, 60%, 70%, more preferably at least 80% or 90% as compared with control insect pests. Hence, the method of the invention can be used to achieve at least 20%, 30%, 40%, preferably at least 50%, 60%, 70%, more preferably at least 80% or 90% pest control.

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides of any of SEQ ID NOs 1, 174, 404, 180, 188, 2, 175, 181, 189, 27 to 30, 282 to 285, 294 to 297 or 310 to 313, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 1, 174, 404, 180, 188, 2, 175, 181, 189, 27 to 30, 282 to 285, 294 to 297 or 310 to 313, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm), wherein the orthologous genes encode a protein having an amino acid sequence which is at least 85%, 90%, 92%, 94%, 96%, 98%, 99% identical the amino acid sequence as presented in any of SEQ ID NOs 79, 349, 405, 352 or 356 (when said encoded proteins are optimally aligned).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 141, 11, 12, 47 to 50, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 141, 11, 12, 47 to 50, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm), wherein the orthologous genes encode a protein having an amino acid sequence which is at least 85%, 90%, 92%, 94%, 96%, 98%, 99% identical to the amino acid sequence as presented in any of SEQ ID NOs 328 or 84 (when said encoded proteins are optimally aligned).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 17, 18, 59 to 62, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 17, 18, 59 to 62, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm), wherein the orthologous genes encode a protein having an amino acid sequence which is at least 85%, 90%, 92%, 94%, 96%, 98%, 99% identical to the amino acid sequence as presented in SEQ ID NOs 87 (when said encoded proteins are optimally aligned).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 19, 20, 63 to 66, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 19, 20, 63 to 66, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm), wherein the orthologous genes encode a protein having an amino acid sequence which is at least 85%, 90%, 92%, 94%, 96%, 98%, 99% identical to the amino acid sequence as presented in SEQ ID NOs 88 (when said encoded proteins are optimally aligned).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 165, 167, 166, 270 to 273, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm). In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 165, 167, 166, 270 to 273, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm), wherein the orthologous genes encode a protein having an amino acid sequence which is at least 85%, 90%, 92%, 94%, 96%, 98%, 99% identical to the amino acid sequence as presented in any of SEQ ID NOs 347 or 348 (when said encoded proteins are optimally aligned).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 143, 121, 142, 176, 182, 130, 177, 183, 206 to 209, 286 to 289, 298 to 301, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 143, 121, 142, 176, 182, 130, 177, 183, 206 to 209, 286 to 289, 298 to 301, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm),
wherein the orthologous genes encode a protein having an amino acid sequence which is at least 85%, 90%, 92%, 94%, 96%, 98%, 99% identical to the amino acid sequence as presented in any of SEQ ID NOs 330, 350 or 353 (when said encoded proteins are optimally aligned).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 145, 122, 144, 178, 131, 179, 210 to 213, 290 to 293, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 145, 122, 144, 178, 131, 179, 210 to 213, 290 to 293, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm), wherein the orthologous genes encode a protein having an amino acid sequence which is at least 85%, 90%, 92%, 94%, 96%, 98%, 99% identical to the amino acid sequence as presented in any of SEQ ID NOs 331 or 351 (when said encoded proteins are optimally aligned).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 128, 149, 184, 137, 185, 234 to 237, 302 to 305, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm).

In the methods described herein to down-regulate expression of a target gene in an insect pest species, double stranded RNA molecules comprising at least 21 bp, one strand of which comprises or consists of a sequence of nucleotides which is complementary to at least 21 contiguous nucleotides in any of SEQ ID NOs 128, 149, 184, 137, 185, 234 to 237, 302 to 305, or the complement thereof, can be used to down-regulate expression of the orthologous target gene in a coleopteran, hemipteran, lepidoteran or dipteran insect chosen from the group comprising but not limited to Leptinotarsa spp. (e.g. L. decemlineata (Colorado potato beetle), L. juncta (false potato beetle), or L. texana (Texan false potato beetle)); Nilaparvata spp. (e.g. N. lugens (brown planthopper)); Lygus spp. (e.g. L. lineolaris (tarnished plant bug) or L. hesperus (western tarnished plant bug)); Myzus spp. (e.g. M. persicae (green peach aphid)); Diabrotica spp. (e.g. D. virgifera virgifera (western corn rootworm), D. barberi (northern corn rootworm), D. undecimpunctata howardi (southern corn rootworm) or D. virgifera zeae (Mexican corn rootworm), wherein the orthologous genes encode a protein having an amino acid sequence which is at least 85%, 90%, 92%, 94%, 96%, 98%, 99% identical to the amino acid sequence as presented in any of SEQ ID NOs 337 or 354 (when said encoded proteins are optimally aligned).

In one embodiment, the plant to be treated is engineered to express the interfering RNA intracellularly via transcription from a polynucleotide incorporated therein. As the pest feeds on tissues of the plant, the cells containing the interfering RNA will be broken down inside the insect's digestive tract and the interfering RNA will thus be distributed within the insect's body resulting in down-regulation of target genes.

Thus, in accordance with another aspect of the present invention is provided a method for generating a transgenic plant resistant to infestation by an insect pest species comprising the steps of (a) transforming a plant cell with a DNA construct comprising a polynucleotide sequence encoding an interfering ribonucleic acid (RNA) that functions upon uptake by an insect pest species to down-regulate expression of a target gene in said insect pest species, (b) regenerating a plant from the transformed plant cell; and (c) growing the transformed plant under conditions suitable for the expression of the interfering RNA from the recombinant DNA construct, said plant thus being resistant to said pest as compared with an untransformed plant.

The interfering RNA expressed by the plant or part thereof may be any of those as disclosed elsewhere herein. Preferably, the interfering RNA comprises or consists of at least one silencing element and said silencing element is a region of double-stranded RNA comprises annealed complementary strands, one strand of which (the sense strand) comprises a sequence of nucleotides which is at least partially complementary to a target nucleotide sequence within a target gene. Wherein part of the interfering RNA is double-stranded, the two strands of the molecule may be expressed from at least two separate polynucleotides or may be encoded by a single polynucleotide encoding an interfering RNA with for example, a stem-loop structure or a so-called hairpin structure as described elsewhere herein.

The interfering RNA expressed by the plant or part thereof may target any of the pest genes as described elsewhere herein. In particular, the target gene may be selected from the group of genes having a nucleotide sequence comprising any of SEQ ID NOs. 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or having a nucleotide sequence that, when the two sequences are optimally aligned and compared, is at least 75%, preferably at least 80%, 85%, 90%, 95%, 98% or 99% identical any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or is selected from the group of genes having a nucleotide sequence consisting of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or is selected from the group of genes having a nucleotide sequence comprising a fragment of at least 20, 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides of any of SEQ ID NOs. 1-26, 121-205, 386-389, 394, 400, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, or is selected from the group of genes having a nucleotide sequence comprising a fragment of at least 20, 21, 22, 23, 24, 25, 26, 27, 28, 29, 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, said nucleotide sequence of said fragment is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof. The target gene may also be an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 23, 24, 71 to 74, 25, 26, 75 to 78, 143, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, 128, 149, 184, 137, 185, 234 to 237, 302 to 305, 129, 138, 238 to 241, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, 168, 170, 169, 274 to 277, 172, 173, 278 to 281, 200, 201, 314 to 317, 402, 186, 202, 187, 203, 306 to 309, 318 to 321, 386, 387, 388, 389.

In one embodiment, the target gene
(i) is selected from the group of genes having a nucleotide sequence comprising any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233or the complement thereof, or having a nucleotide sequence so that, when the two sequences are optimally aligned and compared, is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or
(ii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or
(iii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, said nucleotide sequence of said fragment is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, or
(iv) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401, 121, 142, 176, 182, 130, 177, 183, 206 to 209, 286 to 289, 298 to 301, 145, 122, 144, 178, 131, 179, 210 to 213, 290 to 293, 123, 132, 214 to 217, 124, 133, 218 to 221, 146, 125, 134, 222 to 225, 147, 126, 135, 226 to 229, 127, 148, 136, 230 to 233.

In one embodiment, the target gene
(i) is selected from the group of genes having a nucleotide sequence comprising any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or having a nucleotide sequence so that, when the two sequences are optimally aligned and compared, is at least 75% preferably at least 80%, 85%, 90%, 95%, 98% or 99% identical to any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or
(ii) is selected from the group of genes having a nucleotide sequence comprising a fragment of at least 21 contiguous nucleotides of any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or having a nucleotide sequence so that, when said gene comprising said fragment is optimally aligned and compared with any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, said nucleotide sequence is at least 75% preferably at least 80%, 85%, 90%, 95%, 98% or 99% identical to any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273 or the complement thereof, or
(iii) is selected from the group of genes having a nucleotide sequence comprising a fragment of at least 21 contiguous nucleotides of any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, and wherein when said fragment is optimally aligned and compared with the corresponding fragment in any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, said nucleotide sequence of said fragment is at least 75% preferably at least 80%, 85%, 90%, 95%, 98% or 99% identical to said corresponding fragment of any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, or
(iv) is an insect pest orthologue of a gene having a nucleotide sequence comprising any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273, or
(v) is selected from the group of genes having a nucleotide sequence encoding an amino acid sequence that, when the two amino acid sequences are optimally aligned and compared, is at least 85% preferably at least 90%, 95%, 98% or 99%identical to the amino acid sequence encoded by any of SEQ ID NOs. 3, 4, 31 to 34, 139, 5, 6, 35 to 38, 140, 7, 8, 39 to 42, 9, 10, 43 to 46, 141, 11, 12, 47 to 50, 13, 14, 51 to 54, 15, 204, 16, 205, 55 to 58, 322 to 325, 17, 18, 59 to 62, 19, 20, 63 to 66, 21, 22, 67 to 70, 150, 151, 242 to 245, 152, 153, 246 to 249, 154, 155, 250 to 253, 156, 157, 254 to 257, 158, 159, 258 to 261, 160, 161, 262 to 265, 163, 162, 164, 266 to 269, 165, 167, 166, 270 to 273.

In one embodiment, the target gene
(i) is selected from the group of genes having a nucleotide sequence comprising any of SEQ ID NOs 1, 174, 404, 180, 188, 2, 175, 181, 189, 27 to 30, 282 to 285, 294 to 297, 310 to 313 or 401, or the complement thereof, or having a nucleotide sequence so that, when the two sequences are optimally aligned and compared, is at least 75% preferably at least 80%, 85%, 90%, 95%, 98% or 99% identical to any of SEQ ID NOs 1, 174, 404, 180, 188, 2, 175, 181, 189, or the complement thereof, or
(ii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313 or 401, or the complement thereof, or having a nucleotide sequence so that, when said gene comprising said fragment is optimally aligned and compared with any of SEQ ID NOs 1, 174, 404, 180, 188, 2, 175, 181, 189, 27 to 30, 282 to 285, 294 to 297, 310 to 313, or 401, said nucleotide sequence is at least 75% preferably at least 80%, 85%, 90%, 95%, 98% or 99% identical to any of SEQ ID NOs 1, 174, 404, 180, 188, 2, 175, 181, 189, 27 to 30, 282 to 285, 294 to 297, 310 to 313, or 401, or the complement thereof, or
(iii) is selected from the group of genes having a nucleotide sequence comprising a fragment of at least 21 contiguous nucleotides of any of SEQ ID NOs 1, 174, 404, 180, 188, 2, 175, 181, 189, 27 to 30, 282 to 285, 294 to 297, 310 to 313, or 401, or the complement thereof, and wherein when said fragment is optimally aligned and compared with the corresponding fragment in any of SEQ ID NOs 1, 174, 404, 180, 188, 2, 175, 181, 189, 27 to 30, 282 to 285, 294 to 297, 310 to 313, or 401, said nucleotide sequence of said fragment is at least 75% preferably at least 80%, 85%, 90%, 95%, 98% or 99% identical to said corresponding fragment of any of SEQ ID NOs 1, 174, 404, 180, 188, 2, 175, 181, 189, 27 to 30, 282 to 285, 294 to 297, 310 to 313, or 401, or the complement thereof, or
(iv) is an insect pest orthologue of a gene having a nucleotide sequence comprising any of SEQ ID NOs 1, 174, 404, 180, 188, 2, 175, 181, 189, 27 to 30, 282 to 285, 294 to 297, 310 to 313, or 401, or the complement thereof, wherein the two orthologous genes are similar in sequence to such a degree that when the two genes are optimally aligned and compared, the orthologue has a sequence that is at least 75% preferably at least 80%, 85%, 90%, 95%, 98% or 99% identical to any of the sequences represented by SEQ ID NOs 1, 174, 404, 180, 188, 2, 175, 181, 189, 27 to 30, 282 to 285, 294 to 297, 310 to 313, 401, or
(v) is selected from the group of genes having a nucleotide sequence encoding an amino acid sequence that, when the two amino acid sequences are optimally aligned and compared, is at least 85% preferably at least 90%, 95%, 98% or 99% identical to the amino acid sequence encoded by any of SEQ ID NOs 1, 174, 404, 180, 188, 2, 175, 181, 189, 27 to 30, 282 to 285, 294 to 297, 310 to 313, 401.

Preferably the nucleotide sequence of said target gene is no longer than 10000, 9000, 8000, 7000, 6000, 5000, 4000, 3000, 2000 or 1500 nucleotides. Furthermore, it is important that the interfering RNA does not disrupt expression of any genes of the plant host.

As used herein, the term 'transgenic plant' or 'transgenic plant cell' refers to any plant or plant cell that has been genetically engineered or is descended from a plant that has been genetically engineered so as to carry an exogenous polynucleotide sequence. 'Exogenous' refers to the fact that the polynucleotide originates from outside the plant cell. Typically, the exogenous polynucleotide is non-native to the transgenic plant i.e. it is not found naturally within the genome of the plant.

As used herein, the term 'transformation' refers to the introduction of exogenous polynucleotide molecules into a plant or a cell thereof. Techniques for introducing polynucleotides into plants are known in the art. In one embodiment of the current invention, the plants are 'stably transformed' with a polynucleotide or DNA construct comprising the same, i.e. the polynucleotide or DNA construct introduced into the plant cell integrates into the genome of the plant and is capable of being inherited by the progeny thereof. Transformation protocols for introducing polynucleotides or DNA constructs into the cells of plants may vary depending on the type of plant concerned. Suitable transformation methods include but are not limited to microinjection, electroporation, *Agrobacterium*-mediated transformation, and ballistic particle acceleration. Methods are also known in the art for the targeted insertion of a polynucleotide or DNA construct at a specific location in the plant genome using site-specific recombination systems.

The DNA construct comprising the polynucleotide encoding the active interfering RNA molecule may be any vector suitable for transformation of plant cells. Suitable vectors include but are not limited to bacterial plasmids, for example the Ti plasmid of *Agrobacterium tumefaciens,* and viral vector systems. The DNA construct introduced into the cells of a plant must not be harmful or toxic to the plant and/or must not be harmful or toxic to any organisms higher up the food chain that feed on said plants.

In one embodiment, the DNA construct is an expression construct comprising a polynucleotide encoding an interfering RNA operably linked to a regulatory sequence capable of driving expression of the polynucleotide sequence in plants such as any selected from the group comprising the CaMV35S promoter, doubled CaMV35S promoter, ubiquitin promoter, actin promoter, rubisco promoter, GOS2 promoter, Figwort mosaic virus 34S promoter and the double enhanced CaMV35S promoter. Preferably, the regulatory sequence is a plant promoter selected from those known in the art. In some embodiments, it may be preferred that the plant produces interfering RNA molecules only in the parts of the plant which will come into contact with and/or are damaged by the insect pest species, for example, the aerial parts of the plant, the roots etc. This effect can be achieved through the use of tissue-specific plant promoters including but not limited to leaf-specific promoters, root-specific promoters, stem-specific promoters, flower-specific promoters and fruit-specific promoters known in the art. Suitable examples of a root specific promoter are PsMTA and the Class III Chitinase promoter. Examples of leaf- and stem-specific or photosynthetic tissue-specific promoters that are also photoactivated are promoters of two chlorophyll binding proteins (cab1 and cab2) from sugar beet, ribulose-bisphosphate carboxylase (Rubisco), encoded by rbcS, A (gapA) and B (gapB) subunits of chloroplast glyceraldehyde-3-phosphate dehydrogenase, promoter of the *Solanum tuberosum* gene encoding the leaf and stem specific (ST-LS1) protein, stem-regulated, defense-inducible genes, such as JAS promoters, flower-specific promoters such as chalcone synthase promoter and fruit-specific promoters such as that of RJ39 from strawberry.

In other embodiments, it may be preferred that the plant produces interfering RNA molecules only at a particular stage of its growth. This effect can be achieved through the use of development-specific plant promoters that drive expression only during certain periods of plant development. In particular, it is important to protect plants from pest infestation during the early stages of plant growth or during flowering (for instance in case of rice) or during fructification or fruit maturation or seed-filling, as this is the time when the plant can be most severely damaged.

The DNA construct for use in transformation of a plant according to the present method may comprise more than one polynucleotide encoding an interfering RNA molecule of the current invention. In one embodiment, the different polynucleotides may encode interfering RNA molecules targeting different nucleotide sequences within the same target gene. In a further embodiment, the different polynucleotides may encode interfering RNA molecules targeting different nucleotide sequences within different target genes, wherein the different target genes originate from the same or different insect pest species. Wherein the DNA construct encodes more than one interfering RNA, these RNAs may be expressed differentially within different tissues of the plant by virtue of being under the control of different tissue-specific promoter sequences as described elsewhere herein. In one embodiment, the plant is engineered to express an interfering RNA in the leaves which down-regulates expression of a target gene in an insect that feeds on the leaves, and to additionally express an interfering RNA in the roots which down-regulates expression of a target gene in an insect that colonizes the soil and feeds on the plant roots.

The DNA construct may also comprise at least one other polynucleotide of interest, for example a polynucleotide encoding an additional regulatory RNA molecule, a polynucleotide encoding a protein toxic to insect pest species and/or a polynucleotide encoding a protein conferring herbicide resistance or tolerance.

In accordance with the present method, a plant is regenerated from a transformed plant cell using techniques known in the art. One such technique comprises enzymatic digestion of the plant cell wall to produce a plant protoplast, which can subsequently undergo multiple rounds of cell division and differentiation to produce an adult plant. Adult plants generated in such a way can be subsequently tested for resistance to pest infestation. 'Resistant' as used herein should be interpreted broadly and relates to the ability of the plant to defend against attack from a pest that is typically capable of inflicting damage or loss to the plant. Resistant may either be taken to mean that the plant is no longer susceptible to pest infestation or that any disease symptoms resulting from pest infestation are reduced by at least about 20%, preferably at least 30%, 40% or 50%, more preferably at least 60%, 70% or 80% and most preferably by at least 90%. Techniques to measure the resistance of a plant to insect pest species are commonly known in the art and include but are not limited to measuring over time the average lesion diameter, the pest biomass or weight, the pest survival and/or mortality, and/or the overall percentage of decayed plant tissues.

In one embodiment, the present method of producing a transgenic plant also includes the step of generating offspring or progeny from the transgenic plant and testing the progeny for resistance to the insect pest. Two or more generations may be produced to ensure that expression of the resistance trait is stably maintained and inherited. Seeds may also be harvested from the parent transgenic plant and/or its progeny to test for resistance to an insect pest.

Also encompassed within the present invention is a method for generating transgenic plants resistant to infestation by an insect pest species comprising the steps of crossing a first transgenic plant carrying a DNA construct encoding an interfering RNA that functions to down-regulate expression of a pest target gene, with a second plant lacking said DNA construct, and selecting progeny resistant to said pest. Crossing may be carried out by any methods routinely used in the context of plant breeding. The progeny selected for pest resistance may additionally be self-pollinated or 'selfed' to produce a subsequent generation of pest resistant progeny. In one embodiment, multiple rounds of self pollination or selfing may be carried out to generate 2, 3, 4, 5 or more generations of progeny. The resultant progeny may be tested for pest resistance to ensure that expression of the resistance trait is stably maintained and inherited.

In a further embodiment, any pest resistant progeny plants derived from a cross between a first transgenic parent plant carrying a DNA construct of interest and a second parent plant lacking said DNA construct may be back-crossed to the second parent plant and subsequent progeny tested for resistance to pest infestation. Plants or their progeny may be tested for resistance to pest infestation either by phenotypic analysis as described elsewhere herein or by standard molecular techniques. For example, the pest resistant plants may be identified by the detection of the presence of a polynucleotide sequence encoding an interfering RNA that functions upon uptake by an insect pest species to down-regulate expression of a target gene. Techniques for detecting the presence of specific polynucleotide sequences within cells are known in the art and include PCR, enzymatic digestion and SNP analysis.

The methods of the invention can be used to generate 'stacked transgenic' plants that are resistant to insect pest species and that optionally possess at least one other desirable trait. As used herein, a 'stacked' transgenic plant refers to a plant carrying more than one exogenous polynucleotide sequence. The phrase 'more than one' refers to the possibility of a plant carrying at least 2, at least 3, at least 4 exogenous polynucleotides. In one embodiment, the plant cell transformed with the DNA construct encoding the interfering RNA targeting a pest gene may have previously been engineered to carry a separate exogenous polynucleotide. Alternatively, the method for generating a transgenic plant from a plant cell as described herein may comprise a co-transformation protocol wherein the DNA construct encoding an interfering RNA of the invention is delivered to a plant cell simultaneously or sequentially with a separate exogenous polynucleotide.

Stacked transgenic plants demonstrating pest resistance may also be generated by standard plant breeding techniques. In one embodiment, a first pest-resistant transgenic plant is crossed with a second plant engineered to express an exogenous polynucleotide or heterologous gene conferring a desirable plant trait. Any progeny produced can be tested for pest resistance and acquisition of the additional desirable trait. Alternatively or in addition, any pest-resistant progeny produced from the cross may be back-crossed to the second parent in order to generate further progeny that can be selected for inheritance of the heterologous gene carried by the second parent and thus the additional desirable plant trait. The exogenous polynucleotides carried by a stacked transgenic plant of the invention may be expressed in the same parts of the plant or may be expressed differentially by virtue of the fact that expression of each is controlled by a different tissue-specific promoter.

In one embodiment, the exogenous polynucleotide or heterologous gene conferring a further desirable trait encodes another interfering RNA targeting the same or different insect pest species. In a further embodiment, the heterologous gene encodes a protein harmful or toxic to a plant insect pest species, for example an insecticidal protein selected from the group including but not limited to *Bacillus thuringiensis* insecticidal proteins, *Xenorhabdus* insecticidal proteins, *Photorhabdus* insecticidal proteins, *Bacillus laterosporous* insecticidal proteins, *Bacillus sphaericus* insecticidal proteins, and VIP insecticidal proteins, such as a protein selected from the group including but not limited to Cry1Ab, Cry1C, Cry2Aa, Cry3, CryET70, Cry22, CryET33, CryET34, CryET80, CryET76, TIC100, TIC101, TIC851, TIC900, TIC901, TIC1201, TIC407, TIC417 andPS149B1 insecticidal proteins. In a yet further embodiment, the heterologous gene encodes a protein conferring herbicide resistance or tolerance. Examples of genes conferring herbicide resistance or tolerance include *Bar, EPSPS* which confers glyphosate resistance, *ALS* which confers imidazolinone and sulphonylurea resistance and *bxn* which confers bromoxynil resistance.

Also provided herein is a method for producing hybrid seed from any of the transgenic plants generated by the methods of the current invention, said method comprising the steps of (i) planting the seed obtained from a first inbred plant and the seed obtained from a second inbred plant, wherein at least one of the inbred plants is a transgenic plant resistant to pest infestation (ii) cultivating the seeds into plants that bear flowers, (iii) preventing self-pollination of at least one of the first or second adult plants, (iv) allowing cross-pollination to occur between the first and second plants; and (v) harvesting the seeds resulting from the cross-pollination. Hybrid seed produced by this method and hybrid plants produced by cultivating said seed are within the scope of the current invention. Hybrid plants produced by this method will typically be genetically uniform and are likely to exhibit heterosis or hybrid vigour. Thus, crops with the potential for increased yield may be generated by such a method.

Included within the group of transgenic plants of the current invention are transgenic plants produced by any of the methods described herein. Thus in one embodiment of the invention the transgenic plants comprise stacked transgenic traits carrying a first exogenous polynucleotide conferring pest resistance and at least one other exogenous polynucleotide or heterologous gene conferring an additional desirable plant trait. The additional heterologous genes may comprise genes encoding additional pesticidal agents, genes encoding proteins toxic or harmful to insect pest species and/or genes encoding proteins conferring herbicide resistance as described elsewhere herein.

Preferred transgenic plants according to the invention include but are not limited to cotton, potato, rice, tomato, canola, soy, sunflower, sorghum, pearl millet, corn, alfalfa, strawberries, eggplant, pepper and tobacco.

Also provided herein is the use of the interfering ribonucleic acid (RNA) as described herein or the DNA construct as described herein for preventing and/or controlling insect pest infestation, preferably insect pest infestation of plants.

The invention will be further understood with reference to the following non-limiting examples.

### Examples

### Example 1 Identification of target genes in insect pest species

### 1.1. Lygus hesperus normalized cDNA library and preparation of dsRNAs in multiwell plates for the screening assays

Nucleic acids were isolated from *Lygus hesperus* nymphs of different life stages, including freshly hatched nymphs 2, 4, 6 and 9 days old nymphs and adults. A cDNA library was prepared using the SMARTer™ PCR cDNA Synthesis Kit, following the manufacturer's instructions (Clontech Cat. No 634925). The cDNA library was normalized using the Trimmer kit (Evrogen Cat No NK001) and cloned in the PCR4-TOPO vector (Invitrogen). The normalization of the clones introduced M2 adapters (Trimmer Kit, Evrogen, SEQ ID NO 92: AAGCAGTGGTATCAACGCAG), oppositely oriented at each end of the clones. The recombinant vector constructs were transformed into cells of *Escherichia coli* strain TOP10 (Invitrogen). The transformed cells were subsequently diluted and plated so as to obtain single colonies or clones. The clones were checked to ensure that clone redundancy for the library did not exceed 5%. Single clones were picked in liquid LB (Luria-broth) media, in 96-deep-well plates, and grown overnight at 37°C. The plates also included positive (Lh423) and negative (FP) control clones.

To generate the dsRNA, sense and antisense DNA fragments, containing T7 promoter sequence, were generated by PCR. In brief, per clone, 1µl of bacterial suspension was dispensed in multiwell PCR plates containing REDTaq® (Sigma Cat No D4309) and primers oGCC2738 (SEQ ID NO 93: AAGCAGTGGTATCAACGCAG) and oGCC2739 (SEQ ID NO 94: GCGTAATACGACTCACTATAGGAAGCAGTGGTATCAACGCAG) based on the M2 and the T7-M2 sequences respectively. The PCR reaction was followed by *in vitro* transcription, where per clone, 6µl PCR product were added to 9µl RiboMAX™ Large Scale RNA Production System-T7 (Promega Cat No P1300) and incubated overnight at 37°C. The final dsRNA solution was diluted 2 times in *L. hesperus* sucrose diet, containing 15 % sucrose and 5µg/µl yeast tRNA (Invitrogen Cat No 15401-029) and used for screening. The dsRNA corresponding to the positive Lh423 control clone is SEQ ID NO 101 and to the negative FP control clone is SEQ ID NO 104 (see Table 4).

### 1.2. Screen for novel and potent Lygus hesperus target genes using a dsRNA expression cDNA library

A new screening assay for potent *Lygus hesperus* targets has been developed. The assay set-up was as follows: each well of a 96-well plate houses a one-day-old *L. hesperus* nymph exposed to a parafilm sachet containing sucrose diet which includes either test dsRNA or control dsRNA in the presence of tRNA. Each plate contained dsRNA from 90 different clones, 3 x Lh423 (positive control) and 3 x FP (fluorescent protein; negative control). Each clone (test dsRNA) was replicated over three plates. After three days exposure, the nymphal survival number was recorded and the diet replaced with fresh rearing (complex) diet in absence of dsRNA. The mortality was assessed at days 4, 6 and 8. An identical set up was used for the first and second round confirmation assays, with 8 and 20 insects respectively, with one nymph per well.

The assay system was validated using dsRNA corresponding to Lh423 target as the positive control and a fluorescent protein dsRNA as the negative control: over 90% were true positives and under 5% were false positives, respectively.

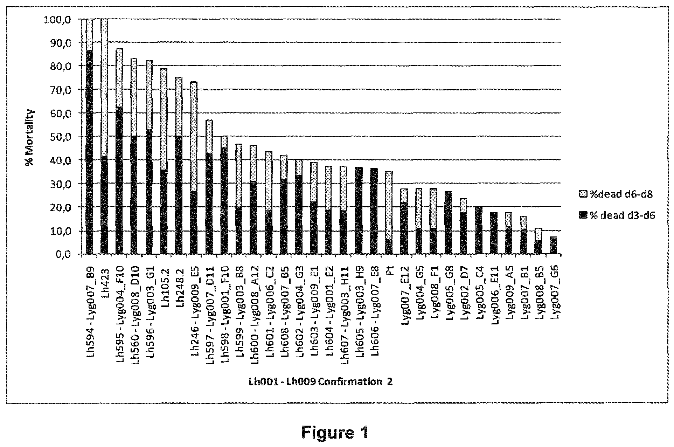

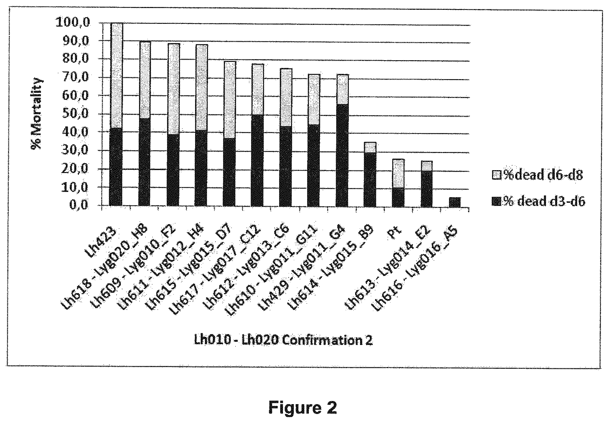

Twenty 96 well-plates, named Lh001 to Lh020 (see bottom line in Figures 1 & 2), containing 1800 individual clones have been tested. 205 candidates were identified and tested in a first confirmation assay. Setting the threshold at showing ≥ 50% mortality, 41 independent clones were identified and progressed to a second round of confirmation. In the assay, the clones were compared to the positive controls Lh423 (RpL19) and Lh105.2 (Sec23) and the negative control Pt (encoding a coral fluorescent protein). The dsRNA corresponding to the positive (Lh423) control clone is SEQ ID NO 101, to the positive Lh105.2 control clone is SEQ ID NO 102 and to the negative (Pt) control clone is SEQ ID NO 104 (see Table 4).

Second round confirmation assays, testing 20 insects / test dsRNA, were initiated for all the test dsRNAs displaying ≥ 50% mortality in the first confirmation (Figures 1 and 2). Candidate targets corresponding to the confirmed test dsRNAs were named with an "Lhxxx number" (see Table 1). Using the same cut-off at ≥ 50% mortality, 15 targets were confirmed in the first screen.

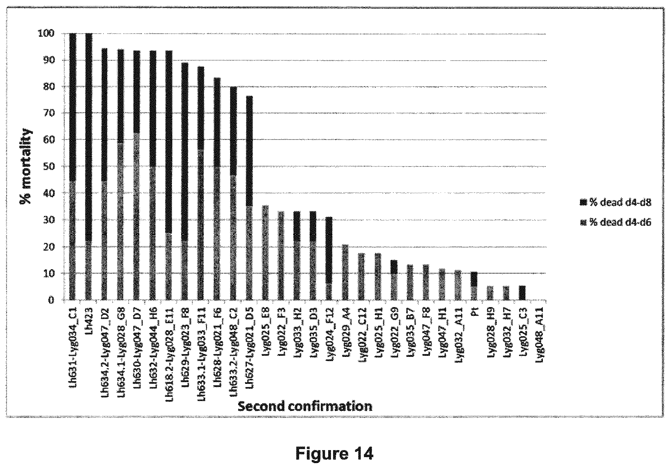

A second screen for identifying more *Lygus hesperus* targets was performed. The results of the second round confirmation assays are represented in Figure 14. Using the same cut-off at ≥ 50% mortality, several targets were confirmed in the second screen (see Table 1 C).

### 1.3. Identification of Lygus targets

In parallel to the confirmation insect assays, the inserts corresponding to the positive clones were sequenced and BlastX searches against both *Drosophila* and *Tribolium* protein databases were used to confirm the identity of the targets. Table 1 provides a summary of the bio-informatics analysis and current annotation of the novel identified *L. hesperus* target sequences.

Fifteen novel *L. hesperus* targets were identified in the first screen and 11 novel *L. Hesperus* targets were identified in the second screen. All targets exhibit high potency against *L. hesperus* nymphs indicating that the cDNAs encoding double-stranded RNAs contained therein are essential for pest survival and thus represent target genes of interest for the purposes of pest control. The DNA sequences and deduced amino acid sequences of these target genes were therefore determined and are provided in Tables 2 and 3 respectively.

Lh594, the *Lygus hesperus* orthologue of *Drosophila* troponin I, involved in muscle contraction - and therefore absent in plants-, represents a novel class of target belonging to an animal specific physiological pathway not yet explored for GM-RNAi. In the fruit fly, troponin I is described as a haplo-insufficient gene, displaying a mutant phenotype in the heterozygote state. Such genes may be particularly susceptible to reduced mRNA expression levels and as such can be considered as ideal RNAi targets.

In this Lh594 pathway, eight targets were selected (see table 1B). For each target, up to 4 pairs of degenerated PCR primers were designed based on the alignments of the sequences of various insects, including bee, *Tribolium* and aphid. The primers are being used to amplify fragments from *Lygus hesperus* targets. The DNA sequences and deduced amino acid sequences of these target genes were determined and are provided in Tables 2 and 3 respectively.

**Table 1: Lygus hesperus novel targets ranked in % mortality according to the second confirmation assay results (first screen).**

| **Target ID** | **rank 2nd confirmati on** | **Best Drosophila hit** | **NAME** | **SYMBOL** |
|---|---|---|---|---|
| Lh594 | 1 | CG7178 | wings up A (troponin I) | wupA |
| Lh618 | 2 | CG2168 | ribosomal protein S3A | RpS3A |
| Lh649 | 3 | CG4087 | ribosomal protein LP1 | RpLP1 |
| Lh595 | 4 | - | no *Drosophila* hit found, *Lygus* specific target/sequence | |
| Lh611 | 5 | CG6779 | ribosomal protein S3 | RpS3 |
| Lh560 | 6 | CG 10423 | ribosomal protein S27 | RpS27 |
| Lh596 | 7 | - | no *Drosophila* hit found, *Lygus* specific target/sequence | RpL34b |
| Lh615 | 8 | CG11522 | ribosomal protein L6 | RpL6 |
| Lh617 | 9 | CG7283 | ribosomal protein L10Ab | RpL10Ab |
| Lh612 | 10 | CG 13389 | ribosomal protein S13 | RpS13 |
| Lh246 | 11 | CG3195 | ribosomal protein L12 | RpL12 |
| Lh429 | 12 | CG8900 | ribosomal protein S18 | RpS18 |
| Lh610 | 13 | CG5502 | ribosomal protein L4 | RpL4 |
| Lh597 | 14 | no hit found | | |
| Lh598 | 15 | CG34069 | mitochondrial cytochrome c oxidase subunit II | mt:Coll |
| Lh614 | - | CG7610 | ATP synthase-γ chain | ATPsyn-γ |

**Table 1B: Lygus hesperus novel targets in Lh594 pathway**

| **Target ID** | **Best Drosophila hit(s)** | **NAME** | **SYMBOL** |
|---|---|---|---|
| Lh619 | CG7107 | troponin T (upheld) | up |
| Lh620 | CG 17927 | myosin heavy chain | Mhc |
| Lh621 | CG4843 | tropomyosin2 (Tm2) | Tm2 |
| Lh622 | CG3201 | myosin light chain cytoplasmic | Mlc-c |
| Lh623 | CG3595 | spaghetti squash | sqh |
| Lh624 | CG 15792 | zipper | zip |
| Lh625 | *CG2981,CG7930,CG9 073,CG6514,CG12408 | troponin C | |
| Lh626 | *CG9073,CG7930,CG2 981,CG12408,CG6514 | troponin C | |

| | | | |
|---|---|---|---|
| *unclear : multiple hits in family - ranked according e-value | | | |

**Table 1C: Lygus hesperus novel targets ranked in % mortality according to the second confirmation assay results (second screen).**

| **Target ID** | **rank 2nd confirmation** | **Best Drosophila hit** | **NAME** | **SYMBOL** |
|---|---|---|---|---|
| **Lh631** | 1 | CG6846 | Ribosomal protein L26 | RpL26 |
| **Lh634.2** | 2 | CG 12775 | Ribosomal protein L21 | RpL21 |
| **Lh634.1** | 3 | CG 12775 | Ribosomal protein L21 | RpL21 |
| **Lh630** | 4 | CG11271 | Ribosomal protein S12 | RpS12 |
| **Lh632** | 5 | CG2998 | Ribosomal protein S28b | RpS28b |
| **Lh618.2** | 6 | CG2168 | Ribosomal protein S3A | RpS3A |
| **Lh629** | 7 | CG4651 | Ribosomal protein L13 | RpL13 |
| **Lh633.2** | 8 | CG17521 | Ribosomal protein L10 | RpL10 |
| **Lh628** | 9 | CG 17489 | Ribosomal protein L5 | RpL5 |
| **Lh633** | 10 | CG17521 | Ribosomal protein L10 | RpL10 |
| **Lh627** | 11 | CG2033 | Ribosomal protein S15Aa | RpS15A |

### 1.4. Full length cDNA cloning by RACE (rapid amplification of cDNA ends)

In order to clone full length cDNA, starting from a known clone of internal fragment from the most potent targets, the 5'/3' RACE kit was used (Roche, Cat. No. 1 734 792; based on Sambrook, J. & Russell, D.M). The standard protocol, described in the Instruction Manual, was followed. Briefly, for a 5' RACE, a target sequence specific antisense primer was designed on the known sequence and used for a first strand cDNA synthesis, using *Lygus* RNA as template. A tail was added to the first strand cDNA and used as an anchor for the second strand synthesis and amplification of an unknown end portion of the transcript. For a 3' RACE, an oligo dT anchor primer was used for the first strand cDNA synthesis. For the 5' and 3' RACEs, nested primers, specific to the target sequence were used in a second PCR reaction. The PCR fragments were analysed on agarose gel, purified, cloned and sequenced for confirmation.

Full length cDNA sequences corresponding to the targets were assembled in VectorNTi, a fully integrated sequence analysis software package for DNA sequence analysis (Invitrogen).

### Example 2 In vitro production of double-stranded RNAs for gene silencing

### 2.2. Production of dsRNAs corresponding to the partial sequences of the Lygus hesperus target genes

Double-stranded RNA was synthesized in milligram quantities. First, two separate 5' T7 RNA polymerase promoter templates (a sense template and an antisense template) were generated by PCR. PCRs were designed and carried out so as to produce sense and antisense template polynucleotides, each having the T7 promoter in a different orientation relative to the target sequence to be transcribed.

For each of the target genes, the sense template was generated using a target-specific T7 forward primer and a target-specific reverse primer. The antisense templates were generated using target-specific forward primers and target-specific T7 reverse primers. The sequences of the respective primers for amplifying the sense and antisense templates via PCR for each of the target genes are provided in Table 4. The PCR products were analysed by agarose gel electrophoresis and purified. The resultant T7 sense and antisense templates were mixed and transcribed by the addition of T7 RNA polymerase. The single-stranded RNAs produced by transcription from the templates were allowed to anneal, were treated with DNase and RNase, and were purified by precipitation. The sense strand of the resulting dsRNA produced from each of the target genes is provided in Table 4.

### 2.2. Survival analysis assays for novel Lygus hesperus targets

To enable ranking according to potency, in vitro dsRNAs corresponding to the novel targets were synthesized and applied to *L. hesperus* in 10 days survival analysis bioassays. Briefly, one day old *L. hesperus* nymphs were placed in 96 well-plates with sucrose seals containing 0.5µg/µl target dsRNA, supplemented with 5µg/µl yeast tRNA. The plates were incubated for 3 days under standard *Lygus* rearing conditions. At day 3, 6 and 8, the diet seals were refreshed with seals containing *Lygus* diet only. Lh423 (RpL19) was used as positive control and GFP dsRNA and sucrose diet were used as negative controls.

The results from the survival analyses confirmed the data from the first and second confirmation assays. Lh594 was established as a highly potent target, with activity and speed-to-kill stronger than the strong control Lh423.

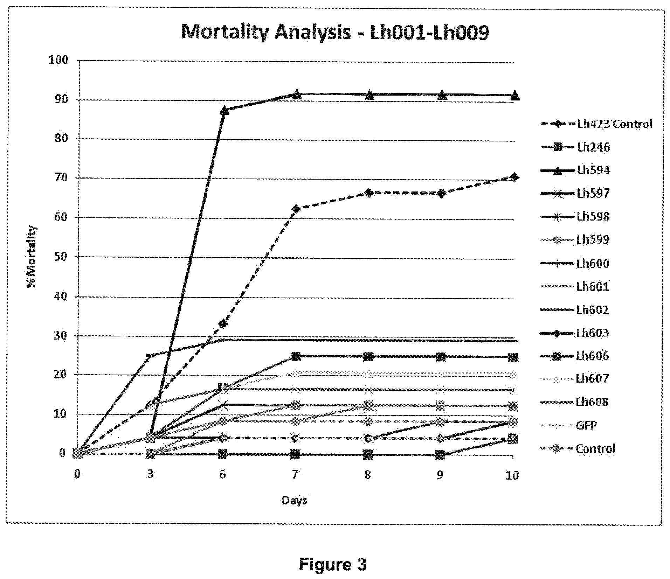

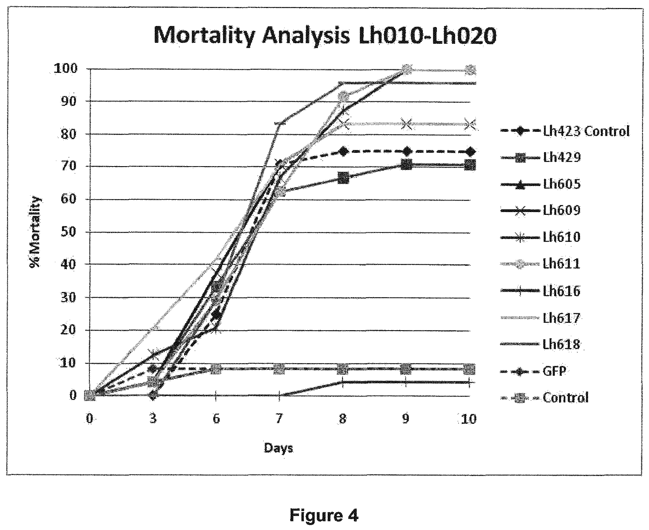

So far, the *Lygus* screen for novel targets identified new targets with activities higher or in the range of the positive control Lh423, these include Lh429, Lh594, Lh609, Lh610, Lh611, Lh617 and Lh618. The mortality induced by these targets is show in the **Figures 3** **and** **4****.**

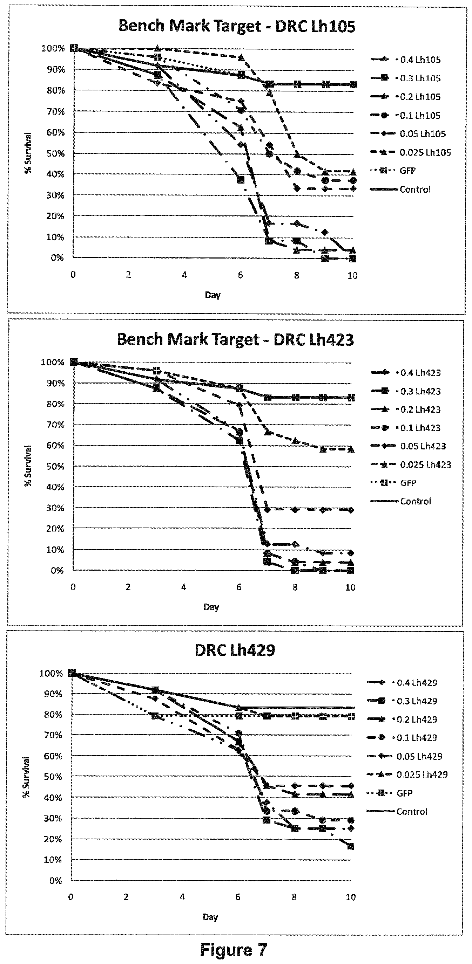

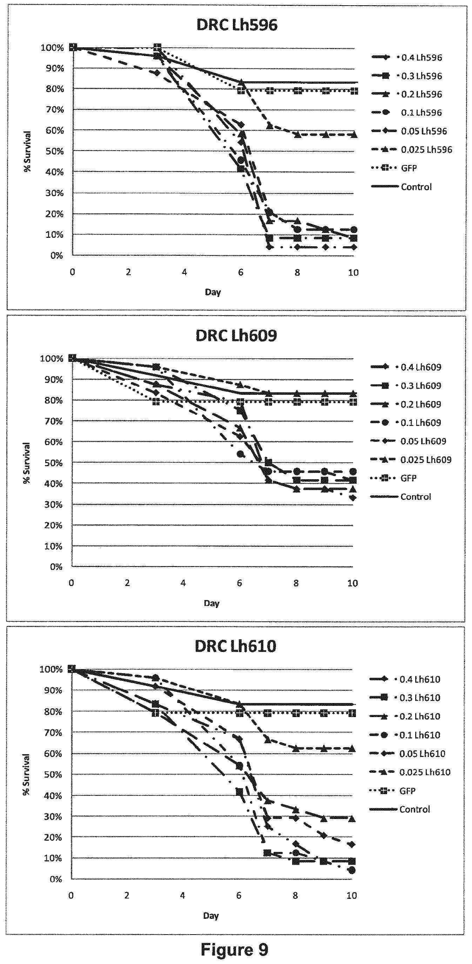

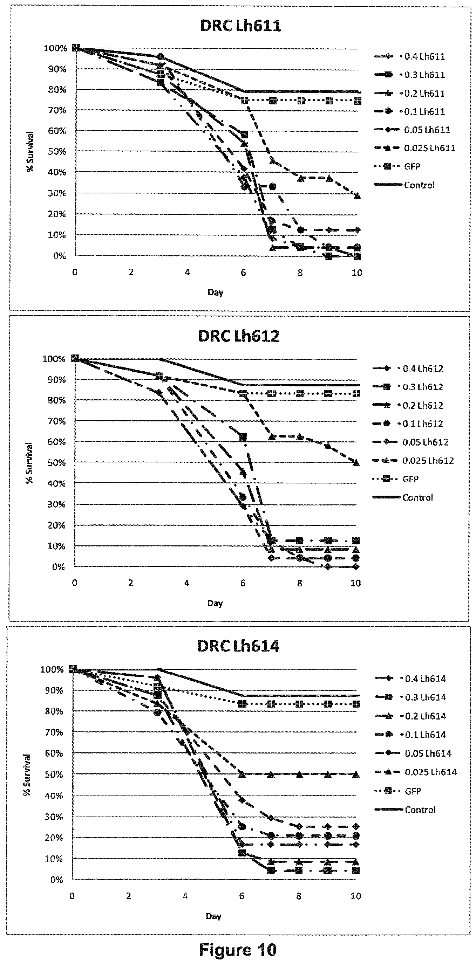

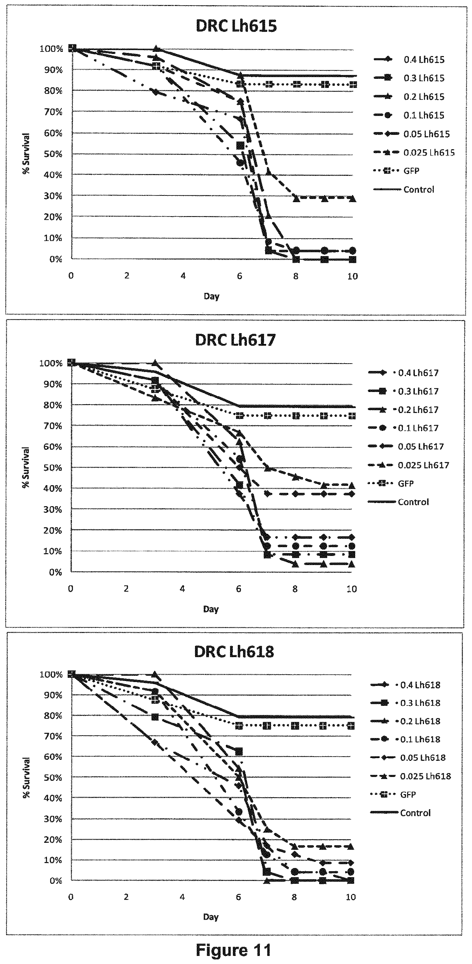

To allow a more precise ranking of the targets according to their activity, dose response concentration analyses were made. The novel targets were tested in *in vitro* assays, with concentrations ranging from 0.4 to 0.025 µg/µl. Per condition, 24 one day old nymphs were tested in the 96 well-plate set-up, in sucrose diet supplemented with dsRNA and tRNA carrier. The results are presented as % survival over a 10 day experiment **(****Figures 7 to 11****)** and are summarized in **Table 5.**

Based on the concentration curve analyses, the targets were ranked by comparison to the bench mark controls Lh423 and Lh105 **(Table 5)**.

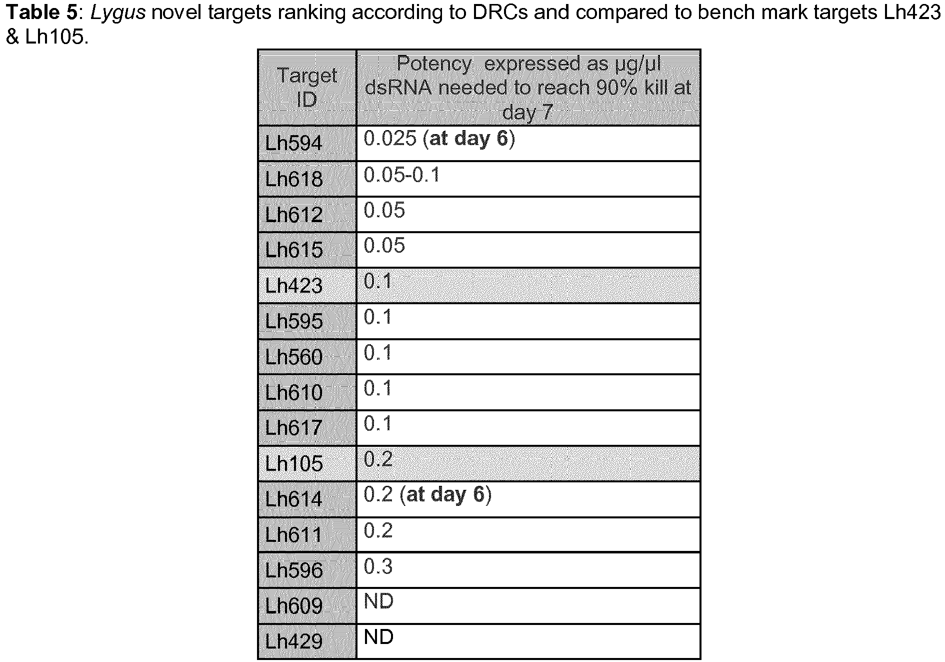

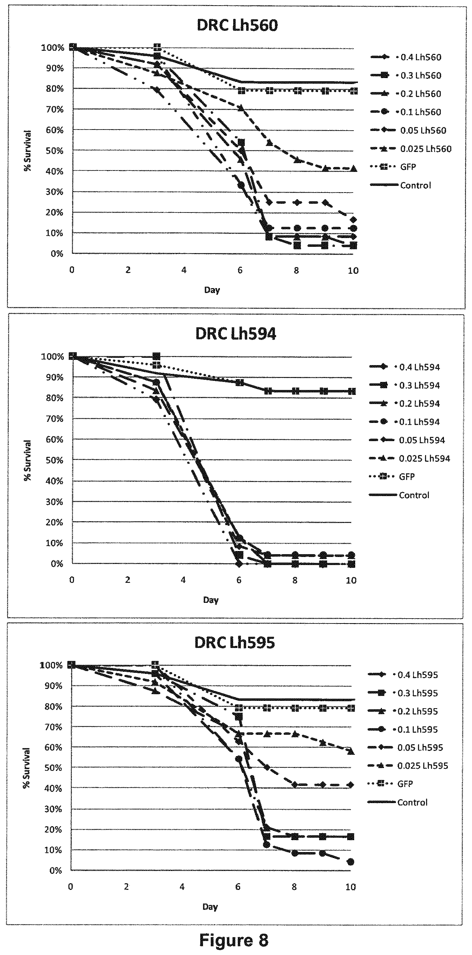

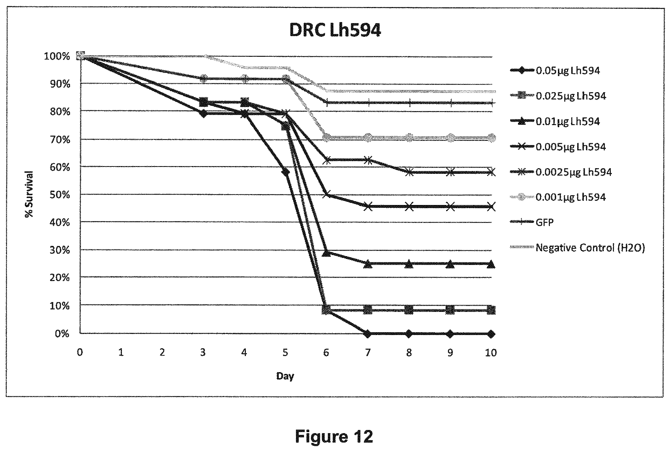

The potency of Lh594 was further confirmed. This target effect is clearly observed at least one day before the other targets and the bench mark positive control Lh105 and Lh423. Because Lh594 was highly potent, the LD50 was not reached in the standard DRC experiment, with concentration ranging from 0.4 to 0.025µg/µl dsRNA **(****Figure 8****),** the Lh594 experiment was therefore repeated, including lower concentrations ranging from 0.05 to 0.001 µg/µl dsRNA **(****Figure 12****).** In conclusion, Lh594 activity was observed at concentration as low as 0.0025 µg/µl and about 90% kill (corresponding to about 10 % survival) was obtained at day 6 with 0.025 µg dsRNA.

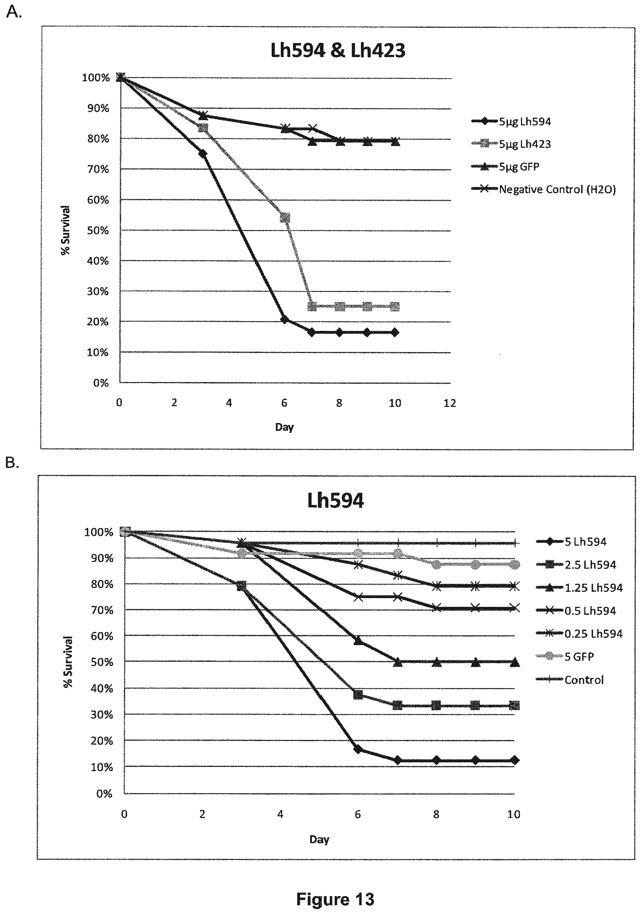

To further explore the potency of Lh594 and the role of tRNA carrier in the RNAi response in *Lygus hesperus,* additional *in vitro* feeding assays were set up in the absence of carrier tRNA. Lh594, Lh423 (bench mark control) and GFP (negative control) dsRNAs were produced *in vitro,* using the standard method. The dsRNAs were purified and tested at 5µg/µl in the absence of tRNA **(****Figure 13 A)****.**

In absence of tRNA, targets Lh594 and Lh423, induced high lethality in *Lygus* nymphs. The results from this experiment have been since reproduced. Target dsRNA was able to induce RNAi-by-feeding effects in *Lygus* nymphs in the absence of tRNA.

To investigate the activity of dsRNA at lower concentrations in the absence of carrier tRNA, additional experiments were set up, using decreasing amounts of dsRNA **(****Figure 13 B)****.**

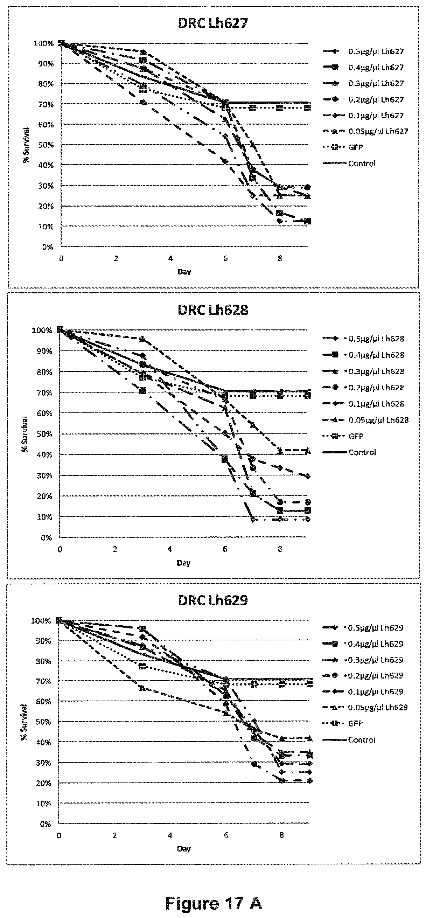

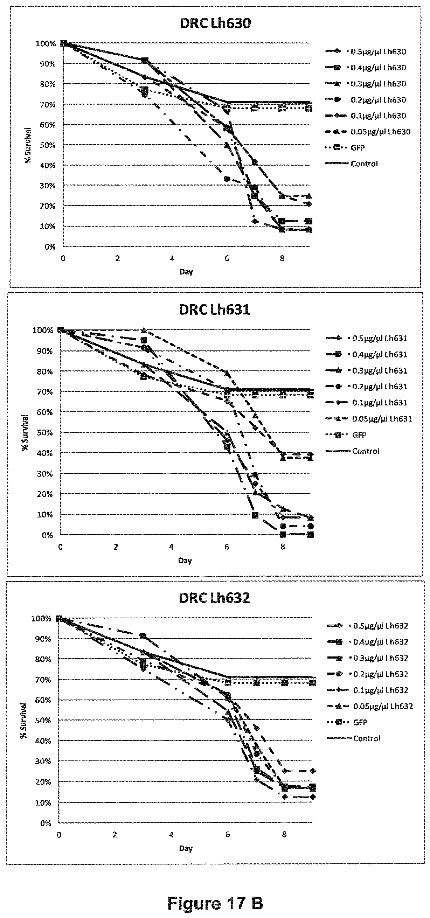

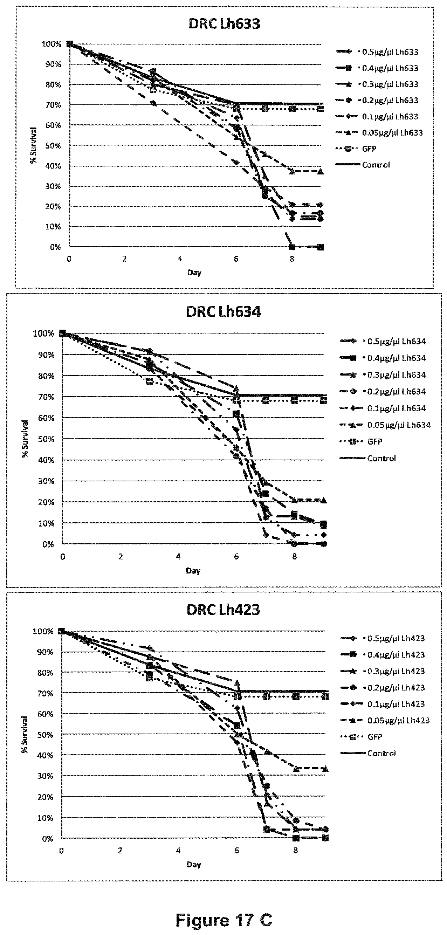

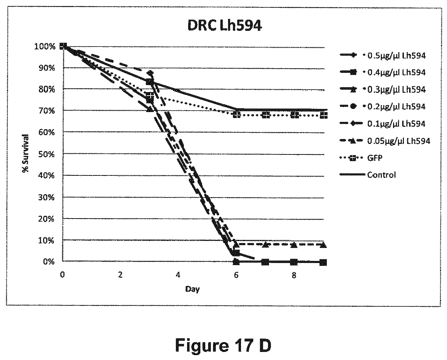

A similar approach was followed for the *lygus* targets that were identified in the second screen. To allow a ranking of the targets according to their activity, dose response concentration analyses were made. The novel targets were tested in *in vitro* assays, with concentrations ranging from 0.5 to 0.05 µg/µl. Per condition, 24 one day old nymphs were tested in the 96 well-plate set-up, in sucrose diet supplemented with dsRNA and tRNA carrier. The results are presented as % survival over a 9 day experiment **(****Figures 17A-D).** Lh594 and Lh423 have been included in the assay as a reference targets. The results are summarized in **Table 6.** Based on the concentration curve analyses, the targets were ranked by comparison to the bench mark control Lh423.

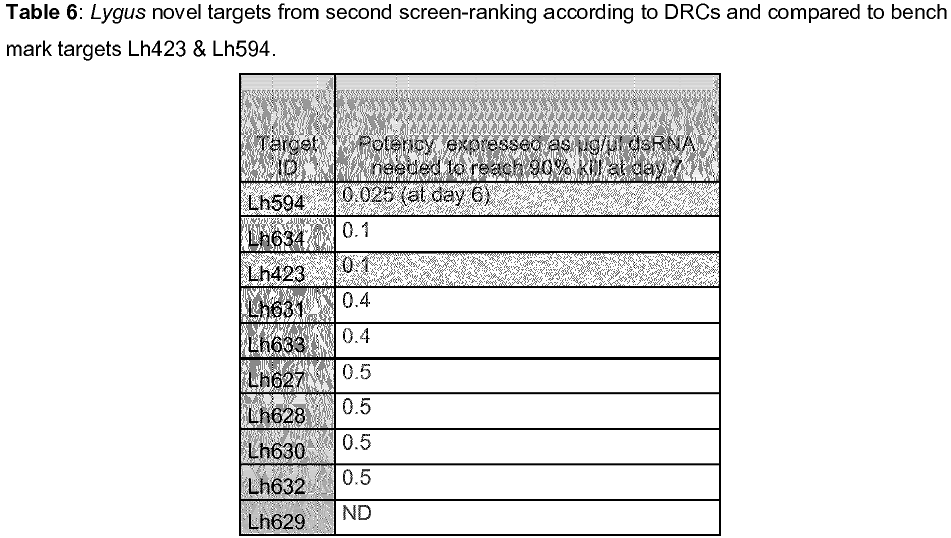

### Example 3 Troponin pathway screen

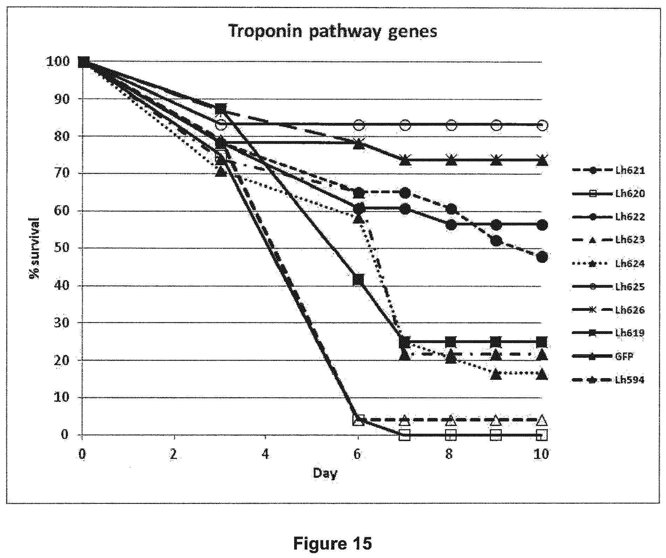

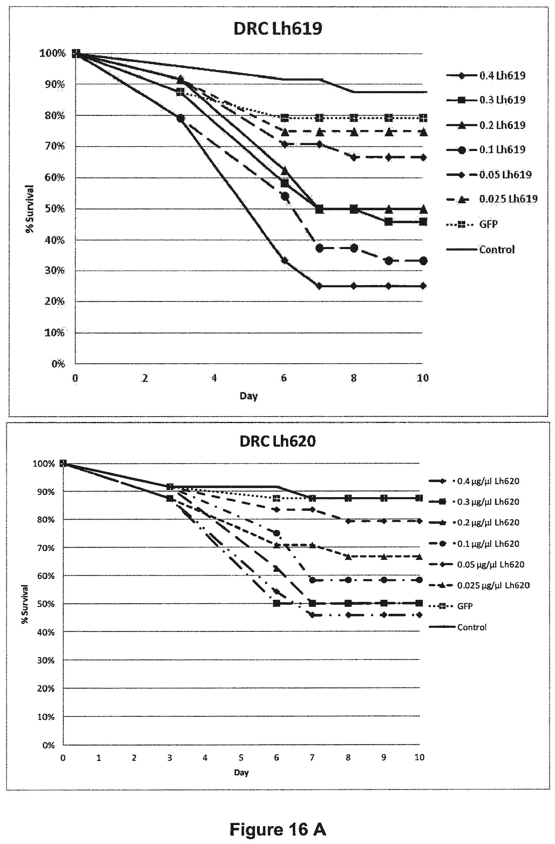

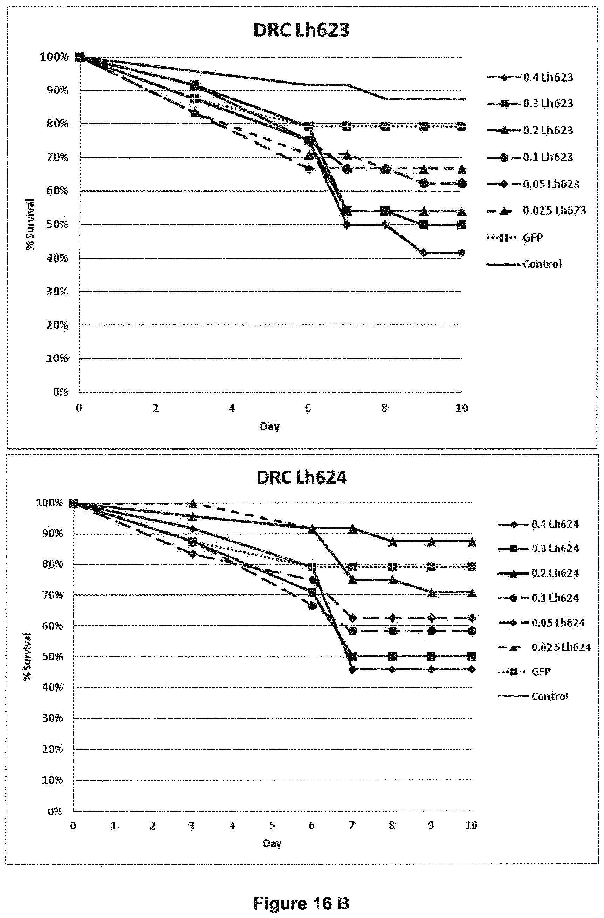

To enable testing of the Troponin pathway targets, in vitro produced dsRNAs corresponding to Lh619, Lh620, Lh621, Lh622, Lh622, Lh623, Lh624, Lh625 and Lh626 were synthesized and applied to *L. hesperus* in 10 days survival analysis bioassays. Briefly, one day old *L. hesperus* nymphs were placed in 96 well-plates with sucrose seals containing 0.5µg/µl target dsRNA, supplemented with 5 µg/µ1 yeast tRNA. The plates were incubated for 3 days under standard *Lygus* rearing conditions. At day 3, 6 and 8, the diet seals were refreshed with seals containing *Lygus* diet only. Lh594 (Troponin I) was used as positive control and GFP dsRNA and sucrose diet were used as negative controls **(****Figure 15****).** Four targets were then included in dose response curve analyses in an *in vitro* assay, with concentrations ranging from 0.4 to 0.025 µg/µl. Per condition, 24 one day old nymphs were tested in the 96 well-plate set-up, in sucrose diet supplemented with dsRNA and tRNA carrier. The results are presented as % survival over a 10 day experiment **(****Figures 16A-B).**

### Example 4 Generation of plants resistant to insect pest species

### 4.1. Assembly of plant expression vectors comprising a Lygus hesperus hairpin sequence for transformation of potato or cotton

Since the mechanism of RNA interference operates through dsRNA fragments, the target polynucleotide sequences were cloned in anti-sense and sense orientation, separated by a spacer
(SEQ ID NO 98: CTCGAGCCTGAGAGAAAAGCATGAAGTATACCCATAACTAACCCATTAGTTATGCATTTATGTTAT ATCTATTCATGCTTCTACTTTAGATAATCAATCACCAAACAATGAGAATCTCAACGGTCGCAATAA TGTTCATGAAAATGTAGTGTGTACACTTACCTTCTAGA, or
SEQ ID NO 385: TCTAGAAGGTAAGTGTACACACTACATTTTCATGAACATTATTGCGACCGTTGAGATTCTCATTGT TTGGTGATTGATTATCTAAAGTAGAAGCATGAATAGATATAACATAAACTAGTAACTAATGGGTTA GTTATGGGTATACTTCATGCTTTTCTCTCAGGCTCGAG), to form a dsRNA hairpin construct. The dsRNA hairpin constructs encoding the *L. hesperus* dsRNA molecules derived from the target genes as mentioned herein were subcloned into a plant expression vector. Similarly a GUS dsRNA hairpin control construct, wherein the sense polynucleotide sequence encoding GUS (SEQ ID NO 97 : CCAGCGTATCGTGCTGCGTTTCGATGCGGTCACTCATTACGGCAAAGTGTGATGGAGCATCAGG GCGGCTATACGCCATTTGAAGCCGATGTCACGCCGTATGTTATTGCCGGGAAAAGTGTACGTAT CTGAAATCAAAAAACTCGACGGCCTGTGGGCATTCAGTCTGGATCGCGAAAACTGTGGAATTGAT CCAGCGCCGTCGTCGGTGAACAGGTATGGAATTTCGCCGATTTTGCGACCTCGCAAGGCATATT CGGGTGAAGGTTATCTCTATGAACTGTGCGTCACAGCCAAAAGCCAGACAGAGT) was cloned in anti-sense and sense orientation, separated by the same intron (SEQ ID NO 98 or SEQ ID NO 385), was subcloned into a plant expression vector.

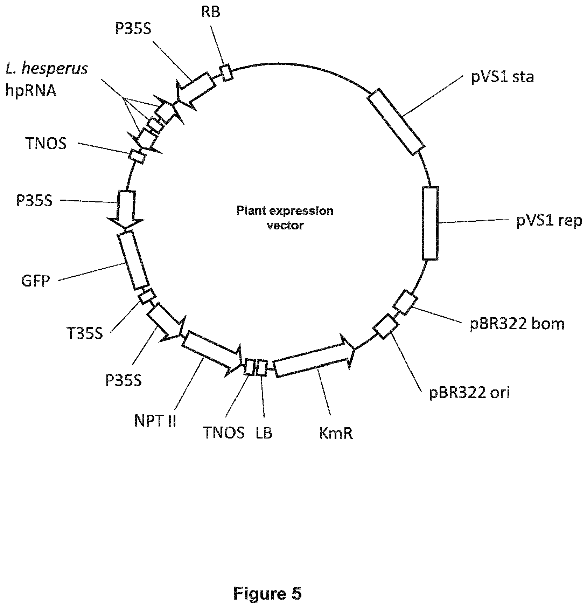

The plant expression vector comprises as well elements necessary for the maintenance of the plasmid in a bacterial cell. The dsRNA hairpin construct is located between the left border (LB) and right border (RB), 3' downstream from the Cauliflower Mosaic Virus 35S promoter (P35S) and 5' upstream from the TNOS terminator. A GFP reporter expression cassette comprising the GFP sequence flanked by the P35S promoter and terminator was subcloned into the plant transformation vector harbouring the *L. hesperus* hairpin cassette. The NPT II expression cassette comprising the NPT II sequence flanked by the P35S promoter and terminator is used for selecting plants that have been effectively transformed. Correct assembly of the genetic fragments in the plant expression vector was confirmed by sequencing (Figure 5).

The plant expression vectors comprising the individual *L. hesperus* target hairpins were subsequently transformed into *Agrobacterium tumefaciens.* For all *L. hesperus* target genes mentioned herein, fragments can be selected and cloned as hairpins in a similar manner.

### 4.2. Transformation of potato with a plant expression vector comprising a Lygus hesperus hairpin sequence and testing of the transformed potato plants for resistance towards L. hesperus

The example provided below is an exemplification of the finding that transgenic potato plants expressing target gene-specific hairpin RNAs adversely affect survival and/or development of insect pest species.

### Lygus hesperus RNAi-by-feeding in planta

Following the positive results obtained in the dsRNA feeding experiments in *L. hesperus,* proof-of-principle *in planta* experiments were performed.

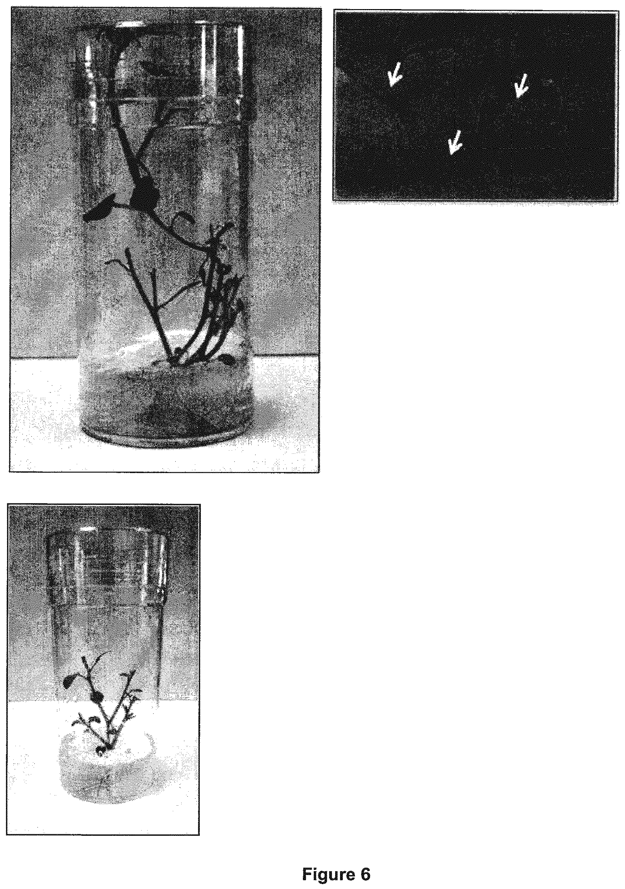

The *in planta* assay was developed with *in vitro* potato plantlets which can sustain insect survival at least 8 days, keeping background mortality low. *L. hesperus* nymphs survive and feed on wild type potato plantlets. This is supported by the visual damage caused by insects which can be observed on the leaves and buds (Figure 6).

In the assay, *L. hesperus* is fed with transgenic potato, expressing hairpin dsRNA targeting the *L. hesperus* targets identified herein. Plasmids carrying hairpin constructs and a GUS control were generated.

The plantlets were analysed by PCR to confirm the integration of the T-DNA and the presence of the hairpin, before being propagated. Excess explants were produced with the aim of obtaining at least 30 independent events for each construct.

### Potato transformation

Stably transformed potato plants were obtained using an adapted protocol received through Julie Gilbert at the NSF Potato Genome Project (http://www.potatogenome.org/nsf5). Stem internode explants of potato 'Line V' (originally obtained from the Laboratory of Plant Breeding at PRI Wageningen, the Netherlands) which is derived from the susceptible diploid *Solanum tuberosum* 6487-9 were used as starting material for transformation. *In vitro*-derived explants were inoculated with *Agrobacterium tumefaciens* C58C₁Rif^{R} containing the hairpin constructs. After three days co-cultivation, the explants were put onto a selective medium containing 100 mg/L Kanamycin and 300 mg/L Timentin. After 6 weeks post-transformation, the first putative shoots were removed and rooted on selective medium. Shoots originating from different explants were treated as independent events, shoots originating from the same callus were termed 'siblings' until their clonal status can be verified by Southern blotting, and nodal cuttings of a shoot were referred to as 'clones'.

The transgenic status of the rooting shoots was checked either by GFP fluorescence or by plus/minus PCR for the inserted target sequence. Positive shoots were then clonally propagated in tissue culture to ensure enough replicates were available for the *Lygus hesperus* assays. These shoots were either kept in tissue culture medium or transferred to soil allowing for greater flexibility to test for resistance towards *L. hesperus* nymphs/adults. The first plants were available to test fourteen weeks post transformation.

### Bioassay

Young transgenic potato plants were either kept in tissue culture medium or were grown in soil in a plant growth room chamber with the following conditions: 25 ± 1°C, 50 ± 5% relative humidity, 16:8 hour light:dark photoperiod. Per construct, a number of events (for example, twenty) were generated with a suitable number of clones (for example, ten) per event. A number of young *Lygus* nymphs/adults were placed on the individually caged young (for example, at the 4 - 5 unfolded leaf stage) potato plants and left for at least seven days before assessing resistance towards *Lygus hesperus* in terms of reduced nymph/larva/adult survival, delayed development and stunted growth, and/or decreased plant feeding damage.

The feasibility of *in planta* RNAi for crop protection against *Lygus* was tested in an assay using transgenic potatoes expressing hairpins corresponding to *Lygus* target genes. **Table 7** summarizes the numbers of transgenic potatoes generated and tested. Transgenic events were generated with hairpins corresponding to *Lygus* targets Lh423 (the hairpin sequence for Lh423 is represented in SEQ ID NO 402; the sense sequence of target Lh423 is represented in SEQ ID NO 101) and Lh594 (the hairpin sequence for Lh594 is represented in SEQ ID NO 401; the sense sequence of target Lh594 is represented in SEQ ID NO 2), and GUS as control (the hairpin sequence for GUS is represented in SEQ ID NO 403; the sense sequence of GUS is represented in SEQ ID NO 97). In this assay SEQ ID NO 385 was used as intron.

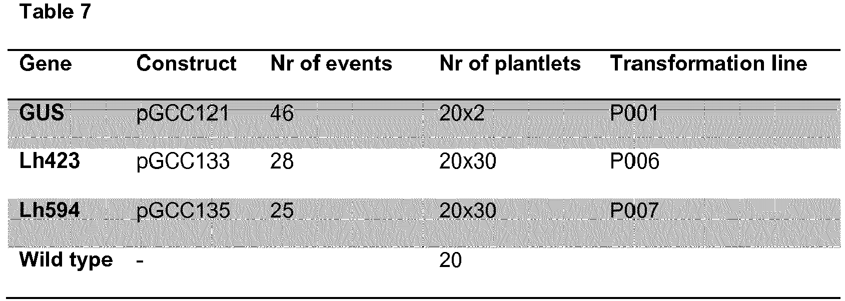

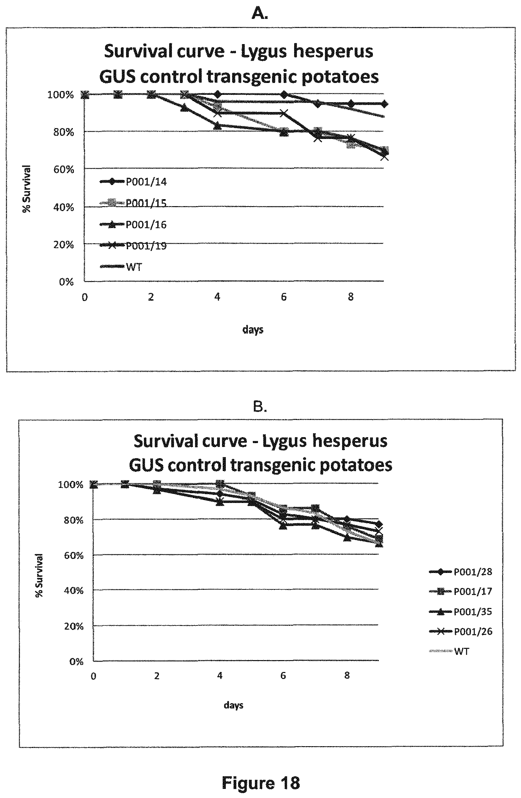

The plantlets were propagated first in boxes then in individual pots, containing MS rooting medium (4.4 g/L MS salts & vitamins, 30 g/L sucrose, 10 g/L Gelrite, pH 5.7), in preparation for the *Lygus* feeding assays. Two independent GUS events were selected from 8 independent events tested in 2 independent experiments **(****Figure 18A-B).** In the assay, 20-30 transgenic plants of the same event, each planted in a separate pot, were tested and compared to WT plantlets. For the transgenic lines carrying the Lh423 and Lh594 hairpins, 28 and 25 independent events were tested respectively and for each independent transgenic event 20 to 30 plantlets, each planted in a separate pot were tested **(****Figure 6****).**

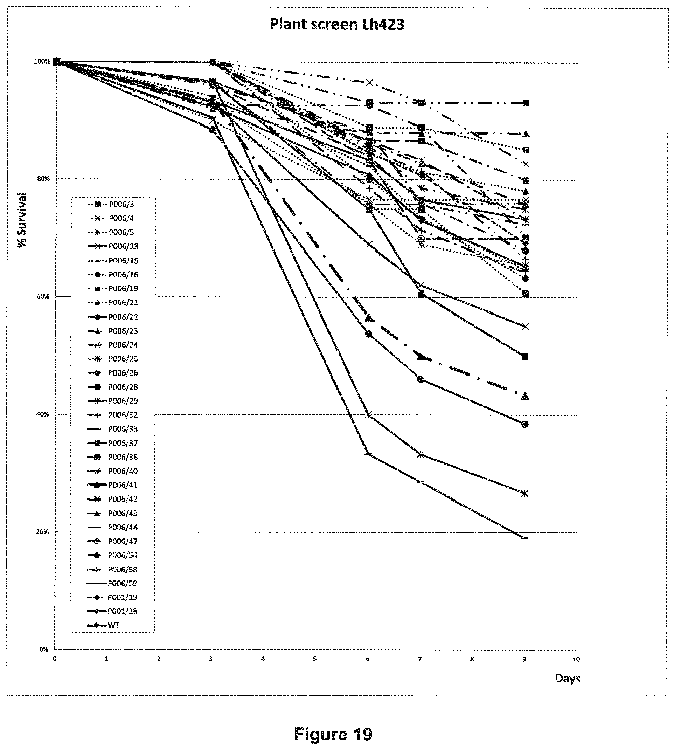

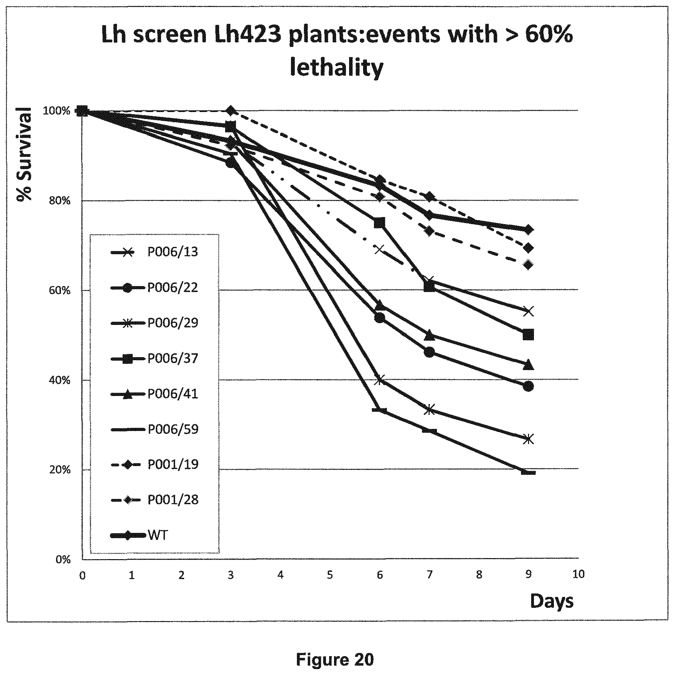

As expected in primary transformants, a range of activity was observed for the 28 independent Lh423 transgenic events **(****Figure 19****);** 6 independent P006 events induced above 60% lethality at day 9 and in one event, lethality reached 80% at day 9 **(****Figure 20****).**

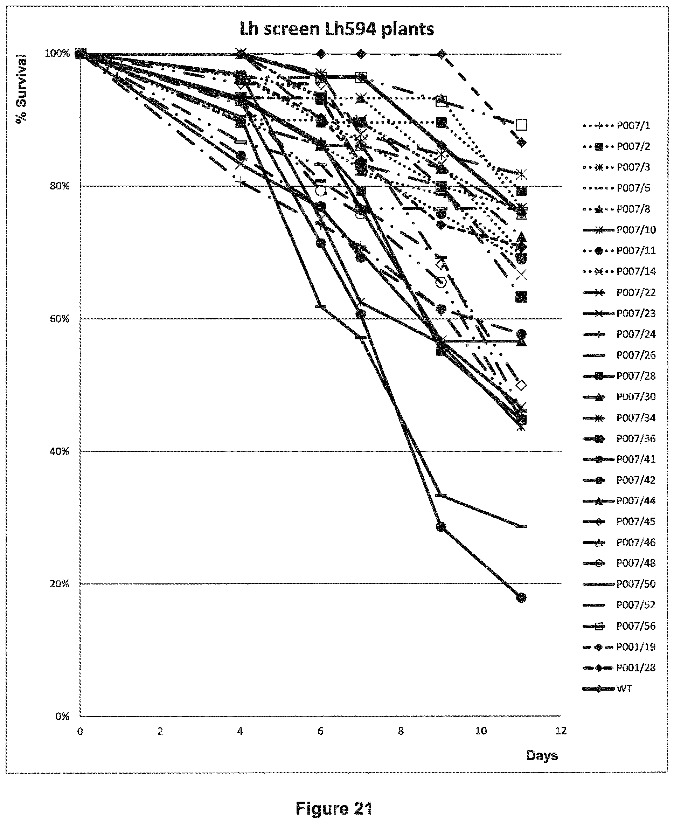

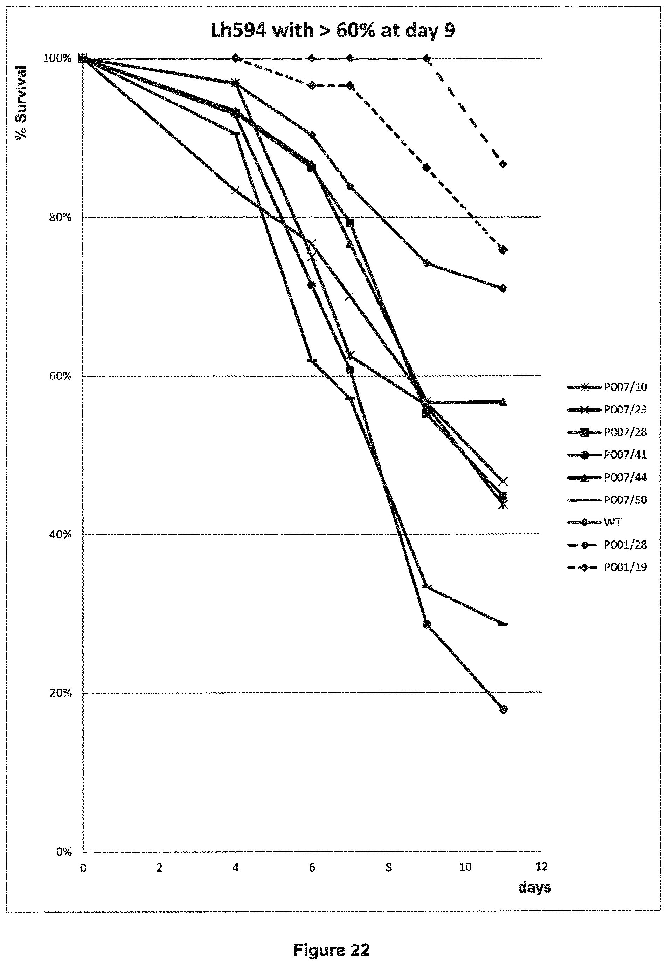

As expected in primary transformants and as seen for the Lh423 primary transformants, a range of activity was also observed for the 25 independent Lh594 transgenic events **(****Figure 21****);** 6 independent P007 events induced above 60% lethality at day 9 and in one event, lethality reached 80% at day 9 **(****Figure 22****).** In addition, growth delays and stunting were clearly observed in the survivor insects.

### Results from qRT-PCR on plants fed Lygus

To prove that the observed decrease in survival of *Lygus* feeding on transgenic potato plantlets expressing hairpins directed against endogenous genes was a true RNAi response, the level of down-regulation of the target mRNA (Lh423) was measured by quantitative real time PCR (qRT-PCR). Insects were allowed to feed on 3 events carrying the Lh423 hairpin (P006/59, /22 and /29) and on one event carrying GUS hairpin control (P001/28) as control. The insects were collected after 5 days and were immediately frozen in liquid nitrogen. Total RNA was extracted from 5 pools of 5 insects using TRI reagent and according to the manufacturer's instructions (SIGMA). After treatment with DNasel (Promega) to remove the genomic DNA followed by phenol-chloroform extraction and ethanol precipitation, precipitated RNAs were dissolved in water. For each sample, 1 µg RNA was reverse transcribed with a mix of random hexamers and anchored oligo dT primers. qRT-PCR PCR was performed on the BioRad I-Cycler, using iQ SYBR Green Supermix (Biorad) and using the manufacture's recommended conditions. The qRT-PCR primers **(Table 8)** were designed using BeaconDesign; to avoid PCR artifacts foreseeable in presence of the plant expressed dsRNA ingested by the insects, the primer sequences were located 3' of the dsRNA sequence. The GeNorm algorithm was used to normalize the level of target mRNA using 2 house-keeping genes, Lh425 (SDHAand) and Lh427 (rpl 11).

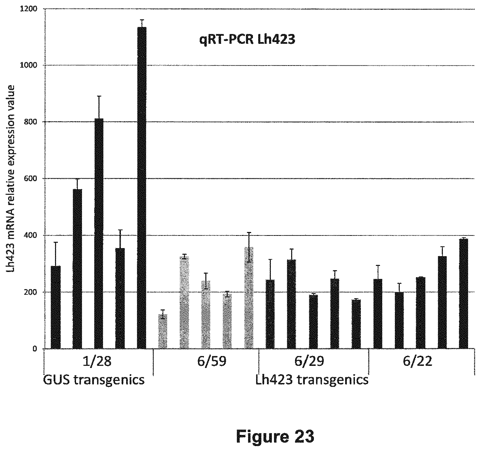

In the control, GUS transgenics, no down-regulation of the insect Lh423 endogenous target mRNA was observed. But the results clearly showed a down-regulation of the endogenous Lygus Lh423 mRNA corresponding to the dsRNA ingested by the animals fed on 3 different events of Lh423 transgenics plants **(****Figure 23****).**

### 4.3. Transformation of cotton with a plant expression vector comprising a Lygus hesperus hairpin sequence and testing of the transformed cotton callus material or plants for resistance towards L. hesperus

The example provided below is an exemplification of the finding that transgenic cotton plants or callus expressing target gene-specific hairpin RNAs adversely affect survival and/or development of insect pest species.

### Cotton transformation

Coker 312 seed is surface sterilized using first, a 5 minute wash in 70% ethanol and then shaking in a 20% bleach (Clorox Co. USA, 1% available chlorine) solution plus 10 drops of the non-ionic detergent, Tween® 20, per litre. The seed is then rinsed 3 times in sterile distilled water before blotting dry on sterile filter papers. The sterile seed is germinated on Germination (SG) medium for 4-6 days, and at this point the hypocotyls are harvested and cut into 0.5cm lengths ready for Agrobacterium inoculation. The cut sections are placed on sterile filter papers overlaying a Murashige and Skoog based medium containing no phytohormones. The explants are incubated on a 16:8 hours day:night cycle at 28°C +/- 2°C for 3 days prior to inoculation.

For the inoculation, an *Agrobacterium tumefaciens* liquid LB culture (10 ml), strain GV3101 (pMP90) Gent^{R} or strain LBA4404 containing the RNA hairpin target of choice and a hygromycin resistance encoding plant selection cassette, is grown up overnight and 5 ml used to inoculate a 100 ml culture the evening prior to the inoculation. The culture is spun down, resuspended and diluted to an OD600 of 0.15 in the bacterial dilution medium.

The hypocotyl segments are inoculated with the *Agrobacterium* suspension for 5 minutes. After this the explants are blotted onto sterile filter paper to remove the excess bacterial suspension. The explants are incubated in the dark on Cotton Co-cultivation Medium (CCM) for 48 hours. The explants are then placed on Selective Callus Induction Medium (SCIM) containing 10mg/l Hygromycin and 500mg/l Cefotaxime and including the phytohormones 2, 4-dichlorophenoxyacetic acid (0.1µg/ml) and kinetin (0.1µg/ml). After 4-6 weeks the first resistant calli are observed, and these can be removed to fresh SCIM and further amplified for molecular assessment and insect bioassays. Plates are refreshed every 4-6 weeks to maintain nutrients and antibiotic selection.

Calli that are shown to give a positive result in the insect feeding bioassay are chosen for regeneration and callus is transferred to non-selective medium for the maturation of the somatic embryos, the recipe is based on the publication of Trolinder and Goodin, 1986. Once the embryos have reached 4mm in length and have differentiated cotyledons and radicles (2-3 months after transfer to maturation medium), they can be transferred Elongation Rooting Medium. This consists of sterilized vermiculite in large test tubes soaked with a Stewart & Hsu (1977) based liquid medium supplemented with kinetin, giberellic acid both added at the final concentration of 0.1 mg/l. The embryos are incubated at 28°C in a 16:8 day/night cycle, and once they reach the 2-3 leaf stage the plantlets can be hardened off in pots of vermiculite enclosed in a propagator to maintain humidity. Once the plants are fully hardened (4-6 true leaf stage) they can be potted into a 50:50 peat:loam mix and grown in a 14:10 light cycle at 30/20°C day/night.

### Bioassay

*L. hesperus* nymphs are placed in a Petri dish containing undifferentiated cotton callus tissue expressing target hairpin RNA. Per construct, a number of transformed cotton calli are generated and tested in a feeding bioassay for reduced nymph/adult survival and/or delayed development and stunted growth. Transgenic calli not expressing *L. hesperus* target hairpin RNA gene fragment serve as a negative control. Furthermore, young regenerated cotton plants from transgenic calli are grown in soil in a plant growth room chamber with the following conditions: 30/20°C day/night, 50 ± 5% relative humidity, 14:10 hour light:dark photoperiod. Per construct, a number of events (for example, twenty) are generated. A number of young *L. hesperus* nymphs/adults are placed on the individually caged young (for example, at the 4 - 5 unfolded leaf stage) plants and left for at least seven days before assessing resistance towards *L. hesperus* in terms of reduced nymph/adult survival, delayed development and stunted growth, and/or decreased plant feeding damage. Cotton plants not transformed with the *L. hesperus* target hairpin RNA gene fragment serve as a negative control.

### Example 5 Identification of target genes in Leptinotarsa decemlineata

### 5.1. Leptinotarsa decemlineata normalized cDNA library and preparation of dsRNAs in multiwell plates for the screening assays

Nucleic acids were isolated from *Leptinotarsa decemlineata* larvae of different stages. A cDNA library was prepared using the SMARTer™ PCR cDNA Synthesis Kit, following the manufacturer's instructions (Clontech Cat. No 634925). The cDNA library was normalized using the Trimmer kit (Evrogen Cat No NK001) and cloned in the PCR®-BLUNTII-TOPO® vector (Invitrogen). The normalization of the clones introduced M2 adapters (Trimmer Kit, Evrogen, SEQ ID NO 92: AAGCAGTGGTATCAACGCAG), oppositely oriented at each end of the clones. The recombinant vector constructs were transformed into cells of *Escherichia coli* strain TOP10 (Invitrogen). The transformed cells were subsequently diluted and plated so as to obtain single colonies or clones. The clones were checked to ensure that clone redundancy for the library did not exceed 5%. Single clones were inoculated into liquid LB (Luria-broth) media, in 96-well plates, and grown overnight at 37°C. The plates also included positive (Ld513) and negative (FP) control clones.

To generate the dsRNA, sense and antisense DNA fragments, containing T7 promoter sequence, were generated by PCR. In brief, per clone, 1 µl of bacterial suspension was dispensed in multiwell PCR plates containing REDTaq® (Sigma Cat No D4309) and primers oGCC2738 (SEQ ID NO 93: AAGCAGTGGTATCAACGCAG) and oGCC2739 (SEQ ID NO 94: GCGTAATACGACTCACTATAGGAAGCAGTGGTATCAACGCAG) based on the M2 and the T7-M2 sequences, respectively. The PCR reaction was followed by *in vitro* transcription, where, per clone, 6 µl PCR product was used in a 20 µl reaction volume containing the transcription reagents provided by the RiboMAX™ Large Scale RNA Production System - T7 kit (Promega Cat No P1300) and incubated overnight at 37°C. The final dsRNA solution was diluted in sterile Milli-Q water and used for screening. The dsRNA corresponding to the positive Ld513 control clone is SEQ ID NO 400 (see **Table 11)** and to the negative FP control clone is SEQ ID NO 104 (see **Table 4**).

### 5.2. Screen for novel and potent Leptinotarsa decemlineata target genes using a dsRNA expression cDNA library

Each well of a 48-well plate contained 0.5 mL artificial diet pretreated with a topical overlay of 25 µl (or 1 µg) of the test or control dsRNA. One L2 larva was placed in each well and 3 larvae were tested per clone. CPB survival numbers were assessed at days 4, 7 and 10.

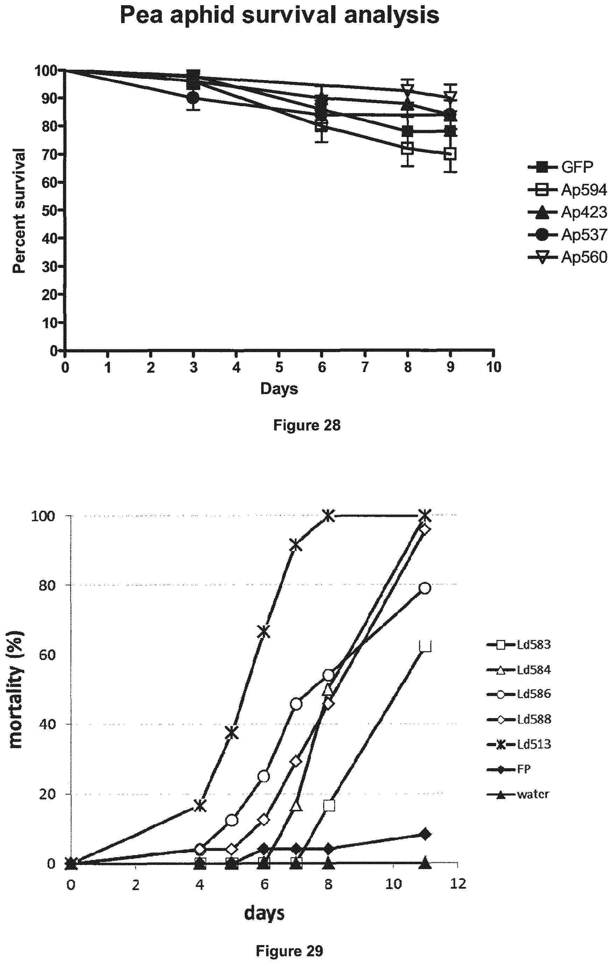

In a second bioassay, CPB larvae were fed on diet treated with topically applied test dsRNA generated from clones derived from a normalized cDNA library. One larva was placed in a well of a 48-well multiplate containing 0.5 mL diet pretreated with a topical overlay of 25 µL of a 40 ng/µL dsRNA solution. A total of twenty-four larvae were tested per treatment (clone). The number of surviving insects were assessed at days 4, 5, 6, 7, 8 & 11. The larval mortality percentage was calculated relative to day 0 (start of assay) (see **Figure 29**).

### 5.3. Identification of L. decemlineata beetle targets

The new target sequences from the screen in 5.2. and the target sequences corresponding to the troponin pathway targets, orthologuous to the *Lygus* Lh594, Lh619 and Lh620 sequences, have been identified in *L. decemlineata.* The primers which provided relevant cDNA fragment for Ld594 are listed in **Table 19.** The cDNA sequences and deduced amino acid sequences of these target genes were determined and are provided in **Tables 9 and 10** respectively.

### 5.4. Production of dsRNAs corresponding to the partial sequences of the L. decemlineata target genes

dsRNA was synthesized using the primers as provided in **Table 11.** The sense strand of the resulting dsRNA produced from the target genes is provided in **Table 11.**

### 5.5. Survival analysis assays for novel L. decemlineata targets

### Early larval assay

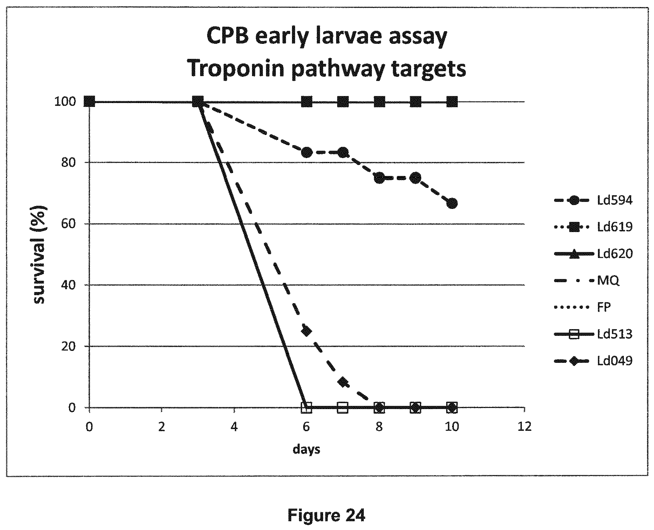

Synthetic dsRNAs were produced for the 3 targets, Ld594, Ld619 and Ld620, and were tested in a feeding assay on CPB larvae (see **Figure 24**). A 10 day assay was performed in 48 well plates, on artificial diet (based on Gelman et al, J Ins Sc,1:7, 1-10: Artificial diets for rearing the Colorado Potato Beetle), supplemented with 1 µg dsRNA /well, with 12 larvae per condition.

A clear effect on the development of the larvae could be observed. A second assay was set up to investigate the effect of these dsRNAs during the course of pupation and metamorphosis (see pupation assay underneath).

### Pupation assay

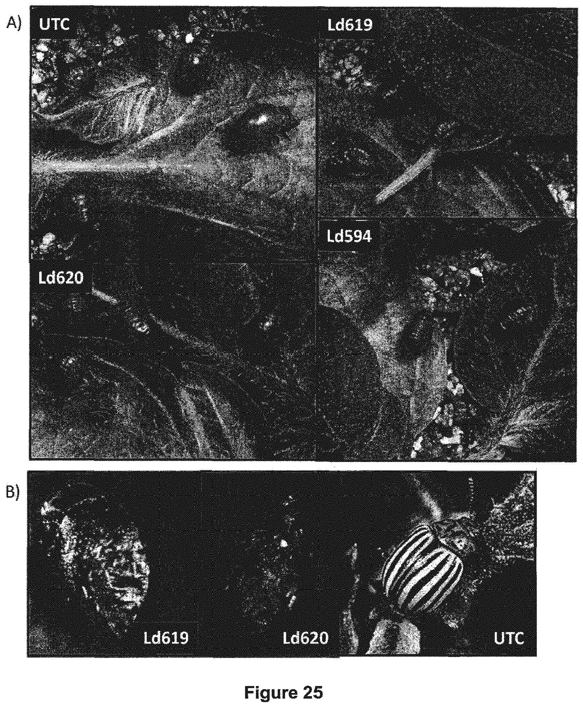

A CPB pupation assay was set up to investigate the effect of RNAi knock-down of Ld594, Ld619 and Ld620 during pupation and metamorphosis. Fourth instar larvae were fed 1 µg *in vitro* synthesized dsRNA dispensed on a potato leaf disk and were then transferred to a box containing untreated fresh potato leaves. Four days later the surviving insects were placed on vermiculite to allow pupation. Lh594 treated insects were slow, smaller and mostly were unable to go through pupation. The hatching of the pupa was assessed at the end of the experiment. For the untreated control 24 larvae pupated and all hatched into healthy adults. For Ld620, a decrease in numbers of larvae progressing into pupation was observed. For the three targets tested, no larvae progressed into healthy pupae and none emerged into adult. Dead insects recovered from the vermiculite showed various degrees of malformations **(****Figure 25****).**

Ld594, Ld619 and Ld620, first appeared as not lethal targets in the CPB larval assay, although a reduction of vitality was clearly observed in the dsRNA treated insects. On the other hand, in the pupation assay, all 3 targets induced strong effects and inhibited the entry in pupation and/or metamorphosis.

### Adult assay

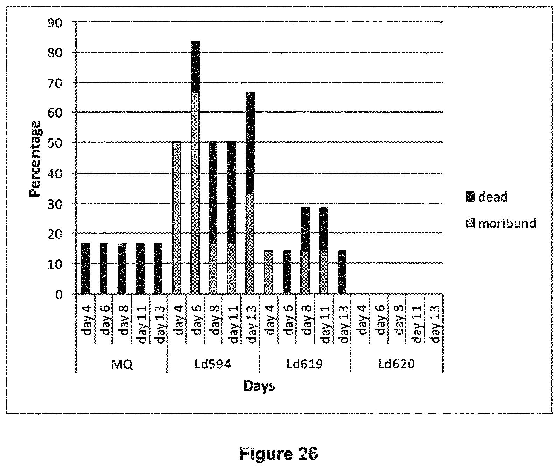

To assess activity of Ld594, Ld619 and Ld620 in CPB adults, a leaf disc assay was set up. A potato leaf disc (1.7 cm diameter) was painted with dsRNA or controls and was placed in a 3.5 cm Petri dish with one adult beetle. The next day a fresh treated leaf disc was provided to the insects. On the third day, the adults were transferred to a box containing enough fresh, untreated potato leaves to sustain the survival of the untreated controls. Per treatment, 6 adults were tested and the numbers of survivors and moribund insects were counted at regular intervals from day 6 to day 13. The insects were considered moribund if they were unable to right themselves after being placed on their back. Despite the relatively high level of background in the negative control in this particular assay, clear effects were observed for the insects that had been exposed to Ld594 or Ld619 dsRNAs **(****Figure 26****).**

### Example 6 Identification of target genes in Nilaparvata lugens

### 6.1. Identification of Nilaparvata lugens targets

New target sequences, corresponding to Troponin pathway targets and named Nl594 (Troponin I), Nl619 (Troponin T) and Nl626 (Troponin C) have been identified in brown plant hopper, *Nilaparvata lugens.* Orthologous sequences of the *Lygus* genes, named Nl594 (Troponin I), Nl619 (Troponin T) and Nl625/626 (Troponin C), were cloned through degenerated primer PCR, using BPH cDNA as template. In addition, full length cDNA was identified for Nl594, using RACE (see above for method). AmpliTaq Gold PCR system (Applied Biosystems) was used following the manufacters' instructions and with standard conditions for the degenerate primer PCR reactions, typically as follows: 1 cycle with 10 minutes at 95°C, followed by 40 cycles with 30 seconds at 95°C, 1 minute at 50°C and 1 minute at 72°C, followed by 10 minutes at 72°C. To increase the rate of success, up to 10 different degenerated primers, forward and reverse, were designed, based on alignments of orthologous sequences in other species, and used in various combinations. PCR fragments obtained were purified from the gel by gel extraction kit (Qiagen Cat. No 28706) and cloned into a TOPO TA vector (Invitrogen). The clones were sequenced and the consensus sequences were used in Blast searches against various available insect sequence databases to confirm the relevance of the insert. The degenerated primers that resulted in successful amplification are listed in **Table 20.**

The DNA sequences and deduced amino acid sequences of these target genes and one other target gene (Nl537) were determined and are provided in **Tables 12 and 13** respectively.

### 6.2. Production of dsRNAs corresponding to the partial sequences of the Nilaparvata lugens target genes

dsRNA was synthesized using the primers as provided in **Table 14.** The sense strand of the resulting dsRNA produced from each of the target genes is provided in **Table 14.**

### 6.3. Survival analysis assays for novel Nilaparvata lugens targets

dsRNAs were synthesized and tested in the previously optimized BPH RNAi-by-feeding assays, in the presence of the zwitterionic detergent, CHAPSO, at 0.1% final concentration. The dsRNAs were tested at 0.5 µg/µl final concentration. Nl537, a potent target in the BPH assays was used as bench mark target in the assay. The insect survival was assessed over the course of 9 days.

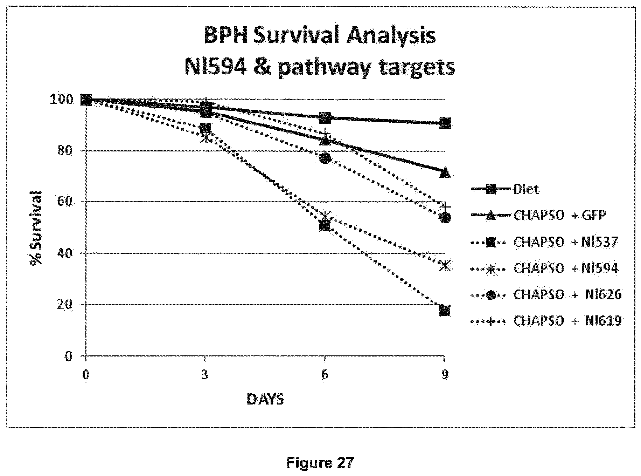

The results of the bioassay showed that in BPH Nl594, Nl619 and Nl626 were also potent RNAi targets in BPH **(****Figure 27****).**

### Example 7 Identification of target genes in Acyrthosiphon pisum

### 7.1. Identification of Acyrthosiphon pisum targets

New target sequences have been identified in aphids and were named Ap423, Ap537, Ap560 and Ap594, following the same nomenclature: "Apxxx", where "Ap" corresponds to *Acyrthosiphon pisum* and "xxx" to the ID of the target. Primers were designed based on public domain gene prediction in AphidBase (ref: http://www.aphidbase.com/) **(Table 15).**

The DNA sequences and deduced amino acid sequences of these target genes were determined and are provided in **Tables 16 and 17** respectively.

### 7.2. Production of dsRNAs corresponding to the partial sequences of the aphid target genes

dsRNA was synthesized using the primers as provided in **Table 18.** The sense strand of the resulting dsRNA produced from each of the target genes is provided in **Table 18.**

### 7.3. Survival analysis assays for novel aphid targets

RNAi-by-feeding was tested in *Acyrthosiphon pisum* (pea aphid) with 4 targets Ap594, Ap423, Ap560, Ap537. The sequences were amplified by PCR using primers, designed on public domain sequence information (http://www.aphidbase.com), and cDNA prepared from aphids. The synthetic dsRNAs were prepared and tested at a final concentration of 0.5 µg/µl in presence of 5 µg/µl yeast tRNA in a sucrose diet. Ten neonate pea aphid nymphs were placed in a small Petri dish (32 mm). Fifty µl diet (with tRNA and dsRNA) was pipetted on top of the first layer of parafilm. A second layer of parafilm covered the diet and created a feeding sachet where the aphids could feed. Per target five replicates of 10 neonate nymphs were set-up. GFP dsRNA was used as a negative control. The diet was refreshed on day 4 and 7 of the assays and survival was assessed **(****Figure 28****).**

**Table 2**

| **Target ID** | **cDNA Sequence (sense strand) 5' → 3'** |
|---|---|
| Lh594 | SEQ ID NO 1 |
| Lh609 | SEQ ID NO 3 |
| Lh610 | SEQ ID NO 5 |
| Lh610 (b) | SEQ ID NO 139 |
| Lh611 | SEQ ID NO 7 |
| Lh611 (b) | SEQ ID NO 140 |
| Lh617 | SEQ ID NO 9 |
| Lh618 | SEQ ID NO 11 |
| Lh618 (b) | SEQ ID NO 141 |
| Lh429 | SEQ ID NO 13 |
| Lh423 | SEQ ID NO 95 |
| Lh105.2 | SEQ ID NO 96 |
| Lh560 | SEQ ID NO 15 |
| Lh615 | SEQ ID NO 17 |
| Lh612 | SEQ ID NO 19 |
| Lh246 | SEQ ID NO 21 |
| Lh597 | SEQ ID NO 23 |
| Lh598 | SEQ ID NO 25 |
| Lh619 | SEQ ID NO 121 |
| Lh619 (b) | SEQ ID NO 142 |
| Lh619 (c) | SEQ ID NO 143 |
| Lh620 | SEQ ID NO 122 |
| Lh620 (b) | SEQ ID NO 144 |
| Lh620 (c) | SEQ ID NO 145 |
| Lh621 | SEQ ID NO 123 |
| Lh622 | SEQ ID NO 124 |
| Lh623 | SEQ ID NO 125 |
| Lh623 (b) | SEQ ID NO 146 |
| Lh624 | SEQ ID NO 126 |
| Lh624 (b) | SEQ ID NO 147 |
| Lh625 | SEQ ID NO 127 |
| Lh625 (b) | SEQ ID NO 148 |
| Lh626 | SEQ ID NO 128 |
| Lh626 (b) | SEQ ID NO 149 |
| Lh614 | SEQ ID NO 129 |
| Lh627 | SEQ ID NO 150 |
| Lh628 | SEQ ID NO 152 |
| Lh629 | SEQ ID NO 154 |
| Lh630 | SEQ ID NO 156 |
| Lh631 | SEQ ID NO 158 |
| Lh632 | SEQ ID NO 160 |
| Lh633.1 | SEQ ID NO 162 |
| Lh633.2 | SEQ ID NO 163 |
| Lh634.1 | SEQ ID NO 165 |
| Lh634.2 | SEQ ID NO 167 |
| Lh595.1 | SEQ ID NO 168 |
| Lh595.2 | SEQ ID NO 170 |
| Lh596 | SEQ ID NO 172 |

**Table 3**

| **Target ID** | **Corresponding amino acid sequence of cDNA clone as represented in Table 2** |
|---|---|
| Lh594 | SEQ ID NO 79 |
| Lh609 | SEQ ID NO 80 |
| Lh610 | SEQ ID NO 81 |
| Lh610 (b) | SEQ ID NO 326 |
| Lh611 | SEQ ID NO 82 |
| Lh611 (b) | SEQ ID NO 327 |
| Lh617 | SEQ ID NO 83 |
| Lh618 | SEQ ID NO 84 |
| Lh618 (b) | SEQ ID NO 328 |
| Lh429 | SEQ ID NO 85 |
| Lh429 (b) | SEQ ID NO 329 |
| Lh423 | SEQ ID NO 99 |
| Lh105.2 | SEQ ID NO 100 |
| Lh560 | SEQ ID NO 86 |
| Lh615 | SEQ ID NO 87 |
| Lh612 | SEQ ID NO 88 |
| Lh246 | SEQ ID NO 89 |
| Lh597 | SEQ ID NO 90 |
| Lh598 | SEQ ID NO 91 |
| Lh619 | SEQ ID NO 330 |
| Lh620 | SEQ ID NO 331 |
| Lh621 | SEQ ID NO 332 |
| Lh622 | SEQ ID NO 333 |
| Lh623 | SEQ ID NO 334 |
| Lh624 | SEQ ID NO 335 |
| Lh625 | SEQ ID NO 336 |
| Lh626 | SEQ ID NO 337 |
| Lh614 | SEQ ID NO 338 |
| Lh627 | SEQ ID NO 339 |
| Lh628 | SEQ ID NO 340 |
| Lh629 | SEQ ID NO 341 |
| Lh630 | SEQ ID NO 342 |
| Lh631 | SEQ ID NO 343 |
| Lh632 | SEQ ID NO 344 |
| Lh633.1 | SEQ ID NO 345 |
| Lh633.2 | SEQ ID NO 346 |
| Lh634.1 | SEQ ID NO 347 |
| Lh634.2 | SEQ ID NO 348 |

**Table 4**

| **Target ID** | **Primers Forward 5' → 3'** | **Primers Reverse 5' → 3'** | **dsRNA: sense strand represented by equivalent DNA Sequence 5' → 3'** |
|---|---|---|---|
| Lh594 | SEQ ID NO 27 | SEQ ID NO 28 | SEQ ID NO 2 |
| | SEQ ID NO 29 | SEQ ID NO 30 | |
| Lh609 | SEQ ID NO 31 | SEQ ID NO 32 | SEQ ID NO 4 |
| | SEQ ID NO 33 | SEQ ID NO 34 | |
| Lh610 | SEQ ID NO 35 | SEQ ID NO 36 | SEQ ID NO 6 |
| | SEQ ID NO 37 | SEQ ID NO 38 | |
| Lh611 | SEQ ID NO 39 | SEQ ID NO 40 | SEQ ID NO 8 |
| | SEQ ID NO 41 | SEQ ID NO 42 | |
| Lh617 | SEQ ID NO 43 | SEQ ID NO 44 | SEQ ID NO 10 |
| | SEQ ID NO 45 | SEQ ID NO 46 | |
| Lh618 | SEQ ID NO 47 | SEQ ID NO 48 | SEQ ID NO 12 |
| | SEQ ID NO 49 | SEQ ID NO 50 | |
| Lh429 | SEQ ID NO 51 | SEQ ID NO 52 | SEQ ID NO 14 |
| | SEQ ID NO 53 | SEQ ID NO 54 | |
| Lh423 | SEQ ID NO 105 | SEQ ID NO 106 | SEQ ID NO 101 |
| | SEQ ID NO 107 | SEQ ID NO 108 | |
| Lh105.2 | SEQ ID NO 109 | SEQ ID NO 110 | SEQ ID NO 102 |
| | SEQ ID NO 111 | SEQ ID NO 112 | |
| GFP | SEQ ID NO 113 | SEQ ID NO 114 | SEQ ID NO 103 |
| | SEQ ID NO 115 | SEQ ID NO 116 | |
| Pt | SEQ ID NO 117 | SEQ ID NO 118 | SEQ ID NO 104 |
| | SEQ ID NO 119 | SEQ ID NO 120 | |
| Lh560 | SEQ ID NO 55 | SEQ ID NO 56 | SEQ ID NO 16 |
| | SEQ ID NO 57 | SEQ ID NO 58 | |
| Lh615 | SEQ ID NO 59 | SEQ ID NO 60 | SEQ ID NO 18 |
| | SEQ ID NO 61 | SEQ ID NO 62 | |
| Lh612 | SEQ ID NO 63 | SEQ ID NO 64 | SEQ ID NO 20 |
| | SEQ ID NO 65 | SEQ ID NO 66 | |
| Lh246 | SEQ ID NO 67 | SEQ ID NO 68 | SEQ ID NO 22 |
| | SEQ ID NO 69 | SEQ ID NO 70 | |
| Lh597 | SEQ ID NO 71 | SEQ ID NO 72 | SEQ ID NO 24 |
| | SEQ ID NO 73 | SEQ ID NO 74 | |
| Lh598 | SEQ ID NO 75 | SEQ ID NO 76 | SEQ ID NO 26 |
| | SEQ ID NO 77 | SEQ ID NO 78 | |
| Lh619 | SEQ ID NO 206 | SEQ ID NO 207 | SEQ ID NO 130 |
| | SEQ ID NO 208 | SEQ ID NO 209 | |
| Lh620 | SEQ ID NO 210 | SEQ ID NO 211 | SEQ ID NO 131 |
| | SEQ ID NO 212 | SEQ ID NO 213 | |
| Lh621 | SEQ ID NO 214 | SEQ ID NO 215 | SEQ ID NO 132 |
| | SEQ ID NO 216 | SEQ ID NO 217 | |
| Lh622 | SEQ ID NO 218 | SEQ ID NO 219 | SEQ ID NO 133 |
| | SEQ ID NO 220 | SEQ ID NO 221 | |
| Lh623 | SEQ ID NO 222 | SEQ ID NO 223 | SEQ ID NO 134 |
| | SEQ ID NO 224 | SEQ ID NO 225 | |
| Lh624 | SEQ ID NO 226 | SEQ ID NO 227 | SEQ ID NO 135 |
| | SEQ ID NO 228 | SEQ ID NO 229 | |
| Lh625 | SEQ ID NO 230 | SEQ ID NO 231 | SEQ ID NO 136 |
| | SEQ ID NO 232 | SEQ ID NO 233 | |
| Lh626 | SEQ ID NO 234 | SEQ ID NO 235 | SEQ ID NO 137 |
| | SEQ ID NO 236 | SEQ ID NO 237 | |
| Lh614 | SEQ ID NO 238 | SEQ ID NO 239 | SEQ ID NO 138 |
| | SEQ ID NO 240 | SEQ ID NO 241 | |
| Lh627 | SEQ ID NO 242 | SEQ ID NO 243 | SEQ ID NO 151 |
| | SEQ ID NO 244 | SEQ ID NO 245 | |
| Lh628 | SEQ ID NO 246 | SEQ ID NO 247 | SEQ ID NO 153 |
| | SEQ ID NO 248 | SEQ ID NO 249 | |
| Lh629 | SEQ ID NO 250 | SEQ ID NO 251 | SEQ ID NO 155 |
| | SEQ ID NO 25 | SEQ ID NO 253 | |
| Lh630 | SEQ ID NO 254 | SEQ ID NO 255 | SEQ ID NO 157 |
| | SEQ ID NO 256 | SEQ ID NO 257 | |
| Lh631 | SEQ ID NO 258 | SEQ ID NO 259 | SEQ ID NO 159 |
| | SEQ ID NO 260 | SEQ ID NO 261 | |
| Lh632 | SEQ ID NO 262 | SEQ ID NO 263 | SEQ ID NO 161 |
| | SEQ ID NO 264 | SEQ ID NO 265 | |
| Lh633.2 | SEQ ID NO 266 | SEQ ID NO 267 | SEQ ID NO 164 |
| | SEQ ID NO 268 | SEQ ID NO 269 | |
| Lh634.1 | SEQ ID NO 270 | SEQ ID NO 271 | SEQ ID NO 166 |
| | SEQ ID NO 272 | SEQ ID NO 273 | |
| Lh595 | SEQ ID NO 274 | SEQ ID NO 275 | SEQ ID NO 169 |
| | SEQ ID NO 276 | SEQ ID NO 277 | |
| Lh596 | SEQ ID NO 278 | SEQ ID NO 279 | SEQ ID NO 173 |
| | SEQ ID NO 280 | SEQ ID NO 281 | |

**Table 8**

| **Target ID** | **Primers Forward 5' → 3'** | **Primers Reverse 5' → 3'** | **qRT-PCR Amplicon 5'** → **3'** |
|---|---|---|---|
| Lh423 | SEQ ID NO 360 | SEQ ID NO 361 | SEQ ID 362 |
| Lh425 | SEQ ID 363 | SEQ ID 364 | SEQ ID 365 |
| Lh427 | SEQ ID 366 | SEQ ID 367 | SEQ ID 368 |

**Table 9**

| **Target ID** | **cDNA sequence (sense strand) 5' → 3'** |
|---|---|
| Ld594 | SEQ ID NO 174 |
| Ld594(b) | SEQ ID NO 404 |
| Ld619 | SEQ ID NO 176 |
| Ld620 | SEQ ID NO 178 |
| Ld583 | SEQ ID NO 386 |
| Ld584 | SEQ ID NO 387 |
| Ld586 | SEQ ID NO 388 |
| Ld588 | SEQ ID NO 389 |
| Ld513 | SEQ ID NO 394 |

**Table 10**

| **Target ID** | **Corresponding amino acid sequence of cDNA clone as represented in Table 9** |
|---|---|
| Ld594 | SEQ ID NO 349 |
| Ld619 | SEQ ID NO 350 |
| Ld594(b) | SEQ ID NO 405 |
| Ld620 | SEQ ID NO 351 |
| Ld583 | SEQ ID NO 390 |
| Ld584 | SEQ ID NO 391 |
| Ld586 | SEQ ID NO 392 |
| Ld588 | SEQ ID NO 393 |
| Ld513 | SEQ ID NO 395 |

**Table 11**

| **Target ID** | **Primer Forward 5' → 3'** | **Primers Reverse 5' → 3'** | **sRNA: sense strand represented by equivalent DNA Sequence 5' → 3'd** |
|---|---|---|---|
| Ld594 | SEQ ID NO 282 | SEQ ID NO 283 | SEQ ID NO 175 |
| | SEQ ID NO 284 | SEQ ID NO 285 | |
| Ld619 | SEQ ID NO 286 | SEQ ID NO 287 | SEQ ID NO 177 |
| | SEQ ID NO 288 | SEQ ID NO 289 | |
| Ld620 | SEQ ID NO 290 | SEQ ID NO 291 | SEQ ID NO 179 |
| | SEQ ID NO 292 | SEQ ID NO 293 | |
| Ld513 | SEQ ID NO 396 | SEQ ID NO 397 | SEQ ID NO 400 |
| | SEQ ID NO 398 | SEQ ID NO 399 | |

**Table12**

| **Target ID** | **cDNA Sequence (sense strand) 5' → 3'** |
|---|---|
| Nl594 | SEQ ID NO 180 |
| Nl619 | SEQ ID NO 182 |
| Nl626 | SEQ ID NO 184 |
| Nl537 | SEQ ID NO 186 |

**Table 13**

| **Target ID** | **Corresponding amino acid sequence of cDNA clone as represented in Table 12** |
|---|---|
| NI594 | SEQ ID NO 352 |
| Nl619 | SEQ ID NO 353 |
| Nl626 | SEQ ID NO 354 |
| Nl537 | SEQ ID NO 355 |

**Table 14**

| **Target ID** | **Primers Forward 5' → 3'** | **Primers Reverse 5' → 3'** | **dsRNA: sense strand represented by equivalent DNA Sequence 5' → 3'** |
|---|---|---|---|
| NI594 | SEQ ID NO 294 | SEQ ID NO 295 | SEQ ID NO 181 |
| | SEQ ID NO 296 | SEQ ID NO 297 | |
| Nl619 | SEQ ID NO 298 | SEQ ID NO 299 | SEQ ID NO 183 |
| | SEQ ID NO 300 | SEQ ID NO 301 | |
| Nl626 | SEQ ID NO 302 | SEQ ID NO 303 | SEQ ID NO 185 |
| | SEQ ID NO 304 | SEQ ID NO 305 | |
| Nl537 | SEQ ID NO 306 | SEQ ID NO 307 | SEQ ID NO 187 |
| | SEQ ID NO 308 | SEQ ID NO 309 | |

**Table 15**

| **Target** | **Fw primer sequence** | **Reverse primer sequence** |
|---|---|---|
| Ap594 | SEQ ID NO 369 | SEQ ID NO 370 |
| Ap423 | SEQ ID NO 371 | SEQ ID NO 372 |
| Ap537 | SEQ ID NO 373 | SEQ ID NO 374 |
| Ap560 | SEQ ID NO 375 | SEQ ID NO 376 |

**Table 16**

| **Target ID** | **cDNA Sequence (sense strand) 5' → 3'** |
|---|---|
| Ap594 | SEQ ID NO 188 |
| Ap423 | SEQ ID NO 200 |
| Ap537 | SEQ ID NO 202 |
| Ap560 | SEQ ID NO 204 |

**Table 17**

| **Target ID** | **Corresponding amino acid sequence of cDNA clone as represented in Table 16** |
|---|---|
| Ap594 | SEQ ID NO 356 |
| Ap423 | SEQ ID NO 357 |
| Ap537 | SEQ ID NO 358 |
| Ap560 | SEQ ID NO 359 |

**Table 18**

| **Target ID** | **Primers Forward 5' → 3'** | **Primers Reverse 5' → 3'** | **dsRNA: sense strand represented by equivalent DNA sequence 5' → 3'** |
|---|---|---|---|
| Ap594 | SEQ ID NO 310 | SEQ ID NO 311 | SEQ ID NO 189 |
| | SEQ ID NO 312 | SEQ ID NO 313 | |
| Ap423 | SEQ ID NO 314 | SEQ ID NO 315 | SEQ ID NO 201 |
| | SEQ ID NO 316 | SEQ ID NO 317 | |
| Ap537 | SEQ ID NO 318 | SEQ ID NO 319 | SEQ ID NO 203 |
| | SEQ ID NO 320 | SEQ ID NO 321 | |
| Ap560 | SEQ ID NO 322 | SEQ ID NO 323 | SEQ ID NO 205 |
| | SEQ ID NO 324 | SEQ ID NO 325 | |

**Table 19**

| **Target** | **Forward primer** | **Reverse primer** |
|---|---|---|
| Ld594 | SEQ ID NO 377 | SEQ ID NO 378 |

**Table 20**

| **Target** | **Forward primer** | **Reverse primer** |
|---|---|---|
| NI594 | seq id no 379 | seq id no 380 |
| Nl619 | seq id no 381 | seq id no 382 |
| Nl626 | seq id no 383 | seq id no 384 |

**Table 21**

| **Target ID** | **Best Drosophila hit** | **NAME** | **SYMBOL** |
|---|---|---|---|
| **Ld583** | CG4759 | Ribosomal protein L27 | RpL27 |
| **Ld584** | CG 17331 | Proteasome, beta-type subunit | |
| Ld586 | CG 13704 | unknown | |
| **Ld588** | CG4157 | Rpn12 | |

**Table 22**

| **Target ID** | **Best Drosophila hit** | **NAME** | **SYMBOL** |
|---|---|---|---|
| **Nl594** | CG7178 | wings up A (troponin I) | wupA |
| | CG7107 | troponin T (upheld) | up |
| **Nl626** | *CG9073, CG7930, CG2981, CG12408, CG6514, CG2981, CG7930, CG9073, CG6514, CG12408 | troponin C | |
| **Nl537** | CG32744 | Ubiquitin-5E; protein modification process | |

| | | | |
|---|---|---|---|
| *unclear : multiple hits in family | | | |

**Table 23**

| **Target ID** | **Best Drosophila hit** | **NAME** | **SYMBOL** |
|---|---|---|---|
| **Ap594** | CG7178 | wings up A (troponin I) | wupA |
| **Ap423** | CG2746 | ribosomal protein L19 | RpL19 |
| **Ap537** | CG32744 | Ubiquitin-5E; protein modification process | |
| **Ap560** | CG 10423 | ribosomal protein S27 | RpS27 |

It will be appreciated by persons skilled in the art that numerous variations and/or modifications may be made to the above mentioned assays without departing from the spirit or scope of this assay as generically described. Those skilled in the art will recognize, or be able to ascertain using no more than routine experimentation, many equivalents to the specific examples, and such equivalents are intended to be encompassed by the present invention. The present example, therefore, is to be considered in all respects as illustrative and not restrictive.

## Claims

1. A transgenic plant, or reproductive or propagation material for a transgenic plant or a cultured transgenic plant cell, which expresses or is capable of expressing at least one interfering ribonucleic acid (RNA) that functions upon uptake by an insect pest species to down-regulate the expression of a target gene within said pest, wherein the interfering RNA comprises at least one silencing element wherein said silencing element is a region of double-stranded RNA comprising annealed complementary strands, one strand of which comprises or consists of a sequence of at least 21 contiguous nucleotides that is at least 75% complementary to any of SEQ ID NOs 122, 131, 144, 145, 178, 179 or the complement thereof.

2. The transgenic plant, reproductive or propagation material for a transgenic plant, or cultured transgenic plant cell of claim 1 wherein the RNA comprises at least two silencing elements, wherein each silencing element comprises or consists of a sequence of nucleotides which is complementary to a target nucleotide sequence within a target gene.

3. The transgenic plant, reproductive or propagation material for a transgenic plant, or cultured transgenic plant cell of any of claims 1-2 wherein said insect pest species is selected from the insect species belonging to the orders: *Coleoptera, Hemiptera, Lepidoptera, Diptera, Dichyoptera, Orthoptera, and Siphonaptera.*

4. The transgenic plant, reproductive or propagation material for a transgenic plant, or cultured transgenic plant cell of claim 3 wherein said insect pest species is selected from the group consisting of *Leptinotarsa* spp. (e.g. *L. decemlineata* (Colorado potato beetle), *L. juncta* (false potato beetle), or *L*. *texana* (Texan false potato beetle)); *Nilaparvata* spp. (e.g. *N. lugens (brown planthopper));* Lygus spp. (e.g. *L. lineolaris* (tarnished plant bug) or *L. hesperus* (western tarnished plant bug)); Myzus spp. (e.g. *M. persicae* (green peach aphid)); *Diabrotica* spp. *(e.g. D. virgifera virgifera* (western corn rootworm), *D. barberi* (northern corn rootworm), *D*. *undecimpunctata howardi* (southern corn rootworm), *D. virgifera* zeae (Mexican corn rootworm)).

5. The transgenic plant, reproductive or propagation material for a transgenic plant, or cultured transgenic plant cell of any of claims 1-4 wherein said target gene encodes the upheld protein.

6. The transgenic plant, reproductive or propagation material for a transgenic plant, or cultured transgenic plant cell of claim 5 wherein down-regulation of expression of said pest target gene causes decreased growth, development, reproduction, or survival of said pest as compared with said pest species exposed to an interfering RNA targeting a non-essential gene or an interfering RNA that does not down-regulate any genes within said pest species.

7. The transgenic plant, reproductive or propagation material for a transgenic plant, or cultured transgenic plant cell of any of claims 1-6 wherein said transgenic plant, material therefrom or plant cell additionally comprises a heterologous gene.

8. The transgenic plant, reproductive or propagation material for a transgenic plant, or cultured transgenic plant cell of claim 7 wherein said heterologous gene encodes a protein toxic to a plant pest species.

9. The transgenic plant, reproductive or propagation material for a transgenic plant, or cultured transgenic plant cell of claim 8 wherein said protein is selected from the group consisting of a patatin, a *Bacillus thuringiensis* insecticidal protein, a *Xenorhabdus* insecticidal protein, a *Photorhabdus* insecticidal protein, a *Bacillus laterosporus* insecticidal protein, and a *Bacillus sphaericus* insecticidal protein.

10. The transgenic plant, reproductive or propagation material for a transgenic plant, or cultured transgenic plant cell of claim 9 wherein said *Bacillus thuringiensis* insecticidal protein is selected from the group consisting of a Cry1, a Cry3, a TIC851, a CryET170, a Cry22, a TIC901, a TIC201, a TIC407, a TIC417, a binary insecticidal protein CryET80 and CryET76, a binary insecticidal protein TIC100 and TIC101, a combination of an insecticidal protein ET29 or ET37 with an insecticidal protein TIC810 or TIC812, and a binary insecticidal protein PS149B1.

11. The transgenic plant, reproductive or propagation material for a transgenic plant, or cultured transgenic plant cell of claim 7 wherein said heterologous gene encodes a protein conferring herbicide tolerance.

12. The transgenic plant, reproductive or propagation material for a transgenic plant, or cultured transgenic plant cell of claim 11 wherein said protein is selected from a glyphosate-insensitive version of a 5-enolpyruvylshikimate-3-phosphate synthase (EPSPS), a catabolic enzyme that is able to break down dicamba such as dicamba monooxygenase or a phosphinothricin acetyl transferase gene that is able to catabolize glufosinate ammonium.

13. Transgenic seed produced from the transgenic plant of any of claims 1-12, wherein said transgenic seed expresses or is capable of expressing an interfering ribonucleic acid (RNA) as defined in claim 1.

14. A method for generating a transgenic plant resistant to infestation by an insect pest species comprising:
(a) transforming a plant cell with a DNA construct comprising a polynucleotide sequence encoding an interfering ribonucleic acid (RNA) as defined in claim 1;
(b) regenerating a plant from the transformed plant cell; and
(c) growing the transformed plant under conditions suitable for the expression of the interfering RNA from the DNA construct, said plant thus being resistant to said pest as compared with an untransformed plant.

15. A method according to claim 14 wherein said plant resistant to infestation by an insect pest is chosen from cotton, potato, rice, canola, sunflower, sorghum, pearl millet, corn, strawberries, soy, alfalfa, tomato, eggplant, pepper and tobacco.

16. A method for preventing and/or controlling insect pest infestation in a field of crop plants, said method comprising expressing in said plants an effective amount of an interfering ribonucleic acid (RNA) as defined in claim 1.


SEQUENCE LISTING

<110>  DEVGEN NV

<120>  DOWN-REGULATING GENE EXPRESSION IN INSECT PESTS

<130>  NLW/P122274WO01

<160>  405

<170>  PatentIn version 3.5

<210>  1
<211>  1096
<212>  DNA
<213>  Lygus hesperus

<400>  1
gcgatctaag gcaggtggca gacagctcga tgacggcagt gggccaagca ataatggata      60

gtcattcata gcaccccagc tttactaagc tctgccgtag tgttggattg ggagcggata     120

caattcacca cagaacagct atgacatgat acgcagtccg aataccctca taaaggacta     180

gtctgcaggt ttaacgatcg cgtagcagtg tatcacgcag agtacatggg gagtgactgt     240

gtgaacctgc tgggtacatc atcacccctc tccttcttca gttatataag acacagtccc     300

taaaggacac cagcaaaaat ggcggatgat gaggcgaaga aggccaaaca ggccgaaatc     360

gagaggaagc gcgctgaagt gcgcaagagg atggaggaag cctctaaggc gaagaaagcc     420

aagaagggtt tcatgacccc ggaaaggaag aagaaactcc gactcctgct gaggaaaaaa     480

gccgctgagg aactgaagaa ggagcaggaa cgcaaagcag ctgagaggag gcgaacgatt     540

gaggagcgct gcgggcaaat tgccgacgtc gacaacgcca atgaagcaac cttgaagaaa     600

ctctgcacag actaccataa gcgaattgac gctctggaga ggagtaaaat tgacatcgaa     660

ttcgaagtgg agagacgtga ccttgagatc gccgacctca acagccaggt caacgacctc     720

cgtggtaaat tcgtcaaacc taccttgaaa aaggtttcca agtacgaaaa caaattcgcc     780

aagctccaga agaaggctgc cgagttcaac ttcagaaacc aactcaaggt cgtcaaaaag     840

aaagaattca ccctggaaga agaagacaaa gagccgaaga aatcggaaaa ggcggagtgg     900

cagaagaaat gaagggaaaa caagcacacc atctcacaaa ataaaataaa cgaaaatctt     960

tcacacgttt accaatttta taacacggtc ctcacaaatt atgttcctta aataatttgt    1020

ataatccatc ctcgcactac aatcaatatt aatatttaaa tacaaaacca aaaaaaaaaa    1080

aaaaaaaaaa aaaaaa                                                    1096

<210>  2
<211>  491
<212>  DNA
<213>  Lygus hesperus

<400>  2

caaacaggcc gaaatcgaga ggaagcgcgc tgaagtgcgc aagaggatgg aggaagcctc 60

taaggcgaag aaagccaaga agggtttcat gaccccggaa aggaagaaga aactccgact 120

cctgctgagg aaaaaagccg ctgaggaact gaagaaggag caggaacgca aagcagctga 180

gaggaggcga acgattgagg agcgctgcgg gcaaattgcc gacgtcgaca acgccaatga 240

agcaaccttg aagaaactct gcacagacta ccataagcga attgacgctc tggagaggag 300

taaaattgac atcgaattcg aagtggagag acgtgacctt gagatcgccg acctcaacag 360

ccaggtcaac gacctccgtg gtaaattcgt caaacctacc ttgaaaaagg tttccaagta 420

cgaaaacaaa ttcgccaagc tccagaagaa ggctgccgag ttcaacttca gaaaccaact 480

caaggtcgtc a 491

<210> 3
<211> 431
<212> DNA
<213> Lygus hesperus

<400> 3
atgggcatca tgtcgaaagc tgaactcgct tgtgtttact ccgctctcat cctcatcgac 60

gacgatgtcg ccgtgacggg tgagaagatt caaaccatcc tgaaggctgc cagtgtcgac 120

atcgagccgt actggcccgg tctgttcgcc aaggccctcg agggtatcaa ccccaaagac 180

ctcatctcct ccattggaag cggagttggt gctggagcgc cggctgtcgg tggagctgca 240

cctgccgccg ctgctgcccc tgccgctgag gctaagaagg aagagaagaa gaaggtcgaa 300

agcgatccag aatccgatga tgacatgggc ttcggtcttt tcgactaaga gcattccaca 360

gcgggttctc atttgttttt aagattttct tttaaaaaat aaaacttcca aaaaaaaaaa 420

aaaaaaaaaa g 431

<210> 4
<211> 332
<212> DNA
<213> Lygus hesperus

<400> 4
gggcatcatg tcgaaagctg aactcgcttg tgtttactcc gctctcatcc tcatcgacga 60

cgatgtcgcc gtgacgggtg agaagattca aaccatcctg aaggctgcca gtgtcgacat 120

cgagccgtac tggcccggtc tgttcgccaa ggccctcgag ggtatcaacc ccaaagacct 180

catctcctcc attggaagcg gagttggtgc tggagcgccg gctgtcggtg gagctgcacc 240

tgccgccgct gctgcccctg ccgctgaggc taagaaggaa gagaagaaga aggtcgaaag 300

cgatccagaa tccgatgatg acatgggctt cg 332

<210> 5
<211> 468

<212>  DNA
<213>  Lygus hesperus

<400>  5
   atgggggcag gtcttctcca taaccataga ttatcttcgt gtatcgtgtc gggctttcgg      60
   ctgaggtcct aattagtaaa taatgattcc gcctacgtcg cggcctcagg tcactgtcta     120
   cagtgacaaa aatgaggcca ccgggactct cctcaacctc ccggctgtct tcaacgcccc     180
   cattcgcccc gatgttgtga acttcgttca ccaaaatgtc gctaaaaacc acaggcagcc     240
   ctactgtgtc tccgctcaag ctggtcatca gacttcagct gagtcctggg gtaccggtcg     300
   tgctgtggct cgtatccccc gtgttcgcgg aggtggtact caccgctcag gtcagggtgc     360
   ttttggcaac atgtgtcgcg gcggtaggat gttcgctccc actcgcccat ggcgtcgttg     420
   gcaccgcaaa atcaacgtta accaaaaaaa aaaaaaaaaa aaaaaaaa                   468


<210>  6
<211>  429
<212>  DNA
<213>  Lygus hesperus

<400>  6
gggcaggtct tctccataac catagattat cttcgtgtat cgtgtcgggc tttcggctga      60

ggtcctaatt agtaaataat gattccgcct acgtcgcggc ctcaggtcac tgtctacagt     120

gacaaaaatg aggccaccgg gactctcctc aacctcccgg ctgtcttcaa cgcccccatt     180

cgccccgatg ttgtgaactt cgttcaccaa aatgtcgcta aaaaccacag gcagccctac     240

tgtgtctccg ctcaagctgg tcatcagact tcagctgagt cctggggtac cggtcgtgct     300

gtggctcgta tcccccgtgt tcgcggaggt ggtactcacc gctcaggtca gggtgctttt     360

ggcaacatgt gtcgcggcgg taggatgttc gctcccactc gcccatggcg tcgttggcac     420

cgcaaaatc                                                             429


<210>  7
<211>  523
<212>  DNA
<213>  Lygus hesperus

<400>  7
atgggatctc tatgctgaaa aggtcgccac cagaggtttg tgtgctattg cacaagctga      60

atccctccgt tacaaactca ttggcggtct tgctgtccga ggggcttgct atggtgtcct     120

tcgcttcatc atggaaaatg gtgccaaggg ttgcgaagtc gtagtatctg gaaaactgcg     180

tggtcagaga gccaagtcaa tgaagttcgt ggatggtttg atgatccaca gtggggatcc     240

ctgtaacgaa tatgttgata ctgctacccg acatgtgctc cttagacaag gtgtcctggg     300

aataaaggtg aagattatgt tgccgtggga cgttaccggc aaaaatgggc cgaagaaccc     360

tcttcccgac cacgtcagcg ttctcttacc taaggaggag ctaccaaatt tggccgttag     420

tgtgcctgga tccgacatca aaccaaagcc tgaagtacca gcacccgctt tgtgaatata 480

aacttctttt ttgtaaaaaa aaaaaaaaaa aaaaaaaaaa aaa 523

<210> 8
<211> 431
<212> DNA
<213> Lygus hesperus

<400> 8
attgcacaag ctgaatccct ccgttacaaa ctcattggcg gtcttgctgt ccgaggggct 60

tgctatggtg tccttcgctt catcatggaa aatggtgcca agggttgcga agtcgtagta 120

tctggaaaac tgcgtggtca gagagccaag tcaatgaagt tcgtggatgg tttgatgatc 180

cacagtgggg atccctgtaa cgaatatgtt gatactgcta cccgacatgt gctccttaga 240

caaggtgtcc tgggaataaa ggtgaagatt atgttgccgt gggacgttac cggcaaaaat 300

gggccgaaga accctcttcc cgaccacgtc agcgttctct tacctaagga ggagctacca 360

aatttggccg ttagtgtgcc tggatccgac atcaaaccaa agcctgaagt accagcaccc 420

gctttgtgaa t 431

<210> 9
<211> 823
<212> DNA
<213> Lygus hesperus

<400> 9
catggggaca ctctcttttt cttcatcgcg tggctcgctg ccgtgtggtt agggagtttc 60

ctactttaat tttttagtgt aattcatctt caaaatgacg tcgaaggttt ctcgtgagac 120

cctctacgag tgcatcaatg gagtcatcca gtcctcccag gagaagaaga ggaacttcgt 180

ggagactgtg gagatccaga tcggtctgaa gaactacgat ccccagaagg acaagcgttt 240

ctcgggaact gtcaagctga agcacattcc aaggcctaaa atgcaggttt gcatcctcgg 300

agatcaacag cattgcgacg aggccaaagc caacaacgtg ccctacatgg acgtcgaggc 360

tctgaagaag ctcaacaaaa acaagaagct cgtcaagaaa ttggccaaga aatacgacgc 420

tttcctcgcc tcagaagccc tcatcaagca gatccccagg ctcctcggac ccggtctcaa 480

caaggcgggc aagttccctg gtctcctctc tcaccaggag tccatgatga tgaagatcga 540

cgaagtcaag gccaccatca agttccaaat gaagaaggtg ttgtgcctct cagtggctgt 600

cggtcacgtc ggcatgactg ctgatgagct cgtccagaac gtgcacttgt cggtcaactt 660

cctcgtttcg ctcctcaaga agcactggca gaacgtcagg tctctccacg tcaaatccac 720

gatgggacct ccccagaggc tttactaaac atcttgtttt ttacttttga cgaataaaat 780

tcgttttatt ctcgaaaaaa aaaaaaaaaa aaaaaaaaaa aaa 823

<210>  10
<211>  607
<212>  DNA
<213>  Lygus hesperus

<400>  10
ccctctacga gtgcatcaat ggagtcatcc agtcctccca ggagaagaag aggaacttcg      60

tggagactgt ggagatccag atcggtctga agaactacga tccccagaag gacaagcgtt     120

tctcgggaac tgtcaagctg aagcacattc caaggcctaa aatgcaggtt tgcatcctcg     180

gagatcaaca gcattgcgac gaggccaaag ccaacaacgt gccctacatg gacgtcgagg     240

ctctgaagaa gctcaacaaa aacaagaagc tcgtcaagaa attggccaag aaatacgacg     300

ctttcctcgc ctcagaagcc ctcatcaagc agatccccag gctcctcgga cccggtctca     360

acaaggcggg caagttccct ggtctcctct ctcaccagga gtccatgatg atgaagatcg     420

acgaagtcaa ggccaccatc aagttccaaa tgaagaaggt gttgtgcctc tcagtggctg     480

tcggtcacgt cggcatgact gctgatgagc tcgtccagaa cgtgcacttg tcggtcaact     540

tcctcgtttc gctcctcaag aagcactggc agaacgtcag gtctctccac gtcaaatcca     600

cgatggg                                                               607


<210>  11
<211>  435
<212>  DNA
<213>  Lygus hesperus

<400>  11
atgggaccaa taaagatcaa ctttcccaga gaaagacttg ctatgcccag cataatcagg      60

tccgagaaat ccgcaaaaag atggttaaaa acatcagtga cagcatttcc agctgtgatt     120

tgaggagtgt tgtgaacaag ctgatcccag actccatcgc taaagatata gaaaagaatt     180

gccaaggaat ctacccactc cacgatgtgt acattcggaa ggtgaaggtg ttgaagaagc     240

cgaggttcga gctcagcaag ctccttgagc ttcacgtcga tggcaaaggg atcgacgaac     300

ccggcgcgaa agtgacgagg actgacgctt acgagcctcc agttcaagag tctgtctaag     360

taaacatttt atataaagtt aacaaaaaat aaaggtgtct cgcctgacta aaaaaaaaaa     420

aaaaaaaaaa aaaaa                                                      435


<210>  12
<211>  353
<212>  DNA
<213>  Lygus hesperus

<400>  12
ccaataaaga tcaactttcc cagagaaaga cttgctatgc ccagcataat caggtccgag      60

aaatccgcaa aaagatggtt aaaaacatca gtgacagcat ttccagctgt gatttgagga     120

gtgttgtgaa caagctgatc ccagactcca tcgctaaaga tatagaaaag aattgccaag 180

gaatctaccc actccacgat gtgtacattc ggaaggtgaa ggtgttgaag aagccgaggt 240

tcgagctcag caagctcctt gagcttcacg tcgatggcaa agggatcgac gaacccggcg 300

cgaaagtgac gaggactgac gcttacgagc ctccagttca agagtctgtc taa 353

<210> 13
<211> 474
<212> DNA
<213> Lygus hesperus

<400> 13
catgggtacg aatatcgacg gtaaaagaaa ggtgatgttc gccatgaccg ccatcaaagg 60

tgtcggcaga cggtacgcca acattgtcct caagaaggcc gatgtcaact tggacaagag 120

ggccggcgaa tgctccgaag aagaagttga aaagatcgtt accatcatgc aaaaccctag 180

gcaatacaaa attcccaact ggttcctcaa cagacaaaaa gacaccgtcg agggcaaata 240

ctctcagttg acttcctccc tgctggattc caagctccgt gacgaccttg agcgactcaa 300

gaagatcagg gcccacagag gcatgaggca ctactggggt ttgagggtgc gtggtcaaca 360

cacgaagacc accggaagga gaggacgaac tgttggtgtg tccaagaaga agtaatttta 420

atttcctaat aaattggttt tttcaaaaaa aaaaaaaaaa aaaaaaaaaa aaaa 474

<210> 14
<211> 332
<212> DNA
<213> Lygus hesperus

<400> 14
gaaaggtgat gttcgccatg accgccatca aaggtgtcgg cagacggtac gccaacattg 60

tcctcaagaa ggccgatgtc aacttggaca agagggccgg cgaatgctcc gaagaagaag 120

ttgaaaagat cgttaccatc atgcaaaacc ctaggcaata caaaattccc aactggttcc 180

tcaacagaca aaaagacacc gtcgagggca aatactctca gttgacttcc tccctgctgg 240

attccaagct ccgtgacgac cttgagcgac tcaagaagat cagggcccac agaggcatga 300

ggcactactg gggtttgagg gtgcgtggtc aa 332

<210> 15
<211> 440
<212> DNA
<213> Lygus hesperus

<400> 15
gtgagttctt ctgttgatta gtttttcctt ccctgaaatt atttcgttga agttaatttg 60

gattaccctg aaagaatccg ctgctttttc tctcgctaaa aatcttttac acccgtcacc 120

acggccccct gtgggcaggc acaagctgaa gcacctgccc gtgcacccta actcgcactt 180

catggacgtc aactgccctg ggtgttataa aatcccaacg gtgttctccc ccgcccagaa 240

cgacttcggc tgctggacct gttccaccat cctctgcctg cccacagggg gccgtgccga 300

cctcaccaaa agatgctcgt ttaggagaaa tcaacattat tattcttggt gggaacactt 360

attttttttg taattaaatt tcaaactaca aaataacttt tccgaaaaac actacaaaaa 420

aaattaaaaa caaaaaaaaa 440

<210> 16
<211> 324
<212> DNA
<213> Lygus hesperus

<400> 16
cttccctgaa attatttcgt tgaagttaat ttggattacc ctgaaagaat ccgctgcttt 60

ttctctcgct aaaaatcttt tacacccgtc accacggccc cctgtgggca ggcacaagct 120

gaagcacctg cccgtgcacc ctaactcgca cttcatggac gtcaactgcc ctgggtgtta 180

taaaatccca acggtgttct cccccgccca gaacgacttc ggctgctgga cctgttccac 240

catcctctgc ctgcccacag ggggccgtgc cgacctcacc aaaagatgct cgtttaggag 300

aaatcaacat tattattctt ggtg 324

<210> 17
<211> 357
<212> DNA
<213> Lygus hesperus

<400> 17
atgggttcaa gagagttaaa gccaagaggg ccaagaagga cgacggtgag atatttgccg 60

ctaaaaagga agtctacaag ccctctgagc agaggaaagc agaccagaaa aacattgaca 120

aacagaccct gaaagccatc aagcgactca agggagacgc ttgcctcatg aggaaatacc 180

tttgcaccat gttcggattc aggagcagtc aatatcccca ccgtatgaag ttttaatatg 240

ttttcagcca ataaataagt gaaagtttct cttttttatt actacagact caaattttta 300

ttttctgaaa attattaaaa attcttaatg gcaaaaaaaa aaaaaaaaaa aaaaaaa 357

<210> 18
<211> 223
<212> DNA
<213> Lygus hesperus

<400> 18
gttcaagaga gttaaagcca agagggccaa gaaggacgac ggtgagatat ttgccgctaa 60

aaaggaagtc tacaagccct ctgagcagag gaaagcagac cagaaaaaca ttgacaaaca 120

gaccctgaaa gccatcaagc gactcaaggg agacgcttgc ctcatgagga aatacctttg 180

caccatgttc ggattcagga gcagtcaata tccccaccgt atg 223

<210>  19
<211>  632
<212>  DNA
<213>  Lygus hesperus

<400>  19
atgggacctt ttttccgtgt gtctggctta ggcctcgcgt gttcttgtat ttttacggga      60

aatttagtga aaaagtgtaa atttaacgcg taaaaatggg tcgtatgcac gcacctggta     120

agggtatttc ccagtcagct ctcccctatc gtcgtagcgt cccaacatgg ctgaagctca     180

ctcctgacga cgtcaaggat cagattttca aactcaccaa gaaaggactg actccatctc     240

agatcggtgt catcctcagg gattctcacg gtgtggctca agtcagattc gtcaccgggt     300

cgaagatcct caggatcatg aaagccatcg gcctcgctcc tgacctccca gaggacctct     360

acttcctcat caaaaaagcc gttgctatca ggaaacatct tgaaagaaat aggaaagaca     420

aagactctaa attcggactt atccccgtcg agtccaggat ccacaggttg gcaagatact     480

acaaaaccaa gggcaccctt ccacccacct ggaaatacga gtccagcacc gcctctgctc     540

tggtggcttg aatattcaac tttttatttg tctactgttt aattaatata atgtgattta     600

gcaaaaaaaa aaaaaaaaaa aaaaaaaaaa aa                                   632


<210>  20
<211>  457
<212>  DNA
<213>  Lygus hesperus

<400>  20
gggtcgtatg cacgcacctg gtaagggtat ttcccagtca gctctcccct atcgtcgtag      60

cgtcccaaca tggctgaagc tcactcctga cgacgtcaag gatcagattt tcaaactcac     120

caagaaagga ctgactccat ctcagatcgg tgtcatcctc agggattctc acggtgtggc     180

tcaagtcaga ttcgtcaccg ggtcgaagat cctcaggatc atgaaagcca tcggcctcgc     240

tcctgacctc ccagaggacc tctacttcct catcaaaaaa gccgttgcta tcaggaaaca     300

tcttgaaaga aataggaaag acaaagactc taaattcgga cttatccccg tcgagtccag     360

gatccacagg ttggcaagat actacaaaac caagggcacc cttccaccca cctggaaata     420

cgagtccagc accgcctctg ctctggtggc ttgaata                              457


<210>  21
<211>  407
<212>  DNA
<213>  Lygus hesperus

<400>  21
atgggaccgt ttgcctcaca atccagaaca gacaggctgc catatccgtc gtcccctctg      60

cagcctccct cgtaatcaag gccctcaaag agcccccgag ggacaggaag aagaacaaga     120

acatcaaaca cgacggtaac ctgagtatgg atgacattct cggaattgcc aaaaccatga 180

ggccgaggtc gatgtccagg aaactggaag gaaccgtcaa ggaaatcctt gggacagctc 240

agtctgtcgg atgcacgatc gaaggccgag ctccccacga cgtcatcgac tccatcaaca 300

acggcgaaat ggaaatccct gacgaataaa ctgttcatga gtttatggat tttatataaa 360

aaataaaaag ttgaaaaatc caaaaaaaaa aaaaaaaaag aaaaaaa 407

<210> 22
<211> 302
<212> DNA
<213> Lygus hesperus

<400> 22
accgtttgcc tcacaatcca gaacagacag gctgccatat ccgtcgtccc ctctgcagcc 60

tccctcgtaa tcaaggccct caaagagccc ccgagggaca ggaagaagaa caagaacatc 120

aaacacgacg gtaacctgag tatggatgac attctcggaa ttgccaaaac catgaggccg 180

aggtcgatgt ccaggaaact ggaaggaacc gtcaaggaaa tccttgggac agctcagtct 240

gtcggatgca cgatcgaagg ccgagctccc cacgacgtca tcgactccat caacaacggc 300

ga 302

<210> 23
<211> 794
<212> DNA
<213> Lygus hesperus

<400> 23
catggggagt caatttggat ctatcgccag atgaagatgt ctcctgccgt gttcgctgtt 60

ctgctggtac tttcagcttc ccaggtcttg ggagatgatg catccaagtt ccaacacgag 120

gaaatcatgg aagtcctcag ctcggtcaac aaaaccgtca acaaattgta cgacttgatg 180

tccacgcaga aggaaagaga tattgacttt atcgagaaga aaatggatga gacgtaccag 240

caactcagga acaagaggga ggcgccggct gagaaccctg aagccattga caagatccaa 300

aacgcgttca aaagctttca agacggcgtc aaggacttcg tcaagtccgc ttcttcctcg 360

gacctctaca agaaggttca ggaaatcggc gaggacctgt agaacaaagg caaagagctc 420

ggagagaagc tgcaagaaac catcaataac gccagaacga aaaactcaga cgagaagaag 480

gactaaactg aggattttga ctctgcacaa acgcccgttg gtgtttaaac gtatttctta 540

cgtttattat catcggggtt catgaaatca aaaatacacc atcgcatacc acctcgaaaa 600

gaacataata tatgtgaaaa gacaagaaaa ggtgttcaat tgtgtcttta actggtggtt 660

atcacgattc acatgaaata ctactaagaa aacccaaaaa ccgtcatgaa acccgaagta 720

tgcttctgta ttacctaatt gtgctgataa ttcttaataa aatattatac tgagaaaaaa 780

aaaaaaaaaa aaaa 794

<210>  24
<211>  278
<212>  DNA
<213>  Lygus hesperus

<400>  24
ttctgctggt actttcagct tcccaggtct tgggagatga tgcatccaag ttccaacacg      60

aggaaatcat ggaagtcctc agctcggtca acaaaaccgt caacaaattg tacgacttga     120

tgtccacgca gaaggaaaga gatattgact ttatcgagaa gaaaatggat gagacgtacc     180

agcaactcag gaacaagagg gaggcgccgg ctgagaaccc tgaagccatt gacaagatcc     240

aaaacgcgtt caaaagcttt caagacggcg tcaaggac                             278


<210>  25
<211>  437
<212>  DNA
<213>  Lygus hesperus

<400>  25
atgggatcca ataataacca ttaaggcaat tggacatcaa tgatactgaa catatgaata      60

ttcagatatc aaaaatatcg aaatagaatc atatataaaa ccaactaacg cattagaaaa     120

taacgaattc cgattacttg aagtagacaa tcgaatcgta ttacctataa aatcaactat     180

ccgaattcta gttacatcat ctgatgtaat tcattcatga accatcccaa gtttgggaat     240

caaaattgat ggcacaccag gacgattaaa tcaagggaga ataaacataa accgaccagg     300

actaatatat gggcaatgtt ctgaaatttg tggagcaaac cacagattta taccaatcgt     360

aattgaaaga gtttcaatta atcaatttat aaactgatta aattcaaaat aaaaaaaaaa     420

aaaaaaaaaa aaaaaaa                                                    437


<210>  26
<211>  327
<212>  DNA
<213>  Lygus hesperus

<400>  26
aacgcagagt acatgggatc caataataac cattaaggca attggacatc aatgatactg      60

aacatatgaa tattcagata tcaaaaatat cgaaatagaa tcatatataa aaccaactaa     120

cgcattagaa aataacgaat tccgattact tgaagtagac aatcgaatcg tattacctat     180

aaaatcaact atccgaattc tagttacatc atctgatgta attcattcat gaaccatccc     240

aagtttggga atcaaaattg atggcacacc aggacgatta aatcaaggga gaataaacat     300

aaaccgacca ggactaatat atgggca                                         327


<210>  27
<211>  42

<212>  DNA
<213>  Artificial

<220>
<223>  Primer

<400>  27
gcgtaatacg actcactata ggcaaacagg ccgaaatcga ga                          42


<210>  28
<211>  23
<212>  DNA
<213>  Artificial

<220>
<223>  Primer

<400>  28
tgacgacctt gagttggttt ctg                                               23


<210>  29
<211>  20
<212>  DNA
<213>  Artificial

<220>
<223>  Primer

<400>  29
caaacaggcc gaaatcgaga                                                   20


<210>  30
<211>  45
<212>  DNA
<213>  Artificial

<220>
<223>  Primer

<400>  30
gcgtaatacg actcactata ggtgacgacc ttgagttggt ttctg                       45


<210>  31
<211>  42
<212>  DNA
<213>  Artificial

<220>
<223>  Primer

<400>  31
gcgtaatacg actcactata gggggcatca tgtcgaaagc tg                          42


<210>  32
<211>  20
<212>  DNA
<213>  Artificial

<220>
<223>  Primer

<400>  32
cgaagcccat gtcatcatcg                                                   20


<210>  33
<211>  20
<212>  DNA
<213>  Artificial

<220>
<223>  Primer

<400>  33
gggcatcatg tcgaaagctg                                                   20


<210>  34
<211>  42
<212>  DNA
<213>  Artificial

<220>
<223>  Primer

<400>  34
gcgtaatacg actcactata ggcgaagccc atgtcatcat cg                          42


<210>  35
<211>  44
<212>  DNA
<213>  Artificial

<220>
<223>  Primer

<400>  35
gcgtaatacg actcactata gggggcaggt cttctccata acca                        44


<210>  36
<211>  20
<212>  DNA
<213>  Artificial

<220>
<223>  Primer

<400>  36
gattttgcgg tgccaacgac                                                   20


<210>  37
<211>  22
<212>  DNA
<213>  Artificial

<220>
<223>  Primer

<400>  37
gggcaggtct tctccataac ca                                                22


<210>  38
<211>  42
<212>  DNA
<213>  Artificial

<220>
<223>  Primer

<400>  38
gcgtaatacg actcactata gggattttgc ggtgccaacg ac                          42


<210>  39
<211>  44
<212>  DNA
<213>  Artificial

<220>
<223>  Primer

<400>  39
gcgtaatacg actcactata ggattgcaca agctgaatcc ctcc                        44


<210>  40
<211>  20
<212>  DNA
<213>  Artificial

<220>
<223>  Primer

<400>  40
attcacaaag cgggtgctgg                                                   20


<210>  41
<211>  22
<212>  DNA
<213>  Artificial

<220>
<223>  Primer

<400>  41
attgcacaag ctgaatccct cc                                                22


<210>  42
<211>  42
<212>  DNA
<213>  Artificial

<220>
<223>  Primer

<400>  42
gcgtaatacg actcactata ggattcacaa agcgggtgct gg                          42

<210>  43
<211>  44
<212>  DNA
<213>  Artificial

<220>
<223>  Primer

<400>  43
gcgtaatacg actcactata ggccctctac gagtgcatca atgg                        44


<210>  44
<211>  20
<212>  DNA
<213>  Artificial

<220>
<223>  Primer

<400>  44
cccatcgtgg atttgacgtg                                                   20


<210>  45
<211>  22
<212>  DNA
<213>  Artificial

<220>
<223>  Primer

<400>  45
ccctctacga gtgcatcaat gg                                                22


<210>  46
<211>  42
<212>  DNA
<213>  Artificial

<220>
<223>  Primer

<400>  46
gcgtaatacg actcactata ggcccatcgt ggatttgacg tg                          42


<210>  47
<211>  47
<212>  DNA
<213>  Artificial

<220>
<223>  Primer

<400>  47
gcgtaatacg actcactata ggccaataaa gatcaacttt cccagag                     47


<210>  48
<211>  25

<212>  DNA
<213>  Artificial

<220>
<223>  Primer

<400>  48
ttagacagac tcttgaactg gaggc                                          25


<210>  49
<211>  25
<212>  DNA
<213>  Artificial

<220>
<223>  Primer

<400>  49
ccaataaaga tcaactttcc cagag                                          25


<210>  50
<211>  47
<212>  DNA
<213>  Artificial

<220>
<223>  Primer

<400>  50
gcgtaatacg actcactata ggttagacag actcttgaac tggaggc                  47


<210>  51
<211>  44
<212>  DNA
<213>  Artificial

<220>
<223>  Primer

<400>  51
gcgtaatacg actcactata gggaaaggtg atgttcgcca tgac                     44


<210>  52
<211>  20
<212>  DNA
<213>  Artificial

<220>
<223>  Primer

<400>  52
ttgaccacgc accctcaaac                                                20


<210>  53
<211>  22
<212>  DNA
<213>  Artificial

<220>
<223>  Primer

<400>  53
gaaaggtgat gttcgccatg ac                                                22


<210>  54
<211>  42
<212>  DNA
<213>  Artificial

<220>
<223>  Primer

<400>  54
gcgtaatacg actcactata ggttgaccac gcaccctcaa ac                          42


<210>  55
<211>  47
<212>  DNA
<213>  Artificial

<220>
<223>  Primer

<400>  55
gcgtaatacg actcactata ggcttccctg aaattatttc gttgaag                     47


<210>  56
<211>  28
<212>  DNA
<213>  Artificial

<220>
<223>  Primer

<400>  56
caccaagaat aataatgttg atttctcc                                          28


<210>  57
<211>  25
<212>  DNA
<213>  Artificial

<220>
<223>  Primer

<400>  57
cttccctgaa attatttcgt tgaag                                             25


<210>  58
<211>  50
<212>  DNA
<213>  Artificial

<220>
<223>  Primer

<400>  58
gcgtaatacg actcactata ggcaccaaga ataataatgt tgatttctcc                50

<210>  59
<211>  47
<212>  DNA
<213>  Artificial

<220>
<223>  Primer

<400>  59
gcgtaatacg actcactata gggttcaaga gagttaaagc caagagg                   47

<210>  60
<211>  22
<212>  DNA
<213>  Artificial

<220>
<223>  Primer

<400>  60
catacggtgg ggatattgac tg                                              22

<210>  61
<211>  25
<212>  DNA
<213>  Artificial

<220>
<223>  Primer

<400>  61
gttcaagaga gttaaagcca agagg                                           25

<210>  62
<211>  44
<212>  DNA
<213>  Artificial

<220>
<223>  Primer

<400>  62
gcgtaatacg actcactata ggcatacggt ggggatattg actg                      44

<210>  63
<211>  42
<212>  DNA
<213>  Artificial

<220>
<223>  Primer

<400>  63
gcgtaatacg actcactata gggggtcgta tgcacgcacc tg                        42

<210>  64
<211>  23
<212>  DNA
<213>  Artificial

<220>
<223>  Primer

<400>  64
tattcaagcc accagagcag agg                                               23


<210>  65
<211>  20
<212>  DNA
<213>  Artificial

<220>
<223>  Primer

<400>  65
gggtcgtatg cacgcacctg                                                   20


<210>  66
<211>  45
<212>  DNA
<213>  Artificial

<220>
<223>  Primer

<400>  66
gcgtaatacg actcactata ggtattcaag ccaccagagc agagg                       45


<210>  67
<211>  42
<212>  DNA
<213>  Artificial

<220>
<223>  Primer

<400>  67
gcgtaatacg actcactata ggaccgtttg cctcacaatc ca                          42


<210>  68
<211>  20
<212>  DNA
<213>  Artificial

<220>
<223>  Primer

<400>  68
tcgccgttgt tgatggagtc                                                   20


<210>  69
<211>  20

<212>  DNA
<213>  Artificial

<220>
<223>  Primer

<400>  69
accgtttgcc tcacaatcca                                                    20


<210>  70
<211>  42
<212>  DNA
<213>  Artificial

<220>
<223>  Primer

<400>  70
gcgtaatacg actcactata ggtcgccgtt gttgatggag tc                           42


<210>  71
<211>  45
<212>  DNA
<213>  Artificial

<220>
<223>  Primer

<400>  71
gcgtaatacg actcactata ggggtatcaa cgcagagtac atggg                        45


<210>  72
<211>  20
<212>  DNA
<213>  Artificial

<220>
<223>  Primer

<400>  72
gtccttgacg ccgtcttgaa                                                    20


<210>  73
<211>  23
<212>  DNA
<213>  Artificial

<220>
<223>  Primer

<400>  73
ggtatcaacg cagagtacat ggg                                                23


<210>  74
<211>  42
<212>  DNA
<213>  Artificial

<220>
<223>  Primer

<400>  74
gcgtaatacg actcactata gggtccttga cgccgtcttg aa                          42


<210>  75
<211>  44
<212>  DNA
<213>  Artificial

<220>
<223>  Primer

<400>  75
gcgtaatacg actcactata ggtgcccata tattagtcct ggtc                        44


<210>  76
<211>  20
<212>  DNA
<213>  Artificial

<220>
<223>  Primer

<400>  76
aacgcagagt acatgggatc                                                   20


<210>  77
<211>  22
<212>  DNA
<213>  Artificial

<220>
<223>  Primer

<400>  77
tgcccatata ttagtcctgg tc                                                22


<210>  78
<211>  42
<212>  DNA
<213>  Artificial

<220>
<223>  Primer

<400>  78
gcgtaatacg actcactata ggaacgcaga gtacatggga tc                          42


<210>  79
<211>  197
<212>  PRT
<213>  Lygus hesperus

<400>  79

Met Ala Asp Asp Glu Ala Lys Lys Ala Lys Gln Ala Glu Ile Glu Arg

1               5                   10                  15

Lys Arg Ala Glu Val Arg Lys Arg Met Glu Glu Ala Ser Lys Ala Lys
            20                  25                  30

Lys Ala Lys Lys Gly Phe Met Thr Pro Glu Arg Lys Lys Lys Leu Arg
        35                  40                  45

Leu Leu Leu Arg Lys Lys Ala Ala Glu Glu Leu Lys Lys Glu Gln Glu
    50                  55                  60

Arg Lys Ala Ala Glu Arg Arg Arg Thr Ile Glu Glu Arg Cys Gly Gln
65                  70                  75                  80

Ile Ala Asp Val Asp Asn Ala Asn Glu Ala Thr Leu Lys Lys Leu Cys
                85                  90                  95

Thr Asp Tyr His Lys Arg Ile Asp Ala Leu Glu Arg Ser Lys Ile Asp
            100                 105                 110

Ile Glu Phe Glu Val Glu Arg Arg Asp Leu Glu Ile Ala Asp Leu Asn
        115                 120                 125

Ser Gln Val Asn Asp Leu Arg Gly Lys Phe Val Lys Pro Thr Leu Lys
    130                 135                 140

Lys Val Ser Lys Tyr Glu Asn Lys Phe Ala Lys Leu Gln Lys Lys Ala
145                 150                 155                 160

Ala Glu Phe Asn Phe Arg Asn Gln Leu Lys Val Val Lys Lys Lys Glu
                165                 170                 175

Phe Thr Leu Glu Glu Glu Asp Lys Glu Pro Lys Lys Ser Glu Lys Ala
            180                 185                 190

Glu Trp Gln Lys Lys
        195

<210>  80
<211>  115
<212>  PRT
<213>  Lygus hesperus

<400>  80

Met Gly Ile Met Ser Lys Ala Glu Leu Ala Cys Val Tyr Ser Ala Leu
1               5                   10                  15

Ile Leu Ile Asp Asp Asp Val Ala Val Thr Gly Glu Lys Ile Gln Thr

20                  25                  30

Ile Leu Lys Ala Ala Ser Val Asp Ile Glu Pro Tyr Trp Pro Gly Leu
        35                  40                  45

Phe Ala Lys Ala Leu Glu Gly Ile Asn Pro Lys Asp Leu Ile Ser Ser
    50                  55                  60

Ile Gly Ser Gly Val Gly Ala Gly Ala Pro Ala Val Gly Gly Ala Ala
65                  70                  75                  80

Pro Ala Ala Ala Ala Ala Pro Ala Ala Glu Ala Lys Lys Glu Glu Lys
                85                  90                  95

Lys Lys Val Glu Ser Asp Pro Glu Ser Asp Asp Asp Met Gly Phe Gly
            100                 105                 110

Leu Phe Asp
        115

<210>  81
<211>  121
<212>  PRT
<213>  Lygus hesperus

<400>  81

Met Ile Pro Pro Thr Ser Arg Pro Gln Val Thr Val Tyr Ser Asp Lys
1               5                   10                  15

Asn Glu Ala Thr Gly Thr Leu Leu Asn Leu Pro Ala Val Phe Asn Ala
            20                  25                  30

Pro Ile Arg Pro Asp Val Val Asn Phe Val His Gln Asn Val Ala Lys
        35                  40                  45

Asn His Arg Gln Pro Tyr Cys Val Ser Ala Gln Ala Gly His Gln Thr
    50                  55                  60

Ser Ala Glu Ser Trp Gly Thr Gly Arg Ala Val Ala Arg Ile Pro Arg
65                  70                  75                  80

Val Arg Gly Gly Gly Thr His Arg Ser Gly Gln Gly Ala Phe Gly Asn
                85                  90                  95

Met Cys Arg Gly Gly Arg Met Phe Ala Pro Thr Arg Pro Trp Arg Arg
            100                 105                 110

Trp His Arg Lys Ile Asn Val Asn Gln

115                 120

<210>  82
<211>  133
<212>  PRT
<213>  Lygus hesperus

<400>  82

Trp Asp Leu Tyr Ala Glu Lys Val Ala Thr Arg Gly Leu Cys Ala Ile
1               5                   10                  15

Ala Gln Ala Glu Ser Leu Arg Tyr Lys Leu Ile Gly Gly Leu Ala Val
            20                  25                  30

Arg Gly Ala Cys Tyr Gly Val Leu Arg Phe Ile Met Glu Asn Gly Ala
        35                  40                  45

Lys Gly Cys Glu Val Val Val Ser Gly Lys Leu Arg Gly Gln Arg Ala
    50                  55                  60

Lys Ser Met Lys Phe Val Asp Gly Leu Met Ile His Ser Gly Asp Pro
65                  70                  75                  80

Cys Asn Glu Tyr Val Asp Thr Ala Thr Arg His Val Leu Leu Arg Gln
                85                  90                  95

Gly Val Leu Gly Ile Lys Val Lys Ile Met Leu Pro Trp Asp Val Thr
            100                 105                 110

Gly Lys Asn Gly Pro Lys Asn Pro Leu Pro Asp His Val Ser Val Leu
        115                 120                 125

Leu Pro Lys Glu Glu
    130

<210>  83
<211>  217
<212>  PRT
<213>  Lygus hesperus

<400>  83

Met Thr Ser Lys Val Ser Arg Glu Thr Leu Tyr Glu Cys Ile Asn Gly
1               5                   10                  15

Val Ile Gln Ser Ser Gln Glu Lys Lys Arg Asn Phe Val Glu Thr Val
            20                  25                  30

Glu Ile Gln Ile Gly Leu Lys Asn Tyr Asp Pro Gln Lys Asp Lys Arg
        35                  40                  45

Phe Ser Gly Thr Val Lys Leu Lys His Ile Pro Arg Pro Lys Met Gln
    50                  55                  60

Val Cys Ile Leu Gly Asp Gln Gln His Cys Asp Glu Ala Lys Ala Asn
65                  70                  75                  80

Asn Val Pro Tyr Met Asp Val Glu Ala Leu Lys Lys Leu Asn Lys Asn
                85                  90                  95

Lys Lys Leu Val Lys Lys Leu Ala Lys Lys Tyr Asp Ala Phe Leu Ala
            100                 105                 110

Ser Glu Ala Leu Ile Lys Gln Ile Pro Arg Leu Leu Gly Pro Gly Leu
        115                 120                 125

Asn Lys Ala Gly Lys Phe Pro Gly Leu Leu Ser His Gln Glu Ser Met
    130                 135                 140

Met Met Lys Ile Asp Glu Val Lys Ala Thr Ile Lys Phe Gln Met Lys
145                 150                 155                 160

Lys Val Leu Cys Leu Ser Val Ala Val Gly His Val Gly Met Thr Ala
                165                 170                 175

Asp Glu Leu Val Gln Asn Val His Leu Ser Val Asn Phe Leu Val Ser
            180                 185                 190

Leu Leu Lys Lys His Trp Gln Asn Val Arg Ser Leu His Val Lys Ser
        195                 200                 205

Thr Met Gly Pro Pro Gln Arg Leu Tyr
    210                 215

<210>  84
<211>  118
<212>  PRT
<213>  Lygus hesperus

<400>  84

Gly Thr Asn Lys Asp Gln Leu Ser Gln Arg Lys Thr Cys Tyr Ala Gln
1               5                   10                  15

His Asn Gln Val Arg Glu Ile Arg Lys Lys Met Val Lys Asn Ile Ser
            20                  25                  30

Asp Ser Ile Ser Ser Cys Asp Leu Arg Ser Val Val Asn Lys Leu Ile
        35                  40                  45

Pro Asp Ser Ile Ala Lys Asp Ile Glu Lys Asn Cys Gln Gly Ile Tyr
    50                  55                  60

Pro Leu His Asp Val Tyr Ile Arg Lys Val Lys Val Leu Lys Lys Pro
65                  70                  75                  80

Arg Phe Glu Leu Ser Lys Leu Leu Glu Leu His Val Asp Gly Lys Gly
                85                  90                  95

Ile Asp Glu Pro Gly Ala Lys Val Thr Arg Thr Asp Ala Tyr Glu Pro
            100                 105                 110

Pro Val Gln Glu Ser Val
        115

<210> 85
<211> 128
<212> PRT
<213> Lygus hesperus

<400> 85

Lys Val Met Phe Ala Met Thr Ala Ile Lys Gly Val Gly Arg Arg Tyr
1               5                   10                  15

Ala Asn Ile Val Leu Lys Lys Ala Asp Val Asn Leu Asp Lys Arg Ala
            20                  25                  30

Gly Glu Cys Ser Glu Glu Glu Val Glu Lys Ile Val Thr Ile Met Gln
        35                  40                  45

Asn Pro Arg Gln Tyr Lys Ile Pro Asn Trp Phe Leu Asn Arg Gln Lys
    50                  55                  60

Asp Thr Val Glu Gly Lys Tyr Ser Gln Leu Thr Ser Ser Leu Leu Asp
65                  70                  75                  80

Ser Lys Leu Arg Asp Asp Leu Glu Arg Leu Lys Lys Ile Arg Ala His
                85                  90                  95

Arg Gly Met Arg His Tyr Trp Gly Leu Arg Val Arg Gly Gln His Thr
            100                 105                 110

Lys Thr Thr Gly Arg Arg Gly Arg Thr Val Gly Val Ser Lys Lys Lys
        115                 120                 125

<210> 86
<211> 122

<212>  PRT
<213>  Lygus hesperus

<400>  86

Val Leu Leu Leu Ile Ser Phe Ser Phe Pro Glu Ile Ile Ser Leu Lys
1               5                   10                  15

Leu Ile Trp Ile Thr Leu Lys Glu Ser Ala Ala Phe Ser Leu Ala Lys
            20                  25                  30

Asn Leu Leu His Pro Ser Pro Arg Pro Pro Val Gly Arg His Lys Leu
        35                  40                  45

Lys His Leu Pro Val His Pro Asn Ser His Phe Met Asp Val Asn Cys
    50                  55                  60

Pro Gly Cys Tyr Lys Ile Pro Thr Val Phe Ser Pro Ala Gln Asn Asp
65                  70                  75                  80

Phe Gly Cys Trp Thr Cys Ser Thr Ile Leu Cys Leu Pro Thr Gly Gly
                85                  90                  95

Arg Ala Asp Leu Thr Lys Arg Cys Ser Phe Arg Arg Asn Gln His Tyr
            100                 105                 110

Tyr Ser Trp Trp Glu His Leu Phe Phe Leu
        115                 120


<210>  87
<211>  77
<212>  PRT
<213>  Lygus hesperus

<400>  87

Gly Phe Lys Arg Val Lys Ala Lys Arg Ala Lys Lys Asp Asp Gly Glu
1               5                   10                  15

Ile Phe Ala Ala Lys Lys Glu Val Tyr Lys Pro Ser Glu Gln Arg Lys
            20                  25                  30

Ala Asp Gln Lys Asn Ile Asp Lys Gln Thr Leu Lys Ala Ile Lys Arg
        35                  40                  45

Leu Lys Gly Asp Ala Cys Leu Met Arg Lys Tyr Leu Cys Thr Met Phe
    50                  55                  60

Gly Phe Arg Ser Ser Gln Tyr Pro His Arg Met Lys Phe
65                  70                  75

<210>  88
<211>  151
<212>  PRT
<213>  Lygus hesperus

<400>  88

Met Gly Arg Met His Ala Pro Gly Lys Gly Ile Ser Gln Ser Ala Leu
1               5                   10                  15

Pro Tyr Arg Arg Ser Val Pro Thr Trp Leu Lys Leu Thr Pro Asp Asp
            20                  25                  30

Val Lys Asp Gln Ile Phe Lys Leu Thr Lys Lys Gly Leu Thr Pro Ser
        35                  40                  45

Gln Ile Gly Val Ile Leu Arg Asp Ser His Gly Val Ala Gln Val Arg
    50                  55                  60

Phe Val Thr Gly Ser Lys Ile Leu Arg Ile Met Lys Ala Ile Gly Leu
65                  70                  75                  80

Ala Pro Asp Leu Pro Glu Asp Leu Tyr Phe Leu Ile Lys Lys Ala Val
                85                  90                  95

Ala Ile Arg Lys His Leu Glu Arg Asn Arg Lys Asp Lys Asp Ser Lys
            100                 105                 110

Phe Gly Leu Ile Pro Val Glu Ser Arg Ile His Arg Leu Ala Arg Tyr
        115                 120                 125

Tyr Lys Thr Lys Gly Thr Leu Pro Pro Thr Trp Lys Tyr Glu Ser Ser
    130                 135                 140

Thr Ala Ser Ala Leu Val Ala
145                 150

<210>  89
<211>  108
<212>  PRT
<213>  Lygus hesperus

<400>  89

Gly Thr Val Cys Leu Thr Ile Gln Asn Arg Gln Ala Ala Ile Ser Val
1               5                   10                  15

Val Pro Ser Ala Ala Ser Leu Val Ile Lys Ala Leu Lys Glu Pro Pro
            20                  25                  30

Arg Asp Arg Lys Lys Asn Lys Asn Ile Lys His Asp Gly Asn Leu Ser
        35                  40                  45

Met Asp Asp Ile Leu Gly Ile Ala Lys Thr Met Arg Pro Arg Ser Met
    50                  55                  60

Ser Arg Lys Leu Glu Gly Thr Val Lys Glu Ile Leu Gly Thr Ala Gln
65                  70                  75                  80

Ser Val Gly Cys Thr Ile Glu Gly Arg Ala Pro His Asp Val Ile Asp
                85                  90                  95

Ser Ile Asn Asn Gly Glu Met Glu Ile Pro Asp Glu
            100                 105

<210>  90
<211>  133
<212>  PRT
<213>  Lygus hesperus

<400>  90

His Gly Glu Ser Ile Trp Ile Tyr Arg Gln Met Lys Met Ser Pro Ala
1               5                   10                  15

Val Phe Ala Val Leu Leu Val Leu Ser Ala Ser Gln Val Leu Gly Asp
            20                  25                  30

Asp Ala Ser Lys Phe Gln His Glu Glu Ile Met Glu Val Leu Ser Ser
        35                  40                  45

Val Asn Lys Thr Val Asn Lys Leu Tyr Asp Leu Met Ser Thr Gln Lys
    50                  55                  60

Glu Arg Asp Ile Asp Phe Ile Glu Lys Lys Met Asp Glu Thr Tyr Gln
65                  70                  75                  80

Gln Leu Arg Asn Lys Arg Glu Ala Pro Ala Glu Asn Pro Glu Ala Ile
                85                  90                  95

Asp Lys Ile Gln Asn Ala Phe Lys Ser Phe Gln Asp Gly Val Lys Asp
            100                 105                 110

Phe Val Lys Ser Ala Ser Ser Ser Asp Leu Tyr Lys Lys Val Gln Glu
        115                 120                 125

Ile Gly Glu Asp Leu
    130

<210>  91
<211>  118
<212>  PRT
<213>  Lygus hesperus

<400>  91

Thr Tyr Glu Tyr Ser Asp Ile Lys Asn Ile Glu Ile Glu Ser Tyr Ile
1               5                   10                  15

Lys Pro Thr Asn Ala Leu Glu Asn Asn Glu Phe Arg Leu Leu Glu Val
            20                  25                  30

Asp Asn Arg Ile Val Leu Pro Ile Lys Ser Thr Ile Arg Ile Leu Val
        35                  40                  45

Thr Ser Ser Asp Val Ile His Ser Thr Ile Pro Ser Leu Gly Ile Lys
    50                  55                  60

Ile Asp Gly Thr Pro Gly Arg Leu Asn Gln Gly Arg Ile Asn Ile Asn
65                  70                  75                  80

Arg Pro Gly Leu Ile Tyr Gly Gln Cys Ser Glu Ile Cys Gly Ala Asn
                85                  90                  95

His Arg Phe Ile Pro Ile Val Ile Glu Arg Val Ser Ile Asn Gln Phe
            100                 105                 110

Ile Asn Leu Asn Ser Lys
        115


<210>  92
<211>  20
<212>  DNA
<213>  Artificial

<220>
<223>  Adaptor

<400>  92
aagcagtggt atcaacgcag                                                   20


<210>  93
<211>  20
<212>  DNA
<213>  Artificial

<220>
<223>  Primer

<400>  93
aagcagtggt atcaacgcag                                                   20

<210> 94
<211> 42
<212> DNA
<213> Artificial

<220>
<223> Primer

<400> 94
gcgtaatacg actcactata ggaagcagtg gtatcaacgc ag 42


<210> 95
<211> 717
<212> DNA
<213> Lygus hesperus

<400> 95
aaagtgtggt tctcttcgtc cgaccatgag ttcgctcaaa ctgcagaaga ggctcgccgc 60

ctcggtgatg agatgcggca agaagaaagt gtggttggac cctaatgaaa tcaacgaaat 120

cgccaacacc aactctaggc aaaacatccg taagctgatc aaggatggtt tgatcatcaa 180

aaagcctgtg gctgtccact ccagagcccg cgtccgtaaa aacacagaag ccagacggaa 240

gggtcgtcat tgtggcttcg gtaagaggaa gggtaccgcc aacgccagaa tgcctgtgaa 300

ggtcctgtgg gtcaacagaa tgagagtcct gcgacggctc cttaaaaaat acagagaagc 360

caagaagatc gataggcaaa tgtaccacga cctttacatg aaagccaaag gtaacgtctt 420

caaaaacaag agggtactga tggacttcat tcacaagaag aaggctgaaa aggcgagatc 480

aaagatgttg aaggaccagg cagaggcgag acgtttcaag gtcaaggagg cgaagaagag 540

gcgcgaggag aggatcgcca ccaagaagca agagatcatg caggcgtacg cccgagaaga 600

cgaggctgcc gtcaaaaagt gatctcgccc cctccgtttt taaattttaa acaaaaaacg 660

tattttgtac aaaaatttac aaaaaaaaaa aaaaaaaaaa aaaaaaaaaa aaaaaaa 717


<210> 96
<211> 2304
<212> DNA
<213> Lygus hesperus

<400> 96
atgacgacct acgaggagtt cattcaacag agcgaggagc gcgacggtat caggttcact 60

tggaacgtct ggccatcaag tcgcatcgaa gccaccaggt tggtcgtacc cgtaggatgt 120

ctctatcaac cactaaaaga acgcacggat cttccagcta ttcaatacga tcccgttcta 180

tgcactagga atacctgtag agccatactc aacccgatgt gccaagtaaa ctatagggca 240

aagttgtggg tgtgtaactt ctgtttccag aggaatccgt tcccaccaca atacgccgca 300

atttccgagc agcatcagcc tgctgagttg attccatcat tctcaactat agagtatact 360

atatctagag ctcaattttt gcctcctata ttcctattgg tggtggatac gtgtttggat 420

gatgacgagc taggagctct gaaagattcg ttacaaacgt ctctatcttt gctaccaacc 480
aactccctag ttggtctgat cacgtttggt aaaatggtcc aagttcacga acttgggtgt 540
gaaggttgtt cccggagcta cgtgttcaga ggcaccaagg atttgacgtc caagcaagta 600
caggacatgc ttgggatcgg aaaggtttcc gcttctcctc agcaacagca gcaaagggca 660
atgggcggtc agcagccatt ccccaccaat cggttcattc agccgattca aagttgtgac 720
atgagcctca ccgacttgtt gggcgaaatg cagcgtgatc catggccagt gggtcagggt 780
aagcgacctc ttagatcaac gggtgctgct ctagctattg ccattgggtt gttggagtgc 840
tcctacccca acacgggagc aaaagtcatg ttgttccttg gtggcccttg ttcccaaggg 900
cctggtcaag ttgtcaatga tgacctgagg gaacctatcc gctctcatca tgacatccag 960
aaagataatg cccgctacat gaaaaaagcc attaaacatt acgattcttt ggcattgaga 1020
gcagccacta atgggcattc agtagacatt tattcctgtg ctttagatca gacaggtttg 1080
gcggaaatga agcaatgttg caattctact gggggtcata tggtgatggg tgacaccttc 1140
aactccactt tgttcaaaca gacgttccag agggtgctct cccgtgatca aaaaggcgaa 1200
ttcaaaatgg ctttcaatgg cgtagttgaa gtcaaaacct cccgagagct aaaagttatg 1260
ggagccattg ggccttgcgt ttcattgaat acgaaaggtc cgtgtgttag tgaaactgac 1320
atagggcttg gaggaacttg ccagtggaag ttctgcacat ttaaccaaaa taccactgct 1380
gccatgttct ttgaggtagt aaaccaacac gctgctccta tccctcaagg tggaagagga 1440
tgtatacagt tcataactca ataccagcat gcgtcgggcc aaaggcgcat ccgagtaacc 1500
actgtagcca ggaattgggc tgatgcgact accaacatgc accatgttag tgcaggattt 1560
gatcaggaag ctggagcggt actcatggcc aggatggtcg ttcacagagc tgaaactgat 1620
gatggacctg atgtcatgag atgggctgat cgcatgttga ttcgtctttg ccagaaattc 1680
ggcgagtaca acaaggatga tccaaatagt ttccgcctcc cagaaaactt ctcgctttac 1740
ccacagttca tgtatcactt gagaaggtcc caattcttgc aggtattcaa caacagccca 1800
gacgaaacgt cgtactatcg tcacatcttg atgcgggaag atttgtcgca gagcttgatc 1860
atgattcagc cgatcctgta cagttacagt ttcaacggtc cagaaccagt ccttttggac 1920
acttccagca ttcaacctga tcggatcctg ctgatggaca ccttcttcca aatcctcatc 1980
ttccacggcg agaccatcgc ccagtggcgt gcccaaaggt accaggacct acctgaatat 2040
gagaacttca agcagctcct acaggctcct gtagacgatg ctaaggaaat cctgcacact 2100
cggttcccca tgccgaggta cattgacacc gaacagggcg gatcacaagc tagattcctt 2160
ctctccaaag tcaacccatc ccaaactcac aacaacatgt acggctatgg aggggaattt 2220
ggagcccctg tgctcactga tgatgtttcc ctccaagtct tcatggaaca ccttaaaaag 2280
ctagccgttt catttactgc ctag 2304

<210> 97
<211> 311
<212> DNA
<213> Artificial

<220>
<223> GUS

<400> 97
ccagcgtatc gtgctgcgtt tcgatgcggt cactcattac ggcaaagtgt gatggagcat 60

cagggcggct atacgccatt tgaagccgat gtcacgccgt atgttattgc cgggaaaagt 120

gtacgtatct gaaatcaaaa aactcgacgg cctgtgggca ttcagtctgg atcgcgaaaa 180

ctgtggaatt gatccagcgc cgtcgtcggt gaacaggtat ggaatttcgc cgattttgcg 240

acctcgcaag gcatattcgg gtgaaggtta tctctatgaa ctgtgcgtca cagccaaaag 300

ccagacagag t 311


<210> 98
<211> 170
<212> DNA
<213> Artificial

<220>
<223> Intron

<400> 98
ctcgagcctg agagaaaagc atgaagtata cccataacta acccattagt tatgcattta 60

tgttatatct attcatgctt ctactttaga taatcaatca ccaaacaatg agaatctcaa 120

cggtcgcaat aatgttcatg aaaatgtagt gtgtacactt accttctaga 170


<210> 99
<211> 198
<212> PRT
<213> Lygus hesperus

<400> 99

Met Ser Ser Leu Lys Leu Gln Lys Arg Leu Ala Ala Ser Val Met Arg
1               5                   10                  15


Cys Gly Lys Lys Lys Val Trp Leu Asp Pro Asn Glu Ile Asn Glu Ile
            20                  25                  30


Ala Asn Thr Asn Ser Arg Gln Asn Ile Arg Lys Leu Ile Lys Asp Gly
        35                  40                  45


Leu Ile Ile Lys Lys Pro Val Ala Val His Ser Arg Ala Arg Val Arg
    50                  55                  60

Lys Asn Thr Glu Ala Arg Arg Lys Gly Arg His Cys Gly Phe Gly Lys
65                  70                  75                  80

Arg Lys Gly Thr Ala Asn Ala Arg Met Pro Val Lys Val Leu Trp Val
                85                  90                  95

Asn Arg Met Arg Val Leu Arg Arg Leu Leu Lys Lys Tyr Arg Glu Ala
            100                 105                 110

Lys Lys Ile Asp Arg Gln Met Tyr His Asp Leu Tyr Met Lys Ala Lys
        115                 120                 125

Gly Asn Val Phe Lys Asn Lys Arg Val Leu Met Asp Phe Ile His Lys
    130                 135                 140

Lys Lys Ala Glu Lys Ala Arg Ser Lys Met Leu Lys Asp Gln Ala Glu
145                 150                 155                 160

Ala Arg Arg Phe Lys Val Lys Glu Ala Lys Lys Arg Arg Glu Glu Arg
                165                 170                 175

Ile Ala Thr Lys Lys Gln Glu Ile Met Gln Ala Tyr Ala Arg Glu Asp
            180                 185                 190

Glu Ala Ala Val Lys Lys
        195

<210>  100
<211>  767
<212>  PRT
<213>  Lygus hesperus

<400>  100
   Met Thr Thr Tyr Glu Glu Phe Ile Gln Gln Ser Glu Glu Arg Asp Gly
   1               5                   10                  15
   Ile Arg Phe Thr Trp Asn Val Trp Pro Ser Ser Arg Ile Glu Ala Thr
               20                  25                  30
   Arg Leu Val Val Pro Val Gly Cys Leu Tyr Gln Pro Leu Lys Glu Arg
           35                  40                  45
   Thr Asp Leu Pro Ala Ile Gln Tyr Asp Pro Val Leu Cys Thr Arg Asn
       50                  55                  60
   Thr Cys Arg Ala Ile Leu Asn Pro Met Cys Gln Val Asn Tyr Arg Ala
   65                  70                  75                  80
   Lys Leu Trp Val Cys Asn Phe Cys Phe Gln Arg Asn Pro Phe Pro Pro
                   85                  90                  95
   Gln Tyr Ala Ala Ile Ser Glu Gln His Gln Pro Ala Glu Leu Ile Pro
               100                 105                 110
   Ser Phe Ser Thr Ile Glu Tyr Thr Ile Ser Arg Ala Gln Phe Leu Pro
           115                 120                 125
   Pro Ile Phe Leu Leu Val Val Asp Thr Cys Leu Asp Asp Asp Glu Leu
       130                 135                 140
   Gly Ala Leu Lys Asp Ser Leu Gln Thr Ser Leu Ser Leu Leu Pro Thr
   145                 150                 155                 160
   Asn Ser Leu Val Gly Leu Ile Thr Phe Gly Lys Met Val Gln Val His
                   165                 170                 175
   Glu Leu Gly Cys Glu Gly Cys Ser Arg Ser Tyr Val Phe Arg Gly Thr
               180                 185                 190
   Lys Asp Leu Thr Ser Lys Gln Val Gln Asp Met Leu Gly Ile Gly Lys
           195                 200                 205
   Val Ser Ala Ser Pro Gln Gln Gln Gln Gln Arg Ala Met Gly Gly Gln
       210                 215                 220
   Gln Pro Phe Pro Thr Asn Arg Phe Ile Gln Pro Ile Gln Ser Cys Asp
   225                 230                 235                 240
   Met Ser Leu Thr Asp Leu Leu Gly Glu Met Gln Arg Asp Pro Trp Pro
                   245                 250                 255
   Val Gly Gln Gly Lys Arg Pro Leu Arg Ser Thr Gly Ala Ala Leu Ala
               260                 265                 270
   Ile Ala Ile Gly Leu Leu Glu Cys Ser Tyr Pro Asn Thr Gly Ala Lys
           275                 280                 285
   Val Met Leu Phe Leu Gly Gly Pro Cys Ser Gln Gly Pro Gly Gln Val
       290                 295                 300
   Val Asn Asp Asp Leu Arg Glu Pro Ile Arg Ser His His Asp Ile Gln
   305                 310                 315                 320
   Lys Asp Asn Ala Arg Tyr Met Lys Lys Ala Ile Lys His Tyr Asp Ser
                   325                 330                 335
   Leu Ala Leu Arg Ala Ala Thr Asn Gly His Ser Val Asp Ile Tyr Ser
   340 345 350
   Cys Ala Leu Asp Gln Thr Gly Leu Ala Glu Met Lys Gln Cys Cys Asn
   355 360 365
   Ser Thr Gly Gly His Met Val Met Gly Asp Thr Phe Asn Ser Thr Leu
   370 375 380
   Phe Lys Gln Thr Phe Gln Arg Val Leu Ser Arg Asp Gln Lys Gly Glu
   385 390 395 400
   Phe Lys Met Ala Phe Asn Gly Val Val Glu Val Lys Thr Ser Arg Glu
   405 410 415
   Leu Lys Val Met Gly Ala Ile Gly Pro Cys Val Ser Leu Asn Thr Lys
   420 425 430
   Gly Pro Cys Val Ser Glu Thr Asp Ile Gly Leu Gly Gly Thr Cys Gln
   435 440 445
   Trp Lys Phe Cys Thr Phe Asn Gln Asn Thr Thr Ala Ala Met Phe Phe
   450 455 460
   Glu Val Val Asn Gln His Ala Ala Pro Ile Pro Gln Gly Gly Arg Gly
   465 470 475 480
   Cys Ile Gln Phe Ile Thr Gln Tyr Gln His Ala Ser Gly Gln Arg Arg
   485 490 495
   Ile Arg Val Thr Thr Val Ala Arg Asn Trp Ala Asp Ala Thr Thr Asn
   500 505 510
   Met His His Val Ser Ala Gly Phe Asp Gln Glu Ala Gly Ala Val Leu
   515 520 525
   Met Ala Arg Met Val Val His Arg Ala Glu Thr Asp Asp Gly Pro Asp
   530 535 540
   Val Met Arg Trp Ala Asp Arg Met Leu Ile Arg Leu Cys Gln Lys Phe
   545 550 555 560
   Gly Glu Tyr Asn Lys Asp Asp Pro Asn Ser Phe Arg Leu Pro Glu Asn
   565 570 575
   Phe Ser Leu Tyr Pro Gln Phe Met Tyr His Leu Arg Arg Ser Gln Phe
   580 585 590
   Leu Gln Val Phe Asn Asn Ser Pro Asp Glu Thr Ser Tyr Tyr Arg His
           595                 600                 605
   Ile Leu Met Arg Glu Asp Leu Ser Gln Ser Leu Ile Met Ile Gln Pro
       610                 615                 620
   Ile Leu Tyr Ser Tyr Ser Phe Asn Gly Pro Glu Pro Val Leu Leu Asp
   625                 630                 635                 640
   Thr Ser Ser Ile Gln Pro Asp Arg Ile Leu Leu Met Asp Thr Phe Phe
                   645                 650                 655
   Gln Ile Leu Ile Phe His Gly Glu Thr Ile Ala Gln Trp Arg Ala Gln
               660                 665                 670
   Arg Tyr Gln Asp Leu Pro Glu Tyr Glu Asn Phe Lys Gln Leu Leu Gln
           675                 680                 685
   Ala Pro Val Asp Asp Ala Lys Glu Ile Leu His Thr Arg Phe Pro Met
       690                 695                 700
   Pro Arg Tyr Ile Asp Thr Glu Gln Gly Gly Ser Gln Ala Arg Phe Leu
   705                 710                 715                 720
   Leu Ser Lys Val Asn Pro Ser Gln Thr His Asn Asn Met Tyr Gly Tyr
                   725                 730                 735
   Gly Gly Glu Phe Gly Ala Pro Val Leu Thr Asp Asp Val Ser Leu Gln
               740                 745                 750
   Val Phe Met Glu His Leu Lys Lys Leu Ala Val Ser Phe Thr Ala
           755                 760                 765

<210>  101
<211>  511
<212>  DNA
<213>  Lygus hesperus

<400>  101
ggtgatgaga tgcggcaaga agaaagtgtg gttggaccct aatgaaatca acgaaatcgc      60

caacaccaac tctaggcaaa acatccgtaa gctgatcaag gatggtttga tcatcaaaaa     120

gcctgtggct gtccactcca gagcccgcgt ccgtaaaaac acagaagcca gacggaaggg     180

tcgtcactgt ggcttcggta agaggaaggg taccgccaac gccagaatgc ctgtgaaggt     240

cctgtgggtc aacagaatga gagtcctgcg acggctcctt aaaaaataca gagaagccaa     300

gaagatcgat aggcaaatgt accacgacct ttacatgaaa gccaaaggta acgtcttcaa     360

aaacaagagg gtactgatgg acttcattca caagaagaag gctgaaaagg cgagatcaaa 420

gatgttgaag gaccaggcag aggcgagacg tctcaaggtc aaggaggcga agaagaggcg 480

cgaggagagg atcgccacca agaagcaaga g 511

<210> 102
<211> 1145
<212> DNA
<213> Lygus hesperus

<400> 102
tgggttgttg gagtgctcct accccaacac gggagcaaaa gtcatgttgt tccttggtgg 60

cccttgttcc caagggcctg gtcaagttgt caatgatgac ctgagggaac ctatccgctc 120

tcatcatgac atccagaaag ataatgcccg ctacatgaaa aaagccatta aacattacga 180

ttctttggca ttgagagcag ccactaatgg gcattcagta gacatttatt cctgtgcttt 240

agatcagaca ggtttggcgg aaatgaagca atgttgcaat tctactgggg gtcatatggt 300

gatgggtgac accttcaact ccactttgtt caaacagacg ttccagaggg tgctctcccg 360

tgatcaaaaa ggcgaattca aaatggcttt caatggcgta gttgaagtca aaacctcccg 420

agagctaaaa gttatgggag ccattgggcc ttgcgtttca ttgaatacga aaggtccgtg 480

tgttagtgaa actgacatag ggcttggagg aacttgccag tggaagttct gcacatttaa 540

ccaaaatacc actgctgcca tgttctttga ggtagtaaac caacacgctg ctcctatccc 600

tcaaggtgga agaggatgta tacagttcat aactcaatac cagcatgcgt cgggccaaag 660

gcgcatccga gtaaccactg tagccaggaa ttgggctgat gcgactacca acatgcacca 720

tgttagtgca ggatttgatc aggaagctgg agcggtactc atggccagga tggtcgttca 780

cagagctgaa actgatgatg gacctgatgt catgagatgg gctgatcgca tgttgattcg 840

tctttgccag aaattcggcg agtacaacaa ggatgatcca aatagtttcc gcctcccaga 900

aaacttctcg ctttacccac agttcatgta tcacttgaga aggtcccaat tcttgcaggt 960

attcaacaac agcccagacg aaacgtcgta ctatcgtcac atcttgatgc gggaagattt 1020

gtcgcagagc ttgatcatga ttcagccgat cctgtacagt tacagtttca acggtccaga 1080

accagtcctt ttggacactt ccagcattca acctgatcgg atcctgctga tggacacctt 1140

cttcc 1145

<210> 103
<211> 258
<212> DNA
<213> Artificial

<220>
<223> GFP

<400>  103
agatacccag atcatatgaa acggcatgac tttttcaaga gtgccatgcc cgaaggttat      60

gtacaggaaa gaactatatt tttcaaagat gacgggaact acaagacacg taagtttaaa     120

cagttcggta ctaactaacc atacatattt aaattttcag gtgctgaagt caagtttgaa     180

ggtgataccc ttgttaatag aatcgagtta aaaggtattg attttaaaga agatggaaac     240

attcttggac acaaattg                                                   258


<210>  104
<211>  745
<212>  DNA
<213>  Artificial

<220>
<223>  Pt coral fluorescent protein

<400>  104
agtgtaataa cttactttga gtctaccgtc atgagtgcaa ttaaaccagt catgaagatt      60

gaattggtca tggaaggaga ggtgaacggg cacaagttca cgatcacggg agagggacaa     120

ggcaagcctt acgagggaac acagactcta aaccttacag tcactaaagg cgtgcccctt     180

cctttcgctt tcgatatctt gtcaacagca ttccagtatg gcaacagggt atttaccaaa     240

tacccagatg atataccgga ctatttcaag cagacctttc cggaaggata ttcgtgggaa     300

agaactttca aatatgaaga gggcgtttgc accacaaaga gtgacataag cctcaagaaa     360

ggccaaccag actgctttca atataaaatt aactttaaag gggagaagct tgaccccaac     420

ggcccaatta tgcagaagaa gaccctgaaa tgggagccat ccactgagag gatgtacatg     480

gacgtggata aagacggtgc aaaggtgctg aagggcgatg ttaatgcggc cctgttgctt     540

gaaggaggtg gccattatcg ttgtgacttt aacagtactt acaaggcgaa gaaaactgtg     600

tccttcccag catatcactt tgtggaccac cgcattgaga ttttgagcca caatacggat     660

tacagcaagg ttacactgta tgaagttgcc gtggctcgca attctcctct tcagattatg     720

gcgccccagt aaaggcttaa cgaaa                                           745


<210>  105
<211>  42
<212>  DNA
<213>  Artificial

<220>
<223>  Primer

<400>  105
gcgtaatacg actcactata ggtgatgaga tgcggcaaga ag                         42


<210>  106
<211>  22
<212>  DNA

<213>  Artificial

<220>
<223>  Primer

<400>  106
ctcttgcttc ttggtggcga tc                                                22


<210>  107
<211>  22
<212>  DNA
<213>  Artificial

<220>
<223>  Primer

<400>  107
ggtgatgaga tgcggcaaga ag                                                22


<210>  108
<211>  44
<212>  DNA
<213>  Artificial

<220>
<223>  Primer

<400>  108
gcgtaatacg actcactata ggctcttgct tcttggtggc gatc                        44


<210>  109
<211>  44
<212>  DNA
<213>  Artificial

<220>
<223>  Primer

<400>  109
gcgtaatacg actcactata ggtgggttgt tggagtgctc ctac                        44


<210>  110
<211>  22
<212>  DNA
<213>  Artificial

<220>
<223>  Primer

<400>  110
ggaagaaggt gtccatcagc ag                                                22


<210>  111
<211>  22
<212>  DNA
<213>  Artificial

<220>

<223>  Primer

<400>  111
tgggttgttg gagtgctcct ac                                                22


<210>  112
<211>  44
<212>  DNA
<213>  Artificial

<220>
<223>  Primer

<400>  112
gcgtaatacg actcactata ggggaagaag gtgtccatca gcag                        44


<210>  113
<211>  46
<212>  DNA
<213>  Artificial

<220>
<223>  Primer

<400>  113
gcgtaatacg actcactata ggagataccc agatcatatg aaacgg                      46


<210>  114
<211>  24
<212>  DNA
<213>  Artificial

<220>
<223>  Primer

<400>  114
caatttgtgt ccaagaatgt ttcc                                              24


<210>  115
<211>  24
<212>  DNA
<213>  Artificial

<220>
<223>  Primer

<400>  115
agatacccag atcatatgaa acgg                                              24


<210>  116
<211>  46
<212>  DNA
<213>  Artificial

<220>
<223>  Primer

<400>  116

gcgtaatacg actcactata ggcaatttgt gtccaagaat gtttcc                46

<210>  117
<211>  43
<212>  DNA
<213>  Artificial

<220>
<223>  Primer

<400>  117
gcgtaatacg actcactata ggagtgtaat aacttacttt gag                   43

<210>  118
<211>  20
<212>  DNA
<213>  Artificial

<220>
<223>  Primer

<400>  118
tttcgttaag cctttactgg                                             20

<210>  119
<211>  21
<212>  DNA
<213>  Artificial

<220>
<223>  Primer

<400>  119
agtgtaataa cttactttga g                                           21

<210>  120
<211>  42
<212>  DNA
<213>  Artificial

<220>
<223>  Primer

<400>  120
gcgtaatacg actcactata ggtttcgtta agcctttact gg                    42

<210>  121
<211>  473
<212>  DNA
<213>  Lygus hesperus

<400>  121
tgccgggccg ctcgccgaac catctgggaa gcttggaatg ggctcgactg ccgaactgat    60

caactttttc ggtccacacc ttttctatca actccttata ccgctccagg atgccgcctt   120

caaacagttt tttcttgtcg tcataagatc tggtgtcaac tcttcgttca tatttggagg   180

cgacttggat tttgggtggg tacttgccgg tgagggcctc agggtccaag ccttttttca 240

aggctttgtg ccgaagttgt tgcttctgtc tttccttcag ttctttaaga tcgtagtctt 300

gcctcttttg cctttcctca agatcgtatt tctcggtctc aagtttgaca atggcttccc 360

agagttcctg agctttgatg cgtagcctgt ctatgctcat attttctatc gccaggggct 420

tgagcctaat gctgagggag atacgtttct cttcctccag ctgctccttg gtc 473

<210> 122
<211> 773
<212> DNA
<213> Lygus hesperus

<400> 122
gctgctcgcc gtccagttcg ttttcgagtt ccctgacacg ttgttccagc ttggcgatgg 60

ccttcttgcc tcccttgagg gcgttgtttt cggcttcgtc caacctgact tggagttcct 120

tgatttgcgt ttccagagcc ttgcggagct tctcctgggt ctgagcgtgg tcctgttctg 180

ccctgagttc atcagctaac ctagcggcat caaccattgc cttcttggcc ttctcttcgg 240

agttcttggc ttcgttgaga agttcgtcga ggtcagcatg aagtgtctgc aactctccct 300

caagcttgcg tttggcggct gaggcgctgg tagcttgggc agccaactcg ttgatctgtt 360

cgtgggcatc tccaagttct tgttcggctt ggcgcctgcc cctgtcggcc tgttcgagga 420

gagtgcgcga ctcctcgagc tcgtttccga gagcgttggc cctcctttcg gcgattccga 480

gttgttcacg agcatcgtcg cgtgcccttt gttcttcctc aagagcggtc tgtacgtcct 540

tgagttgttg ttggtatttc ttgatggtct tctgggcttc ggcgttagcc ttgttggcgt 600

ggtcgagagc gatttcgagt tcgttgatgt cggcttcaag cttcttcttc atgcgaagag 660

cctcagcctt acccttggct tcagcctcca agctggcttg catggagtcg agtgcccgtt 720

ggtggttctt cctggtgttc tcgaactcct cctccttttc ctggatccgc cgg 773

<210> 123
<211> 771
<212> DNA
<213> Lygus hesperus

<400> 123
tggacgccat caagaagaaa atgcaggcga tgaagatgga gaaggacacg gccatggaca 60

aggccgacac ctgcgagggg caggccaagg acgctaacac ccgcgccgac aaaatccttg 120

aagatgtgag ggacctccaa aagaaactca accaggtaga aagtgatctc gaaaggacca 180

agagggaact cgagacgaaa accaccgaac tcgaagagaa ggagaaggcc aacaccaacg 240

ctgagagcga ggtcgcctcc ctcaacagga aagtccagat ggttgaagag gacttggaaa 300

gatctgaaga aaggtccggc accgcacaac aaaaactgtc cgaagcctcc cacgccgctg 360

atgaagcctc tcgtatgtgc aaagtattgg agaacaggtc acaacaggat gaggagagga 420

tggaccagct caccaaccag ctgaaagaag cccgactcct cgctgaagac gccgacggca 480

aatcggatga ggtatcaagg aagctggcct tcgttgaaga cgaactggaa gtagctgaag 540

atcgtgtcaa atctggagac tcgaagatca tggagcttga ggaggagttg aaagttgtcg 600

gtaacagctt gaaatctctc gaagtttcag aggagaaggc caaccagcga gtcgaagagt 660

acaaacgtca aatcaagcaa ctgactgtca agttgaagga ggctgaagct cgcgctgagt 720

tcgccgaaaa gacagtcaag aagttgcaga aagaggtgga ccggctggag g 771

<210> 124
<211> 257
<212> DNA
<213> Lygus hesperus

<400> 124
tgcgggccct ggggcagaat cccacagaat ctgacgtgaa gaagttcacc caccagcaca 60

aaccagatga aagaatcagc ttcgaggtgt ttctcccgat ataccaagcc atatcgaagg 120

gtaggacgtc agacacagct gaagacttca tcgagggtct cagacacttt gacaaagatg 180

gaaatggctt catttcaaca gctgagcttc gccacttgct cacaactttg ggcgaaaaac 240

tgaccgacga cgaggtg 257

<210> 125
<211> 410
<212> DNA
<213> Lygus hesperus

<400> 125
gccacctcca acgtgtttgc catgttcgat caggctcaga ttcaagaatt caaggaggca 60

ttcaacatga tcgaccagaa cagggacggc ttcgtggata aggaagacct ccatgacatg 120

ctcgcttccc taggtaagaa cccctcagac gagtatctcg aggggatgat gaacgaggcg 180

cctggtccca tcaacttcac aatgttcctc accctcttcg gtgagcggct tcagggaact 240

gatccggagg aggttatcaa gaacgcattt gggtgttttg acgaagacaa caacggattc 300

atcaacgagg aaagactgcg cgagctgctc acctccatgg gggacaggtt cactgatgaa 360

gacgtggacg aaatgtaccg agaggccccc atcaagaacg gcatgttcga 410

<210> 126
<211> 1021
<212> DNA
<213> Lygus hesperus

<400> 126
tgttcatcct ggagcaggag gagtatcaga gagaaggtat tgaatggaag ttcatcgact 60

tcggacttga tcttcagccg accattgatc tcattgataa gccaatggga gtcatggctc 120

tcctggatga agaatgttgg ttccccaaag ccactgacaa gaccttcgtt gagaagctgg 180

tcggtgctca cagcgttcac cccaaattca tcaaaactga tttccgtgga gtcgccgact 240

ttgctgtcgt ccattatgcc ggaaaagtcg attattcggc ggcgcagtgg ctgatgaaga 300

acatggaccc tctgaacgaa aacgtcgtgc agctcctcca gaactcgcaa gatccgttcg 360

tcatccacat ctggaaggac gcagagatcg tcggcatggc tcaccaagct ctcagcgaca 420

ctcagtttgg agctcgtacc aggaagggta tgttccgaac cgtgtctcaa ctctacaaag 480

accagctgtc caaactcatg atcacacttc gcaacacgaa ccccaacttc gtccgttgca 540

tcctccccaa ccacgagaag agagctggca agatcgatgc tcctttggtg ctggatcagc 600

tcagatgcaa cggtgtgttg gaaggcatca gaatttgcag acaaggtttc ccgaatagaa 660

tcccattcca ggaattccgg caaagatacg agctcttaac tcccaatgtc atccccaaag 720

ggttcatgga cggtaaaaag gcttgcgaga agatgatcaa cgctctcgaa ctggacccta 780

atctctacag agttggtcag tccaagatat tcttcagagc tggagtctta gctcatctag 840

aagaagagcg cgactataag attactgatc tgatagccaa tttccgggct ttctgtaggg 900

gatatcttgc ccgaaggaac taccaaaagc gtcttcagca gctcaacgcc attcgtatta 960

tccagcgaaa ttgctcagct tacttgaagt tgaggaactg gcaatggtgg cggctgtaca 1020

c 1021

<210> 127
<211> 325
<212> DNA
<213> Lygus hesperus

<400> 127
cggtcatcat ctccatgaac tcgtcgaagt caacagttcc ggaaccgtca gaatcaattt 60

cagcaatcat catgtcaagt tcttgggagg tgattttgtc gtcgagttcc ttcaggattt 120

ccctcaagac gtcagtggta atgtaaccgt tcccttcctt gtcgtagagc ctgaaggcct 180

ccctcagttc ttgctgcatg gcctcagcat cttgtgtctc atcttctgtc aggaaaccgg 240

cagccaaggc tacgaactcc tcaaattcaa gttgtccaga gccatcagcg tcgacctccg 300

caatgatctc ctccaggatc ttctt 325

<210> 128
<211> 463
<212> DNA
<213> Lygus hesperus

<400> 128
cggtcatcat ctccatgaac tcgtcgaagt cgacagttcc ggatccgtca gagtcgatct 60

cctcgatgat catgtccagc tcctcgttgg tcagctgctc gtccaattca tgaaggattt 120

ctttgaggca ggaggtcggg atgtagccat taccttcttt gtcgtagaga cggaaggctt 180

ctcgcagctc tttctgcatg gcttcatcgt cttcctcaac aatgaacttg gctgccaacg 240

tgatgaactc ttcaaactcc agccttcccg atttgtcagc gtcaacttct tcgatgagtt 300

catcgagaat cttcttgttg aagggttgac ccatgagtct gaggatgtcg gccaccatgt 360

ccgtcgggat ggaacccgag tgatcccggt cgaaagcgtt caacgcgatg gtcatgatgg 420

ggataattcg gttaattctg ttagaccagt ccgattagtg acg 463

<210> 129
<211> 413
<212> DNA
<213> Lygus hesperus

<400> 129
atgggtgaag gagggtgcct gctcagagca gtcctccagg atgacggcta tggacaacgc 60

ctcgaagaac gccgctgaga tgatcgacaa gctgaccttg acgttcaaca ggactcggca 120

agccgtcatc accagggagc tcatcgaaat catctccggt gcctctgctt tggagtaacg 180

tctcagctca cccagccacc tcccgtagat ccactagtgc tgcgagagac cgagtacctc 240

gttctattca ccctgtacat ttcttaatca atattattgg aattcgattc gatagtcgta 300

tgctgggaaa tatcttgttc atattcatga tacttgttca acattgttct ggtaaataat 360

ttatgtaata caggttgagt taccaaaaaa aaaaaaaaaa aaaaaaaaaa aaa 413

<210> 130
<211> 449
<212> DNA
<213> Lygus hesperus

<400> 130
gcagctggag gaagagaaac gtatctccct cagcattagg ctcaagcccc tggcgataga 60

aaatatgagc atagacaggc tacgcatcaa agctcaggaa ctctgggaag ccattgtcaa 120

acttgagacc gagaaatacg atcttgagga aaggcaaaag aggcaagact acgatcttaa 180

agaactgaag gaaagacaga agcaacaact tcggcacaaa gccttgaaaa aaggcttgga 240

ccctgaggcc ctcaccggca agtacccacc caaaatccaa gtcgcctcca aatatgaacg 300

aagagttgac accagatctt atgacgacaa gaaaaaactg tttgaaggcg gcatcctgga 360

gcggtataag gagttgatag aaaaggtgtg gaccgaaaaa gttgatcagt tcggcagtcg 420

agcccattcc aagcttccca gatggttcg 449

<210> 131
<211> 719
<212> DNA
<213> Lygus hesperus

<400> 131
aggagttcga gaacaccagg aagaaccacc aacgggcact cgactccatg caagccagct 60

tggaggctga agccaagggt aaggctgagg ctcttcgcat gaagaagaag cttgaagccg 120

acatcaacga actcgaaatc gctctcgacc acgccaacaa ggctaacgcc gaagcccaga 180

agaccatcaa gaaataccaa caacaactca aggacgtaca gaccgctctt gaggaagaac 240

aaagggcacg cgacgatgct cgtgaacaac tcggaatcgc cgaaaggagg gccaacgctc 300

tcggaaacga gctcgaggag tcgcgcactc tcctcgaaca ggccgacagg ggcaggcgcc 360

aagccgaaca agaacttgga gatgcccacg aacagatcaa cgagttggct gcccaagcta 420

ccagcgcctc agccgccaaa cgcaagcttg agggagagtt gcagacactt catgctgacc 480

tcgacgaact tctcaacgaa gccaagaact ccgaagagaa ggccaagaag gcaatggttg 540

atgccgctag gttagctgat gaactcaggg cagaacagga ccacgctcag acccaggaga 600

agctccgcaa ggctctggaa acgcaaatca aggaactcca agtcaggttg gacgaagccg 660

aaaacaacgc cctcaaggga ggcaagaagg ccatcgccaa gctggaacaa cgtgtcagg 719

<210> 132
<211> 737
<212> DNA
<213> Lygus hesperus

<400> 132
gcaggcgatg aagatggaga aggacacggc catggacaag gccgacacct gcgaggggca 60

ggccaaggac gctaacaccc gcgccgacaa aatccttgaa gatgtgaggg acctccaaaa 120

gaaactcaac caggtagaaa gtgatctcga aaggaccaag agggaactcg agacgaaaac 180

caccgaactc gaagagaagg agaaggccaa caccaacgct gagagcgagg tcgcctccct 240

caacaggaaa gtccagatgg ttgaagagga cttggaaaga tctgaagaaa ggtccggcac 300

cgcacaacaa aaactgtccg aagcctccca cgccgctgat gaagcctctc gtatgtgcaa 360

agtattggag aacaggtcac aacaggatga ggagaggatg gaccagctca ccaaccagct 420

gaaagaagcc cgactcctcg ctgaagacgc cgacggcaaa tcggatgagg tatcaaggaa 480

gctggccttc gttgaagacg aactggaagt agctgaagat cgtgtcaaat ctggagactc 540

gaagatcatg gagcttgagg aggagttgaa agttgtcggt aacagcttga aatctctcga 600

agtttcagag gagaaggcca accagcgagt cgaagagtac aaacgtcaaa tcaagcaact 660

gactgtcaag ttgaaggagg ctgaagctcg cgctgagttc gccgaaaaga cagtcaagaa 720

gttgcagaaa gaggtgg 737

<210> 133
<211> 205
<212> DNA
<213> Lygus hesperus

<400> 133
cagaatccca cagaatctga cgtgaagaag ttcacccacc agcacaaacc agatgaaaga 60

atcagcttcg aggtgtttct cccgatatac caagccatat cgaagggtag gacgtcagac 120

acagctgaag acttcatcga gggtctcaga cactttgaca aagatggaaa tggcttcatt 180

tcaacagctg agcttcgcca cttgc 205

<210> 134
<211> 326
<212> DNA
<213> Lygus hesperus

<400> 134
ggaggcattc aacatgatcg accagaacag ggacggcttc gtggataagg aagacctcca 60

tgacatgctc gcttccctag gtaagaaccc ctcagacgag tatctcgagg ggatgatgaa 120

cgaggcgcct ggtcccatca acttcacaat gttcctcacc ctcttcggtg agcggcttca 180

gggaactgat ccggaggagg ttatcaagaa cgcatttggg tgttttgacg aagacaacaa 240

cggattcatc aacgaggaaa gactgcgcga gctgctcacc tccatggggg acaggttcac 300

tgatgaagac gtggacgaaa tgtacc 326

<210> 135
<211> 944
<212> DNA
<213> Lygus hesperus

<400> 135
gacttgatct tcagccgacc attgatctca ttgataagcc aatgggagtc atggctctcc 60

tggatgaaga atgttggttc cccaaagcca ctgacaagac cttcgttgag aagctggtcg 120

gtgctcacag cgttcacccc aaattcatca aaactgattt ccgtggagtc gccgactttg 180

ctgtcgtcca ttatgccgga aaagtcgatt attcggcggc gcagtggctg atgaagaaca 240

tggaccctct gaacgaaaac gtcgtgcagc tcctccagaa ctcgcaagat ccgttcgtca 300

tccacatctg gaaggacgca gagatcgtcg gcatggctca ccaagctctc agcgacactc 360

agtttggagc tcgtaccagg aagggtatgt tccgaaccgt gtctcaactc tacaaagacc 420

agctgtccaa actcatgatc acacttcgca acacgaaccc caacttcgtc cgttgcatcc 480

tccccaacca cgagaagaga gctggcaaga tcgatgctcc tttggtgctg gatcagctca 540

gatgcaacgg tgtgttggaa ggcatcagaa tttgcagaca aggtttcccg aatagaatcc 600

cattccagga attccggcaa agatacgagc tcttaactcc caatgtcatc cccaaagggt 660

tcatggacgg taaaaaggct tgcgagaaga tgatcaacgc tctcgaactg gaccctaatc 720

tctacagagt tggtcagtcc aagatattct tcagagctgg agtcttagct catctagaag 780

aagagcgcga ctataagatt actgatctga tagccaattt ccgggctttc tgtaggggat 840

atcttgcccg aaggaactac caaaagcgtc ttcagcagct caacgccatt cgtattatcc 900

agcgaaattg ctcagcttac ttgaagttga ggaactggca atgg 944

<210> 136
<211> 318
<212> DNA
<213> Lygus hesperus

<400> 136
atcctggagg agatcattgc ggaggtcgac gctgatggct ctggacaact tgaatttgag 60

gagttcgtag ccttggctgc cggtttcctg acagaagatg agacacaaga tgctgaggcc 120

atgcagcaag aactgaggga ggccttcagg ctctacgaca aggaagggaa cggttacatt 180

accactgacg tcttgaggga aatcctgaag gaactcgacg acaaaatcac ctcccaagaa 240

cttgacatga tgattgctga aattgattct gacggttccg gaactgttga cttcgacgag 300

ttcatggaga tgatgacc 318

<210> 137
<211> 423
<212> DNA
<213> Lygus hesperus

<400> 137
atccccatca tgaccatcgc gttgaacgct ttcgaccggg atcactcggg ttccatcccg 60

acggacatgg tggccgacat cctcagactc atgggtcaac ccttcaacaa gaagattctc 120

gatgaactca tcgaagaagt tgacgctgac aaatcgggaa ggctggagtt tgaagagttc 180

atcacgttgg cagccaagtt cattgttgag gaagacgatg aagccatgca gaaagagctg 240

cgagaagcct tccgtctcta cgacaaagaa ggtaatggct acatcccgac ctcctgcctc 300

aaagaaatcc ttcatgaatt ggacgagcag ctgaccaacg aggagctgga catgatcatc 360

gaggagatcg actctgacgg atccggaact gtcgacttcg acgagttcat ggagatgatg 420

acc 423

<210> 138
<211> 252
<212> DNA
<213> Lygus hesperus

<400> 138
ggtgaaggag ggtgcctgct cagagcagtc ctccaggatg acggctatgg acaacgcctc 60

gaagaacgcc gctgagatga tcgacaagct gaccttgacg ttcaacagga ctcggcaagc 120

cgtcatcacc agggagctca tcgaaatcat ctccggtgcc tctgctttgg agtaacgtct 180

cagctcaccc agccacctcc cgtagatcca ctagtgctgc gagagaccga gtacctcgtt 240

ctattcaccc tg 252

<210> 139

<211>  1110
<212>  DNA
<213>  Lygus hesperus

<400>  139
gtctccgctc aagctggtca tcagacttca gctgagtcct ggggtaccgg tcgtgctgtg      60

gctcgtatcc cccgtgttcg cggaggtggt actcaccgct caggtcaggg tgcttttggc     120

aacatgtgtc gcggcggtag gatgttcgct cccactcgcc catggcgtcg ttggcaccgc     180

aagatcaacg ttaaccaaaa acgttatgcc gtcgtgtccg ccatcgctgc atccggcgtc     240

ccagccctcg tcatgtccaa aggacacatg gtgcaaagcg tccctgaatt cccccttgtt     300

gtgtctgaca aagttcagga atacactaaa accaaacagg ctgtcatctt ccttcaccgc     360

atcaaagcct ggcaagacat ccagaaagtg tacaagtcga agaggttccg tgctggtaag     420

ggtaaaatga ggaaccgcag gaggatccag aggcgtggac ccctcatcat ctacgaccag     480

gatcagggtc tgaacagggc tttccgtaac attcccggcg tcgatttgat cgaagtgagc     540

cgcctcaact tgctgaagct cgctccagga ggtcacatcg gccggttcgt catctggact     600

cagtcggcct tcgagaagtt ggacgccctc tacggcacct ggaagaagaa gtccaccctc     660

aaggctggat acaatctccc catgcccaag atggccaaca ccgacctttc ccgcctcttc     720

aaggccccgg agatcaaggc tgtcctcagg aatcccaaga agaccatcgt acgacgagtg     780

cgcaaactga accctctccg caacaccagg gctatgctgc gtctcaaccc atacgctgct     840

gtcctcaaga ggaaggccat ccttgatcaa aggaagttga aactccagaa gctcgtagaa     900

gctgccaaga agggagatac caagctgtcg ccccgcgtcg agcgtcacct gaagatgatc     960

gagagaagga aagccctgat caagaaagcc aaggctgcca agcccaagaa gcccaaaacg    1020

gccaagaaac ccaagaccgc cgagaaggca ccagcacccg ccaagaaggc ggcagcgccc    1080

aaaaaggcca ccacccctgc caagaaatga                                     1110


<210>  140
<211>  729
<212>  DNA
<213>  Lygus hesperus

<400>  140
atggccaatg ctaagcctat ttctaagaag aagaagtttg tgtctgacgg tgtcttcaaa      60

gccgaattga acgaatttct taccagagaa ctcgctgaag aggggtactc aggtgttgag     120

gtccgagtga cccccaacaa gacagaaatt atcatcatgg cgacaaggac acaaagcgtt     180

cttggtgata agggccgccg aatcagggag ctcacgtctg tagttcagaa aagattcaat     240

ttcaagcctc agactttgga tctctatgct gaaaaggtcg ccaccagagg tttgtgtgct     300

attgcacaag ctgaatccct ccgttacaaa ctcattggcg gtcttgctgt ccgaggggct     360

tgctatggtg tccttcgctt catcatggaa aatggtgcca agggttgcga agtcgtagta     420

tctggaaaac tgcgtggtca gagagccaag tcaatgaagt tcgtggatgg tttgatgatc 480

cacagtgggg atccctgtaa cgaatatgtt gatactgcta cccgacatgt gctccttaga 540

caaggtgtcc tgggaataaa ggtgaagatt atgttgccgt gggacgttac cggcaaaaat 600

gggccgaaga accctcttcc cgaccacgtc agcgttctct tacctaagga ggagctacca 660

aatttggccg ttagtgtgcc tggatccgac atcaaaccaa agcctgaagt accagcaccc 720

gctttgtga 729

<210> 141
<211> 789
<212> DNA
<213> Lygus hesperus

<400> 141
atggctgttg gtaaaaataa gggtctatcg aaaggaggaa agaagggagt taaaaaaaag 60

gtagtggacc ctttcaccag gaaggattgg tacgatgtta aggctccttc catgttcaaa 120

aagcgtcaag ttggcaaaac tttggtcaac cgaactcagg gaaccaagat tgcttctgaa 180

gggttgaaag gacgagtttt cgaagtttcg ctcgctgata tccaggagga cactgatgcc 240

gagcgctcct tcaggaaatt caggctcatc gctgaagatg tccaagccag aaacgtcctt 300

accaatttcc acggtatgga tttgaccact gacaaactcc ggagcatggt caagaagtgg 360

cagactctca tcgaagccaa cgttgacgtc aagaccaccg acggctacct cctgcgcgtc 420

ttctgcatag gattcaccaa taaagatcaa ctttcccaga gaaagacttg ctatgcccag 480

cataatcagg tccgagaaat ccgcaaaaag atggttaaaa acatcagtga cagcatttcc 540

agctgtgatt tgaggagtgt tgtgaacaag ctgatcccag actccatcgc taaagatata 600

gaaaagaatt gccaaggaat ctacccactc cacgatgtgt acattcggaa ggtgaaggtg 660

ttgaagaagc cgaggttcga gctcagcaag ctccttgagc ttcacgtcga tggcaaaggg 720

atcgacgaac ccggcgcgaa agtgacgagg actgacgctt acgagcctcc agttcaagag 780

tctgtctaa 789

<210> 142
<211> 473
<212> DNA
<213> Lygus hesperus

<400> 142
gaccaaggag cagctggagg aagagaaacg tatctccctc agcattaggc tcaagcccct 60

ggcgatagaa aatatgagca tagacaggct acgcatcaaa gctcaggaac tctgggaagc 120

cattgtcaaa cttgagaccg agaaatacga tcttgaggaa aggcaaaaga ggcaagacta 180

cgatcttaaa gaactgaagg aaagacagaa gcaacaactt cggcacaaag ccttgaaaaa 240

aggcttggac cctgaggccc tcaccggcaa gtacccaccc aaaatccaag tcgcctccaa 300

atatgaacga agagttgaca ccagatctta tgacgacaag aaaaaactgt ttgaaggcgg 360

catcctggag cggtataagg agttgataga aaaggtgtgg accgaaaaag ttgatcagtt 420

cggcagtcga gcccattcca agcttcccag atggttcggc gagcggcccg gca 473

<210> 143
<211> 1463
<212> DNA
<213> Lygus hesperus

<400> 143
gggtctcagc tgaggcacat tccatctcgt cgcaaatctt tcctgcatct ctcctgggtg 60

acctttaggt gaccaatcac atccatcatg tcggacgagg agtattcgga gtcggaggaa 120

gagacccagc cggaaccaca gaaaaaacca gaggctgaag gaggcggcga cccagaattc 180

gtcaagcgta aggaagccca gacctcagcc ttagacgagc agcttaaaga ctatatcgca 240

gaatggagga aacaaagagc tcgcgaagaa gaagacctca agaagctgaa ggagaagcaa 300

gccaagcgca aggtcgctcg ggcagaagaa gaaaagagat tggcggaaaa gaagaagcag 360

gaagaagaac gacgtgtgag ggaagcagaa gagaagaaac agagggaaat cgaagagaag 420

aggcgaaggc ttgaagaggc cgagaagaag agacaagcca tgatggctgc tctcaaggac 480

cagagcaaaa cgaagggacc caattttgtc gttaataaga aagccgaaac ccttggcatg 540

tcctccgctc aaattgagcg caacaagact aaggaacagc ttgaggaaga aaaacgtatc 600

tccctcagca ttaggctcaa gcccctggcg atagaaaata tgagcataga caggctacgc 660

ataaaagctc aggaactctg ggaagccatt gtcaaacttg agaccgagaa atacgatctt 720

gaggaaaggc aaaagaggca agactacgat cttaaagaac tgaaggaaag acagaagcaa 780

caacttcggc acaaagcctt gaaaaaaggc ttggaccctg aggccctcac cggcaagtac 840

ccacccaaaa tccaagtcgc ctccaaatat gaacgaagag ttgacaccag atcttatgac 900

gacaagaaaa aactgtttga aggcggcatc ctggagcggt ataaggagtt gatagaaaag 960

gtgtggaccg aaaaagttga tcagttcggc agtcgagccc attccaagct tcccagatgg 1020

ttcggcgagc ggcccggcaa gaagaaggat gcccctgaaa gcccggaaga agaggaagtg 1080

aaggtagaag atgaacctga agctgaacca agcttcatgc tcgacgaaga agaagaagaa 1140

gcggaagaag aggaggcgga agaggaagag gaagccgagg aagaggagga agaagaagag 1200

gaagaggaag aggaggagga ggaagaagaa taggtctttt tcaacatttc actgcaccca 1260

cagttccacg gtctttccgc ccacaaactc aatctgtgct cacgagatct tagcaggaaa 1320

agtattgcga cccgataaga acaaattaaa ttatttttgg aatatctcgt tcagttattt 1380

cgtgagaaac aattttattc atgtaaacga ttaaaagatc ccatacattt ccaaaaaaaa 1440

aaaaaaaaaa aaaaaaaaaa aaa 1463

<210> 144
<211> 773
<212> DNA
<213> Lygus hesperus

<400> 144
ccggcggatc caggaaaagg aggaggagtt cgagaacacc aggaagaacc accaacgggc 60

actcgactcc atgcaagcca gcttggaggc tgaagccaag ggtaaggctg aggctcttcg 120

catgaagaag aagcttgaag ccgacatcaa cgaactcgaa atcgctctcg accacgccaa 180

caaggctaac gccgaagccc agaagaccat caagaaatac caacaacaac tcaaggacgt 240

acagaccgct cttgaggaag aacaaagggc acgcgacgat gctcgtgaac aactcggaat 300

cgccgaaagg agggccaacg ctctcggaaa cgagctcgag gagtcgcgca ctctcctcga 360

acaggccgac aggggcaggc gccaagccga acaagaactt ggagatgccc acgaacagat 420

caacgagttg gctgcccaag ctaccagcgc ctcagccgcc aaacgcaagc ttgagggaga 480

gttgcagaca cttcatgctg acctcgacga acttctcaac gaagccaaga actccgaaga 540

gaaggccaag aaggcaatgg ttgatgccgc taggttagct gatgaactca gggcagaaca 600

ggaccacgct cagacccagg agaagctccg caaggctctg gaaacgcaaa tcaaggaact 660

ccaagtcagg ttggacgaag ccgaaaacaa cgccctcaag ggaggcaaga aggccatcgc 720

caagctggaa caacgtgtca gggaactcga aaacgaactg gacggcgagc agc 773

<210> 145
<211> 5446
<212> DNA
<213> Lygus hesperus

<400> 145
tcaggaaaac tggctggtgc tgatattgag acctatctgc tggagaaggc tcgtgtcatc 60

tcccaacaaa cactcgagag atcctaccac attttctacc agatgatgtc tggagctgtc 120

aagggcgtca aggaaatgtg cttgctggtc gacgatatct atacgtacaa cttcatatcc 180

cagggtaaag tcagcattgc aggcgttgat gacggagagg aaatggttct gaccgatcaa 240

gccttcgaca tcttgggttt caccaagcaa gagaaggaag acatctacaa gatcaccgcc 300

gctgtcattc acatgggtac catgaagttc aagcaaaggg gtcgtgaaga gcaggctgaa 360

gccgatggaa ctgaggaagg cggtaaggtc ggtgtgctcc tcggtatcga cggtgacgac 420

ttgtacaaga atatgtgcaa gcccagaatc aaggtcggaa ctgagttcgt gacccaggga 480

aagaacgtca accaggtctc atactctctc ggtgccatgt ccaagggtat gttcgatcgt 540

ctcttcaaat tcttggtcaa gaaatgtaac gaaactctgg acaccaaaca gaagagacag 600

cacttcattg gtgtactgga tattgccggg ttcgaaattt tcgacttcaa cggttttgag 660

caactgtgta tcaacttcac caacgagaaa ttgcaacaat tcttcaacca ccacatgttc 720

gtactcgagc aagaagagta caagagggaa ggcattaact gggctttcat tgatttcgga 780

atggacttgc tcgcttgtat tgaactgatt gagaagccca tgggtatctt gtccatcctt 840

gaagaagagt ctatgttccc caaggctact gacaagacct ttgaggacaa actcatcacc 900

aaccacttgg gcaaatctcc caacttcagg aagcccgccg ttccaaagcc tggccaacaa 960

gctggtcact tcgccatcgc tcactacgct ggttgcgtgt catacaacat caccggctgg 1020

cttgagaaga acaaggatcc gttgaacgac actgttgtcg atcagtacaa gaagggaacc 1080

aacaaactgt tgtgcgagat cttcgctgat catcctggcc aatctggtgc ccctggtggt 1140

gatgctggtg gcaagggtgg tcgtggcaag aaaggtggtg gcttcgccac tgtgtcatct 1200

tcctacaagg aacaattgaa caacttgatg accactttga agagcacaca gcctcacttc 1260

gtccgttgta tcatccccaa cgaattgaaa cagcccggtg ttattgattc tcacttggtc 1320

atgcaccagc tgacttgtaa cggtgtactt gaaggcatcc gtatttgccg taaaggcttc 1380

cccaacagga tgaactaccc tgacttcaag ctccgataca agatccttaa ccccgctgcc 1440

gtggacagag agagtgatat cctcaaggct gctggtctcg tccttgagtc aactgggctc 1500

gaccctgata tgtaccgtct cggccacacc aaggtgttct tcagggccgg agttttgggt 1560

caacttgaag aattgcgtga cgacaggctt agcaagatca tcggatggat gcaggccttc 1620

atgcgcggtt acctcgtcag gaaggagtac aagaagctcc aggaacagag gttagccctc 1680

caagttgtcc agcgcaactt gagaaggtac ctccaactga ggacctggcc ctggtggaag 1740

atgtggtcca gggtcaagcc cctcctcaac gtcgccaacg tcgaagagga gatgcggaaa 1800

ctcgaagagt tggtcgccga gacccaggcc gctttggaga aggaggagaa gctgaggaag 1860

gaggccgaag cccttaacgc caagcttctc caagagaaga ccgaccttct caggaacttg 1920

gaaggagaga agggatccat cagcggtatc caggaacgat gtgccaagct gcaagcccaa 1980

aaggccgatc ttgagtctca actcatggac acccaagaaa ggctgcagaa cgaagaagat 2040

gccaggaacc agctcttcca acagaagaag aaattggaac aagaagccgc tgccctcaag 2100

aaggacatcg aagatctcga actctccaac caaaagaccg accaagataa ggccagcaag 2160

gaacaccaaa tcagaaacct caatgacgag atcgctcacc aagatgactt gatcaacaag 2220

ctcaacaagg agaagaaaat ccagagcgaa ctcaaccaaa agactgctga agaacttcag 2280

gccgctgaag acaaaatcaa ccacctcacc aaggttaagg tcaagcttga acagaccttg 2340

gatgaactcg aagacaccct cgaacgtgaa aagaaactcc gaggagatgt cgaaaaggcc 2400

aagaggaaga ctgaaggcga cctcaagctc actcaggaag ccgttgccga tcttgaaagg 2460

aacaagaaag aactcgaaca gaccatccag aggaaagaca aggaaattgc ttccctcacc 2520

gccaagctcg aagacgaaca atccatcgtc aacaagactg gcaaacagat caaggaactc 2580

cagagccgca ttgaagagct cgaggaggaa gtcgaggctg agaggcaagc ccgcggaaag 2640

gctgagaagc aacgtgctga cctcgcccgc gaacttgagg aactcggcga gaggttagag 2700

gaagctggtg gtgccacctc tgcccagatc gagctcaaca agaagcgtga agctgagatg 2760

agcaaactca ggagggacct ggaagaagcc aacatccagc acgaaggcac gctcgccaac 2820

ctccgcaaga agcacaacga tgctgtcagt gagatgggag accaaatcga ccagctcaac 2880

aaacttaaga ccaaggttga aaaggagaag tctcaatacc tcggtgaact caacgacgtc 2940

cgcgcctcca ttgaccactt gaccaacgag aaggctgcca ctgaaaaggt tgccaagcaa 3000

ctgcaacacc aaatcaatga agttcaaggc aaacttgatg aagctaacag gacgctcaac 3060

gacttcgatg ctgccaagaa gaagttgtct attgagaact ctgacctcct cagacagttg 3120

gaggaagctg agagccaagt ttctcaactt agcaagatca agatctccct caccactcaa 3180

ctcgaggaca ctaagcgtct cgccgatgag gaagctaggg aacgcgcaac ccttcttggc 3240

aagttccgca acttggaaca cgaccttgac aacctgaggg aacaggtgga ggaagaagcc 3300

gaagctaagg ctgatatcca acgtcaactc agcaaggcca acgctgaagc tcagttgtgg 3360

cgcagcaagt acgaaagcga gggtgttgcc cgcgctgagg agcttgagga ggccaagagg 3420

aaactccagg cccgtttggc tgaggctgag gagaccattg agtccctcaa ccagaaggtt 3480

atcgcccttg agaagacgaa gcagcgcctt gccactgaag tcgaggatct gcagctcgag 3540

gtcgaccgtg ccaacgccat tgccaatgcc gctgaaaaga aggctaaggc tattgacaag 3600

atcattggtg aatggaaact caaggttgat gaccttgctg ctgagcttga tgctagtcaa 3660

aaggaatgca gaaactactc cactgagctc ttcaggctca agggagctta tgaagaagga 3720

caggaacaac ttgaagctgt ccgcagggag aacaagaacc ttgctgatga agtcaaggac 3780

ttgctcgacc agatcggtga gggtggccgc aacatccacg aaattgagaa gcagcgcaag 3840

aggctcgaag ttgagaagga cgaacttcag gccgctcttg aggaggctga agccgctctt 3900

gaacaggagg agaacaaagt actcagggct caacttgagc tcagccaggt gcgtcaagaa 3960

attgaccgcc gcatccagga gaaggaagag gagttcgaga acaccaggaa gaaccaccaa 4020

cgggcactcg actccatgca agccagcttg gaggctgaag ccaagggtaa ggctgaggct 4080

cttcgcatga agaagaagct tgaagccgac atcaacgaac tcgaaatcgc tctcgaccac 4140

gccaacaagg ctaacgccga agcccagaag accatcaaga aataccaaca acaactcaag 4200

gacgtacaga ccgctcttga ggaagaacaa agggcacgcg acgatgctcg tgaacaactc 4260

ggaatcgccg aaaggagggc caacgctctc ggaaacgagc tcgaggagtc gcgcactctc 4320

ctcgaacagg ccgacagggg caggcgccaa gccgaacaag aacttggaga tgcccacgaa 4380

cagatcaacg agttggctgc ccaagctacc agcgcctcag ccgccaaacg caagcttgag 4440

ggagagttgc agacacttca tgctgacctc gacgaacttc tcaacgaagc caagaactcc 4500

gaagagaagg ccaagaaggc aatggttgat gccgctaggt tagctgatga actcagggca 4560

gaacaggacc acgctcagac ccaggagaag ctccgcaagg ctctggaaac gcaaatcaag 4620

gaactccaag tcaggttgga cgaagccgaa aacaacgccc tcaagggagg caagaaggcc 4680

atcgccaagc tggaacaacg tgtcagggaa ctcgaaaacg aactggacgg cgagcagagg 4740

agacacgccg acgcacaaaa gaacctccgt aaatccgagc gtagaattaa ggagctcagt 4800

ttccagtccg acgaggaccg taagaaccac gaacgcatgc aagacctcgt agacaaactg 4860

caacagaaga tcaagactta caagaggcag attgaagaag ccgaagaaat cgcggccctt 4920

aacctcgcca aattccgcaa agcacaacaa gaactcgaag aagctgaaga acgcgctgat 4980

ctcgctgaac aggctgtttc caaattcaga acaaagggtg gacgcgcagg atctgctgcc 5040

agagcgatga gccctgtcgg ccagaagtga aggaacgaat aagcggacgt ataagctatc 5100

aatacctcgc acacaaacct gccaggcctc aatttgacgg caatgccttc ccaccacgat 5160

tcgatctaca tcccgacgac ttttaagatc tttgatagca acgcaaaaca tcaaatgaaa 5220

atcttttaaa ttttatgtat ttattttgac ctattttatt aagttattgt taatacaaac 5280

ataattccat gagctagata tctagccaac gaaccatcac aatcacgatt attcgaactg 5340

tacgatagaa gcattatttg tacagctgga ccatttacaa aatatttttg cttcgaataa 5400

taaagagttt atatcgcgaa aaaaaaaaaa aaaaaaaaaa aaaaaa 5446

<210> 146
<211> 964
<212> DNA
<213> Lygus hesperus

<400> 146
tcctcctctg gtgcccgact cttcaaatac ccaaatccag tcatgtcttc ccgtaaaacc 60

gctggccgca gggcgaccac caagaagcgc gctcagcgtg cgacgtcaaa cgtattcgcc 120

atgttcgatc aggctcagat tcaagaattc aaggaggcat tcaacatgat cgaccagaac 180

agggacggct tcgtggataa ggaagacctc catgacatgc tcgcttccct aggtaagaac 240

ccctcagacg agtatctcga ggggatgatg aacgaggcgc ctggtcccat caacttcaca 300

atgttcctca ccctcttcgg tgagcggctt cagggaactg atccggagga ggttatcaag 360

aacgcatttg ggtgttttga cgaagacaac aacggattca tcaacgagga aagactgcgc 420

gagctgctca cctccatggg ggacaggttc actgatgaag acgtggacga aatgtaccga 480

gaggccccca tcaagaacgg catgttcgac tacatcgaat tcactcggat cctcaagcac 540

ggagccaaag acaaagacga gcagtgacct atcaaatcct cgtcaacctc ccttcagtaa 600

tttgaaacca atccatcaaa ttttgtttaa aactcttact taaaatccga tcatctacgt 660

cactttgcca ccaatcggta ttattttttg agccgttcct acataaatcg aattaatttt 720

atacctacga atcatattgt tggaaatttc tctcttgtac ttatactttc tgttatttcc 780

taatttttct aactaaccaa gttagtcgtt agtttttatt cattccttta taaattatta 840

gttatccatt tttaatcatc ttgaagttat ttgtttttcg agtggtagaa tatttataca 900

ttttccaata tataatggtt tattcattct taaaaaacga aaaaaaagaa aaaaaaaaaa 960

aaaa 964

<210> 147
<211> 5872
<212> DNA
<213> Lygus hesperus

<400> 147
gatcttacct gcctgaacga ggcgtccgtt cttcacaaca tcaaggacag atattactcc 60

ggattgattt atacgtattc gggactcttc tgcgtggtgg tcaaccctta caagaaactg 120

ccaatctaca cagagagaat catggagaaa tacaaaggcg tcaaaagaca cgacctccct 180

ccacacgtat tcgccatcac agacacagct taccgttcta tgctgcaaga tagggaagat 240

caatcgatac tctgcaccgg cgaatcgggt gcggggaaaa ccgaaaacac gaaaaaagta 300

atccagtact tggcctacgt tgcagcctcg aaacccaaat cttccgcatc cccacatacg 360

gcccagagtc aagctctgat cattggagaa ctcgaacaac agctgcttca agctaaccca 420

attttggaag cattcggaaa cgccaagact gttaaaaacg ataattcttc tcgattcggt 480

aaattcattc gtatcaattt cgacgcatca ggctacatcg caggagccaa catagaaacg 540

tatcttctag agaaatctag ggccatcaga caagcgaaag atgagcgaac gttccacatc 600

ttttaccaac ttctggccgg agcatctgca gaacaaagaa aggagttcat cctcgaagat 660

ccgaaaaact accctttcct cagcagcggg atggtgtctg tgcctggagt tgacgatggt 720

gttgatttcc aagcaactat cgcctccatg tccatcatgg gcatgaccaa cgacgatctt 780

tccgctctct tccgcatcgt cagtgccgtc atgctgttcg gcagcatgca gttcaagcag 840

gagcgaaaca gcgaccaggc gacgctccca gacaacactg tagcgcaaaa aatcgcccac 900

ctccttggtc tctcaatcac agagatgacc aaagcgttcc tcaggcctag aatcaaagta 960

ggacgggatt tcgtcaccaa ggctcaaact aaggaacaag ttgagttcgc agtggaagcc 1020

atttcgaaag cctgctacga acgtatgttc cgatggctcg tcaacagaat caaccgctcc 1080

ctggatcgta ccaaaaggca gggagcatct ttcattggta ttcttgatat ggctggtttc 1140

gaaatctttg agatcaactc cttcgagcag ctttgtatca attacaccaa tgagaaactt 1200

caacaactct tcaaccacac catgttcatt ttggagcaag aggagtacca gagagaaggt 1260

attgaatgga agttcatcga cttcggactt gatcttcagc cgaccattga tctcattgat 1320

aagccaatgg gagtcatggc tctcctggat gaagaatgtt ggttccccaa agccactgac 1380

aagaccttcg ttgagaagct ggtcggtgct cacagcgttc accccaaatt catcaaaact 1440

gatttccgtg gagtcgccga ctttgctgtc gtccattatg ccggaaaagt cgattattcg 1500

gcggcgcagt ggctgatgaa gaacatggac cctctgaacg aaaacgtcgt gcagctcctc 1560

cagaactcgc aagatccgtt cgtcatccac atctggaagg acgcagagat cgtcggcatg 1620

gctcaccaag ctctcagcga cactcagttt ggagctcgta ccaggaaggg tatgttccga 1680

accgtgtctc aactctacaa agaccagctg tccaaactca tgatcacact tcgcaacacg 1740

aaccccaact tcgtccgttg catcctcccc aaccacgaga agagagctgg caagatcgat 1800

gctcctttgg tgctggatca gctcagatgc aacggtgtgt tggaaggcat cagaatttgc 1860

agacaaggtt tcccgaatag aatcccattc caggaattcc ggcaaagata cgagctctta 1920

actcccaatg tcatccccaa agggttcatg gacggtaaaa aggcttgcga gaagatgatc 1980

aacgctctcg aactggaccc taatctctac agagttggtc agtccaagat attcttcaga 2040

gctggagtct tagctcatct agaagaagag cgcgactata agattactga tctgatagcc 2100

aatttccggg ctttctgtag ggatatcttg cccgaaggaa ctaccaaaag cgtcttcag 2160

cagctcaacg ccattcgtat tatccagcga aattgctcag cttacttgaa gttgaggaac 2220

tggcaatggt ggcggctgta caccaaggtc aaacctctgc ttgaagtgac gaaacaagaa 2280

gagaagctga cgcaaaagga agacgaactg aagcaggtcc gcgagaaact ggacaaccag 2340

gtgaggtcca aggaagagta tgaaaagagg cttcaggacg ctttggagga gaaagctgct 2400

ctggcagagc aacttcaggc agaagtagag ctgtgtgcgg aagccgaaga aatgagagcc 2460

aggctcgctg tgaggaagca agaactagag gaaattctcc acgatctaga agccagaata 2520

gaggaagaag agcaacgaaa cacggtcctc atcaacgaaa agaagaagtt gaccctcaac 2580

atcgccgacc tcgaagaaca actggaagag gaagaaggag ctcgacagaa actccaactc 2640

gaaaaagtcc agatcgaagc tcggctgaag aaaatggaag aggacctcgc tctggccgaa 2700

gacaccaaca ccaaagtcgt aaaggagaag aaagtgttgg aagagagggc tagtgacttg 2760

gcccagaccc tcgctgagga agaagaaaaa gctaaacacc tcgcgaagct caagaccaag 2820

cacgagacga cgatagcgga attggaagag aggttgctca aagacaatca gcagaggcag 2880

gaaatggata ggaacaagag gaagatcgaa tcagaggtga atgatttgaa agaacaaatt 2940

aacgagaaga aggtccaagt agaggagctt cagttgcaac tcgggaagag ggaagaggaa 3000

atcgctcaag ctctgatgag aattgacgag gaaggagcag gcaaagctca gactcaaaag 3060

gctctcaggg aattggagtc tcagctggct gagctacaag aggatctaga ggctgaaaag 3120

gccgctcgcg ccaaggccga aaagcagaag cgcgacctca acgaagaact cgagtccctc 3180

aagaatgaac ttcttgactc actggacacg acagcagctc aacaggaatt gaggaccaag 3240

agagaacacg aactggcaac gctcaagaaa acattagaag aggaaacgca cattcacgaa 3300

gtatctctca ccgaaatgag gcacaaacac actcaagaag tcgctgcact caacgaacag 3360

ttggagcaac tcaaaaaggc caaatctgca ctcgaaaaat cgaaagcaca acttgaaggg 3420

gaagctgctg agctcgccaa cgaactggaa acagcaggaa cgagcaaggg cgagagtgaa 3480

aggaaacgga agcaggccga atcgtctctg caggagctct cgtcgcgact cttggaaatg 3540

gagagaacca aagccgagct ccaagagagg gtccagaaac tgtctgcaga agccgactct 3600

gtcaatcagc agttggaagc agcggaactg aaagcatcag cagccctcaa ggcatctggt 3660

accttggaga ctcagctcca ggaggcgcaa gtgctcctgg aagaggaaac tcggcagaag 3720

ctgtcgttga ccaccaaact gaaaggcctc gaaagcgaaa gagatgctct caaagagcaa 3780

ctctacgaag aggacgaggg taggaagaac ctagaaaaac agatggcgat actcaatcaa 3840

caagtagctg aaagcaagaa gaagtctgaa gaagaaacgg aaaaaataac tgaactcgaa 3900

gaaagtcgca aaaaattgct caaagacata gaaattcttc aaaggcaagt cgaagaactt 3960

caagttacca acgacaaatt agagaaaggc aagaagaagc tgcagtcaga actggaagac 4020

ctcaccatcg acctggagtc tcagagaaca aaggtggtcg agctcgagaa gaaacaaaga 4080

aatttcgaca aagttttggc cgaagaaaaa gcgttgtcgc aacaaatcac gcacgagagg 4140

gatgcggctg aaagagaagc ccgtgaaaag gaaactagag tactgtcgct gacgcgagaa 4200

ctcgatgaat tcatggagaa aatcgaggaa ctggagagaa gcaaacggca actccaggct 4260

gaactagacg agctggtcaa caaccaaggc accaccgaca aaagcgtgca cgaattggaa 4320

agggcgaaac gagttctgga gtcacaactt gcagagcaga aagcacaaaa tgaagagctt 4380

gaagatgaac tccaaatgac ggaagacgcc aaattgaggc tcgaagtcaa catgcaagct 4440

ctgagagctc aattcgaaag agatctacag ggcaaagaag agtcgggaga agaaaagagg 4500

agaggattgc tgaaacagct gagggacatt gaggctgaac ttgaagacga gagaaaacaa 4560

aggaccgctg ctgttgcctc tagaaagaag attgaagcgg atttcaaaga tgtagaacag 4620

caactggaaa tgcacactaa ggtaaaggaa gatcttcaga agcaactgaa gaaatgccag 4680

gtccaactga aggacgcaat cagagacgcg gaagaggctc ggctcggtcg ggaagagctg 4740

caggctgccg ctaaagaggc cgaaaggaag tggaagggtt tggaaacgga gctcattcaa 4800

gtgcaagagg atttgatggc gagcgaaagg cagcggcggg cagcggaagc cgaaagggat 4860

gaagtcgttg aagaagccaa caagaatgtc aagagcttat cgaatcttct cgacgaaaag 4920

aagaggctcg aagcccaatg ctcaggcctg gaagaggaac tcgaagaaga acttagcaac 4980

aatgaggccc tccaagacaa agcgagaaaa gcacaactca gcgttgagca acttaatgca 5040

gaacttgctg ccgaacggag taatgtgcag aaacttgagg gaacgagatt gtcgatggaa 5100

aggcaaaaca aggaactgaa ggccaaactg aacgaactgg aaacgttaca acgcaacaag 5160

ttcaaggcca atgcgtctct ggaggctaag attaccaatc ttgaagagca actggaaaat 5220

gaagccaagg aaaagctact tctccagaaa ggcaacagga agctcgacaa gaaaatcaaa 5280

gacctcctcg ttcaattgga ggatgaaagg aggcatgccg accagtataa agaacaagtc 5340

gagaagatca acgtcagggt gaagacgcta aagcgaactt tggacgacgc cgaagaagaa 5400

atgagtaggg agaagaccca gaagaggaaa gcacttcgcg aattggaaga cctcagggag 5460

aactacgatt ccctactccg agagaacgat aacctcaaaa acaaactcag gcggggcggc 5520

ggtatttccg ggatctcgag caggctcgga ggctccaagc gaggttccat ccccggagag 5580

gattcccagg gtctcaacaa caccacagac gaatcagtcg atggtgacga tatctcgaat 5640

ccttaaacgc tacttggatt taccagccag catccaactt tccactgaag acgtctccca 5700

taaacgttga aagagacccg tcgaggaaga aaaaaaggct ctttaagaaa aactattctg 5760

cctttttcaa aactttgtac ttaaaagtac tttcgcttaa caatgaaaga agaataaaaa 5820

tgtaaagttt tcatttatac aaaaaaaaaa aaaaaaaaaa aaaaaaaaaa aa 5872

<210> 148
<211> 325
<212> DNA
<213> Lygus hesperus

<400> 148
aagaagatcc tggaggagat cattgcggag gtcgacgctg atggctctgg acaacttgaa 60

tttgaggagt tcgtagcctt ggctgccggt ttcctgacag aagatgagac acaagatgct 120

gaggccatgc agcaagaact gagggaggcc ttcaggctct acgacaagga agggaacggt 180

tacattacca ctgacgtctt gagggaaatc ctgaaggaac tcgacgacaa aatcacctcc 240

caagaacttg acatgatgat tgctgaaatt gattctgacg gttccggaac tgttgacttc 300

gacgagttca tggagatgat gaccg 325

<210> 149
<211> 428
<212> DNA
<213> Lygus hesperus

<400> 149
aattatcccc atcatgacca tcgcgttgaa cgctttcgac cgggatcact cgggttccat 60

cccgacggac atggtggccg acatcctcag actcatgggt caacccttca acaagaagat 120

tctcgatgaa ctcatcgaag aagttgacgc tgacaaatcg ggaaggctgg agtttgaaga 180

gttcatcacg ttggcagcca agttcattgt tgaggaagac gatgaagcca tgcagaaaga 240

gctgcgagaa gccttccgtc tctacgacaa agaaggtaat ggctacatcc cgacctcctg 300

cctcaaagaa atccttcatg aattggacga gcagctgacc aacgaggagc tggacatgat 360

catcgaggag atcgactctg acggatccgg aactgtcgac ttcgacgagt tcatggagat 420

gatgaccg 428

<210> 150
<211> 538
<212> DNA
<213> Lygus hesperus

<400> 150
gcttctttta caaatcgcac cacgccgact taattcattc ccggagggtt taaattttat 60

cgaagcagca tggtgcggat gaatgtgctg agcgatgctc tgaaaagcat caacaatgct 120

gagaagaggg gcaaaaggca ggtgctcctg aggccttgtt ccaaagtcat cattaaattc 180

cttacagtga tgatgaagaa aggttatatc ggcgaattcg aaatagtaga tgatcacaga 240

tctggtaaaa tcgtcgtcaa cctcaacggc agattgaaca aatgtggagt tatatcgccc 300

agattcgacg tacccatcac acaaatcgaa aaatggacga acaacctcct gccttcccga 360

cagttcggtt atgtcgtact caccactagt ggagggatca tggatcacga agaagccagg 420

cgaaaacatc ttgggggtaa aatattaggg tttttctttt aataaaaaaa gacgagatgt 480

aaattaataa aactctttta cgtttcgcta aaaaaaaaaa aaaaaaaaaa aaaaaaaa 538

<210> 151
<211> 405
<212> DNA
<213> Lygus hesperus

<400> 151
ccacgccgac ttaattcatt cccggagggt ttaaatttta tcgaagcagc atggtgcgga 60

tgaatgtgct gagcgatgct ctgaaaagca tcaacaatgc tgagaagagg ggcaaaaggc 120

aggtgctcct gaggccttgt tccaaagtca tcattaaatt ccttacagtg atgatgaaga 180

aaggttatat cggcgaattc gaaatagtag atgatcacag atctggtaaa atcgtcgtca 240

acctcaacgg cagattgaac aaatgtggag ttatatcgcc cagattcgac gtacccatca 300

cacaaatcga aaaatggacg aacaacctcc tgccttcccg acagttcggt tatgtcgtac 360

tcaccactag tggagggatc atggatcacg aagaagccag gcgaa 405

<210> 152
<211> 470
<212> DNA
<213> Lygus hesperus

<400> 152
tgtcgatggc ggtcttaaca tcccccattc caccaagagg ttccctgggt acgacagtga 60

gtctaaggaa ttcaacgctg aggtccacag gaagcacatt ttcggcattc acgtcgctga 120

ctacatgcgt cagctggctg aagaggatga cgatgcttac aagaagcagt tctcgcagta 180

tgtcaagaac ggagtcactg ctgacagcat tgaaagtatc tacaagaagg ctcacgaagc 240

aatccgagct gatccaactc gcaaaccact tgagaagaag gaagtcaaga agaagaggtg 300

gaaccgcgcc aagctttcct tgtctgaaag gaagaacacc atcaaccaaa agaaggcaac 360

ttatctcaag aaagtggaag ctggagaaat cgaataagtt tttatattcc tgacattacc 420

cattaaaggt ttcgttttaa cctaaaaaaa aaaaaaaaaa aaaaaaaaaa 470

<210> 153
<211> 387
<212> DNA
<213> Lygus hesperus

<400> 153
tgtcgatggc ggtcttaaca tcccccattc caccaagagg ttccctgggt acgacagtga 60

gtctaaggaa ttcaacgctg aggtccacag gaagcacatt ttcggcattc acgtcgctga 120

ctacatgcgt cagctggctg aagaggatga cgatgcttac aagaagcagt tctcgcagta 180

tgtcaagaac ggagtcactg ctgacagcat tgaaagtatc tacaagaagg ctcacgaagc 240

aatccgagct gatccaactc gcaaaccact tgagaagaag gaagtcaaga agaagaggtg 300

gaaccgcgcc aagctttcct tgtctgaaag gaagaacacc atcaaccaaa agaaggcaac 360

ttatctcaag aaagtggaag ctggaga 387

<210> 154
<211> 745
<212> DNA
<213> Lygus hesperus

<400> 154
gtcctacgtg tttccggaaa aacgtgcatt tcgcgtaccc ctcgtggtga tccgttttca 60

tagaaataat ccaaaatggc tcccaagggg aataatatga ttcccaatgg ccatttccac 120

aaggattggc agaggttcat caaaacctgg ttcaaccagc ctgcccgcaa gttgaggagg 180

agaaacaaga ggttggagaa ggcccaacgg ctcgcgcccc gccccgcggg acctcttcgc 240

cccgctgtca gatgtcccac cgtcaggtac cacaccaagc tacgacctgg acgtggcttc 300

accttggaag aaatcaagag agccggtctg tgcaaaggat tcgcgatgtc catcggaatc 360

gctgtcgacc ccagaagaag gaataaatcc atcgagtccc tccaactcaa tgtacagaga 420

ctcaaggagt acagggctaa gcttatcctc ttcccacaca agaatgccaa gaaactgaag 480

aagggagaag ctactgagga agagaggaag gtggccaccc aacagcccct gccagttatg 540

cccatcaagc aaccagtcat caaattcaag gctcgcgtca ttacagacga tgagaagaaa 600

tactctgcct tcaccgccct ccgcaaggga cgagcagacc aaaggttggt cggtatccgt 660

gctaagcgcg caaaggaagc cgcagaaaac gccgaagacc cctctaaagc tcctaaaaaa 720

aaaaaaaaaa aaaaaaaaaa aaaaa 745

<210> 155
<211> 527
<212> DNA
<213> Lygus hesperus

<400> 155
aacgtgcatt tcgcgtaccc ctcgtggtga tccgttttca tagaaataat ccaaaatggc 60

tcccaagggg aataatatga ttcccaatgg ccatttccac aaggattggc agaggttcat 120

caaaacctgg ttcaaccagc ctgcccgcaa gttgaggagg agaaacaaga ggttggagaa 180

ggcccaacgg ctcgcgcccc gccccgcggg acctcttcgc cccgctgtca gatgtcccac 240

cgtcaggtac cacaccaagc tacgacctgg acgtggcttc accttggaag aaatcaagag 300

agccggtctg tgcaaaggat tcgcgatgtc catcggaatc gctgtcgacc ccagaagaag 360

gaataaatcc atcgagtccc tccaactcaa tgtacagaga ctcaaggagt acagggctaa 420

gcttatcctc ttcccacaca agaatgccaa gaaactgaag aagggagaag ctactgagga 480

agagaggaag gtggccaccc aacagcccct gccagttatg cccatca 527

<210> 156
<211> 576
<212> DNA
<213> Lygus hesperus

<400> 156
gccttattga acgtggtcga cagaaaactc ggtttctgag ctcatctcaa catggatatc 60

gaagaaccgg ccgcggcccc tacggagccc tcggacgtca acaccgccct tcaagaggtc 120

ctcaaggccg cccttcaaca cggagtcgtc gtccacggta tccacgagtc cgccaaggcc 180

ctcgacaaga ggcaagcttt gttgtgcgtc ctcgctgaga actgcgacga gccgatgtac 240

aagaagctgg tacaagccct ctgctcagag caccacatcc ccctcgtcaa agtagattcc 300

aataagaaac tcggcgaatg gacgggcctt tgcaagatcg acaagaccgg caaatctagg 360

aaaatcgtcg gctgctcttg tgtcgtcatc aaggactggg gtgaggacac gccccacttg 420

gacctcctca aggactacat cagggacgtc ttctaagaag tttctcctca atttcctttt 480

tataatgatt taacaactga gaattaataa taaaaatgtt aaattaaaca aaaaaatctc 540

aaaactgtaa aaaaaaagaa gaaaaaaaaa aaaaaa 576

<210> 157
<211> 442
<212> DNA
<213> Lygus hesperus

<400> 157
ccttattgaa cgtggtcgac agaaaactcg gtttctgagc tcatctcaac atggatatcg 60

aagaaccggc cgcggcccct acggagccct cggacgtcaa caccgccctt caagaggtcc 120

tcaaggccgc ccttcaacac ggagtcgtcg tccacggtat ccacgagtcc gccaaggccc 180

tcgacaagag gcaagctttg ttgtgcgtcc tcgctgagaa ctgcgacgag ccgatgtaca 240

agaagctggt acaagccctc tgctcagagc accacatccc cctcgtcaaa gtagattcca 300

ataagaaact cggcgaatgg acgggccttt gcaagatcga caagaccggc aaatctagga 360

aaatcgtcgg ctgctcttgt gtcgtcatca aggactgggg tgaggacacg ccccacttgg 420

acctcctcaa ggactacatc ag 442

<210> 158
<211> 601
<212> DNA
<213> Lygus hesperus

<400> 158
ctttcatttg tatagtacgg acgggtagtt tagttgtgtc ggttcatcgt aattcatcgg 60

ctgaatcatg aagatgaata aattggtcac ttcctcgagg aggaagaaca ggaagaggca 120

cttcaccgcc ccatcccaca tccgtagaaa gttgatgtcg gcaccactgt ccaaagaact 180

taggcagaag tacaacgtcc gaactatgcc tgtgaggaag gacgatgaag tccaggttgt 240

acgaggacac tacaaaggcc aacaggttgg caaagtcctc caggtgtaca ggaagaagtt 300

cattatttac attgagcgga tccaaagaga aaaagccaat ggtgccagcg tttacgttgg 360

cattcacccc tcaaagtgtg tgatcgtcaa attgaaggtc gacaaggata ggaaagaaat 420

ccttgacaga agatccaaag gacgtgactt ggcacttggc aaggacaagg gcaaatacac 480

cgaagacagt acgactgcta tggacacgtc ttaaattaat ttggtttatt tggttcctta 540

actccgttct tctttaataa tgactttttt aaagcaaaaa aaaaaaaaaa aaaaaaaaaa 600

a 601

<210> 159
<211> 448
<212> DNA
<213> Lygus hesperus

<400> 159
gtacggacgg gtagtttagt tgtgtcggtt catcgtaatt catcggctga atcatgaaga 60

tgaataaatt ggtcacttcc tcgaggagga agaacaggaa gaggcacttc accgccccat 120

cccacatccg tagaaagttg atgtcggcac cactgtccaa agaacttagg cagaagtaca 180

acgtccgaac tatgcctgtg aggaaggacg atgaagtcca ggttgtacga ggacactaca 240

aaggccaaca ggttggcaaa gtcctccagg tgtacaggaa gaagttcatt atttacattg 300

agcggatcca aagagaaaaa gccaatggtg ccagcgttta cgttggcatt cacccctcaa 360

agtgtgtgat cgtcaaattg aaggtcgaca aggataggaa agaaatcctt gacagaagat 420

ccaaaggacg tgacttggca cttggcaa 448

<210> 160
<211> 456
<212> DNA
<213> Lygus hesperus

<400> 160
ggctgttgtc ggctggtcat atcccgtttt ccacgtggtg tgtcgagtta tttttcttgt 60

aaattcgcat ttaaaatcgg atttataacc gaaattcatt atggaaaagc cagtagtttt 120

ggcccgtgtc atcaaaatcc tcggacgtac cggctcacag ggccaatgta cgcaagtgaa 180

ggtggagttc attggtgagc agaaccgaca gatcatcagg aacgtgaaag gaccagttag 240

agaaggcgac atcctcacac tcctagagtc tgaaagagaa gcgagaagac tgaggtagtg 300

ggaggtggcg atgcgttacg ttattttact tcattcaaca tttgaaaaaa accatcttcg 360

tgacaaaaaa catcttcacg caactatttg tattacctat gtttcgtaaa taaagtaacc 420

tcgttactta aaaaaaaaaa aaaaaaaaaa aaaaaa 456

<210> 161
<211> 321
<212> DNA
<213> Lygus hesperus

<400> 161
ctgttgtcgg ctggtcatat cccgttttcc acgtggtgtg tcgagttatt tttcttgtaa 60

attcgcattt aaaatcggat ttataaccga aattcattat ggaaaagcca gtagttttgg 120

cccgtgtcat caaaatcctc ggacgtaccg gctcacaggg ccaatgtacg caagtgaagg 180

tggagttcat tggtgagcag aaccgacaga tcatcaggaa cgtgaaagga ccagttagag 240

aaggcgacat cctcacactc ctagagtctg aaagagaagc gagaagactg aggtagtggg 300

aggtggcgat gcgttacgtt a 321

<210> 162
<211> 865
<212> DNA
<213> Lygus hesperus

<400> 162
aatcccggat tcatcgtttt attgaattgt ttttcgaagt ttctggtatt atcgttaaat 60

tagtctgtta agccctcatc cgtgatttgg caagttgttg attgttctat tttccttttt 120

ccagaaaatg gggagacgtc cagcgaggtg ttatcggtac tgtaaaaaca agccataccc 180

ccaaatcccg gttctgtcgt ggtgtccccg accccaagat caggatcttc gatctgggaa 240

agaagaaggc ccgcgtggaa gacttccccc tctgcgttca cctcgtctcc gatgagtacg 300

agcagctgtc ctccgaagcc ctcgaggcag gacgtatctg ctgcaacaag tacctcgtca 360

agaactgcgg caaggaccag ttccacatca ggatgaggct ccaccccttc cacgtcatta 420

ggatcaacaa aatgttatcg tgcgctggag ctgataggct ccagacaggg atgagaggag 480

cgttcggaaa gccgcaagga accgtcgctc gcgtccgcat cggtcagccc atcatgagcg 540

tccgctcgtc cgacaggtac aaggccgccg tcatcaaggc tctgaggaga gccaaattca 600

agttccctgg tcgccagaag atctacgttt ccaagaaatg gggcttcacc aagttcgacc 660

gcgaagagta cgagggcctt aggaacgaca acaaactagc gaatgacggc tgcaacgtca 720

aattgaggcc ggatcacgga cctttgcagg cgtggaggaa ggctcagctt gacatcgctg 780

ctggcctcta aattactttc caatggtttt ataaatcaac aaataaaact cgttttatgt 840

aaaaaaaaaa aaaaaaaaaa aaaaa 865

<210> 163
<211> 792
<212> DNA
<213> Lygus hesperus

<400> 163
ggttcctttc tcagattttg actttgccgt gttgtctctc ccaattttcc aaaatgggga 60

gacgtccagc gaggtgttat cggtactgta aaaacaagcc ataccccaaa tcccggttct 120

gtcgtggtgt ccccgacccc aagatcagga tcttcgatct gggaaagaag aaggcccgcg 180

tggaagactt ccccctctgc gttcacctcg tctccgatga gtacgagcag ctgtcctccg 240

aagccctcga ggcaggacgt atctgctgca acaagtacct cgtcaagaac tgcggcaagg 300

accagttcca catcaggatg aggctccacc ccttccacgt cattaggatc aacaaaatgt 360

tatcgtgcgc tggagctgat aggctccaga cagggatgag aggagcattc ggaaagccgc 420

aaggaaccgt cgctcgcgtc cgcatcggtc agcccatcat gagcgtccgc tcgtccgaca 480

ggtacaaggc cgccgtcatc gaggctctga ggagagccaa attcaagttc cctggtcgcc 540

agaagatcta cgtttccaag aaatggggct tcaccaagtt cgaccgcgaa gagtacgagg 600

gccttaggaa cgacaacaaa ctagcgaatg gcggctgcaa cgtcaaattg aggccggatc 660

acggaccttt gcaggcgtgg aggaaggctc agcttgacat cgctgctggc ctctaaatta 720

ctttccaatg gttttataaa tcaacaaata aaactcgttt tatctaaaaa aaaaaaaaaa 780

aaaaaaaaaa aa 792

<210> 164
<211> 645
<212> DNA
<213> Lygus hesperus

<400> 164
agccctcatc cgtgatttgg caagttgttg attgttctat tttccttttt ccagaaaatg 60

gggagacgtc cagcgaggtg ttatcggtac tgtaaaaaca agccataccc ccaaatcccg 120

gttctgtcgt ggtgtccccg accccaagat caggatcttc gatctgggaa agaagaaggc 180

ccgcgtggaa gacttccccc tctgcgttca cctcgtctcc gatgagtacg agcagctgtc 240

ctccgaagcc ctcgaggcag gacgtatctg ctgcaacaag tacctcgtca agaactgcgg 300

caaggaccag ttccacatca ggatgaggct ccaccccttc cacgtcatta ggatcaacaa 360

aatgttatcg tgcgctggag ctgataggct ccagacaggg atgagaggag cgttcggaaa 420

gccgcaagga accgtcgctc gcgtccgcat cggtcagccc atcatgagcg tccgctcgtc 480

cgacaggtac aaggccgccg tcatcaaggc tctgaggaga gccaaattca agttccctgg 540

tcgccagaag atctacgttt ccaagaaatg gggcttcacc aagttcgacc gcgaagagta 600

cgagggcctt aggaacgaca acaaactagc gaatgacggc tgcaa 645

<210> 165
<211> 619
<212> DNA
<213> Lygus hesperus

<400> 165
gctttaccga ttccgttctt gtttagtcca cgtttctctg ctcattcgtg cagattttaa 60

aacatgacca actccaaagg ttatcgtcgc ggaacgaggg atctcttctc gaggcccttc 120

cgtcaccatg gtgtcatccc actctcaacg tacatgaaag tataccgagt aggagacatc 180

gtatctatca aaggtaatgg agcagtgcaa aaaggtatgc cccacaaagt ttaccacggc 240

aagaccggac gagtctacaa tgttacacct cgcgcccttg gtgttattgt caacaagagg 300

gttcgtggaa aaatccttcc caagaggatc aacatcagga ttgaacacgt caaccacagt 360

aaatgcagag aagatttctt gaagcgagtg cgagaaaatg aaaggctccg caaattcgcc 420

aaagaaactg gcaccagggt tgaactcaaa agacagcctg ctcagccacg ccctgcacac 480

tttgtacaag ctaaagaagt cccagagctg ctggccccca taccttacga gttcatcgct 540

taaaaaattt tcaattccat cttaacttta tatatttgaa taaaattgtg ttctcaaaaa 600

aaaaaaaaaa aaaaaaaaa 619

<210> 166
<211> 461
<212> DNA
<213> Lygus hesperus

<400> 166
acgtttctct gctcattcgt gcagatttta aaacatgacc aactccaaag gttatcgtcg 60

cggaacgagg gatctcttct cgaggccctt ccgtcaccat ggtgtcatcc cactctcaac 120

gtacatgaaa gtataccgag taggagacat cgtatctatc aaaggtaatg gagcagtgca 180

aaaaggtatg ccccacaaag tttaccacgg caagaccgga cgagtctaca atgttacacc 240

tcgcgccctt ggtgttattg tcaacaagag ggttcgtgga aaaatccttc ccaagaggat 300

caacatcagg attgaacacg tcaaccacag taaatgcaga gaagatttct tgaagcgagt 360

gcgagaaaat gaaaggctcc gcaaattcgc caaagaaact ggcaccaggg ttgaactcaa 420

aagacagcct gctcagccac gccctgcaca ctttgtacaa g 461

<210> 167
<211> 481
<212> DNA
<213> Lygus hesperus

<400> 167
caacgtacat gaaagtatac cgagtaggag acatcgtatc tatcaaaggt aatggagcag 60

tgcaaaaagg tatgccccac aaagtttacc acggcaagac cggacgagtc tataatgtta 120

cacctcgcgc ccttggtgtt attgtcaaca agagggttcg tggaaaaatc cttcccaaga 180

ggatcaacat caggattgaa cacgtcaacc acagtaaatg cagagaagat ttcttgaagc 240

gagtgcgaga aaatgagagg ctccgcaaat tcgccaaaga aactggcacc agggttgaac 300

tcaaaagaca gcctgctcag ccacgccctg cacactttgt acaagctaaa gaagtcccag 360

agctgctggc ccccatacct tacgagttca tcgcttaaac aattttcaat tccatcttaa 420

ctttatatat ttgaataaaa ttgtgttccc taaaaaaaaa aaaaaaaaaa aaaaaaaaaa 480

a 481

<210> 168
<211> 747
<212> DNA
<213> Lygus hesperus

<400> 168
gcataaatat atagggcgat tgatttagcg gccgcgaatt cgcccttaag cagtggtatc 60

aacgcagagg gggggtcttc tctcccggtt ttcttcttgc ccgaatcgtc catcctgatg 120

ttggggtcac tgtcaccacg accatacccc aatttggggt atggcttggt tgtcccctac 180

ccataaatcc tgattggaca tctccccatt atgaaagact gcgagaaaca cccctgcccc 240

cggctttaaa cccacggcta aggggggatt cgcgggcggc aaatttcatt cggcccatag 300

tgagtcgtat tacaattcac tgggcgtcct ttttacacct tcggaccggg aaaaacctgg 360

cggttaccca aaatccgtta tttgccacat ccccctttac tccactgggt tatataacaa 420

agaggcccct tccaatgtcc tttcccaaaa gtgcgcagcc ctatactaat ggcctttaaa 480

ggaaccccta ttaaaaaaaa aacccttaac cacaggttgg tgatgtaacc aaggaaaata 540

atgaacacac cgggccaaag aaggtgatac ccctggtctt ggcgaccgcc tgtcaaatct 600

tcctcccgga acgaaacccg tagtggcatc gaggaataac cttgcgcatc atagactcca 660

aatggccact gtggccgctc tcgattcatg gaagaaatga gatgacccct accccgcgca 720

aaaggattca gaaccaatac cagaatc 747

<210> 169
<211> 1052
<212> DNA
<213> Lygus hesperus

<220>
<221> misc_feature
<222> (662)..(665)
<223> n is a, c, g, or t

<400> 169
ggttttcttc ttgcccgaat cgtccatcct gatgttgggg tcactgtcac cacgaccata 60

ccccaatttg gggtatggct tggttgtccc ctacccataa atcctgattg gacatctccc 120

cattatgaaa gactgcgaga aacacccctg cccccggctt taaacccacg gctaaggggg 180

gattcgcggg cggcaaattt cattcggccc atagtgagtc gtattacaat tcactgggcg 240

tcctttttac accttcggac cgggaaaaac ctggcggtta cccaaaatcc gttatttgcc 300

acatccccct ttactccact gggttatata acaaagaggc cccttccaat gtcctttccc 360

aaaagtgcgc agccctatac taatggcctt taaaggaacc cctattaaaa aaaaaaccct 420

taaccacagg ttggtgatgt aaccaaggaa aataatgaac acaccgggcc aaagaaggtg 480

atacccctgg tcttggcgac cgcctgtcaa atcttcctcc cggaacgaaa cccgtagtgg 540

catcgaggaa taaccttgcg catcatagac tccaaatggc cactgtggcc gctctcgatt 600

catggaagaa atgagatgac ccctaccccg cgcaaaagga ttcagaacca ataccagaat 660

cnnnntagca aaacggctat ttcccggttc tttgtcggat tcttttgcca gggccatgcc 720

ttttcccgga atggaaggcg ggctgtttga gaaacgcatt aaatgggatt agtccattca 780

taggccaccc aaggaaacca ctttaatttc gggttggtag gttgagagaa atggtgaggg 840

gtaacaattt tacaccggga accgtttatg cccagaatta ccccagcttc gaattaaccc 900

cccctaaagg ggatagttcc gccgggttaa aagaaattcg ccttaaacca gtgttttaaa 960

gcaggagaca gaagtgtttc tcgcaagctt tcaaaatggg gagatgtcca atcaggattt 1020

atgggtaggg tacaaccaag ccgaacccca aa 1052

<210> 170
<211> 555
<212> DNA
<213> Lygus hesperus

<400> 170
tagcaaaacg gctatttccc ggttctttgt cggattcttt tgccagggcc atgccttttc 60

ccggaatgga aggcgggctg tttgagaaac gcattaaatg ggattagtcc attcataggc 120

cacccaagga aaccacttta atttcgggtt ggtaggttga gagaaatggt gaggggtaac 180

aattttacac cgggaaccgt ttatgcccag aattacccca gcttcgaatt aaccccccct 240

aaaggggata gttccgccgg gttaaaagaa attcgcctta aaccagtgtt ttaaagcagg 300

agacagaagt gtttctcgca agctttcaaa atggggagat gtccaatcag gatttatggg 360

tagggtacaa ccaagccgaa ccccaaatcc ctgttctgtc gtggtgacag tgaccccaag 420

atctggatgt tcgttttggg aaagaagaaa accgggaggg accacttcct cctctgcgtt 480

gataccactg cttaagggcg aattcgttta aacctgcagg actagtccct tagtgagggt 540

aatctagcag cccac 555

<210> 171
<211> 1052
<212> DNA
<213> Lygus hesperus

<220>
<221> misc_feature
<222> (662)..(665)
<223> n is a, c, g, or t

<400> 171
ggttttcttc ttgcccgaat cgtccatcct gatgttgggg tcactgtcac cacgaccata 60

ccccaatttg gggtatggct tggttgtccc ctacccataa atcctgattg gacatctccc 120

cattatgaaa gactgcgaga aacacccctg cccccggctt taaacccacg gctaaggggg 180

gattcgcggg cggcaaattt cattcggccc atagtgagtc gtattacaat tcactgggcg 240

tcctttttac accttcggac cgggaaaaac ctggcggtta cccaaaatcc gttatttgcc 300

acatccccct ttactccact gggttatata acaaagaggc cccttccaat gtcctttccc 360

aaaagtgcgc agccctatac taatggcctt taaaggaacc cctattaaaa aaaaaaccct 420

taaccacagg ttggtgatgt aaccaaggaa aataatgaac acaccgggcc aaagaaggtg 480

atacccctgg tcttggcgac cgcctgtcaa atcttcctcc cggaacgaaa cccgtagtgg 540

catcgaggaa taaccttgcg catcatagac tccaaatggc cactgtggcc gctctcgatt 600

catggaagaa atgagatgac ccctaccccg cgcaaaagga ttcagaacca ataccagaat 660

cnnnntagca aaacggctat ttcccggttc tttgtcggat tcttttgcca gggccatgcc 720

ttttcccgga atggaaggcg ggctgtttga gaaacgcatt aaatgggatt agtccattca 780

taggccaccc aaggaaacca ctttaatttc gggttggtag gttgagagaa atggtgaggg 840

gtaacaattt tacaccggga accgtttatg cccagaatta ccccagcttc gaattaaccc 900

cccctaaagg ggatagttcc gccgggttaa aagaaattcg ccttaaacca gtgttttaaa 960

gcaggagaca gaagtgtttc tcgcaagctt tcaaaatggg gagatgtcca atcaggattt 1020

atgggtaggg tacaaccaag ccgaacccca aa 1052

<210> 172
<211> 1175
<212> DNA
<213> Lygus hesperus

<400> 172
ctcagcgaga tccctaagac aacgcctgcc acgtgggaga atatcggaca cgcctcccca 60

gagtgcggaa aggggaacgg cgttccgtat cggtcaaggt gcaagcttcg gaaccggagg 120

acgaccgttg caaggtgcaa ggggcaggta tcttgtattt tcattgtgcg tgtcgacatc 180

taccaaactg agacttggag ttcgatattt tgacgatggg gccgggggcc ggaggcaaaa 240

cgacaaacac aggcaccgtg accgtgttcc ggtccctggc ctgcgttgcc ttacgttcac 300

atcttgttct tgcgctttct ctggttttac gataacccta ctacgagttt agtagagccg 360

atcccgtagc cgaagccaaa gcccaagcgc tccgtatccg agaacgcgga agagcacgaa 420

ctccccaaac ccctccgccc ctcccccgcg cgtatccgaa acacaaatgc agcgggcagt 480

acaggttttg gaaggggacg cgggcagtga gcgcaatgca agtaaatgtg attagctcat 540

ggctacgcag ccctgctttt tcagtttcgg ttcggatcgt tagggggtgt gggattggga 600

gcggattcaa tctggacagg aaacagctat gaccaaggtc acgccaagct ctgaattaac 660

cctcaggaaa gggactagtc cggcaggttg aaacgaactc gcccctaagc agtggtatca 720

gagcacagtg gttttttttt tttgtttttt ttcgtagaaa aaaatatgta ttaagtcaat 780

taattaaatc attggttttc tggcttcaca acaggtggca cgtgctgtgc tcggagaaat 840

ttatgaacta tgttctgttc ttcaatgagg aaagatgaga tgatccattc tcagacacat 900

tcagacagag gacaccaccg taagccctat ccacagtctg tccacgtaag gggatcgtgt 960

ccccttccat gggcagagca gggagagggc cgtaagcttg ttcttgcgtc atcaacatgt 1020

gggggtaatg ttggtcatag cgatgttcgg tacacaagag aaccacctgg tgtaatcatt 1080

acagcacagc aatactctgt gttttgtaag ataacaaaaa aggtacttaa gacgctgaac 1140

cattttctac gatcggaaaa caaaaaaaaa gaaaa 1175

<210> 173
<211> 1023
<212> DNA
<213> Lygus hesperus

<400> 173
tcagcgagat ccctaagaca acgcctgcca cgtgggagaa tatcggacac gcctccccag 60

agtgcggaaa ggggaacggc gttccgtatc ggtcaaggtg caagcttcgg aaccggagga 120

cgaccgttgc aaggtgcaag gggcaggtat cttgtatttt cattgtgcgt gtcgacatct 180

accaaactga gacttggagt tcgatatttt gacgatgggg ccgggggccg gaggcaaaac 240

gacaaacaca ggcaccgtga ccgtgttccg gtccctggcc tgcgttgcct tacgttcaca 300

tcttgttctt gcgctttctc tggttttacg ataaccctac tacgagttta gtagagccga 360

tcccgtagcc gaagccaaag cccaagcgct ccgtatccga gaacgcggaa gagcacgaac 420

tccccaaacc cctccgcccc tcccccgcgc gtatccgaaa cacaaatgca gcgggcagta 480

caggttttgg aaggggacgc gggcagtgag cgcaatgcaa gtaaatgtga ttagctcatg 540

gctacgcagc cctgcttttt cagtttcggt tcggatcgtt agggggtgtg ggattgggag 600

cggattcaat ctggacagga aacagctatg accaaggtca cgccaagctc tgaattaacc 660

ctcaggaaag ggactagtcc ggcaggttga aacgaactcg cccctaagca gtggtatcag 720

agcacagtgg tttttttttt ttgttttttt tcgtagaaaa aaatatgtat taagtcaatt 780

aattaaatca ttggttttct ggcttcacaa caggtggcac gtgctgtgct cggagaaatt 840

tatgaactat gttctgttct tcaatgagga aagatgagat gatccattct cagacacatt 900

cagacagagg acaccaccgt aagccctatc cacagtctgt ccacgtaagg ggatcgtgtc 960

cccttccatg ggcagagcag ggagagggcc gtaagcttgt tcttgcgtca tcaacatgtg 1020

ggg 1023

<210> 174
<211> 454
<212> DNA
<213> leptinotarsa decemlineata

<400> 174
ccaagaaggc caagaagggg tttatgaccc ctgagaggaa gaagaaactt aggttattgc 60

tgagaaagaa agcagcagaa gaactgaaaa aagaacaaga acgcaaagct gccgaaagga 120

gacgtattat tgaagagaga tgcggaaaac caaaactcat tgatgaggca aatgaagagc 180

aggtgaggaa ctattgcaag ttatatcacg gtagaatagc taaactggag gaccagaaat 240

ttgatttgga ataccttgtc aaaaagaaag acatggagat cgccgaattg aacagtcaag 300

tcaacgacct caggggtaaa ttcgtcaaac ccactctcaa gaaagtatcc aaatacgaga 360

acaaatttgc taaactccaa aagaaagcag cagaattcaa tttccgtaat caactgaaag 420

ttgtaaagaa gaaggagttc accctggagg agga 454

<210> 175
<211> 431
<212> DNA
<213> leptinotarsa decemlineata

<400> 175
ggtttatgac ccctgagagg aagaagaaac ttaggttatt gctgagaaag aaagcagcag 60

aagaactgaa aaaagaacaa gaacgcaaag ctgccgaaag gagacgtatt attgaagaga 120

gatgcggaaa accaaaactc attgatgagg caaatgaaga gcaggtgagg aactattgca 180

agttatatca cggtagaata gctaaactgg aggaccagaa atttgatttg gaataccttg 240

tcaaaaagaa agacatggag atcgccgaat tgaacagtca agtcaacgac ctcaggggta 300

aattcgtcaa acccactctc aagaaagtat ccaaatacga gaacaaattt gctaaactcc 360

aaaagaaagc agcagaattc aatttccgta atcaactgaa agttgtaaag aagaaggagt 420

tcaccctgga g 431

<210> 176
<211> 888
<212> DNA
<213> leptinotarsa decemlineata

<400> 176
agcagtggta tcaacgcaga gtacgcgggg acatcgagga gaagaggcaa cgcctcgaag 60

aggctgaaaa gaaacgccag gccatgatgc aggccctcaa ggaccagaac aagaacaagg 120

ggcccaactt caccatcacc aagagggatg cttcatctaa cctttctgcc gctcagttgg 180

aacgcaacaa gaccaaggag caactcgagg aagagaagaa aatttccctt tccatccgca 240

tcaagccctt ggtcgttgat ggtctgggcg tagataaact ccgtctgaaa gcacaagaac 300

tttgggaatg catcgtcaag ttggagactg aaaagtacga cttggaagag aggcagaaac 360

gtcaagacta cgatctcaaa gagctgaaag aaagacagaa acaacagctg agacacaaag 420

ccttgaagaa gggtctagac ccagaagccc taaccggcaa atacccgcct aaaatccaag 480

tagcctccaa atatgaacgt cgtgttgaca cgaggtcgta tggagacaaa aagaagctat 540

tcgaaggggg attagaagaa atcattaaag agaccaatga aaagagctgg aaagagaaat 600

ttggacagtt cgattccaga caaaaggcaa gacttcccaa gtggttcggt gaacgtcctg 660

gcaaaaaacc tggagatccc gaaactccag aaggcgagga ggagggcaaa caagtcattg 720

atgaggatga cgacctcaag gagcctgtaa tcgaagctga aattgaagaa gaggaggaag 780

aagaggaagt cgaggtcgat gaagaagaag aggatgacga agaagaagaa gaagaagagt 840

gaatgccaaa ggcagaagat aatcatgaaa tcaacattag ataacgtc 888

<210> 177
<211> 404
<212> DNA
<213> leptinotarsa decemlineata

<400> 177
caaggaccag aacaagaaca aggggcccaa cttcaccatc accaagaggg atgcttcatc 60

taacctttct gccgctcagt tggaacgcaa caagaccaag gagcaactcg aggaagagaa 120

gaaaatttcc ctttccatcc gcatcaagcc cttggtcgtt gatggtctgg gcgtagataa 180

actccgtctg aaagcacaag aactttggga atgcatcgtc aagttggaga ctgaaaagta 240

cgacttggaa gagaggcaga aacgtcaaga ctacgatctc aaagagctga aagaaagaca 300

gaaacaacag ctgagacaca aagccttgaa gaagggtcta gacccagaag ccctaaccgg 360

caaatacccg cctaaaatcc aagtagcctc caaatatgaa cgtc 404

<210> 178
<211> 1155
<212> DNA
<213> leptinotarsa decemlineata

<400> 178
gctcttcaga atgaacttga agaatctcgt acactgttgg aacaagctga ccgtgcccgt 60

cgccaagcag aacaagaatt gggagatgct cacgaacaat tgaatgatct tggtgcacag 120

aatggttctc tgtctgccgc caagaggaaa ctggaaactg aactccaaac tctccattcc 180

gatcttgatg aacttctcaa tgaagccaag aactctgagg agaaggctaa gaaagccatg 240

gtcgatgcag ctcgtcttgc agatgaactg agagcagaac aagatcatgc acaaactcag 300

gagaaacttc gtaaagcctt agaatcacaa atcaaggacc ttcaagttcg tctcgacgag 360

gctgaagcta acgccctcaa aggaggtaag aaagcaatcg ctaaacttga acaacgcgtc 420

agggaattgg agaatgagtt agatggtgaa caaagacgac acgccgatgc tcaaaagaat 480

ttgagaaagt ccgaacgtcg catcaaggag ctcagcctcc aagctgaaga agaccgtaag 540

aaccacgaaa aaatgcaaga cttagtcgac aaacttcaac agaaaatcaa gacccacaag 600

aggcaaatag aagaagctga agaaatagcg gctctcaatt tggccaaatt ccgtaaagca 660

caacaggaat tggaagaagc agaagagcgt gcagaccttg ctgaacaagc aattgtcaaa 720

ttccgtacca agggacgttc tggatcagca gctaggggag ccagccctgc gcctcagcga 780

cagcgtccca cattcggaat gggagattca cttggaggtg ccttccctcc aaggttcgat 840

cttgcacccg actttgaatg aatctgacat tgtgttataa gtgtaaggtg aacattctat 900

cgcagtgtaa atatcatccc aatgcgaatc aattctacat tcagtttaag tcattctatc 960

tctcaaaata ataatagtgt catccattct cactatcaaa tcaagacaag agatgatgat 1020

cagagaacac gtatcacatc tacagcaaac cctcagtcct cggcatctct gataatattt 1080

tcaattatcg agattgatga tatcgggtgt tgaatgctga tgaatagaag gcgccctatg 1140

gaaataagag agaag 1155

<210> 179
<211> 523
<212> DNA
<213> leptinotarsa decemlineata

<400> 179

gaatctcgta cactgttgga acaagctgac cgtgcccgtc gccaagcaga acaagaattg 60

ggagatgctc acgaacaatt gaatgatctt ggtgcacaga atggttctct gtctgccgcc 120

aagaggaaac tggaaactga actccaaact ctccattccg atcttgatga acttctcaat 180

gaagccaaga actctgagga gaaggctaag aaagccatgg tcgatgcagc tcgtcttgca 240

gatgaactga gagcagaaca agatcatgca caaactcagg agaaacttcg taaagcctta 300

gaatcacaaa tcaaggacct tcaagttcgt ctcgacgagg ctgaagctaa cgccctcaaa 360

ggaggtaaga aagcaatcgc taaacttgaa caacgcgtca gggaattgga gaatgagtta 420

gatggtgaac aaagacgaca cgccgatgct caaaagaatt tgagaaagtc cgaacgtcgc 480

atcaaggagc tcagcctcca agctgaagaa gaccgtaaga acc 523

<210> 180
<211> 865
<212> DNA
<213> nilaparvata lugens

<400> 180
ctaggagtat ctcctacgta attcggtgct tgagccaact gcagctactc acttttttcc 60

aggttcagtg gtagggacgc aaacacagct aaaatggcgg acgatgaggc aaagaaggca 120

aagcaggcgg aaatcgaccg caagagagcc gaggtccgca agcggatgga ggaagcctcc 180

aaggccaaga aggccaagaa aggtttcatg acgcctgaca gaaagaagaa gctcaggttg 240

ttgctgagga aaaaggctgc tgaggaattg aagaaggaac aggagaggaa agccgcggaa 300

aggagaagga tcatcgagga gaggtgtggc aaggctgttg atctcgatga cggaagtgaa 360

gagaaagtca aggcaacttt aaaaacctat cacgacagaa ttggaaaatt ggaggatgaa 420

aaatttgacc tggaatatat tgtaaaaaag aaagacttcg agatcgctga cctcaacagc 480

caggtgaatg acctccgtgg taaatttgtc aagccaacct tgaaaaaagt ctccaaatat 540

gagaacaaat tcgccaagct ccagaagaaa gcagctgaat tcaatttcag aaatcagctc 600

aaagttgtca agaagaagga attcaccttg gaagaagaag acaaggagcc gaagaaatcg 660

gagaaagccg aatggcagaa gaaatgaact cacatcacct cttcataata ttgtcccaca 720

cttctacaac cttcatcaaa taacttttat tcgagtaaac ttactgttac taacaaaatt 780

acaaaaccaa actcttatca tcaacgtagg caatgtgctc aacttatttc ttaaacatat 840

tgtccagcta tttattgaaa ttaaa 865

<210> 181
<211> 269
<212> DNA
<213> nilaparvata lugens

<400> 181
aagaagaagc tcaggttgtt gctgaggaaa aaggctgctg aggaattgaa gaaggaacag 60

gagaggaaag ccgcggaaag gagaaggatc atcgaggaga ggtgtggcaa ggctgttgat 120

ctcgatgacg gaagtgaaga gaaagtcaag gcaactttaa aaacctatca cgacagaatt 180

ggaaaattgg aggatgaaaa atttgacctg gaatatattg taaaaaagaa agacttcgag 240

atcgctgacc tcaacagcca ggtgaatga 269

<210> 182
<211> 553
<212> DNA
<213> nilaparvata lugens

<400> 182
aatgatggcg gctctcaagg accagagcaa atcgaaagga cccaacttca ccgtaaacaa 60

gaaaacagac ttgaacatga cgtcagctca aatggaaagg aacaagacta aggagcagct 120

ggaggaggag aagaagatct ctctgtcgtt ccgcatcaag ccgttggcca tcgagaacat 180

gagcatcaac gcactgcgcg ccaaggccca ggaactgtgg gactgcatcg tcaagctcga 240

aactgagaag tacgatctgg aggaacgcca gaagaggcag gactacgatc tcaaagaatt 300

gaaagaaaga caaaagcaac agctgaggca taaagccctc aaaaaaggtc tagaccctga 360

ggctctcaca ggaaagtacc caccaaaaat ccaagttgcc tccaaatatg aaagacgtgt 420

agatacaagg tcatacgacg acaagaagaa gctcttcgaa ggtggctggg acacattaac 480

atcagaaacc aatgagaaaa tatggaagag cagaaacgat cagttttcaa atcgtagcaa 540

ggctaaactg cca 553

<210> 183
<211> 470
<212> DNA
<213> nilaparvata lugens

<400> 183
atgatggcgg ctctcaagga ccagagcaaa tcgaaaggac ccaacttcac cgtaaacaag 60

aaaacagact tgaacatgac gtcagctcaa atggaaagga acaagactaa ggagcagctg 120

gaggaggaga agaagatctc tctgtcgttc cgcatcaagc cgttggccat cgagaacatg 180

agcatcaacg cactgcgcgc caaggcccag gaactgtggg actgcatcgt caagctcgaa 240

actgagaagt acgatctgga ggaacgccag aagaggcagg actacgatct caaagaattg 300

aaagaaagac aaaagcaaca gctgaggcat aaagccctca aaaaaggtct agaccctgag 360

gctctcacag gaaagtaccc accaaaaatc caagttgcct ccaaatatga aagacgtgta 420

gatacaaggt catacgacga caagaagaag ctcttcgaag gtggctggga 470

<210> 184
<211> 367
<212> DNA

<213>  nilaparvata lugens

<400>  184
tgccttcgac cgtgaaaggt ctggaagtat cccaacagac atggtcgccg acatcctcag      60

gctcatggga cagcctttca acaagaagat cctcgacgaa ctcattgagg aagttgatgc     120

tgacaaatct ggccgtcttg agtttgacga attcgtgact ctggccgcca aattcattgt     180

tgaggaagac gatgaggcaa tgcagaagga attgaaggaa gctttcagat tatacgacaa     240

ggaaggtaac ggctacatcc ccacatcatg tctgaaggaa atcttaaggg aacttgacga     300

tcagctgaca aacgaggaac tcaacatgat gattgatgag atcgactctg acggatcagg     360

aactgtt                                                               367


<210>  185
<211>  204
<212>  DNA
<213>  nilaparvata lugens

<400>  185
acatcctcag gctcatggga cagcctttca acaagaagat cctcgacgaa cttattgagg      60

aggttgatgc tgacaagtct ggccgtctag agtttgacga attcgtgact ctggccgcca     120

aattcattgt tgaggaagac gatgaggcaa tgcagaagga attgaaggaa gctttcagat     180

tatacgacaa ggaaggtaac ggct                                            204


<210>  186
<211>  221
<212>  DNA
<213>  nilaparvata lugens

<400>  186
cgtaaaaact ctgaccggca agaccatcac cttggaagtg gagccttccg ataccattga      60

aaacgtgaag gccaagatcc aagacaagga gggaattcct cccgaccagc agagacttat     120

cttcgctgga aagcaactgg aggatggcag aaccctgtcc gactacaaca tccaaaaaga     180

atctacactc cacttggttc tcagacttcg tggtggaact a                         221


<210>  187
<211>  221
<212>  DNA
<213>  nilaparvata lugens

<400>  187
cgtaaaaact ctgaccggca agaccatcac cttggaagtg gagccttccg ataccattga      60

aaacgtgaag gccaagatcc aagacaagga gggaattcct cccgaccagc agagacttat     120

cttcgctgga aagcaactgg aggatggcag aaccctgtcc gactacaaca tccaaaaaga     180

atctacactc cacttggttc tcagacttcg tggtggaact a                         221

<210>  188
<211>  759
<212>  DNA
<213>  Acyrthosiphon pisum

<400>  188
atggccgacg atgaagctaa gaaagcaaaa caggcggaaa tcgaccgcaa gagggccgaa      60

gtgcgcaagc gtatggaaga ggcgtccaag gccaagaagg ccaagaaggg tttcatgacc     120

ccagacagaa agaagaaact ccgtctgttg ttgaaaaaaa aggcggccga agagttgaag     180

aaagaacaag aacgcaaagc tgccgaacga aggcggatca tcgaagagcg gtgcggacaa     240

ccgaagaaca tcgacgacgc cggcgaagag gagcttgcgg aaatctgcga agaactatgg     300

aaacgggttt acaccgtaga gggcataaaa tttgacttgg aaagggatat caggatgaaa     360

gttttcgaga tcagcgaatt gaacagccaa gtcaatgact tacgaggaaa attcgtcaaa     420

ccaacattga agaaggtttc caaatacgaa aacaaattcg caaaactcca aaagaaagcg     480

gcggagttca acttcagaaa ccaactgaaa gtagtgaaga aaaaggagtt caccttggaa     540

gaagaagaca aagagaaaaa acccgattgg tccaaaaagg gagacgaaaa gaagggcgaa      600

ggagaagacg gcgacggtac cgaagacgaa aagaccgacg acggtttgac caccgaaggc     660

gaatcggtcg cgggcgatct aacggacgcg acggaagacg cgcagagcga caacgagata     720

ctcgaaccag aacccgtggt tgaacccgaa ccagaacca                            759

<210>  189
<211>  759
<212>  DNA
<213>  Acyrthosiphon pisum

<400>  189
atggccgacg atgaagctaa gaaagcaaaa caggcggaaa tcgaccgcaa gagggccgaa      60

gtgcgcaagc gtatggaaga ggcgtccaag gccaagaagg ccaagaaggg tttcatgacc     120

ccagacagaa agaagaaact ccgtctgttg ttgaaaaaaa aggcggccga agagttgaag     180

aaagaacaag aacgcaaagc tgccgaacga aggcggatca tcgaagagcg gtgcggacaa     240

ccgaagaaca tcgacgacgc cggcgaagag gagcttgcgg aaatctgcga agaactatgg     300

aaacgggttt acaccgtaga gggcataaaa tttgacttgg aaagggatat caggatgaaa     360

gttttcgaga tcagcgaatt gaacagccaa gtcaatgact tacgaggaaa attcgtcaaa     420

ccaacattga agaaggtttc caaatacgaa aacaaattcg caaaactcca aaagaaagcg     480

gcggagttca acttcagaaa ccaactgaaa gtagtgaaga aaaaggagtt caccttggaa     540

gaagaagaca aagagaaaaa acccgattgg tccaaaaagg gagacgaaaa gaagggcgaa      600

ggagaagacg gcgacggtac cgaagacgaa aagaccgacg acggtttgac caccgaaggc     660

gaatcggtcg cgggcgatct aacggacgcg acggaagacg cgcagagcga caacgagata     720

ctcgaaccag aacccgtggt tgaacccgaa ccagaacca 759

<210> 190
<211> 759
<212> DNA
<213> Acyrthosiphon pisum

<400> 190
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 191
<211> 759
<212> DNA
<213> Acyrthosiphon pisum

<400> 191
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 192
<211> 759
<212> DNA
<213> Acyrthosiphon pisum

<400> 192
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 193
<211> 759
<212> DNA
<213> Acyrthosiphon pisum

<400> 193
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 194
<211> 759
<212> DNA
<213> Acyrthosiphon pisum

<400> 194
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 195
<211> 759
<212> DNA
<213> Acyrthosiphon pisum

<400> 195
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 196
<211> 759
<212> DNA
<213> Acyrthosiphon pisum

<400> 196
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 197
<211> 759
<212> DNA
<213> Acyrthosiphon pisum

<400> 197
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 198
<211> 759
<212> DNA
<213> Acyrthosiphon pisum

<400> 198
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 199
<211> 759
<212> DNA
<213> Acyrthosiphon pisum

<400> 199
atggccgacg atgaagctaa gaaagcaaaa caggcggaaa tcgaccgcaa gagggccgaa 60

gtgcgcaagc gtatggaaga ggcgtccaag gccaagaagg ccaagaaggg tttcatgacc 120

ccagacagaa agaagaaact ccgtctgttg ttgaaaaaaa aggcggccga agagttgaag 180

aaagaacaag aacgcaaagc tgccgaacga aggcggatca tcgaagagcg gtgcggacaa 240

ccgaagaaca tcgacgacgc cggcgaagag gagcttgcgg aaatctgcga agaactatgg 300

aaacgggttt acaccgtaga gggcataaaa tttgacttgg aaagggatat caggatgaaa 360

gttttcgaga tcagcgaatt gaacagccaa gtcaatgact tacgaggaaa attcgtcaaa 420

ccaacattga agaaggtttc caaatacgaa aacaaattcg caaaactcca aaagaaagcg 480

gcggagttca acttcagaaa ccaactgaaa gtagtgaaga aaaaggagtt caccttggaa 540

gaagaagaca aagagaaaaa acccgattgg tccaaaaagg gagacgaaaa gaagggcgaa 600

ggagaagacg gcgacggtac cgaagacgaa aagaccgacg acggtttgac caccgaaggc 660

gaatcggtcg cgggcgatct aacggacgcg acggaagacg cgcagagcga caacgagata 720

ctcgaaccag aacccgtggt tgaacccgaa ccagaacca 759

<210> 200
<211> 541
<212> DNA
<213> Acyrthosiphon pisum

<400> 200
cggtaatgcg atgcggtaag aagaaggtat ggttggatcc aaacgaaata aatgaaattg 60

ccaacaccaa ttccagacaa aatattcgta agttgatcaa agatggtttg atcattaaaa 120

agccagtagc tgtacactct agggctcgtg cacgtaaaaa tgcagatgcc agaagaaaag 180

gtcgtcattg tggttttggt aaaaggaagg gtactgctaa tgctcgaaca cctcaaaaag 240

acctttgggt gaaaagaatg cgagtattaa ggcggttgct taaaaaatac cgtgaagcaa 300

agaaaattga caaccatctt taccatcagt tatacatgaa ggctaagggt aatgttttca 360

agaacaaacg tgtattgatg gagttcatcc acaaaaagaa ggcagagaag gcccgtgcca 420

agatgttgag tgatcaagct gaagctagac gtcaaaaggt taaggaagct aggaaacgta 480

aagaagcaag atttttacaa aataggaagg aacttttggc tgcatacgcc cgagaagatg 540

a 541

<210> 201
<211> 541
<212> DNA
<213> Acyrthosiphon pisum

<400> 201
cggtaatgcg atgcggtaag aagaaggtat ggttggatcc aaacgaaata aatgaaattg 60

ccaacaccaa ttccagacaa aatattcgta agttgatcaa agatggtttg atcattaaaa 120

agccagtagc tgtacactct agggctcgtg cacgtaaaaa tgcagatgcc agaagaaaag 180

gtcgtcattg tggttttggt aaaaggaagg gtactgctaa tgctcgaaca cctcaaaaag 240

acctttgggt gaaaagaatg cgagtattaa ggcggttgct taaaaaatac cgtgaagcaa 300

agaaaattga caaccatctt taccatcagt tatacatgaa ggctaagggt aatgttttca 360

agaacaaacg tgtattgatg gagttcatcc acaaaaagaa ggcagagaag gcccgtgcca 420

agatgttgag tgatcaagct gaagctagac gtcaaaaggt taaggaagct aggaaacgta 480

aagaagcaag atttttacaa aataggaagg aacttttggc tgcatacgcc cgagaagatg 540

a 541

<210> 202
<211> 823
<212> DNA
<213> Acyrthosiphon pisum

<400> 202
gttgtagtcg gaaagggtac gtccgtcttc aagttgtttt ccggcaaaga tcaaacgttg 60

ttggtctggt gggatacctt ctttgtcttg gatcttggct tttacatttt caatggaatc 120

agatgattcc acctccaatg taatggtctt tccagtgagg gtctttacaa agatttgcat 180

accaccacgg agacgcaaca ctaagtgaag ggtagattct ttctggatgt tgtagtcaga 240

aagtgtgcgt ccgtcttcaa gttgctttcc ggcaaagatc aaacgttgtt ggtcaggtgg 300

aataccttct ttgtcttgga tcttagcttt tacattttca atggaatctg atgactcaac 360

ttccaatgta atggtctttc cagtgagggt ctttacaaag atttgcatac caccacggag 420

acgcaacact aagtgaaggg tagattcttt ctggatgttg tagtcggaaa gggtacgtcc 480

gtcttcaagt tgctttccgg caaagatcaa acgttgttgg tctggtggga taccttcttt 540

gtcttggatc ttggctttta cattttcaat ggaatcagat gattccacct ccaatgtaat 600

ggtctttcca gtgagggtct ttacaaagat ttgcatacca ccacggagac gcaacactaa 660

gtgaagggta gattctttct ggatgttgta gtcggaaagg gtacgtccgt cttcaagttg 720

ctttccagca aagatcaaac gttgctggtc tggtgggata ccttccttgt cttggatctt 780

ggccttaaca ttttcaatgg aatctgatga ctcaacttcc aaa 823

<210> 203
<211> 823
<212> DNA
<213> Acyrthosiphon pisum

<400> 203
gttgtagtcg gaaagggtac gtccgtcttc aagttgtttt ccggcaaaga tcaaacgttg 60

ttggtctggt gggatacctt ctttgtcttg gatcttggct tttacatttt caatggaatc 120

agatgattcc acctccaatg taatggtctt tccagtgagg gtctttacaa agatttgcat 180

accaccacgg agacgcaaca ctaagtgaag ggtagattct ttctggatgt tgtagtcaga 240

aagtgtgcgt ccgtcttcaa gttgctttcc ggcaaagatc aaacgttgtt ggtcaggtgg 300

aataccttct ttgtcttgga tcttagcttt tacattttca atggaatctg atgactcaac 360

ttccaatgta atggtctttc cagtgagggt ctttacaaag atttgcatac caccacggag 420

acgcaacact aagtgaaggg tagattcttt ctggatgttg tagtcggaaa gggtacgtcc 480

gtcttcaagt tgctttccgg caaagatcaa acgttgttgg tctggtggga taccttcttt 540

gtcttggatc ttggctttta cattttcaat ggaatcagat gattccacct ccaatgtaat 600

ggtctttcca gtgagggtct ttacaaagat ttgcatacca ccacggagac gcaacactaa 660

gtgaagggta gattctttct ggatgttgta gtcggaaagg gtacgtccgt cttcaagttg 720

ctttccagca aagatcaaac gttgctggtc tggtgggata ccttccttgt cttggatctt 780

ggccttaaca ttttcaatgg aatctgatga ctcaacttcc aaa 823

<210> 204
<211> 172
<212> DNA
<213> Acyrthosiphon pisum

<400> 204
aagacttgct tcatcctact gcaattgaag aacgcaggaa acacaaatta aagcgccttg 60

ttcaacaccc aaactctttt ttcatggatg tcaaatgccc tggatgttat aaaattacaa 120

ctgtattcag tcacgctcag agtgtagtta tatgtaccgg atgttccaca at 172

<210> 205
<211> 172
<212> DNA
<213> Acyrthosiphon pisum

<400> 205
aagacttgct tcatcctact gcaattgaag aacgcaggaa acacaaatta aagcgccttg 60

ttcaacaccc aaactctttt ttcatggatg tcaaatgccc tggatgttat aaaattacaa 120

ctgtattcag tcacgctcag agtgtagtta tatgtaccgg atgttccaca at 172

<210> 206
<211> 25
<212> DNA
<213> Artificial

<220>
<223> Primer

<400> 206
cgaaccatct gggaagcttg gaatg 25

<210> 207
<211> 25
<212> DNA
<213> Artificial

<220>
<223> Primer

<400> 207

gcagctggag gaagagaaac gtatc                                                25

<210>  208
<211>  47
<212>  DNA
<213>  Artificial

<220>
<223>  Primer

<400>  208
gcgtaatacg actcactata ggcgaaccat ctgggaagct tggaatg                        47

<210>  209
<211>  46
<212>  DNA
<213>  Artificial

<220>
<223>  Primer

<400>  209
gcgtaatacg actcactata ggcagctgga ggaagagaaa cgtatc                         46

<210>  210
<211>  20
<212>  DNA
<213>  Artificial

<220>
<223>  Primer

<400>  210
agttcgagaa caccaggaag                                                      20

<210>  211
<211>  22
<212>  DNA
<213>  Artificial

<220>
<223>  Primer

<400>  211
cctgacacgt tgttccagct tg                                                   22

<210>  212
<211>  45
<212>  DNA
<213>  Artificial

<220>
<223>  Primer

<400>  212
gcgtaatacg actcactata ggaggagttc gagaacacca ggaag                          45

<210>  213
<211>  44
<212>  DNA
<213>  Artificial

<220>
<223>  Primer

<400>  213
gcgtaatacg actcactata ggcctgacac gttgttccag cttg                        44


<210>  214
<211>  20
<212>  DNA
<213>  Artificial

<220>
<223>  Primer

<400>  214
gcaggcgatg aagatggaga                                                   20


<210>  215
<211>  24
<212>  DNA
<213>  Artificial

<220>
<223>  Primer

<400>  215
ccacctcttt ctgcaacttc ttga                                              24


<210>  216
<211>  42
<212>  DNA
<213>  Artificial

<220>
<223>  Primer

<400>  216
gcgtaatacg actcactata gggcaggcga tgaagatgga ga                          42


<210>  217
<211>  46
<212>  DNA
<213>  Artificial

<220>
<223>  Primer

<400>  217
gcgtaatacg actcactata ggccacctct ttctgcaact tcttga                      46


<210>  218
<211>  25
<212>  DNA

<213>  Artificial

<220>
<223>  Primer

<400>  218
cagaatccca cagaatctga cgtga                                          25


<210>  219
<211>  20
<212>  DNA
<213>  Artificial

<220>
<223>  Primer

<400>  219
gcaagtggcg aagctcagct                                                20


<210>  220
<211>  47
<212>  DNA
<213>  Artificial

<220>
<223>  Primer

<400>  220
gcgtaatacg actcactata ggcagaatcc cacagaatct gacgtga                  47


<210>  221
<211>  41
<212>  DNA
<213>  Artificial

<220>
<223>  Primer

<400>  221
gcgtaatacg actcactata ggcaagtggc gaagctcagc t                        41


<210>  222
<211>  21
<212>  DNA
<213>  Artificial

<220>
<223>  Primer

<400>  222
cgtgtttgcc atgttcgatc a                                              21


<210>  223
<211>  23
<212>  DNA
<213>  Artificial

<220>

<223>  Primer

<400>  223
ggtacatttc gtccacgtct tca                                              23


<210>  224
<211>  43
<212>  DNA
<213>  Artificial

<220>
<223>  Primer

<400>  224
gcgtaatacg actcactata ggcgtgtttg ccatgttcga tca                        43


<210>  225
<211>  43
<212>  DNA
<213>  Artificial

<220>
<223>  Primer

<400>  225
gcgtaatacg actcactata ggtacatttc gtccacgtct tca                        43


<210>  226
<211>  23
<212>  DNA
<213>  Artificial

<220>
<223>  Primer

<400>  226
gacttgatct tcagccgacc att                                              23


<210>  227
<211>  23
<212>  DNA
<213>  Artificial

<220>
<223>  Primer

<400>  227
ccattgccag ttcctcaact tca                                              23


<210>  228
<211>  44
<212>  DNA
<213>  Artificial

<220>
<223>  Primer

<400>  228

gcgtaatacg actcactata ggacttgatc ttcagccgac catt 44

<210> 229
<211> 45
<212> DNA
<213> Artificial

<220>
<223> Primer

<400> 229
gcgtaatacg actcactata ggccattgcc agttcctcaa cttca 45

<210> 230
<211> 21
<212> DNA
<213> Artificial

<220>
<223> Primer

<400> 230
cgcaatgatc tcctccagga t 21

<210> 231
<211> 24
<212> DNA
<213> Artificial

<220>
<223> Primer

<400> 231
ggtcatcatc tccatgaact cgtc 24

<210> 232
<211> 43
<212> DNA
<213> Artificial

<220>
<223> Primer

<400> 232
gcgtaatacg actcactata ggcgcaatga tctcctccag gat 43

<210> 233
<211> 45
<212> DNA
<213> Artificial

<220>
<223> Primer

<400> 233
gcgtaatacg actcactata gggtcatcat ctccatgaac tcgtc 45

<210>  234
<211>  26
<212>  DNA
<213>  Artificial

<220>
<223>  Primer

<400>  234
cgtcactaat cggactggtc taacag                                            26


<210>  235
<211>  24
<212>  DNA
<213>  Artificial

<220>
<223>  Primer

<400>  235
ggtcatcatc tccatgaact cgtc                                              24


<210>  236
<211>  48
<212>  DNA
<213>  Artificial

<220>
<223>  Primer

<400>  236
gcgtaatacg actcactata ggcgtcacta atcggactgg tctaacag                    48


<210>  237
<211>  45
<212>  DNA
<213>  Artificial

<220>
<223>  Primer

<400>  237
gcgtaatacg actcactata gggtcatcat ctccatgaac tcgtc                       45


<210>  238
<211>  23
<212>  DNA
<213>  Artificial

<220>
<223>  Primer

<400>  238
ggtgaaggag ggtgcctgct cag                                               23


<210>  239
<211>  25
<212>  DNA

<213>  Artificial

<220>
<223>  Primer

<400>  239
cagggtgaat agaacgaggt actcg                                              25


<210>  240
<211>  40
<212>  DNA
<213>  Artificial

<220>
<223>  Primer

<400>  240
aatacgactc actatagggc gctatgaaat tccaagcaca                              40


<210>  241
<211>  47
<212>  DNA
<213>  Artificial

<220>
<223>  Primer

<400>  241
gcgtaatacg actcactata ggcagggtga atagaacgag gtactcg                      47


<210>  242
<211>  29
<212>  DNA
<213>  Artificial

<220>
<223>  Primer

<400>  242
ctcaacgaag gtcttgtcag tggctttgg                                          29


<210>  243
<211>  20
<212>  DNA
<213>  Artificial

<220>
<223>  Primer

<400>  243
ttcgcctggc ttcttcgtga                                                    20


<210>  244
<211>  44
<212>  DNA
<213>  Artificial

<220>

<223>  Primer

<400>  244
gcgtaatacg actcactata ggccacgccg acttaattca ttcc                        44


<210>  245
<211>  42
<212>  DNA
<213>  Artificial

<220>
<223>  Primer

<400>  245
gcgtaatacg actcactata ggttcgcctg gcttcttcgt ga                          42


<210>  246
<211>  22
<212>  DNA
<213>  Artificial

<220>
<223>  Primer

<400>  246
tgtcgatggc ggtcttaaca tc                                                22


<210>  247
<211>  24
<212>  DNA
<213>  Artificial

<220>
<223>  Primer

<400>  247
tctccagctt ccactttctt gaga                                              24


<210>  248
<211>  44
<212>  DNA
<213>  Artificial

<220>
<223>  Primer

<400>  248
gcgtaatacg actcactata ggtgtcgatg gcggtcttaa catc                        44


<210>  249
<211>  46
<212>  DNA
<213>  Artificial

<220>
<223>  Primer

<400>  249

gcgtaatacg actcactata ggtctccagc ttccactttc ttgaga 46

<210> 250
<211> 20
<212> DNA
<213> Artificial

<220>
<223> Primer

<400> 250
aacgtgcatt tcgcgtaccc 20

<210> 251
<211> 20
<212> DNA
<213> Artificial

<220>
<223> Primer

<400> 251
tgatgggcat aactggcagg 20

<210> 252
<211> 42
<212> DNA
<213> Artificial

<220>
<223> Primer

<400> 252
gcgtaatacg actcactata ggaacgtgca tttcgcgtac cc 42

<210> 253
<211> 42
<212> DNA
<213> Artificial

<220>
<223> Primer

<400> 253
gcgtaatacg actcactata ggtgatgggc ataactggca gg 42

<210> 254
<211> 22
<212> DNA
<213> Artificial

<220>
<223> Primer

<400> 254
ccttattgaa cgtggtcgac ag 22

<210>  255
<211>  20
<212>  DNA
<213>  Artificial

<220>
<223>  Primer

<400>  255
ctgatgtagt ccttgaggag                                                    20


<210>  256
<211>  44
<212>  DNA
<213>  Artificial

<220>
<223>  Primer

<400>  256
gcgtaatacg actcactata ggccttattg aacgtggtcg acag                         44


<210>  257
<211>  42
<212>  DNA
<213>  Artificial

<220>
<223>  Primer

<400>  257
gcgtaatacg actcactata ggctgatgta gtccttgagg ag                           42


<210>  258
<211>  26
<212>  DNA
<213>  Artificial

<220>
<223>  Primer

<400>  258
gtacggacgg gtagtttagt tgtgtc                                             26


<210>  259
<211>  20
<212>  DNA
<213>  Artificial

<220>
<223>  Primer

<400>  259
ttgccaagtg ccaagtcacg                                                    20


<210>  260
<211>  48
<212>  DNA

<213>  Artificial

<220>
<223>  Primer

<400>  260
gcgtaatacg actcactata gggtacggac gggtagttta gttgtgtc                    48


<210>  261
<211>  42
<212>  DNA
<213>  Artificial

<220>
<223>  Primer

<400>  261
gcgtaatacg actcactata ggttgccaag tgccaagtca cg                          42


<210>  262
<211>  22
<212>  DNA
<213>  Artificial

<220>
<223>  Primer

<400>  262
ctgttgtcgg ctggtcatat cc                                                22


<210>  263
<211>  20
<212>  DNA
<213>  Artificial

<220>
<223>  Primer

<400>  263
taacgtaacg catcgccacc                                                   20


<210>  264
<211>  44
<212>  DNA
<213>  Artificial

<220>
<223>  Primer

<400>  264
gcgtaatacg actcactata ggctgttgtc ggctggtcat atcc                        44


<210>  265
<211>  42
<212>  DNA
<213>  Artificial

<220>

<223>  Primer

<400>  265
gcgtaatacg actcactata ggtaacgtaa cgcatcgcca cc                    42


<210>  266
<211>  20
<212>  DNA
<213>  Artificial

<220>
<223>  Primer

<400>  266
agccctcatc cgtgatttgg                                              20


<210>  267
<211>  20
<212>  DNA
<213>  Artificial

<220>
<223>  Primer

<400>  267
gatccggcct caatttgacg                                              20


<210>  268
<211>  42
<212>  DNA
<213>  Artificial

<220>
<223>  Primer

<400>  268
gcgtaatacg actcactata ggagccctca tccgtgattt gg                    42


<210>  269
<211>  42
<212>  DNA
<213>  Artificial

<220>
<223>  Primer

<400>  269
gcgtaatacg actcactata gggatccggc ctcaatttga cg                    42


<210>  270
<211>  22
<212>  DNA
<213>  Artificial

<220>
<223>  Primer

<400>  270

acgtttctct gctcattcgt gc                                                22

<210>  271
<211>  20
<212>  DNA
<213>  Artificial

<220>
<223>  Primer

<400>  271
cttgtacaaa gtgtgcaggg                                                   20

<210>  272
<211>  44
<212>  DNA
<213>  Artificial

<220>
<223>  Primer

<400>  272
gcgtaatacg actcactata ggacgtttct ctgctcattc gtgc                        44

<210>  273
<211>  42
<212>  DNA
<213>  Artificial

<220>
<223>  Primer

<400>  273
gcgtaatacg actcactata ggcttgtaca aagtgtgcag gg                          42

<210>  274
<211>  22
<212>  DNA
<213>  Artificial

<220>
<223>  Primer

<400>  274
ggttttcttc ttgcccgaat cg                                                22

<210>  275
<211>  20
<212>  DNA
<213>  Artificial

<220>
<223>  Primer

<400>  275
tttggggttc ggcttggttg                                                   20

<210>  276
<211>  44
<212>  DNA
<213>  Artificial

<220>
<223>  Primer

<400>  276
gcgtaatacg actcactata ggggttttct tcttgcccga atcg                        44


<210>  277
<211>  42
<212>  DNA
<213>  Artificial

<220>
<223>  Primer

<400>  277
gcgtaatacg actcactata ggtttggggt tcggcttggt tg                          42


<210>  278
<211>  23
<212>  DNA
<213>  Artificial

<220>
<223>  Primer

<400>  278
tcagcgagat ccctaagaca acg                                               23


<210>  279
<211>  20
<212>  DNA
<213>  Artificial

<220>
<223>  Primer

<400>  279
ccccacatgt tgatgacgca                                                   20


<210>  280
<211>  45
<212>  DNA
<213>  Artificial

<220>
<223>  Primer

<400>  280
gcgtaatacg actcactata ggtcagcgag atccctaaga caacg                       45


<210>  281
<211>  42
<212>  DNA

<213>  Artificial

<220>
<223>  Primer

<400>  281
gcgtaatacg actcactata ggccccacat gttgatgacg ca                          42


<210>  282
<211>  23
<212>  DNA
<213>  Artificial

<220>
<223>  Primer

<400>  282
ggtttatgac ccctgagagg aag                                               23


<210>  283
<211>  22
<212>  DNA
<213>  Artificial

<220>
<223>  Primer

<400>  283
ctccagggtg aactccttct tc                                                22


<210>  284
<211>  43
<212>  DNA
<213>  Artificial

<220>
<223>  Primer

<400>  284
gcgtaatacg actcactata ggtttatgac ccctgagagg aag                         43


<210>  285
<211>  44
<212>  DNA
<213>  Artificial

<220>
<223>  Primer

<400>  285
gcgtaatacg actcactata ggctccaggg tgaactcctt cttc                        44


<210>  286
<211>  24
<212>  DNA
<213>  Artificial

<220>

<223>  Primer

<400>  286
caaggaccag aacaagaaca aggg                                              24


<210>  287
<211>  26
<212>  DNA
<213>  Artificial

<220>
<223>  Primer

<400>  287
gacgttcata tttggaggct acttgg                                            26


<210>  288
<211>  46
<212>  DNA
<213>  Artificial

<220>
<223>  Primer

<400>  288
gcgtaatacg actcactata ggcaaggacc agaacaagaa caaggg                      46


<210>  289
<211>  47
<212>  DNA
<213>  Artificial

<220>
<223>  Primer

<400>  289
gcgtaatacg actcactata ggacgttcat atttggaggc tacttgg                     47


<210>  290
<211>  25
<212>  DNA
<213>  Artificial

<220>
<223>  Primer

<400>  290
aatctcgtac actgttggaa caagc                                             25


<210>  291
<211>  25
<212>  DNA
<213>  Artificial

<220>
<223>  Primer

<400>  291

ggttcttacg gtcttcttca gcttg                                                25

<210>  292
<211>  47
<212>  DNA
<213>  Artificial

<220>
<223>  Primer

<400>  292
gcgtaatacg actcactata ggaatctcgt acactgttgg aacaagc                        47

<210>  293
<211>  45
<212>  DNA
<213>  Artificial

<220>
<223>  Primer

<400>  293
gcgtaatacg actcactata ggttcttacg gtcttcttca gcttg                          45

<210>  294
<211>  22
<212>  DNA
<213>  Artificial

<220>
<223>  Primer

<400>  294
aagaagaagc tcaggttgtt gc                                                   22

<210>  295
<211>  20
<212>  DNA
<213>  Artificial

<220>
<223>  Primer

<400>  295
tcattcacct ggctgttgag                                                      20

<210>  296
<211>  44
<212>  DNA
<213>  Artificial

<220>
<223>  Primer

<400>  296
gcgtaatacg actcactata ggaagaagaa gctcaggttg ttgc                           44

<210>  297
<211>  42
<212>  DNA
<213>  Artificial

<220>
<223>  Primer

<400>  297
gcgtaatacg actcactata ggtcattcac ctggctgttg ag                          42


<210>  298
<211>  20
<212>  DNA
<213>  Artificial

<220>
<223>  Primer

<400>  298
acatcctcag gctcatggga                                                   20


<210>  299
<211>  20
<212>  DNA
<213>  Artificial

<220>
<223>  Primer

<400>  299
agccgttacc ttccttgtcg                                                   20


<210>  300
<211>  42
<212>  DNA
<213>  Artificial

<220>
<223>  Primer

<400>  300
gcgtaatacg actcactata ggacatcctc aggctcatgg ga                          42


<210>  301
<211>  42
<212>  DNA
<213>  Artificial

<220>
<223>  Primer

<400>  301
gcgtaatacg actcactata ggagccgtta ccttccttgt cg                          42


<210>  302
<211>  20
<212>  DNA

<213>  Artificial

<220>
<223>  Primer

<400>  302
acatcctcag gctcatggga                                                    20


<210>  303
<211>  20
<212>  DNA
<213>  Artificial

<220>
<223>  Primer

<400>  303
agccgttacc ttccttgtcg                                                    20


<210>  304
<211>  42
<212>  DNA
<213>  Artificial

<220>
<223>  Primer

<400>  304
gcgtaatacg actcactata ggacatcctc aggctcatgg ga                           42


<210>  305
<211>  42
<212>  DNA
<213>  Artificial

<220>
<223>  Primer

<400>  305
gcgtaatacg actcactata ggagccgtta ccttccttgt cg                           42


<210>  306
<211>  24
<212>  DNA
<213>  Artificial

<220>
<223>  Primer

<400>  306
cgtaaaaact ctgaccggca agac                                               24


<210>  307
<211>  26
<212>  DNA
<213>  Artificial

<220>

<223>  Primer

<400>  307
tagttccacc acgaagtctg agaacc                                          26


<210>  308
<211>  46
<212>  DNA
<213>  Artificial

<220>
<223>  Primer

<400>  308
gcgtaatacg actcactata ggcgtaaaaa ctctgaccgg caagac                    46


<210>  309
<211>  48
<212>  DNA
<213>  Artificial

<220>
<223>  Primer

<400>  309
gcgtaatacg actcactata ggtagttcca ccacgaagtc tgagaacc                  48


<210>  310
<211>  21
<212>  DNA
<213>  Artificial

<220>
<223>  Primer

<400>  310
atggccgacg atgaagctaa g                                               21


<210>  311
<211>  20
<212>  DNA
<213>  Artificial

<220>
<223>  Primer

<400>  311
tggttgtggt tctggttcgg                                                 20


<210>  312
<211>  43
<212>  DNA
<213>  Artificial

<220>
<223>  Primer

<400>  312

gcgtaatacg actcactata ggatggccga cgatgaagct aag                43

<210>  313
<211>  42
<212>  DNA
<213>  Artificial

<220>
<223>  Primer

<400>  313
gcgtaatacg actcactata ggtggttctg gttcgggttc aa                42

<210>  314
<211>  20
<212>  DNA
<213>  Artificial

<220>
<223>  Primer

<400>  314
cggtaatgcg atgcggtaag                                         20

<210>  315
<211>  20
<212>  DNA
<213>  Artificial

<220>
<223>  Primer

<400>  315
tcatcttctc gggcgtatgc                                         20

<210>  316
<211>  42
<212>  DNA
<213>  Artificial

<220>
<223>  Primer

<400>  316
gcgtaatacg actcactata ggcggtaatg cgatgcggta ag                42

<210>  317
<211>  42
<212>  DNA
<213>  Artificial

<220>
<223>  Primer

<400>  317
gcgtaatacg actcactata ggtcatcttc tcgggcgtat gc                42

<210>  318
<211>  25
<212>  DNA
<213>  Artificial

<220>
<223>  Primer

<400>  318
tttggaagtt gagtcatcag attcc                                          25


<210>  319
<211>  24
<212>  DNA
<213>  Artificial

<220>
<223>  Primer

<400>  319
gttgtagtcg gaaagggtac gtcc                                           24


<210>  320
<211>  47
<212>  DNA
<213>  Artificial

<220>
<223>  Primer

<400>  320
gcgtaatacg actcactata ggtttggaag ttgagtcatc agattcc                  47


<210>  321
<211>  46
<212>  DNA
<213>  Artificial

<220>
<223>  Primer

<400>  321
gcgtaatacg actcactata gggttgtagt cggaaagggt acgtcc                   46


<210>  322
<211>  23
<212>  DNA
<213>  Artificial

<220>
<223>  Primer

<400>  322
aagacttgct tcatcctact gca                                            23


<210>  323
<211>  20
<212>  DNA

<213>  Artificial

<220>
<223>  Primer

<400>  323
attgtggaac atccggtaca                                                   20


<210>  324
<211>  45
<212>  DNA
<213>  Artificial

<220>
<223>  Primer

<400>  324
gcgtaatacg actcactata ggaagacttg cttcatccta ctgca                       45


<210>  325
<211>  42
<212>  DNA
<213>  Artificial

<220>
<223>  Primer

<400>  325
gcgtaatacg actcactata ggattgtgga acatccggta ca                          42


<210>  326
<211>  424
<212>  PRT
<213>  Lygus hesperus

<400>  326

Met Ile Pro Pro Thr Ser Arg Pro Gln Val Thr Val Tyr Ser Asp Lys
1               5                   10                  15


Asn Glu Ala Thr Gly Thr Leu Leu Asn Leu Pro Ala Val Phe Asn Ala
            20                  25                  30


Pro Ile Arg Pro Asp Val Val Asn Phe Val His Gln Asn Val Ala Lys
        35                  40                  45


Asn His Arg Gln Pro Tyr Cys Val Ser Ala Gln Ala Gly His Gln Thr
    50                  55                  60


Ser Ala Glu Ser Trp Gly Thr Gly Arg Ala Val Ala Arg Ile Pro Arg
65                  70                  75                  80


Val Arg Gly Gly Gly Thr His Arg Ser Gly Gln Gly Ala Phe Gly Asn
                85                  90                  95

Met Cys Arg Gly Gly Arg Met Phe Ala Pro Thr Arg Pro Trp Arg Arg
            100                 105                 110

Trp His Arg Lys Ile Asn Val Asn Gln Lys Arg Tyr Ala Val Val Ser
        115                 120                 125

Ala Ile Ala Ala Ser Gly Val Pro Ala Leu Val Met Ser Lys Gly His
    130                 135                 140

Met Val Gln Ser Val Pro Glu Phe Pro Leu Val Val Ser Asp Lys Val
145                 150                 155                 160

Gln Glu Tyr Thr Lys Thr Lys Gln Ala Val Ile Phe Leu His Arg Ile
                165                 170                 175

Lys Ala Trp Gln Asp Ile Gln Lys Val Tyr Lys Ser Lys Arg Phe Arg
            180                 185                 190

Ala Gly Lys Gly Lys Met Arg Asn Arg Arg Arg Ile Gln Arg Arg Gly
        195                 200                 205

Pro Leu Ile Ile Tyr Asp Gln Asp Gln Gly Leu Asn Arg Ala Phe Arg
    210                 215                 220

Asn Ile Pro Gly Val Asp Leu Ile Glu Val Ser Arg Leu Asn Leu Leu
225                 230                 235                 240

Lys Leu Ala Pro Gly Gly His Ile Gly Arg Phe Val Ile Trp Thr Gln
                245                 250                 255

Ser Ala Phe Glu Lys Leu Asp Ala Leu Tyr Gly Thr Trp Lys Lys Lys
            260                 265                 270

Ser Thr Leu Lys Ala Gly Tyr Asn Leu Pro Met Pro Lys Met Ala Asn
        275                 280                 285

Thr Asp Leu Ser Arg Leu Phe Lys Ala Pro Glu Ile Lys Ala Val Leu
    290                 295                 300

Arg Asn Pro Lys Lys Thr Ile Val Arg Arg Val Arg Lys Leu Asn Pro
305                 310                 315                 320

Leu Arg Asn Thr Arg Ala Met Leu Arg Leu Asn Pro Tyr Ala Ala Val
                325                 330                 335

Leu Lys Arg Lys Ala Ile Leu Asp Gln Arg Lys Leu Lys Leu Gln Lys
            340                 345                 350

Leu Val Glu Ala Ala Lys Lys Gly Asp Thr Lys Leu Ser Pro Arg Val
        355                 360                 365

Glu Arg His Leu Lys Met Ile Glu Arg Arg Lys Ala Leu Ile Lys Lys
    370                 375                 380

Ala Lys Ala Ala Lys Pro Lys Lys Pro Lys Thr Ala Lys Lys Pro Lys
385                 390                 395                 400

Thr Ala Glu Lys Ala Pro Ala Pro Ala Lys Lys Ala Ala Ala Pro Lys
                405                 410                 415

Lys Ala Thr Thr Pro Ala Lys Lys
            420

<210>  327
<211>  242
<212>  PRT
<213>  Lygus hesperus

<400>  327

Met Ala Asn Ala Lys Pro Ile Ser Lys Lys Lys Lys Phe Val Ser Asp
1               5                   10                  15

Gly Val Phe Lys Ala Glu Leu Asn Glu Phe Leu Thr Arg Glu Leu Ala
            20                  25                  30

Glu Glu Gly Tyr Ser Gly Val Glu Val Arg Val Thr Pro Asn Lys Thr
        35                  40                  45

Glu Ile Ile Ile Met Ala Thr Arg Thr Gln Ser Val Leu Gly Asp Lys
    50                  55                  60

Gly Arg Arg Ile Arg Glu Leu Thr Ser Val Val Gln Lys Arg Phe Asn
65                  70                  75                  80

Phe Lys Pro Gln Thr Leu Asp Leu Tyr Ala Glu Lys Val Ala Thr Arg
                85                  90                  95

Gly Leu Cys Ala Ile Ala Gln Ala Glu Ser Leu Arg Tyr Lys Leu Ile
            100                 105                 110

Gly Gly Leu Ala Val Arg Gly Ala Cys Tyr Gly Val Leu Arg Phe Ile
        115                 120                 125

Met Glu Asn Gly Ala Lys Gly Cys Glu Val Val Val Ser Gly Lys Leu
    130                 135                 140

Arg Gly Gln Arg Ala Lys Ser Met Lys Phe Val Asp Gly Leu Met Ile
145                 150                 155                 160

His Ser Gly Asp Pro Cys Asn Glu Tyr Val Asp Thr Ala Thr Arg His
                165                 170                 175

Val Leu Leu Arg Gln Gly Val Leu Gly Ile Lys Val Lys Ile Met Leu
            180                 185                 190

Pro Trp Asp Val Thr Gly Lys Asn Gly Pro Lys Asn Pro Leu Pro Asp
        195                 200                 205

His Val Ser Val Leu Leu Pro Lys Glu Glu Leu Pro Asn Leu Ala Val
    210                 215                 220

Ser Val Pro Gly Ser Asp Ile Lys Pro Lys Pro Glu Val Pro Ala Pro
225                 230                 235                 240

Ala Leu

<210>  328
<211>  262
<212>  PRT
<213>  Lygus hesperus

<400>  328

Met Ala Val Gly Lys Asn Lys Gly Leu Ser Lys Gly Gly Lys Lys Gly
1               5                   10                  15

Val Lys Lys Lys Val Val Asp Pro Phe Thr Arg Lys Asp Trp Tyr Asp
            20                  25                  30

Val Lys Ala Pro Ser Met Phe Lys Lys Arg Gln Val Gly Lys Thr Leu
        35                  40                  45

Val Asn Arg Thr Gln Gly Thr Lys Ile Ala Ser Glu Gly Leu Lys Gly
    50                  55                  60

Arg Val Phe Glu Val Ser Leu Ala Asp Ile Gln Glu Asp Thr Asp Ala
65                  70                  75                  80

Glu Arg Ser Phe Arg Lys Phe Arg Leu Ile Ala Glu Asp Val Gln Ala
                85                  90                  95

Arg Asn Val Leu Thr Asn Phe His Gly Met Asp Leu Thr Thr Asp Lys
            100                 105                 110

Leu Arg Ser Met Val Lys Lys Trp Gln Thr Leu Ile Glu Ala Asn Val
        115                 120                 125

Asp Val Lys Thr Thr Asp Gly Tyr Leu Leu Arg Val Phe Cys Ile Gly
    130                 135                 140

Phe Thr Asn Lys Asp Gln Leu Ser Gln Arg Lys Thr Cys Tyr Ala Gln
145                 150                 155                 160

His Asn Gln Val Arg Glu Ile Arg Lys Lys Met Val Lys Asn Ile Ser
                165                 170                 175

Asp Ser Ile Ser Ser Cys Asp Leu Arg Ser Val Val Asn Lys Leu Ile
            180                 185                 190

Pro Asp Ser Ile Ala Lys Asp Ile Glu Lys Asn Cys Gln Gly Ile Tyr
        195                 200                 205

Pro Leu His Asp Val Tyr Ile Arg Lys Val Lys Val Leu Lys Lys Pro
    210                 215                 220

Arg Phe Glu Leu Ser Lys Leu Leu Glu Leu His Val Asp Gly Lys Gly
225                 230                 235                 240

Ile Asp Glu Pro Gly Ala Lys Val Thr Arg Thr Asp Ala Tyr Glu Pro
                245                 250                 255

Pro Val Gln Glu Ser Val
            260


<210>  329
<211>  152
<212>  PRT
<213>  Lygus hesperus

<400>  329

Met Ser Leu Met Leu Pro Glu Lys Phe Gln His Ile Leu Arg Ile Met
1               5                   10                  15

Gly Thr Asn Ile Asp Gly Lys Arg Lys Val Met Phe Ala Met Thr Ala
            20                  25                  30

Ile Lys Gly Val Gly Arg Arg Tyr Ala Asn Ile Val Leu Lys Lys Ala
        35                  40                  45

Asp Val Asn Leu Asp Lys Arg Ala Gly Glu Cys Ser Glu Glu Glu Val
    50                  55                  60

Glu Lys Ile Val Thr Ile Met Gln Asn Pro Arg Gln Tyr Lys Ile Pro
65                  70                  75                  80

Asn Trp Phe Leu Asn Arg Gln Lys Asp Thr Val Glu Gly Lys Tyr Ser
                85                  90                  95

Gln Leu Thr Ser Ser Leu Leu Asp Ser Lys Leu Arg Asp Asp Leu Glu
            100                 105                 110

Arg Leu Lys Lys Ile Arg Ala His Arg Gly Met Arg His Tyr Trp Gly
        115                 120                 125

Leu Arg Val Arg Gly Gln His Thr Lys Thr Thr Gly Arg Arg Gly Arg
    130                 135                 140

Thr Val Gly Val Ser Lys Lys Lys
145                 150

<210>  330
<211>  381
<212>  PRT
<213>  Lygus hesperus

<400>  330

Met Ser Asp Glu Glu Tyr Ser Glu Ser Glu Glu Glu Thr Gln Pro Glu
1               5                   10                  15

Pro Gln Lys Lys Pro Glu Ala Glu Gly Gly Gly Asp Pro Glu Phe Val
            20                  25                  30

Lys Arg Lys Glu Ala Gln Thr Ser Ala Leu Asp Glu Gln Leu Lys Asp
        35                  40                  45

Tyr Ile Ala Glu Trp Arg Lys Gln Arg Ala Arg Glu Glu Glu Asp Leu
    50                  55                  60

Lys Lys Leu Lys Glu Lys Gln Ala Lys Arg Lys Val Ala Arg Ala Glu
65                  70                  75                  80

Glu Glu Lys Arg Leu Ala Glu Lys Lys Lys Gln Glu Glu Glu Arg Arg
                85                  90                  95

Val Arg Glu Ala Glu Glu Lys Lys Gln Arg Glu Ile Glu Glu Lys Arg
            100                 105                 110

Arg Arg Leu Glu Glu Ala Glu Lys Lys Arg Gln Ala Met Met Ala Ala
        115                 120                 125

Leu Lys Asp Gln Ser Lys Thr Lys Gly Pro Asn Phe Val Val Asn Lys
    130                 135                 140

Lys Ala Glu Thr Leu Gly Met Ser Ser Ala Gln Ile Glu Arg Asn Lys
145                 150                 155                 160

Thr Lys Glu Gln Leu Glu Glu Glu Lys Arg Ile Ser Leu Ser Ile Arg
                165                 170                 175

Leu Lys Pro Leu Ala Ile Glu Asn Met Ser Ile Asp Arg Leu Arg Ile
            180                 185                 190

Lys Ala Gln Glu Leu Trp Glu Ala Ile Val Lys Leu Glu Thr Glu Lys
        195                 200                 205

Tyr Asp Leu Glu Glu Arg Gln Lys Arg Gln Asp Tyr Asp Leu Lys Glu
    210                 215                 220

Leu Lys Glu Arg Gln Lys Gln Gln Leu Arg His Lys Ala Leu Lys Lys
225                 230                 235                 240

Gly Leu Asp Pro Glu Ala Leu Thr Gly Lys Tyr Pro Pro Lys Ile Gln
                245                 250                 255

Val Ala Ser Lys Tyr Glu Arg Arg Val Asp Thr Arg Ser Tyr Asp Asp
            260                 265                 270

Lys Lys Lys Leu Phe Glu Gly Gly Ile Leu Glu Arg Tyr Lys Glu Leu
        275                 280                 285

Ile Glu Lys Val Trp Thr Glu Lys Val Asp Gln Phe Gly Ser Arg Ala
    290                 295                 300

His Ser Lys Leu Pro Arg Trp Phe Gly Glu Arg Pro Gly Lys Lys Lys
305                 310                 315                 320

Asp Ala Pro Glu Ser Pro Glu Glu Glu Glu Val Lys Val Glu Asp Glu
                325                 330                 335

Pro Glu Ala Glu Pro Ser Phe Met Leu Asp Glu Glu Glu Glu Ala
            340                 345                 350

Glu Glu Glu Glu Ala Glu Glu Glu Glu Glu Ala Glu Glu Glu Glu Glu
        355                 360                 365

Glu Glu Glu Glu Glu Glu Glu Glu Glu Glu Glu Glu Glu

370                 375                 380

<210>  331
<211>  1689
<212>  PRT
<213>  Lygus hesperus

<400>  331

Ser Gly Lys Leu Ala Gly Ala Asp Ile Glu Thr Tyr Leu Leu Glu Lys
1               5                   10                  15

Ala Arg Val Ile Ser Gln Gln Thr Leu Glu Arg Ser Tyr His Ile Phe
            20                  25                  30

Tyr Gln Met Met Ser Gly Ala Val Lys Gly Val Lys Glu Met Cys Leu
        35                  40                  45

Leu Val Asp Asp Ile Tyr Thr Tyr Asn Phe Ile Ser Gln Gly Lys Val
    50                  55                  60

Ser Ile Ala Gly Val Asp Asp Gly Glu Glu Met Val Leu Thr Asp Gln
65                  70                  75                  80

Ala Phe Asp Ile Leu Gly Phe Thr Lys Gln Glu Lys Glu Asp Ile Tyr
                85                  90                  95

Lys Ile Thr Ala Ala Val Ile His Met Gly Thr Met Lys Phe Lys Gln
            100                 105                 110

Arg Gly Arg Glu Glu Gln Ala Glu Ala Asp Gly Thr Glu Glu Gly Gly
        115                 120                 125

Lys Val Gly Val Leu Leu Gly Ile Asp Gly Asp Asp Leu Tyr Lys Asn
    130                 135                 140

Met Cys Lys Pro Arg Ile Lys Val Gly Thr Glu Phe Val Thr Gln Gly
145                 150                 155                 160

Lys Asn Val Asn Gln Val Ser Tyr Ser Leu Gly Ala Met Ser Lys Gly
                165                 170                 175

Met Phe Asp Arg Leu Phe Lys Phe Leu Val Lys Lys Cys Asn Glu Thr
            180                 185                 190

Leu Asp Thr Lys Gln Lys Arg Gln His Phe Ile Gly Val Leu Asp Ile
        195                 200                 205

Ala Gly Phe Glu Ile Phe Asp Phe Asn Gly Phe Glu Gln Leu Cys Ile

210                 215                 220

Asn Phe Thr Asn Glu Lys Leu Gln Gln Phe Phe Asn His His Met Phe
225                 230                 235                 240

Val Leu Glu Gln Glu Glu Tyr Lys Arg Glu Gly Ile Asn Trp Ala Phe
                245                 250                 255

Ile Asp Phe Gly Met Asp Leu Leu Ala Cys Ile Glu Leu Ile Glu Lys
            260                 265                 270

Pro Met Gly Ile Leu Ser Ile Leu Glu Glu Glu Ser Met Phe Pro Lys
        275                 280                 285

Ala Thr Asp Lys Thr Phe Glu Asp Lys Leu Ile Thr Asn His Leu Gly
    290                 295                 300

Lys Ser Pro Asn Phe Arg Lys Pro Ala Val Pro Lys Pro Gly Gln Gln
305                 310                 315                 320

Ala Gly His Phe Ala Ile Ala His Tyr Ala Gly Cys Val Ser Tyr Asn
                325                 330                 335

Ile Thr Gly Trp Leu Glu Lys Asn Lys Asp Pro Leu Asn Asp Thr Val
            340                 345                 350

Val Asp Gln Tyr Lys Lys Gly Thr Asn Lys Leu Leu Cys Glu Ile Phe
        355                 360                 365

Ala Asp His Pro Gly Gln Ser Gly Ala Pro Gly Gly Asp Ala Gly Gly
    370                 375                 380

Lys Gly Gly Arg Gly Lys Lys Gly Gly Gly Phe Ala Thr Val Ser Ser
385                 390                 395                 400

Ser Tyr Lys Glu Gln Leu Asn Asn Leu Met Thr Thr Leu Lys Ser Thr
                405                 410                 415

Gln Pro His Phe Val Arg Cys Ile Ile Pro Asn Glu Leu Lys Gln Pro
            420                 425                 430

Gly Val Ile Asp Ser His Leu Val Met His Gln Leu Thr Cys Asn Gly
        435                 440                 445

Val Leu Glu Gly Ile Arg Ile Cys Arg Lys Gly Phe Pro Asn Arg Met
    450                 455                 460

Asn Tyr Pro Asp Phe Lys Leu Arg Tyr Lys Ile Leu Asn Pro Ala Ala
465                 470                 475                 480

Val Asp Arg Glu Ser Asp Ile Leu Lys Ala Ala Gly Leu Val Leu Glu
                485                 490                 495

Ser Thr Gly Leu Asp Pro Asp Met Tyr Arg Leu Gly His Thr Lys Val
            500                 505                 510

Phe Phe Arg Ala Gly Val Leu Gly Gln Leu Glu Glu Leu Arg Asp Asp
        515                 520                 525

Arg Leu Ser Lys Ile Ile Gly Trp Met Gln Ala Phe Met Arg Gly Tyr
    530                 535                 540

Leu Val Arg Lys Glu Tyr Lys Lys Leu Gln Glu Gln Arg Leu Ala Leu
545                 550                 555                 560

Gln Val Val Gln Arg Asn Leu Arg Arg Tyr Leu Gln Leu Arg Thr Trp
                565                 570                 575

Pro Trp Trp Lys Met Trp Ser Arg Val Lys Pro Leu Leu Asn Val Ala
            580                 585                 590

Asn Val Glu Glu Glu Met Arg Lys Leu Glu Glu Leu Val Ala Glu Thr
        595                 600                 605

Gln Ala Ala Leu Glu Lys Glu Glu Lys Leu Arg Lys Glu Ala Glu Ala
    610                 615                 620

Leu Asn Ala Lys Leu Leu Gln Glu Lys Thr Asp Leu Leu Arg Asn Leu
625                 630                 635                 640

Glu Gly Glu Lys Gly Ser Ile Ser Gly Ile Gln Glu Arg Cys Ala Lys
                645                 650                 655

Leu Gln Ala Gln Lys Ala Asp Leu Glu Ser Gln Leu Met Asp Thr Gln
            660                 665                 670

Glu Arg Leu Gln Asn Glu Glu Asp Ala Arg Asn Gln Leu Phe Gln Gln
        675                 680                 685

Lys Lys Lys Leu Glu Gln Glu Ala Ala Ala Leu Lys Lys Asp Ile Glu
    690                 695                 700

Asp Leu Glu Leu Ser Asn Gln Lys Thr Asp Gln Asp Lys Ala Ser Lys
705                 710                 715                 720

Glu His Gln Ile Arg Asn Leu Asn Asp Glu Ile Ala His Gln Asp Asp
                725                 730                 735

Leu Ile Asn Lys Leu Asn Lys Glu Lys Lys Ile Gln Ser Glu Leu Asn
            740                 745                 750

Gln Lys Thr Ala Glu Glu Leu Gln Ala Ala Glu Asp Lys Ile Asn His
        755                 760                 765

Leu Thr Lys Val Lys Val Lys Leu Glu Gln Thr Leu Asp Glu Leu Glu
    770                 775                 780

Asp Thr Leu Glu Arg Glu Lys Lys Leu Arg Gly Asp Val Glu Lys Ala
785                 790                 795                 800

Lys Arg Lys Thr Glu Gly Asp Leu Lys Leu Thr Gln Glu Ala Val Ala
                805                 810                 815

Asp Leu Glu Arg Asn Lys Lys Glu Leu Glu Gln Thr Ile Gln Arg Lys
            820                 825                 830

Asp Lys Glu Ile Ala Ser Leu Thr Ala Lys Leu Glu Asp Glu Gln Ser
        835                 840                 845

Ile Val Asn Lys Thr Gly Lys Gln Ile Lys Glu Leu Gln Ser Arg Ile
    850                 855                 860

Glu Glu Leu Glu Glu Glu Val Glu Ala Glu Arg Gln Ala Arg Gly Lys
865                 870                 875                 880

Ala Glu Lys Gln Arg Ala Asp Leu Ala Arg Glu Leu Glu Glu Leu Gly
                885                 890                 895

Glu Arg Leu Glu Glu Ala Gly Gly Ala Thr Ser Ala Gln Ile Glu Leu
            900                 905                 910

Asn Lys Lys Arg Glu Ala Glu Met Ser Lys Leu Arg Arg Asp Leu Glu
        915                 920                 925

Glu Ala Asn Ile Gln His Glu Gly Thr Leu Ala Asn Leu Arg Lys Lys
    930                 935                 940

His Asn Asp Ala Val Ser Glu Met Gly Asp Gln Ile Asp Gln Leu Asn
945                 950                 955                 960

Lys Leu Lys Thr Lys Val Glu Lys Glu Lys Ser Gln Tyr Leu Gly Glu
                965                 970                 975

Leu Asn Asp Val Arg Ala Ser Ile Asp His Leu Thr Asn Glu Lys Ala
            980                 985                 990

Ala Thr Glu Lys Val Ala Lys Gln Leu Gln His Gln Ile Asn Glu Val
        995                 1000                1005

Gln Gly Lys Leu Asp Glu Ala Asn Arg Thr Leu Asn Asp Phe Asp
    1010                1015                1020

Ala Ala Lys Lys Lys Leu Ser Ile Glu Asn Ser Asp Leu Leu Arg
    1025                1030                1035

Gln Leu Glu Glu Ala Glu Ser Gln Val Ser Gln Leu Ser Lys Ile
    1040                1045                1050

Lys Ile Ser Leu Thr Thr Gln Leu Glu Asp Thr Lys Arg Leu Ala
    1055                1060                1065

Asp Glu Glu Ala Arg Glu Arg Ala Thr Leu Leu Gly Lys Phe Arg
    1070                1075                1080

Asn Leu Glu His Asp Leu Asp Asn Leu Arg Glu Gln Val Glu Glu
    1085                1090                1095

Glu Ala Glu Ala Lys Ala Asp Ile Gln Arg Gln Leu Ser Lys Ala
    1100                1105                1110

Asn Ala Glu Ala Gln Leu Trp Arg Ser Lys Tyr Glu Ser Glu Gly
    1115                1120                1125

Val Ala Arg Ala Glu Glu Leu Glu Glu Ala Lys Arg Lys Leu Gln
    1130                1135                1140

Ala Arg Leu Ala Glu Ala Glu Glu Thr Ile Glu Ser Leu Asn Gln
    1145                1150                1155

Lys Val Ile Ala Leu Glu Lys Thr Lys Gln Arg Leu Ala Thr Glu
    1160                1165                1170

Val Glu Asp Leu Gln Leu Glu Val Asp Arg Ala Asn Ala Ile Ala
    1175                1180                1185

Asn Ala Ala Glu Lys Lys Ala Lys Ala Ile Asp Lys Ile Ile Gly
    1190                1195                1200

Glu Trp Lys Leu Lys Val Asp Asp Leu Ala Ala Glu Leu Asp Ala

1205                1210                1215

Ser Gln Lys Glu Cys Arg Asn Tyr Ser Thr Glu Leu Phe Arg Leu
    1220                1225                1230

Lys Gly Ala Tyr Glu Glu Gly Gln Glu Gln Leu Glu Ala Val Arg
    1235                1240                1245

Arg Glu Asn Lys Asn Leu Ala Asp Glu Val Lys Asp Leu Leu Asp
    1250                1255                1260

Gln Ile Gly Glu Gly Gly Arg Asn Ile His Glu Ile Glu Lys Gln
    1265                1270                1275

Arg Lys Arg Leu Glu Val Glu Lys Asp Glu Leu Gln Ala Ala Leu
    1280                1285                1290

Glu Glu Ala Glu Ala Ala Leu Glu Gln Glu Glu Asn Lys Val Leu
    1295                1300                1305

Arg Ala Gln Leu Glu Leu Ser Gln Val Arg Gln Glu Ile Asp Arg
    1310                1315                1320

Arg Ile Gln Glu Lys Glu Glu Glu Phe Glu Asn Thr Arg Lys Asn
    1325                1330                1335

His Gln Arg Ala Leu Asp Ser Met Gln Ala Ser Leu Glu Ala Glu
    1340                1345                1350

Ala Lys Gly Lys Ala Glu Ala Leu Arg Met Lys Lys Lys Leu Glu
    1355                1360                1365

Ala Asp Ile Asn Glu Leu Glu Ile Ala Leu Asp His Ala Asn Lys
    1370                1375                1380

Ala Asn Ala Glu Ala Gln Lys Thr Ile Lys Lys Tyr Gln Gln Gln
    1385                1390                1395

Leu Lys Asp Val Gln Thr Ala Leu Glu Glu Glu Gln Arg Ala Arg
    1400                1405                1410

Asp Asp Ala Arg Glu Gln Leu Gly Ile Ala Glu Arg Arg Ala Asn
    1415                1420                1425

Ala Leu Gly Asn Glu Leu Glu Glu Ser Arg Thr Leu Leu Glu Gln
    1430                1435                1440

Ala Asp Arg Gly Arg Arg Gln Ala Glu Gln Glu Leu Gly Asp Ala
    1445                1450                1455

His Glu Gln Ile Asn Glu Leu Ala Ala Gln Ala Thr Ser Ala Ser
    1460                1465                1470

Ala Ala Lys Arg Lys Leu Glu Gly Glu Leu Gln Thr Leu His Ala
    1475                1480                1485

Asp Leu Asp Glu Leu Leu Asn Glu Ala Lys Asn Ser Glu Glu Lys
    1490                1495                1500

Ala Lys Lys Ala Met Val Asp Ala Ala Arg Leu Ala Asp Glu Leu
    1505                1510                1515

Arg Ala Glu Gln Asp His Ala Gln Thr Gln Glu Lys Leu Arg Lys
    1520                1525                1530

Ala Leu Glu Thr Gln Ile Lys Glu Leu Gln Val Arg Leu Asp Glu
    1535                1540                1545

Ala Glu Asn Asn Ala Leu Lys Gly Gly Lys Lys Ala Ile Ala Lys
    1550                1555                1560

Leu Glu Gln Arg Val Arg Glu Leu Glu Asn Glu Leu Asp Gly Glu
    1565                1570                1575

Gln Arg Arg His Ala Asp Ala Gln Lys Asn Leu Arg Lys Ser Glu
    1580                1585                1590

Arg Arg Ile Lys Glu Leu Ser Phe Gln Ser Asp Glu Asp Arg Lys
    1595                1600                1605

Asn His Glu Arg Met Gln Asp Leu Val Asp Lys Leu Gln Gln Lys
    1610                1615                1620

Ile Lys Thr Tyr Lys Arg Gln Ile Glu Glu Ala Glu Glu Ile Ala
    1625                1630                1635

Ala Leu Asn Leu Ala Lys Phe Arg Lys Ala Gln Gln Glu Leu Glu
    1640                1645                1650

Glu Ala Glu Glu Arg Ala Asp Leu Ala Glu Gln Ala Val Ser Lys
    1655                1660                1665

Phe Arg Thr Lys Gly Gly Arg Ala Gly Ser Ala Ala Arg Ala Met
    1670                1675                1680

Ser Pro  Val Gly Gln Lys
    1685

<210>  332
<211>  256
<212>  PRT
<213>  Lygus hesperus

<400>  332

Asp Ala Ile Lys Lys Lys Met Gln Ala Met Lys Met Glu Lys Asp Thr
1               5                   10                  15

Ala Met Asp Lys Ala Asp Thr Cys Glu Gly Gln Ala Lys Asp Ala Asn
            20                  25                  30

Thr Arg Ala Asp Lys Ile Leu Glu Asp Val Arg Asp Leu Gln Lys Lys
        35                  40                  45

Leu Asn Gln Val Glu Ser Asp Leu Glu Arg Thr Lys Arg Glu Leu Glu
    50                  55                  60

Thr Lys Thr Thr Glu Leu Glu Glu Lys Glu Lys Ala Asn Thr Asn Ala
65                  70                  75                  80

Glu Ser Glu Val Ala Ser Leu Asn Arg Lys Val Gln Met Val Glu Glu
                85                  90                  95

Asp Leu Glu Arg Ser Glu Glu Arg Ser Gly Thr Ala Gln Gln Lys Leu
            100                 105                 110

Ser Glu Ala Ser His Ala Ala Asp Glu Ala Ser Arg Met Cys Lys Val
        115                 120                 125

Leu Glu Asn Arg Ser Gln Gln Asp Glu Glu Arg Met Asp Gln Leu Thr
    130                 135                 140

Asn Gln Leu Lys Glu Ala Arg Leu Leu Ala Glu Asp Ala Asp Gly Lys
145                 150                 155                 160

Ser Asp Glu Val Ser Arg Lys Leu Ala Phe Val Glu Asp Glu Leu Glu
                165                 170                 175

Val Ala Glu Asp Arg Val Lys Ser Gly Asp Ser Lys Ile Met Glu Leu
            180                 185                 190

Glu Glu Glu Leu Lys Val Val Gly Asn Ser Leu Lys Ser Leu Glu Val
        195                 200                 205

Ser Glu Glu Lys Ala Asn Gln Arg Val Glu Glu Tyr Lys Arg Gln Ile
    210                 215                 220

Lys Gln Leu Thr Val Lys Leu Lys Glu Ala Glu Ala Arg Ala Glu Phe
225                 230                 235                 240

Ala Glu Lys Thr Val Lys Lys Leu Gln Lys Glu Val Asp Arg Leu Glu
                245                 250                 255

<210>  333
<211>  85
<212>  PRT
<213>  Lygus hesperus

<400>  333

Arg Ala Leu Gly Gln Asn Pro Thr Glu Ser Asp Val Lys Lys Phe Thr
1               5                   10                  15

His Gln His Lys Pro Asp Glu Arg Ile Ser Phe Glu Val Phe Leu Pro
            20                  25                  30

Ile Tyr Gln Ala Ile Ser Lys Gly Arg Thr Ser Asp Thr Ala Glu Asp
        35                  40                  45

Phe Ile Glu Gly Leu Arg His Phe Asp Lys Asp Gly Asn Gly Phe Ile
    50                  55                  60

Ser Thr Ala Glu Leu Arg His Leu Leu Thr Thr Leu Gly Glu Lys Leu
65                  70                  75                  80

Thr Asp Asp Glu Val
                85

<210>  334
<211>  174
<212>  PRT
<213>  Lygus hesperus

<400>  334

Met Ser Ser Arg Lys Thr Ala Gly Arg Arg Ala Thr Thr Lys Lys Arg
1               5                   10                  15

Ala Gln Arg Ala Thr Ser Asn Val Phe Ala Met Phe Asp Gln Ala Gln
            20                  25                  30

Ile Gln Glu Phe Lys Glu Ala Phe Asn Met Ile Asp Gln Asn Arg Asp
        35                  40                  45

Gly Phe Val Asp Lys Glu Asp Leu His Asp Met Leu Ala Ser Leu Gly
    50                  55                  60

Lys Asn Pro Ser Asp Glu Tyr Leu Glu Gly Met Met Asn Glu Ala Pro
65                  70                  75                  80

Gly Pro Ile Asn Phe Thr Met Phe Leu Thr Leu Phe Gly Glu Arg Leu
                85                  90                  95

Gln Gly Thr Asp Pro Glu Glu Val Ile Lys Asn Ala Phe Gly Cys Phe
            100                 105                 110

Asp Glu Asp Asn Asn Gly Phe Ile Asn Glu Glu Arg Leu Arg Glu Leu
        115                 120                 125

Leu Thr Ser Met Gly Asp Arg Phe Thr Asp Glu Asp Val Asp Glu Met
    130                 135                 140

Tyr Arg Glu Ala Pro Ile Lys Asn Gly Met Phe Asp Tyr Ile Glu Phe
145                 150                 155                 160

Thr Arg Ile Leu Lys His Gly Ala Lys Asp Lys Asp Glu Gln
                165                 170


<210>  335
<211>  1881
<212>  PRT
<213>  Lygus hesperus

<400>  335

Asp Leu Thr Cys Leu Asn Glu Ala Ser Val Leu His Asn Ile Lys Asp
1               5                   10                  15

Arg Tyr Tyr Ser Gly Leu Ile Tyr Thr Tyr Ser Gly Leu Phe Cys Val
            20                  25                  30

Val Val Asn Pro Tyr Lys Lys Leu Pro Ile Tyr Thr Glu Arg Ile Met
        35                  40                  45

Glu Lys Tyr Lys Gly Val Lys Arg His Asp Leu Pro Pro His Val Phe
    50                  55                  60

Ala Ile Thr Asp Thr Ala Tyr Arg Ser Met Leu Gln Asp Arg Glu Asp
65                  70                  75                  80

Gln Ser Ile Leu Cys Thr Gly Glu Ser Gly Ala Gly Lys Thr Glu Asn
                85                  90                  95

Thr Lys Lys Val Ile Gln Tyr Leu Ala Tyr Val Ala Ala Ser Lys Pro
            100                 105                 110

Lys Ser Ser Ala Ser Pro His Thr Ala Gln Ser Gln Ala Leu Ile Ile
        115                 120                 125

Gly Glu Leu Glu Gln Gln Leu Leu Gln Ala Asn Pro Ile Leu Glu Ala
    130                 135                 140

Phe Gly Asn Ala Lys Thr Val Lys Asn Asp Asn Ser Ser Arg Phe Gly
145                 150                 155                 160

Lys Phe Ile Arg Ile Asn Phe Asp Ala Ser Gly Tyr Ile Ala Gly Ala
                165                 170                 175

Asn Ile Glu Thr Tyr Leu Leu Glu Lys Ser Arg Ala Ile Arg Gln Ala
            180                 185                 190

Lys Asp Glu Arg Thr Phe His Ile Phe Tyr Gln Leu Leu Ala Gly Ala
        195                 200                 205

Ser Ala Glu Gln Arg Lys Glu Phe Ile Leu Glu Asp Pro Lys Asn Tyr
    210                 215                 220

Pro Phe Leu Ser Ser Gly Met Val Ser Val Pro Gly Val Asp Asp Gly
225                 230                 235                 240

Val Asp Phe Gln Ala Thr Ile Ala Ser Met Ser Ile Met Gly Met Thr
                245                 250                 255

Asn Asp Asp Leu Ser Ala Leu Phe Arg Ile Val Ser Ala Val Met Leu
            260                 265                 270

Phe Gly Ser Met Gln Phe Lys Gln Glu Arg Asn Ser Asp Gln Ala Thr
        275                 280                 285

Leu Pro Asp Asn Thr Val Ala Gln Lys Ile Ala His Leu Leu Gly Leu
    290                 295                 300

Ser Ile Thr Glu Met Thr Lys Ala Phe Leu Arg Pro Arg Ile Lys Val
305                 310                 315                 320

Gly Arg Asp Phe Val Thr Lys Ala Gln Thr Lys Glu Gln Val Glu Phe
                325                 330                 335

Ala Val Glu Ala Ile Ser Lys Ala Cys Tyr Glu Arg Met Phe Arg Trp
            340                 345                 350

Leu Val Asn Arg Ile Asn Arg Ser Leu Asp Arg Thr Lys Arg Gln Gly
        355                 360                 365

Ala Ser Phe Ile Gly Ile Leu Asp Met Ala Gly Phe Glu Ile Phe Glu
    370                 375                 380

Ile Asn Ser Phe Glu Gln Leu Cys Ile Asn Tyr Thr Asn Glu Lys Leu
385                 390                 395                 400

Gln Gln Leu Phe Asn His Thr Met Phe Ile Leu Glu Gln Glu Glu Tyr
                405                 410                 415

Gln Arg Glu Gly Ile Glu Trp Lys Phe Ile Asp Phe Gly Leu Asp Leu
            420                 425                 430

Gln Pro Thr Ile Asp Leu Ile Asp Lys Pro Met Gly Val Met Ala Leu
        435                 440                 445

Leu Asp Glu Glu Cys Trp Phe Pro Lys Ala Thr Asp Lys Thr Phe Val
    450                 455                 460

Glu Lys Leu Val Gly Ala His Ser Val His Pro Lys Phe Ile Lys Thr
465                 470                 475                 480

Asp Phe Arg Gly Val Ala Asp Phe Ala Val Val His Tyr Ala Gly Lys
                485                 490                 495

Val Asp Tyr Ser Ala Ala Gln Trp Leu Met Lys Asn Met Asp Pro Leu
            500                 505                 510

Asn Glu Asn Val Val Gln Leu Leu Gln Asn Ser Gln Asp Pro Phe Val
        515                 520                 525

Ile His Ile Trp Lys Asp Ala Glu Ile Val Gly Met Ala His Gln Ala
    530                 535                 540

Leu Ser Asp Thr Gln Phe Gly Ala Arg Thr Arg Lys Gly Met Phe Arg
545                 550                 555                 560

Thr Val Ser Gln Leu Tyr Lys Asp Gln Leu Ser Lys Leu Met Ile Thr
                565                 570                 575

Leu Arg Asn Thr Asn Pro Asn Phe Val Arg Cys Ile Leu Pro Asn His
            580                 585                 590

Glu Lys Arg Ala Gly Lys Ile Asp Ala Pro Leu Val Leu Asp Gln Leu
        595                 600                 605

Arg Cys Asn Gly Val Leu Glu Gly Ile Arg Ile Cys Arg Gln Gly Phe
    610                 615                 620

Pro Asn Arg Ile Pro Phe Gln Glu Phe Arg Gln Arg Tyr Glu Leu Leu
625                 630                 635                 640

Thr Pro Asn Val Ile Pro Lys Gly Phe Met Asp Gly Lys Lys Ala Cys
                645                 650                 655

Glu Lys Met Ile Asn Ala Leu Glu Leu Asp Pro Asn Leu Tyr Arg Val
            660                 665                 670

Gly Gln Ser Lys Ile Phe Phe Arg Ala Gly Val Leu Ala His Leu Glu
        675                 680                 685

Glu Glu Arg Asp Tyr Lys Ile Thr Asp Leu Ile Ala Asn Phe Arg Ala
    690                 695                 700

Phe Cys Arg Gly Tyr Leu Ala Arg Arg Asn Tyr Gln Lys Arg Leu Gln
705                 710                 715                 720

Gln Leu Asn Ala Ile Arg Ile Ile Gln Arg Asn Cys Ser Ala Tyr Leu
                725                 730                 735

Lys Leu Arg Asn Trp Gln Trp Trp Arg Leu Tyr Thr Lys Val Lys Pro
            740                 745                 750

Leu Leu Glu Val Thr Lys Gln Glu Glu Lys Leu Thr Gln Lys Glu Asp
        755                 760                 765

Glu Leu Lys Gln Val Arg Glu Lys Leu Asp Asn Gln Val Arg Ser Lys
    770                 775                 780

Glu Glu Tyr Glu Lys Arg Leu Gln Asp Ala Leu Glu Glu Lys Ala Ala
785                 790                 795                 800

Leu Ala Glu Gln Leu Gln Ala Glu Val Glu Leu Cys Ala Glu Ala Glu
                805                 810                 815

Glu Met Arg Ala Arg Leu Ala Val Arg Lys Gln Glu Leu Glu Glu Ile
            820                 825                 830

Leu His Asp Leu Glu Ala Arg Ile Glu Glu Glu Glu Gln Arg Asn Thr
        835                 840                 845

Val Leu Ile Asn Glu Lys Lys Lys Leu Thr Leu Asn Ile Ala Asp Leu

850                 855                 860

Glu Glu Gln Leu Glu Glu Glu Glu Gly Ala Arg Gln Lys Leu Gln Leu
865                 870                 875                 880

Glu Lys Val Gln Ile Glu Ala Arg Leu Lys Lys Met Glu Glu Asp Leu
                885                 890                 895

Ala Leu Ala Glu Asp Thr Asn Thr Lys Val Val Lys Glu Lys Lys Val
            900                 905                 910

Leu Glu Glu Arg Ala Ser Asp Leu Ala Gln Thr Leu Ala Glu Glu Glu
        915                 920                 925

Glu Lys Ala Lys His Leu Ala Lys Leu Lys Thr Lys His Glu Thr Thr
    930                 935                 940

Ile Ala Glu Leu Glu Glu Arg Leu Leu Lys Asp Asn Gln Gln Arg Gln
945                 950                 955                 960

Glu Met Asp Arg Asn Lys Arg Lys Ile Glu Ser Glu Val Asn Asp Leu
                965                 970                 975

Lys Glu Gln Ile Asn Glu Lys Lys Val Gln Val Glu Glu Leu Gln Leu
            980                 985                 990

Gln Leu Gly Lys Arg Glu Glu Glu Ile Ala Gln Ala Leu Met Arg Ile
        995                 1000                1005

Asp Glu Glu Gly Ala Gly Lys Ala Gln Thr Gln Lys Ala Leu Arg
    1010                1015                1020

Glu Leu Glu Ser Gln Leu Ala Glu Leu Gln Glu Asp Leu Glu Ala
    1025                1030                1035

Glu Lys Ala Ala Arg Ala Lys Ala Glu Lys Gln Lys Arg Asp Leu
    1040                1045                1050

Asn Glu Glu Leu Glu Ser Leu Lys Asn Glu Leu Leu Asp Ser Leu
    1055                1060                1065

Asp Thr Thr Ala Ala Gln Gln Glu Leu Arg Thr Lys Arg Glu His
    1070                1075                1080

Glu Leu Ala Thr Leu Lys Lys Thr Leu Glu Glu Glu Thr His Ile
    1085                1090                1095

His Glu Val Ser Leu Thr Glu Met Arg His Lys His Thr Gln Glu
    1100                1105                1110

Val Ala Ala Leu Asn Glu Gln Leu Glu Gln Leu Lys Lys Ala Lys
    1115                1120                1125

Ser Ala Leu Glu Lys Ser Lys Ala Gln Leu Glu Gly Glu Ala Ala
    1130                1135                1140

Glu Leu Ala Asn Glu Leu Glu Thr Ala Gly Thr Ser Lys Gly Glu
    1145                1150                1155

Ser Glu Arg Lys Arg Lys Gln Ala Glu Ser Ser Leu Gln Glu Leu
    1160                1165                1170

Ser Ser Arg Leu Leu Glu Met Glu Arg Thr Lys Ala Glu Leu Gln
    1175                1180                1185

Glu Arg Val Gln Lys Leu Ser Ala Glu Ala Asp Ser Val Asn Gln
    1190                1195                1200

Gln Leu Glu Ala Ala Glu Leu Lys Ala Ser Ala Ala Leu Lys Ala
    1205                1210                1215

Ser Gly Thr Leu Glu Thr Gln Leu Gln Glu Ala Gln Val Leu Leu
    1220                1225                1230

Glu Glu Glu Thr Arg Gln Lys Leu Ser Leu Thr Thr Lys Leu Lys
    1235                1240                1245

Gly Leu Glu Ser Glu Arg Asp Ala Leu Lys Glu Gln Leu Tyr Glu
    1250                1255                1260

Glu Asp Glu Gly Arg Lys Asn Leu Glu Lys Gln Met Ala Ile Leu
    1265                1270                1275

Asn Gln Gln Val Ala Glu Ser Lys Lys Lys Ser Glu Glu Glu Thr
    1280                1285                1290

Glu Lys Ile Thr Glu Leu Glu Glu Ser Arg Lys Lys Leu Leu Lys
    1295                1300                1305

Asp Ile Glu Ile Leu Gln Arg Gln Val Glu Glu Leu Gln Val Thr
    1310                1315                1320

Asn Asp Lys Leu Glu Lys Gly Lys Lys Lys Leu Gln Ser Glu Leu
    1325                1330                1335

Glu Asp Leu Thr Ile Asp Leu Glu Ser Gln Arg Thr Lys Val Val
    1340                1345                1350

Glu Leu Glu Lys Lys Gln Arg Asn Phe Asp Lys Val Leu Ala Glu
    1355                1360                1365

Glu Lys Ala Leu Ser Gln Gln Ile Thr His Glu Arg Asp Ala Ala
    1370                1375                1380

Glu Arg Glu Ala Arg Glu Lys Glu Thr Arg Val Leu Ser Leu Thr
    1385                1390                1395

Arg Glu Leu Asp Glu Phe Met Glu Lys Ile Glu Glu Leu Glu Arg
    1400                1405                1410

Ser Lys Arg Gln Leu Gln Ala Glu Leu Asp Glu Leu Val Asn Asn
    1415                1420                1425

Gln Gly Thr Thr Asp Lys Ser Val His Glu Leu Glu Arg Ala Lys
    1430                1435                1440

Arg Val Leu Glu Ser Gln Leu Ala Glu Gln Lys Ala Gln Asn Glu
    1445                1450                1455

Glu Leu Glu Asp Glu Leu Gln Met Thr Glu Asp Ala Lys Leu Arg
    1460                1465                1470

Leu Glu Val Asn Met Gln Ala Leu Arg Ala Gln Phe Glu Arg Asp
    1475                1480                1485

Leu Gln Gly Lys Glu Glu Ser Gly Glu Glu Lys Arg Arg Gly Leu
    1490                1495                1500

Leu Lys Gln Leu Arg Asp Ile Glu Ala Glu Leu Glu Asp Glu Arg
    1505                1510                1515

Lys Gln Arg Thr Ala Ala Val Ala Ser Arg Lys Lys Ile Glu Ala
    1520                1525                1530

Asp Phe Lys Asp Val Glu Gln Gln Leu Glu Met His Thr Lys Val
    1535                1540                1545

Lys Glu Asp Leu Gln Lys Gln Leu Lys Lys Cys Gln Val Gln Leu
    1550                1555                1560

Lys Asp Ala Ile Arg Asp Ala Glu Glu Ala Arg Leu Gly Arg Glu
    1565                1570                1575

Glu Leu Gln Ala Ala Ala Lys Glu Ala Glu Arg Lys Trp Lys Gly
    1580                1585                1590

Leu Glu Thr Glu Leu Ile Gln Val Gln Glu Asp Leu Met Ala Ser
    1595                1600                1605

Glu Arg Gln Arg Arg Ala Ala Glu Ala Glu Arg Asp Glu Val Val
    1610                1615                1620

Glu Glu Ala Asn Lys Asn Val Lys Ser Leu Ser Asn Leu Leu Asp
    1625                1630                1635

Glu Lys Lys Arg Leu Glu Ala Gln Cys Ser Gly Leu Glu Glu Glu
    1640                1645                1650

Leu Glu Glu Glu Leu Ser Asn Asn Glu Ala Leu Gln Asp Lys Ala
    1655                1660                1665

Arg Lys Ala Gln Leu Ser Val Glu Gln Leu Asn Ala Glu Leu Ala
    1670                1675                1680

Ala Glu Arg Ser Asn Val Gln Lys Leu Glu Gly Thr Arg Leu Ser
    1685                1690                1695

Met Glu Arg Gln Asn Lys Glu Leu Lys Ala Lys Leu Asn Glu Leu
    1700                1705                1710

Glu Thr Leu Gln Arg Asn Lys Phe Lys Ala Asn Ala Ser Leu Glu
    1715                1720                1725

Ala Lys Ile Thr Asn Leu Glu Glu Gln Leu Glu Asn Glu Ala Lys
    1730                1735                1740

Glu Lys Leu Leu Leu Gln Lys Gly Asn Arg Lys Leu Asp Lys Lys
    1745                1750                1755

Ile Lys Asp Leu Leu Val Gln Leu Glu Asp Glu Arg Arg His Ala
    1760                1765                1770

Asp Gln Tyr Lys Glu Gln Val Glu Lys Ile Asn Val Arg Val Lys
    1775                1780                1785

Thr Leu Lys Arg Thr Leu Asp Asp Ala Glu Glu Glu Met Ser Arg
    1790                1795                1800

Glu Lys Thr Gln Lys Arg Lys Ala Leu Arg Glu Leu Glu Asp Leu

1805                1810                1815

Arg Glu  Asn Tyr Asp Ser Leu  Leu Arg Glu Asn Asp  Asn Leu Lys
    1820                1825                1830

Asn Lys  Leu Arg Arg Gly Gly  Gly Ile Ser Gly Ile  Ser Ser Arg
    1835                1840                1845

Leu Gly  Gly Ser Lys Arg Gly  Ser Ile Pro Gly Glu  Asp Ser Gln
    1850                1855                1860

Gly Leu  Asn Asn Thr Thr Asp  Glu Ser Val Asp Gly  Asp Asp Ile
    1865                1870                1875

Ser Asn  Pro
    1880

<210>  336
<211>  108
<212>  PRT
<213>  Lygus hesperus

<400>  336

Lys Lys Ile Leu Glu Glu Ile Ile Ala Glu Val Asp Ala Asp Gly Ser
1               5                   10                  15

Gly Gln Leu Glu Phe Glu Glu Phe Val Ala Leu Ala Ala Gly Phe Leu
            20                  25                  30

Thr Glu Asp Glu Thr Gln Asp Ala Glu Ala Met Gln Gln Glu Leu Arg
        35                  40                  45

Glu Ala Phe Arg Leu Tyr Asp Lys Glu Gly Asn Gly Tyr Ile Thr Thr
    50                  55                  60

Asp Val Leu Arg Glu Ile Leu Lys Glu Leu Asp Asp Lys Ile Thr Ser
65                  70                  75                  80

Gln Glu Leu Asp Met Met Ile Ala Glu Ile Asp Ser Asp Gly Ser Gly
                85                  90                  95

Thr Val Asp Phe Asp Glu Phe Met Glu Met Met Thr
            100                 105

<210>  337
<211>  141
<212>  PRT
<213>  Lygus hesperus

<400>  337

Ile Pro Ile Met Thr Ile Ala Leu Asn Ala Phe Asp Arg Asp His Ser
1               5                   10                  15

Gly Ser Ile Pro Thr Asp Met Val Ala Asp Ile Leu Arg Leu Met Gly
            20                  25                  30

Gln Pro Phe Asn Lys Lys Ile Leu Asp Glu Leu Ile Glu Glu Val Asp
        35                  40                  45

Ala Asp Lys Ser Gly Arg Leu Glu Phe Glu Glu Phe Ile Thr Leu Ala
    50                  55                  60

Ala Lys Phe Ile Val Glu Glu Asp Asp Glu Ala Met Gln Lys Glu Leu
65                  70                  75                  80

Arg Glu Ala Phe Arg Leu Tyr Asp Lys Glu Gly Asn Gly Tyr Ile Pro
                85                  90                  95

Thr Ser Cys Leu Lys Glu Ile Leu His Glu Leu Asp Glu Gln Leu Thr
            100                 105                 110

Asn Glu Glu Leu Asp Met Ile Ile Glu Glu Ile Asp Ser Asp Gly Ser
        115                 120                 125

Gly Thr Val Asp Phe Asp Glu Phe Met Glu Met Met Thr
    130                 135                 140


<210>  338
<211>  58
<212>  PRT
<213>  Lygus hesperus

<400>  338

Trp Val Lys Glu Gly Ala Cys Ser Glu Gln Ser Ser Arg Met Thr Ala
1               5                   10                  15

Met Asp Asn Ala Ser Lys Asn Ala Ala Glu Met Ile Asp Lys Leu Thr
            20                  25                  30

Leu Thr Phe Asn Arg Thr Arg Gln Ala Val Ile Thr Arg Glu Leu Ile
        35                  40                  45

Glu Ile Ile Ser Gly Ala Ser Ala Leu Glu
    50                  55


<210>  339
<211>  130

<212>   PRT
<213>   Lygus hesperus

<400>   339

Met Val Arg Met Asn Val Leu Ser Asp Ala Leu Lys Ser Ile Asn Asn
1               5                   10                  15

Ala Glu Lys Arg Gly Lys Arg Gln Val Leu Leu Arg Pro Cys Ser Lys
            20                  25                  30

Val Ile Ile Lys Phe Leu Thr Val Met Met Lys Lys Gly Tyr Ile Gly
        35                  40                  45

Glu Phe Glu Ile Val Asp Asp His Arg Ser Gly Lys Ile Val Val Asn
    50                  55                  60

Leu Asn Gly Arg Leu Asn Lys Cys Gly Val Ile Ser Pro Arg Phe Asp
65                  70                  75                  80

Val Pro Ile Thr Gln Ile Glu Lys Trp Thr Asn Asn Leu Leu Pro Ser
                85                  90                  95

Arg Gln Phe Gly Tyr Val Val Leu Thr Thr Ser Gly Gly Ile Met Asp
            100                 105                 110

His Glu Glu Ala Arg Arg Lys His Leu Gly Gly Lys Ile Leu Gly Phe
        115                 120                 125

Phe Phe
    130


<210>   340
<211>   131
<212>   PRT
<213>   Lygus hesperus

<400>   340

Val Asp Gly Gly Leu Asn Ile Pro His Ser Thr Lys Arg Phe Pro Gly
1               5                   10                  15

Tyr Asp Ser Glu Ser Lys Glu Phe Asn Ala Glu Val His Arg Lys His
            20                  25                  30

Ile Phe Gly Ile His Val Ala Asp Tyr Met Arg Gln Leu Ala Glu Glu
        35                  40                  45

Asp Asp Asp Ala Tyr Lys Lys Gln Phe Ser Gln Tyr Val Lys Asn Gly
    50                  55                  60

Val Thr Ala Asp Ser Ile Glu Ser Ile Tyr Lys Lys Ala His Glu Ala
65                  70                  75                  80

Ile Arg Ala Asp Pro Thr Arg Lys Pro Leu Glu Lys Lys Glu Val Lys
                85                  90                  95

Lys Lys Arg Trp Asn Arg Ala Lys Leu Ser Leu Ser Glu Arg Lys Asn
            100                 105                 110

Thr Ile Asn Gln Lys Lys Ala Thr Tyr Leu Lys Lys Val Glu Ala Gly
        115                 120                 125

Glu Ile Glu
    130

<210>  341
<211>  214
<212>  PRT
<213>  Lygus hesperus

<400>  341

Met Ala Pro Lys Gly Asn Asn Met Ile Pro Asn Gly His Phe His Lys
1               5                   10                  15

Asp Trp Gln Arg Phe Ile Lys Thr Trp Phe Asn Gln Pro Ala Arg Lys
            20                  25                  30

Leu Arg Arg Arg Asn Lys Arg Leu Glu Lys Ala Gln Arg Leu Ala Pro
        35                  40                  45

Arg Pro Ala Gly Pro Leu Arg Pro Ala Val Arg Cys Pro Thr Val Arg
    50                  55                  60

Tyr His Thr Lys Leu Arg Pro Gly Arg Gly Phe Thr Leu Glu Glu Ile
65                  70                  75                  80

Lys Arg Ala Gly Leu Cys Lys Gly Phe Ala Met Ser Ile Gly Ile Ala
                85                  90                  95

Val Asp Pro Arg Arg Arg Asn Lys Ser Ile Glu Ser Leu Gln Leu Asn
            100                 105                 110

Val Gln Arg Leu Lys Glu Tyr Arg Ala Lys Leu Ile Leu Phe Pro His
        115                 120                 125

Lys Asn Ala Lys Lys Leu Lys Lys Gly Glu Ala Thr Glu Glu Glu Arg
    130                 135                 140

Lys Val Ala Thr Gln Gln Pro Leu Pro Val Met Pro Ile Lys Gln Pro
145                 150                 155                 160

Val Ile Lys Phe Lys Ala Arg Val Ile Thr Asp Asp Glu Lys Lys Tyr
                165                 170                 175

Ser Ala Phe Thr Ala Leu Arg Lys Gly Arg Ala Asp Gln Arg Leu Val
            180                 185                 190

Gly Ile Arg Ala Lys Arg Ala Lys Glu Ala Ala Glu Asn Ala Glu Asp
        195                 200                 205

Pro Ser Lys Ala Pro Lys
    210

<210>  342
<211>  134
<212>  PRT
<213>  Lygus hesperus

<400>  342

Met Asp Ile Glu Glu Pro Ala Ala Ala Pro Thr Glu Pro Ser Asp Val
1               5                   10                  15

Asn Thr Ala Leu Gln Glu Val Leu Lys Ala Ala Leu Gln His Gly Val
            20                  25                  30

Val Val His Gly Ile His Glu Ser Ala Lys Ala Leu Asp Lys Arg Gln
        35                  40                  45

Ala Leu Leu Cys Val Leu Ala Glu Asn Cys Asp Glu Pro Met Tyr Lys
    50                  55                  60

Lys Leu Val Gln Ala Leu Cys Ser Glu His His Ile Pro Leu Val Lys
65                  70                  75                  80

Val Asp Ser Asn Lys Lys Leu Gly Glu Trp Thr Gly Leu Cys Lys Ile
                85                  90                  95

Asp Lys Thr Gly Lys Ser Arg Lys Ile Val Gly Cys Ser Cys Val Val
            100                 105                 110

Ile Lys Asp Trp Gly Glu Asp Thr Pro His Leu Asp Leu Leu Lys Asp
        115                 120                 125

Tyr Ile Arg Asp Val Phe
    130

<210>  343
<211>  148
<212>  PRT
<213>  Lygus hesperus

<400>  343

Met Lys Met Asn Lys Leu Val Thr Ser Ser Arg Arg Lys Asn Arg Lys
1               5                   10                  15

Arg His Phe Thr Ala Pro Ser His Ile Arg Arg Lys Leu Met Ser Ala
            20                  25                  30

Pro Leu Ser Lys Glu Leu Arg Gln Lys Tyr Asn Val Arg Thr Met Pro
        35                  40                  45

Val Arg Lys Asp Asp Glu Val Gln Val Val Arg Gly His Tyr Lys Gly
    50                  55                  60

Gln Gln Val Gly Lys Val Leu Gln Val Tyr Arg Lys Lys Phe Ile Ile
65                  70                  75                  80

Tyr Ile Glu Arg Ile Gln Arg Glu Lys Ala Asn Gly Ala Ser Val Tyr
                85                  90                  95

Val Gly Ile His Pro Ser Lys Cys Val Ile Val Lys Leu Lys Val Asp
            100                 105                 110

Lys Asp Arg Lys Glu Ile Leu Asp Arg Arg Ser Lys Gly Arg Asp Leu
        115                 120                 125

Ala Leu Gly Lys Asp Lys Gly Lys Tyr Thr Glu Asp Ser Thr Thr Ala
    130                 135                 140

Met Asp Thr Ser
145

<210>  344
<211>  65
<212>  PRT
<213>  Lygus hesperus

<400>  344

Met Glu Lys Pro Val Val Leu Ala Arg Val Ile Lys Ile Leu Gly Arg
1               5                   10                  15

Thr Gly Ser Gln Gly Gln Cys Thr Gln Val Lys Val Glu Phe Ile Gly
            20                  25                  30

Glu Gln Asn Arg Gln Ile Ile Arg Asn Val Lys Gly Pro Val Arg Glu
        35                  40                  45

Gly Asp Ile Leu Thr Leu Leu Glu Ser Glu Arg Glu Ala Arg Arg Leu
    50                  55                  60

Arg
65

<210>  345
<211>  229
<212>  PRT
<213>  Lygus hesperus

<400>  345

Leu Phe Tyr Phe Pro Phe Ser Arg Lys Trp Gly Asp Val Gln Arg Gly
1               5                   10                  15

Val Ile Gly Thr Val Lys Thr Ser His Thr Pro Lys Ser Arg Phe Cys
            20                  25                  30

Arg Gly Val Pro Asp Pro Lys Ile Arg Ile Phe Asp Leu Gly Lys Lys
        35                  40                  45

Lys Ala Arg Val Glu Asp Phe Pro Leu Cys Val His Leu Val Ser Asp
    50                  55                  60

Glu Tyr Glu Gln Leu Ser Ser Glu Ala Leu Glu Ala Gly Arg Ile Cys
65                  70                  75                  80

Cys Asn Lys Tyr Leu Val Lys Asn Cys Gly Lys Asp Gln Phe His Ile
                85                  90                  95

Arg Met Arg Leu His Pro Phe His Val Ile Arg Ile Asn Lys Met Leu
            100                 105                 110

Ser Cys Ala Gly Ala Asp Arg Leu Gln Thr Gly Met Arg Gly Ala Phe
        115                 120                 125

Gly Lys Pro Gln Gly Thr Val Ala Arg Val Arg Ile Gly Gln Pro Ile
    130                 135                 140

Met Ser Val Arg Ser Ser Asp Arg Tyr Lys Ala Ala Val Ile Lys Ala
145                 150                 155                 160

Leu Arg Arg Ala Lys Phe Lys Phe Pro Gly Arg Gln Lys Ile Tyr Val
                165                 170                 175

Ser Lys Lys Trp Gly Phe Thr Lys Phe Asp Arg Glu Glu Tyr Glu Gly
            180                 185                 190

Leu Arg Asn Asp Asn Lys Leu Ala Asn Asp Gly Cys Asn Val Lys Leu
        195                 200                 205

Arg Pro Asp His Gly Pro Leu Gln Ala Trp Arg Lys Ala Gln Leu Asp
    210                 215                 220

Ile Ala Ala Gly Leu
225

<210>  346
<211>  220
<212>  PRT
<213>  Lygus hesperus

<400>  346

Met Gly Arg Arg Pro Ala Arg Cys Tyr Arg Tyr Cys Lys Asn Lys Pro
1               5                   10                  15

Tyr Pro Lys Ser Arg Phe Cys Arg Gly Val Pro Asp Pro Lys Ile Arg
            20                  25                  30

Ile Phe Asp Leu Gly Lys Lys Lys Ala Arg Val Glu Asp Phe Pro Leu
        35                  40                  45

Cys Val His Leu Val Ser Asp Glu Tyr Glu Gln Leu Ser Ser Glu Ala
    50                  55                  60

Leu Glu Ala Gly Arg Ile Cys Cys Asn Lys Tyr Leu Val Lys Asn Cys
65                  70                  75                  80

Gly Lys Asp Gln Phe His Ile Arg Met Arg Leu His Pro Phe His Val
                85                  90                  95

Ile Arg Ile Asn Lys Met Leu Ser Cys Ala Gly Ala Asp Arg Leu Gln
            100                 105                 110

Thr Gly Met Arg Gly Ala Phe Gly Lys Pro Gln Gly Thr Val Ala Arg
        115                 120                 125

Val Arg Ile Gly Gln Pro Ile Met Ser Val Arg Ser Ser Asp Arg Tyr
    130                 135                 140

Lys Ala Ala Val Ile Glu Ala Leu Arg Arg Ala Lys Phe Lys Phe Pro
145                 150                 155                 160

Gly Arg Gln Lys Ile Tyr Val Ser Lys Lys Trp Gly Phe Thr Lys Phe
                165                 170                 175

Asp Arg Glu Glu Tyr Glu Gly Leu Arg Asn Asp Asn Lys Leu Ala Asn
            180                 185                 190

Gly Gly Cys Asn Val Lys Leu Arg Pro Asp His Gly Pro Leu Gln Ala
        195                 200                 205

Trp Arg Lys Ala Gln Leu Asp Ile Ala Ala Gly Leu
    210                 215                 220

<210>  347
<211>  159
<212>  PRT
<213>  Lygus hesperus

<400>  347

Met Thr Asn Ser Lys Gly Tyr Arg Arg Gly Thr Arg Asp Leu Phe Ser
1               5                   10                  15

Arg Pro Phe Arg His His Gly Val Ile Pro Leu Ser Thr Tyr Met Lys
            20                  25                  30

Val Tyr Arg Val Gly Asp Ile Val Ser Ile Lys Gly Asn Gly Ala Val
        35                  40                  45

Gln Lys Gly Met Pro His Lys Val Tyr His Gly Lys Thr Gly Arg Val
    50                  55                  60

Tyr Asn Val Thr Pro Arg Ala Leu Gly Val Ile Val Asn Lys Arg Val
65                  70                  75                  80

Arg Gly Lys Ile Leu Pro Lys Arg Ile Asn Ile Arg Ile Glu His Val
                85                  90                  95

Asn His Ser Lys Cys Arg Glu Asp Phe Leu Lys Arg Val Arg Glu Asn
            100                 105                 110

Glu Arg Leu Arg Lys Phe Ala Lys Glu Thr Gly Thr Arg Val Glu Leu
        115                 120                 125

Lys Arg Gln Pro Ala Gln Pro Arg Pro Ala His Phe Val Gln Ala Lys
    130                 135                 140

Glu Val Pro Glu Leu Leu Ala Pro Ile Pro Tyr Glu Phe Ile Ala
145                 150                 155

<210>  348
<211>  131
<212>  PRT
<213>  Lygus hesperus

<400>  348

Thr Tyr Met Lys Val Tyr Arg Val Gly Asp Ile Val Ser Ile Lys Gly
1               5                   10                  15

Asn Gly Ala Val Gln Lys Gly Met Pro His Lys Val Tyr His Gly Lys
            20                  25                  30

Thr Gly Arg Val Tyr Asn Val Thr Pro Arg Ala Leu Gly Val Ile Val
        35                  40                  45

Asn Lys Arg Val Arg Gly Lys Ile Leu Pro Lys Arg Ile Asn Ile Arg
    50                  55                  60

Ile Glu His Val Asn His Ser Lys Cys Arg Glu Asp Phe Leu Lys Arg
65                  70                  75                  80

Val Arg Glu Asn Glu Arg Leu Arg Lys Phe Ala Lys Glu Thr Gly Thr
                85                  90                  95

Arg Val Glu Leu Lys Arg Gln Pro Ala Gln Pro Arg Pro Ala His Phe
            100                 105                 110

Val Gln Ala Lys Glu Val Pro Glu Leu Leu Ala Pro Ile Pro Tyr Glu
        115                 120                 125

Phe Ile Ala
    130

<210>  349
<211>  150
<212>  PRT
<213>  leptinotarsa decemlineata

<400>  349

Lys Lys Ala Lys Lys Gly Phe Met Thr Pro Glu Arg Lys Lys Lys Leu
1               5                   10                  15

Arg Leu Leu Leu Arg Lys Lys Ala Ala Glu Glu Leu Lys Lys Glu Gln
            20                  25                  30

Glu Arg Lys Ala Ala Glu Arg Arg Arg Ile Ile Glu Glu Arg Cys Gly
        35                  40                  45

Lys Pro Lys Leu Ile Asp Glu Ala Asn Glu Glu Gln Val Arg Asn Tyr

50                  55                  60

Cys Lys Leu Tyr His Gly Arg Ile Ala Lys Leu Glu Asp Gln Lys Phe
65                  70                  75                  80

Asp Leu Glu Tyr Leu Val Lys Lys Lys Asp Met Glu Ile Ala Glu Leu
                85                  90                  95

Asn Ser Gln Val Asn Asp Leu Arg Gly Lys Phe Val Lys Pro Thr Leu
            100                 105                 110

Lys Lys Val Ser Lys Tyr Glu Asn Lys Phe Ala Lys Leu Gln Lys Lys
        115                 120                 125

Ala Ala Glu Phe Asn Phe Arg Asn Gln Leu Lys Val Val Lys Lys Lys
    130                 135                 140

Glu Phe Thr Leu Glu Glu
145                 150

<210>  350
<211>  279
<212>  PRT
<213>  leptinotarsa decemlineata

<400>  350

Gln Trp Tyr Gln Arg Arg Val Arg Gly Asp Ile Glu Glu Lys Arg Gln
1               5                   10                  15

Arg Leu Glu Glu Ala Glu Lys Lys Arg Gln Ala Met Met Gln Ala Leu
            20                  25                  30

Lys Asp Gln Asn Lys Asn Lys Gly Pro Asn Phe Thr Ile Thr Lys Arg
        35                  40                  45

Asp Ala Ser Ser Asn Leu Ser Ala Ala Gln Leu Glu Arg Asn Lys Thr
    50                  55                  60

Lys Glu Gln Leu Glu Glu Glu Lys Lys Ile Ser Leu Ser Ile Arg Ile
65                  70                  75                  80

Lys Pro Leu Val Val Asp Gly Leu Gly Val Asp Lys Leu Arg Leu Lys
                85                  90                  95

Ala Gln Glu Leu Trp Glu Cys Ile Val Lys Leu Glu Thr Glu Lys Tyr
            100                 105                 110

Asp Leu Glu Glu Arg Gln Lys Arg Gln Asp Tyr Asp Leu Lys Glu Leu

115                 120                 125

Lys Glu Arg Gln Lys Gln Gln Leu Arg His Lys Ala Leu Lys Lys Gly
    130                 135                 140

Leu Asp Pro Glu Ala Leu Thr Gly Lys Tyr Pro Pro Lys Ile Gln Val
145                 150                 155                 160

Ala Ser Lys Tyr Glu Arg Arg Val Asp Thr Arg Ser Tyr Gly Asp Lys
                165                 170                 175

Lys Lys Leu Phe Glu Gly Gly Leu Glu Glu Ile Ile Lys Glu Thr Asn
            180                 185                 190

Glu Lys Ser Trp Lys Glu Lys Phe Gly Gln Phe Asp Ser Arg Gln Lys
        195                 200                 205

Ala Arg Leu Pro Lys Trp Phe Gly Glu Arg Pro Gly Lys Lys Pro Gly
    210                 215                 220

Asp Pro Glu Thr Pro Glu Gly Glu Glu Glu Gly Lys Gln Val Ile Asp
225                 230                 235                 240

Glu Asp Asp Asp Leu Lys Glu Pro Val Ile Glu Ala Glu Ile Glu Glu
                245                 250                 255

Glu Glu Glu Glu Glu Glu Val Glu Val Asp Glu Glu Glu Glu Asp Asp
            260                 265                 270

Glu Glu Glu Glu Glu Glu Glu
        275

<210>  351
<211>  286
<212>  PRT
<213>  leptinotarsa decemlineata

<400>  351

Ala Leu Gln Asn Glu Leu Glu Glu Ser Arg Thr Leu Leu Glu Gln Ala
1               5                   10                  15

Asp Arg Ala Arg Arg Gln Ala Glu Gln Glu Leu Gly Asp Ala His Glu
            20                  25                  30

Gln Leu Asn Asp Leu Gly Ala Gln Asn Gly Ser Leu Ser Ala Ala Lys
        35                  40                  45

Arg Lys Leu Glu Thr Glu Leu Gln Thr Leu His Ser Asp Leu Asp Glu

50                  55                  60

Leu Leu Asn Glu Ala Lys Asn Ser Glu Glu Lys Ala Lys Lys Ala Met
65                  70                  75                  80

Val Asp Ala Ala Arg Leu Ala Asp Glu Leu Arg Ala Glu Gln Asp His
                85                  90                  95

Ala Gln Thr Gln Glu Lys Leu Arg Lys Ala Leu Glu Ser Gln Ile Lys
            100                 105                 110

Asp Leu Gln Val Arg Leu Asp Glu Ala Glu Ala Asn Ala Leu Lys Gly
        115                 120                 125

Gly Lys Lys Ala Ile Ala Lys Leu Glu Gln Arg Val Arg Glu Leu Glu
    130                 135                 140

Asn Glu Leu Asp Gly Glu Gln Arg Arg His Ala Asp Ala Gln Lys Asn
145                 150                 155                 160

Leu Arg Lys Ser Glu Arg Arg Ile Lys Glu Leu Ser Leu Gln Ala Glu
                165                 170                 175

Glu Asp Arg Lys Asn His Glu Lys Met Gln Asp Leu Val Asp Lys Leu
            180                 185                 190

Gln Gln Lys Ile Lys Thr His Lys Arg Gln Ile Glu Glu Ala Glu Glu
        195                 200                 205

Ile Ala Ala Leu Asn Leu Ala Lys Phe Arg Lys Ala Gln Gln Glu Leu
    210                 215                 220

Glu Glu Ala Glu Glu Arg Ala Asp Leu Ala Glu Gln Ala Ile Val Lys
225                 230                 235                 240

Phe Arg Thr Lys Gly Arg Ser Gly Ser Ala Ala Arg Gly Ala Ser Pro
                245                 250                 255

Ala Pro Gln Arg Gln Arg Pro Thr Phe Gly Met Gly Asp Ser Leu Gly
            260                 265                 270

Gly Ala Phe Pro Pro Arg Phe Asp Leu Ala Pro Asp Phe Glu
        275                 280                 285

<210>  352
<211>  197
<212>  PRT
<213>  nilaparvata lugens

<400>  352

Met Ala Asp Asp Glu Ala Lys Lys Ala Lys Gln Ala Glu Ile Asp Arg
1               5                   10                  15

Lys Arg Ala Glu Val Arg Lys Arg Met Glu Glu Ala Ser Lys Ala Lys
            20                  25                  30

Lys Ala Lys Lys Gly Phe Met Thr Pro Asp Arg Lys Lys Lys Leu Arg
        35                  40                  45

Leu Leu Leu Arg Lys Lys Ala Ala Glu Glu Leu Lys Lys Glu Gln Glu
    50                  55                  60

Arg Lys Ala Ala Glu Arg Arg Arg Ile Ile Glu Glu Arg Cys Gly Lys
65                  70                  75                  80

Ala Val Asp Leu Asp Asp Gly Ser Glu Glu Lys Val Lys Ala Thr Leu
                85                  90                  95

Lys Thr Tyr His Asp Arg Ile Gly Lys Leu Glu Asp Glu Lys Phe Asp
            100                 105                 110

Leu Glu Tyr Ile Val Lys Lys Lys Asp Phe Glu Ile Ala Asp Leu Asn
        115                 120                 125

Ser Gln Val Asn Asp Leu Arg Gly Lys Phe Val Lys Pro Thr Leu Lys
    130                 135                 140

Lys Val Ser Lys Tyr Glu Asn Lys Phe Ala Lys Leu Gln Lys Lys Ala
145                 150                 155                 160

Ala Glu Phe Asn Phe Arg Asn Gln Leu Lys Val Val Lys Lys Lys Glu
                165                 170                 175

Phe Thr Leu Glu Glu Glu Asp Lys Glu Pro Lys Lys Ser Glu Lys Ala
            180                 185                 190

Glu Trp Gln Lys Lys
        195


<210>  353
<211>  184
<212>  PRT
<213>  nilaparvata lugens

<400>  353

Met Met Ala Ala Leu Lys Asp Gln Ser Lys Ser Lys Gly Pro Asn Phe

1               5                   10                  15

Thr Val Asn Lys Lys Thr Asp Leu Asn Met Thr Ser Ala Gln Met Glu
            20                  25                  30

Arg Asn Lys Thr Lys Glu Gln Leu Glu Glu Glu Lys Lys Ile Ser Leu
        35                  40                  45

Ser Phe Arg Ile Lys Pro Leu Ala Ile Glu Asn Met Ser Ile Asn Ala
    50                  55                  60

Leu Arg Ala Lys Ala Gln Glu Leu Trp Asp Cys Ile Val Lys Leu Glu
65                  70                  75                  80

Thr Glu Lys Tyr Asp Leu Glu Glu Arg Gln Lys Arg Gln Asp Tyr Asp
                85                  90                  95

Leu Lys Glu Leu Lys Glu Arg Gln Lys Gln Gln Leu Arg His Lys Ala
            100                 105                 110

Leu Lys Lys Gly Leu Asp Pro Glu Ala Leu Thr Gly Lys Tyr Pro Pro
        115                 120                 125

Lys Ile Gln Val Ala Ser Lys Tyr Glu Arg Arg Val Asp Thr Arg Ser
    130                 135                 140

Tyr Asp Asp Lys Lys Lys Leu Phe Glu Gly Gly Trp Asp Thr Leu Thr
145                 150                 155                 160

Ser Glu Thr Asn Glu Lys Ile Trp Lys Ser Arg Asn Asp Gln Phe Ser
                165                 170                 175

Asn Arg Ser Lys Ala Lys Leu Pro
            180

<210>  354
<211>  122
<212>  PRT
<213>  nilaparvata lugens

<400>  354

Ala Phe Asp Arg Glu Arg Ser Gly Ser Ile Pro Thr Asp Met Val Ala
1               5                   10                  15

Asp Ile Leu Arg Leu Met Gly Gln Pro Phe Asn Lys Lys Ile Leu Asp
            20                  25                  30

Glu Leu Ile Glu Glu Val Asp Ala Asp Lys Ser Gly Arg Leu Glu Phe

35                  40                  45

Asp Glu Phe Val Thr Leu Ala Ala Lys Phe Ile Val Glu Glu Asp Asp
    50                  55                  60

Glu Ala Met Gln Lys Glu Leu Lys Glu Ala Phe Arg Leu Tyr Asp Lys
65                  70                  75                  80

Glu Gly Asn Gly Tyr Ile Pro Thr Ser Cys Leu Lys Glu Ile Leu Arg
                85                  90                  95

Glu Leu Asp Asp Gln Leu Thr Asn Glu Glu Leu Asn Met Met Ile Asp
            100                 105                 110

Glu Ile Asp Ser Asp Gly Ser Gly Thr Val
        115                 120

<210>  355
<211>  73
<212>  PRT
<213>  nilaparvata lugens

<400>  355

Val Lys Thr Leu Thr Gly Lys Thr Ile Thr Leu Glu Val Glu Pro Ser
1               5                   10                  15

Asp Thr Ile Glu Asn Val Lys Ala Lys Ile Gln Asp Lys Glu Gly Ile
            20                  25                  30

Pro Pro Asp Gln Gln Arg Leu Ile Phe Ala Gly Lys Gln Leu Glu Asp
        35                  40                  45

Gly Arg Thr Leu Ser Asp Tyr Asn Ile Gln Lys Glu Ser Thr Leu His
    50                  55                  60

Leu Val Leu Arg Leu Arg Gly Gly Thr
65                  70

<210>  356
<211>  253
<212>  PRT
<213>  Acyrthosiphon pisum

<400>  356

Met Ala Asp Asp Glu Ala Lys Lys Ala Lys Gln Ala Glu Ile Asp Arg
1               5                   10                  15

Lys Arg Ala Glu Val Arg Lys Arg Met Glu Glu Ala Ser Lys Ala Lys
            20                  25                  30

Lys Ala Lys Lys Gly Phe Met Thr Pro Asp Arg Lys Lys Lys Leu Arg
        35                  40                  45

Leu Leu Leu Lys Lys Lys Ala Ala Glu Glu Leu Lys Lys Glu Gln Glu
    50                  55                  60

Arg Lys Ala Ala Glu Arg Arg Arg Ile Ile Glu Glu Arg Cys Gly Gln
65                  70                  75                  80

Pro Lys Asn Ile Asp Asp Ala Gly Glu Glu Glu Leu Ala Glu Ile Cys
                85                  90                  95

Glu Glu Leu Trp Lys Arg Val Tyr Thr Val Glu Gly Ile Lys Phe Asp
            100                 105                 110

Leu Glu Arg Asp Ile Arg Met Lys Val Phe Glu Ile Ser Glu Leu Asn
        115                 120                 125

Ser Gln Val Asn Asp Leu Arg Gly Lys Phe Val Lys Pro Thr Leu Lys
    130                 135                 140

Lys Val Ser Lys Tyr Glu Asn Lys Phe Ala Lys Leu Gln Lys Lys Ala
145                 150                 155                 160

Ala Glu Phe Asn Phe Arg Asn Gln Leu Lys Val Val Lys Lys Lys Glu
                165                 170                 175

Phe Thr Leu Glu Glu Glu Asp Lys Glu Lys Lys Pro Asp Trp Ser Lys
            180                 185                 190

Lys Gly Asp Glu Lys Lys Gly Glu Gly Glu Asp Gly Asp Gly Thr Glu
        195                 200                 205

Asp Glu Lys Thr Asp Asp Gly Leu Thr Thr Glu Gly Glu Ser Val Ala
    210                 215                 220

Gly Asp Leu Thr Asp Ala Thr Glu Asp Ala Gln Ser Asp Asn Glu Ile
225                 230                 235                 240

Leu Glu Pro Glu Pro Val Val Glu Pro Glu Pro Glu Pro
                245                 250

<210>  357
<211>  179
<212>  PRT
<213>  Acyrthosiphon pisum

<400>  357

Val Met Arg Cys Gly Lys Lys Lys Val Trp Leu Asp Pro Asn Glu Ile
1               5                   10                  15

Asn Glu Ile Ala Asn Thr Asn Ser Arg Gln Asn Ile Arg Lys Leu Ile
            20                  25                  30

Lys Asp Gly Leu Ile Ile Lys Lys Pro Val Ala Val His Ser Arg Ala
        35                  40                  45

Arg Ala Arg Lys Asn Ala Asp Ala Arg Arg Lys Gly Arg His Cys Gly
    50                  55                  60

Phe Gly Lys Arg Lys Gly Thr Ala Asn Ala Arg Thr Pro Gln Lys Asp
65                  70                  75                  80

Leu Trp Val Lys Arg Met Arg Val Leu Arg Arg Leu Leu Lys Lys Tyr
                85                  90                  95

Arg Glu Ala Lys Lys Ile Asp Asn His Leu Tyr His Gln Leu Tyr Met
            100                 105                 110

Lys Ala Lys Gly Asn Val Phe Lys Asn Lys Arg Val Leu Met Glu Phe
        115                 120                 125

Ile His Lys Lys Lys Ala Glu Lys Ala Arg Ala Lys Met Leu Ser Asp
    130                 135                 140

Gln Ala Glu Ala Arg Arg Gln Lys Val Lys Glu Ala Arg Lys Arg Lys
145                 150                 155                 160

Glu Ala Arg Phe Leu Gln Asn Arg Lys Glu Leu Leu Ala Ala Tyr Ala
                165                 170                 175

Arg Glu Asp

<210>  358
<211>  275
<212>  PRT
<213>  Acyrthosiphon pisum

<400>  358

Gly Leu Glu Val Glu Ser Ser Asp Ser Ile Glu Asn Val Lys Ala Lys
1               5                   10                  15

Ile Gln Asp Lys Glu Gly Ile Pro Pro Asp Gln Gln Arg Leu Ile Phe
            20                  25                  30

Ala Gly Lys Gln Leu Glu Asp Gly Arg Thr Leu Ser Asp Tyr Asn Ile
        35                  40                  45

Gln Lys Glu Ser Thr Leu His Leu Val Leu Arg Leu Arg Gly Gly Met
    50                  55                  60

Gln Ile Phe Val Lys Thr Leu Thr Gly Lys Thr Ile Thr Leu Glu Val
65                  70                  75                  80

Glu Ser Ser Asp Ser Ile Glu Asn Val Lys Ala Lys Ile Gln Asp Lys
                85                  90                  95

Glu Gly Ile Pro Pro Asp Gln Gln Arg Leu Ile Phe Ala Gly Lys Gln
            100                 105                 110

Leu Glu Asp Gly Arg Thr Leu Ser Asp Tyr Asn Ile Gln Lys Glu Ser
        115                 120                 125

Thr Leu His Leu Val Leu Arg Leu Arg Gly Gly Met Gln Ile Phe Val
    130                 135                 140

Lys Thr Leu Thr Gly Lys Thr Ile Thr Leu Glu Val Glu Ser Ser Asp
145                 150                 155                 160

Ser Ile Glu Asn Val Lys Ala Lys Ile Gln Asp Lys Glu Gly Ile Pro
                165                 170                 175

Pro Asp Gln Gln Arg Leu Ile Phe Ala Gly Lys Gln Leu Glu Asp Gly
            180                 185                 190

Arg Thr Leu Ser Asp Tyr Asn Ile Gln Lys Glu Ser Thr Leu His Leu
        195                 200                 205

Val Leu Arg Leu Arg Gly Gly Met Gln Ile Phe Val Lys Thr Leu Thr
    210                 215                 220

Gly Lys Thr Ile Thr Leu Glu Val Glu Ser Ser Asp Ser Ile Glu Asn
225                 230                 235                 240

Val Lys Ala Lys Ile Gln Asp Lys Glu Gly Ile Pro Pro Asp Gln Gln
                245                 250                 255

Arg Leu Ile Phe Ala Gly Lys Gln Leu Glu Asp Gly Arg Thr Leu Ser
            260                 265                 270

Asp Tyr Asn

        275

<210>  359
<211>  56
<212>  PRT
<213>  Acyrthosiphon pisum

<400>  359

Asp Leu Leu His Pro Thr Ala Ile Glu Glu Arg Arg Lys His Lys Leu
1               5                   10                  15

Lys Arg Leu Val Gln His Pro Asn Ser Phe Phe Met Asp Val Lys Cys
            20                  25                  30

Pro Gly Cys Tyr Lys Ile Thr Thr Val Phe Ser His Ala Gln Ser Val
        35                  40                  45

Val Ile Cys Thr Gly Cys Ser Thr
    50                  55

<210>  360
<211>  20
<212>  DNA
<213>  Artificial

<220>
<223>  Primer

<400>  360
atcatgcagg cgtacgcccg                                                   20

<210>  361
<211>  19
<212>  DNA
<213>  Artificial

<220>
<223>  Primer

<400>  361
cggagggggc gagatcact                                                    19

<210>  362
<211>  62
<212>  DNA
<213>  Artificial

<220>
<223>  Amplicon

<400>  362
   atcatgcagg cgtacgcccg agaagacgag gctgccgtca aaagtgatct tcgccccctc        60
   cg                                                                      62

<210>  363
<211>  22
<212>  DNA
<213>  Artificial

<220>
<223>  Primer

<400>  363
tgtgttggct actggtggct ac                                                22


<210>  364
<211>  25
<212>  DNA
<213>  Artificial

<220>
<223>  Primer

<400>  364
tcggatggaa ctggacaaat tcaag                                             25


<210>  365
<211>  137
<212>  DNA
<213>  Artificial

<220>
<223>  Amplicon

<400>  365
tgtgttggct actggtggct acggcagagc ttacttttca tgcacttcag ctcacacttg      60

cacgggagat ggccaagcaa tggtttcacg agctgggctt cccaacgaag atcttgaatt     120

tgtccagttc catccga                                                     137


<210>  366
<211>  21
<212>  DNA
<213>  Artificial

<220>
<223>  Primer

<400>  366
gcaacccgtg ttctccaaag c                                                 21


<210>  367
<211>  28
<212>  DNA
<213>  Artificial

<220>
<223>  Primer

<400>  367
tcaactcgta ttctcgtact ttcaaacc                                          28

<210>  368
<211>  146
<212>  DNA
<213>  Artificial

<220>
<223>  Amplicon

<400>  368
gcaacccgtg ttctccaaag ccagatacac tgtgcgatcc ttcggtatca ggcgtaacga      60

aaaaatcgcc gttcactgca ctgtcagggg cgccaaagca gaggaaattc tggagcgtgg     120

tttgaaagta cgagaatacg agttga                                          146


<210>  369
<211>  21
<212>  DNA
<213>  Artificial

<220>
<223>  Primer

<400>  369
atggccgacg atgaagctaa g                                                21


<210>  370
<211>  20
<212>  DNA
<213>  Artificial

<220>
<223>  Primer

<400>  370
tggttctggt tcgggttcaa                                                  20


<210>  371
<211>  20
<212>  DNA
<213>  Artificial

<220>
<223>  Primer

<400>  371
cggtaatgcg atgcggtaag                                                  20


<210>  372
<211>  20
<212>  DNA
<213>  Artificial

<220>
<223>  Primer

<400>  372

tcatcttctc gggcgtatgc                                                    20

<210>  373
<211>  25
<212>  DNA
<213>  Artificial

<220>
<223>  Primer

<400>  373
tttggaagtt gagtcatcag attcc                                             25

<210>  374
<211>  24
<212>  DNA
<213>  Artificial

<220>
<223>  Primer

<400>  374
gttgtagtcg gaaagggtac gtcc                                              24

<210>  375
<211>  20
<212>  DNA
<213>  Artificial

<220>
<223>  Primer

<400>  375
attgtggaac atccggtaca                                                   20

<210>  376
<211>  23
<212>  DNA
<213>  Artificial

<220>
<223>  Primer

<400>  376
aagacttgct tcatcctact gca                                               23

<210>  377
<211>  28
<212>  DNA
<213>  Artificial

<220>
<223>  Primer

<220>
<221>  misc_feature
<222>  (20)..(20)

<223>  n is a, c, g, or t

<400>  377
ccaagaaggc caagaagggn ttyatgac                                          28


<210>  378
<211>  29
<212>  DNA
<213>  Artificial

<220>
<223>  Primer

<400>  378
tcctcctcca gggtgaactc yttyttytt                                         29


<210>  379
<211>  26
<212>  DNA
<213>  Artificial

<220>
<223>  Primer


<220>
<221>  misc_feature
<222>  (21)..(21)
<223>  n is a, c, g, or t

<220>
<221>  misc_feature
<222>  (24)..(24)
<223>  n is a, c, g, or t

<400>  379
gccaagaagg gcttcatgac nccnga                                            26


<210>  380
<211>  27
<212>  DNA
<213>  Artificial

<220>
<223>  Primer

<400>  380
gaagttgaac tcggcggcyt tyttytg                                           27


<210>  381
<211>  26
<212>  DNA
<213>  Artificial

<220>
<223>  Primer


<220>

<221>  misc_feature
<222>  (24)..(24)
<223>  n is a, c, g, or t

<400>  381
ctggaggagg ccgagaaraa rmgnca                                            26


<210>  382
<211>  22
<212>  DNA
<213>  Artificial

<220>
<223>  Primer


<220>
<221>  misc_feature
<222>  (14)..(14)
<223>  n is a, c, g, or t

<400>  382
tgccgggccg ctcnccraac ca                                                22


<210>  383
<211>  28
<212>  DNA
<213>  Artificial

<220>
<223>  Primer


<220>
<221>  misc_feature
<222>  (20)..(20)
<223>  n is a, c, g, or t

<220>
<221>  misc_feature
<222>  (23)..(23)
<223>  n is a, c, g, or t

<400>  383
agatcgccat cctgaggaan gcnttyra                                          28


<210>  384
<211>  31
<212>  DNA
<213>  Artificial

<220>
<223>  Primer

<400>  384
cggtcatcat ctccatgaac tcrtcraart c                                      31


<210>  385
<211>  170

<212>  DNA
<213>  Artificial

<220>
<223>  Intron

<400>  385
tctagaaggt aagtgtacac actacatttt catgaacatt attgcgaccg ttgagattct      60

cattgtttgg tgattgatta tctaaagtag aagcatgaat agatataaca taaactagta     120

actaatgggt tagttatggg tatacttcat gcttttctct caggctcgag               170


<210>  386
<211>  521
<212>  DNA
<213>  leptinotarsa decemlineata

<400>  386
tcgatttttc atttttcttt tattatttgg agtgggcctg ttgtggtcgt tatcaaaatg      60

ggtaaaataa tgaaatctgg taaagtcgta ttggtccttg gaggccgata cgctggaaga     120

aaggcagtag tcataaaaaa ttacgatgat gggacgtcag ataaacaata tggacatgcc     180

gtggtggctg gaatcgatag gtaccctaga aaaatccaca aacgtatggg caaaggaaaa     240

atgcacaaga ggtccaaaat caagcccttc cttaaggtgc tcaactataa ccatttgatg     300

cctacaagat attcagtgga tttgacttcg gacttgaaag tggcgcccaa agacctcaag     360

gatccagtga agaggaagaa gattaggttc caaaccagag ttaaattcga agagagatac     420

aagcaaggaa aacacaaatg gtttttccag aaattgaggt tctagattct ataaatttaa     480

ccattttgta atccacccac ctttttgttc aaataaattg t                         521


<210>  387
<211>  475
<212>  DNA
<213>  leptinotarsa decemlineata

<400>  387
tatcgcgaaa aatatacaac ttacaaaatg aggaacacgt atgagttgag ccctaaagaa      60

gcagcaaatt tcactcgtcg aaatttagca gatactcttc gaagcaggag tccatatcat     120

gttaatcttc tcttggctgg atatgacaag aaagacgggg ctcagttgta ttacatggat     180

tatctagcgt ctgttgctag tgttgattac gctgcccatg gatacggagg atatttctcc     240

ctttccataa tggatcgcaa ttatttgaaa accctgtcga aagatcaagg atacgaactt     300

ctgaaggaat gtgttaaaga agttcaaaag agacttgcta taaatttacc aaatttcaaa     360

gttcaggtta ttgataaaga tggtattaag gatatgccta atataacttc aaaaggtttg     420

aattgattaa gcaacttcag tttcagattt ttttctaaat aaacatttaa agtgt          475


<210>  388

<211>  467
<212>  DNA
<213>  leptinotarsa decemlineata

<400>  388
gcggggactg gatacatctc taaaacacag aaaaatgaaa ttcttcaagt caggaatata      60

ttctgttgta tttttggcaa ttatattttc tttggtcact gaggaagtgg aaggtcgaag      120

gactatttta agagggcgta aaacactgac gagaacctat tttcgtgaca atgcagtccc      180

agcatacgtc atagtgatac tcgttggaat aggagaaatc attttgggag ctatcctgta      240

tgttataatg aggaaaacga taatagattt tcctttatca gggagttacg cagtggcccc      300

tactcaagaa gcataaatcc cattgaaatt gtgactgttt actttctttg gaaaaatgtg      360

tataataaat acaattcatt tataatattt atatttggaa cttaaaatac ttacaaaatt      420

accatttaca tgatcaaata actaataaag ttctgtctca attataa                    467


<210>  389
<211>  906
<212>  DNA
<213>  leptinotarsa decemlineata

<400>  389
ggattggaag taaaaatata caattcatgc tgtagctgta gtgtaaaaac tgaactgaaa      60

gccataaaat aaagaccttg caagaaacat gtccaagatt aatgaggtgt ctaatttgta      120

caaacaactg aaatcagaat ggaacacatc caatccaaat ttaagcaaat gtgaaaagct      180

tttgtcagat ttgaagcttg agctaacaca cttaatgttc cttccaactt caaacgccac      240

tgcttcaaaa caagaacttc ttctggcaag agatgttctg gaaattgggg tacaatggag      300

tatagctgca aatgatatac ctgcctttga aagatacatg gcacagttga aatgttatta      360

tttcgattat aagaatcaac ttcccgaatc ttctttcaaa tatcagttac tgggtctgaa      420

tttactattt ttgttatcac aaaatagagt ggcagagttc cacacagaat tagaattgtt      480

gcctgctgac cacattcaga atgatgtata catcaggcac cctccatcta ttgaacagta      540

ccttatggaa ggaagttata ataagatatt tctggcaaag ggaaatgtcc cagcaacaaa      600

ttacaatttt tttatggata tacttctaga tactatcaga ggggagattg cagattgtct      660

agagaaagca tatgaaaaaa tatcaattaa agatgttgct aggatgctat acttgggcag      720

tgaagaatcg gccaaggcct ttgtaacaaa gagtaagaca tggaaattag aaaaggacaa      780

cttctttcac ttcacgcccg aggttaaaaa gacacatgag ccaattctat ccaaagaatt      840

ggcacaacaa gctattgaat atgcaaaaga actggaaatg attgtttaaa gtaataaagt      900

ttttca                                                                 906


<210>  390
<211>  135

<212>  PRT
<213>  leptinotarsa decemlineata

<400>  390

Met Gly Lys Ile Met Lys Ser Gly Lys Val Val Leu Val Leu Gly Gly
1               5                   10                  15

Arg Tyr Ala Gly Arg Lys Ala Val Val Ile Lys Asn Tyr Asp Asp Gly
            20                  25                  30

Thr Ser Asp Lys Gln Tyr Gly His Ala Val Val Ala Gly Ile Asp Arg
        35                  40                  45

Tyr Pro Arg Lys Ile His Lys Arg Met Gly Lys Gly Lys Met His Lys
    50                  55                  60

Arg Ser Lys Ile Lys Pro Phe Leu Lys Val Leu Asn Tyr Asn His Leu
65                  70                  75                  80

Met Pro Thr Arg Tyr Ser Val Asp Leu Thr Ser Asp Leu Lys Val Ala
                85                  90                  95

Pro Lys Asp Leu Lys Asp Pro Val Lys Arg Lys Lys Ile Arg Phe Gln
            100                 105                 110

Thr Arg Val Lys Phe Glu Glu Arg Tyr Lys Gln Gly Lys His Lys Trp
        115                 120                 125

Phe Phe Gln Lys Leu Arg Phe
    130                 135


<210>  391
<211>  141
<212>  PRT
<213>  leptinotarsa decemlineata

<400>  391

Tyr Arg Glu Lys Tyr Thr Thr Tyr Lys Met Arg Asn Thr Tyr Glu Leu
1               5                   10                  15

Ser Pro Lys Glu Ala Ala Asn Phe Thr Arg Arg Asn Leu Ala Asp Thr
            20                  25                  30

Leu Arg Ser Arg Ser Pro Tyr His Val Asn Leu Leu Leu Ala Gly Tyr
        35                  40                  45

Asp Lys Lys Asp Gly Ala Gln Leu Tyr Tyr Met Asp Tyr Leu Ala Ser
    50                  55                  60

Val Ala Ser Val Asp Tyr Ala Ala His Gly Tyr Gly Gly Tyr Phe Ser
65                  70                  75                  80

Leu Ser Ile Met Asp Arg Asn Tyr Leu Lys Thr Leu Ser Lys Asp Gln
                85                  90                  95

Gly Tyr Glu Leu Leu Lys Glu Cys Val Lys Glu Val Gln Lys Arg Leu
            100                 105                 110

Ala Ile Asn Leu Pro Asn Phe Lys Val Gln Val Ile Asp Lys Asp Gly
        115                 120                 125

Ile Lys Asp Met Pro Asn Ile Thr Ser Lys Gly Leu Asn
    130                 135                 140

<210>  392
<211>  104
<212>  PRT
<213>  leptinotarsa decemlineata

<400>  392

Arg Gly Leu Asp Thr Ser Leu Lys His Arg Lys Met Lys Phe Phe Lys
1               5                   10                  15

Ser Gly Ile Tyr Ser Val Val Phe Leu Ala Ile Ile Phe Ser Leu Val
            20                  25                  30

Thr Glu Glu Val Glu Gly Arg Arg Thr Ile Leu Arg Gly Arg Lys Thr
        35                  40                  45

Leu Thr Arg Thr Tyr Phe Arg Asp Asn Ala Val Pro Ala Tyr Val Ile
    50                  55                  60

Val Ile Leu Val Gly Ile Gly Glu Ile Ile Leu Gly Ala Ile Leu Tyr
65                  70                  75                  80

Val Ile Met Arg Lys Thr Ile Ile Asp Phe Pro Leu Ser Gly Ser Tyr
                85                  90                  95

Ala Val Ala Pro Thr Gln Glu Ala
            100

<210>  393
<211>  266
<212>  PRT
<213>  leptinotarsa decemlineata

<400>  393

Met Ser Lys Ile Asn Glu Val Ser Asn Leu Tyr Lys Gln Leu Lys Ser
1               5                   10                  15

Glu Trp Asn Thr Ser Asn Pro Asn Leu Ser Lys Cys Glu Lys Leu Leu
            20                  25                  30

Ser Asp Leu Lys Leu Glu Leu Thr His Leu Met Phe Leu Pro Thr Ser
        35                  40                  45

Asn Ala Thr Ala Ser Lys Gln Glu Leu Leu Leu Ala Arg Asp Val Leu
    50                  55                  60

Glu Ile Gly Val Gln Trp Ser Ile Ala Ala Asn Asp Ile Pro Ala Phe
65                  70                  75                  80

Glu Arg Tyr Met Ala Gln Leu Lys Cys Tyr Tyr Phe Asp Tyr Lys Asn
                85                  90                  95

Gln Leu Pro Glu Ser Ser Phe Lys Tyr Gln Leu Leu Gly Leu Asn Leu
            100                 105                 110

Leu Phe Leu Leu Ser Gln Asn Arg Val Ala Glu Phe His Thr Glu Leu
        115                 120                 125

Glu Leu Leu Pro Ala Asp His Ile Gln Asn Asp Val Tyr Ile Arg His
    130                 135                 140

Pro Pro Ser Ile Glu Gln Tyr Leu Met Glu Gly Ser Tyr Asn Lys Ile
145                 150                 155                 160

Phe Leu Ala Lys Gly Asn Val Pro Ala Thr Asn Tyr Asn Phe Phe Met
                165                 170                 175

Asp Ile Leu Leu Asp Thr Ile Arg Gly Glu Ile Ala Asp Cys Leu Glu
            180                 185                 190

Lys Ala Tyr Glu Lys Ile Ser Ile Lys Asp Val Ala Arg Met Leu Tyr
        195                 200                 205

Leu Gly Ser Glu Glu Ser Ala Lys Ala Phe Val Thr Lys Ser Lys Thr
    210                 215                 220

Trp Lys Leu Glu Lys Asp Asn Phe Phe His Phe Thr Pro Glu Val Lys
225                 230                 235                 240

Lys Thr His Glu Pro Ile Leu Ser Lys Glu Leu Ala Gln Gln Ala Ile
                245                 250                 255

Glu Tyr Ala Lys Glu Leu Glu Met Ile Val
            260                 265

<210>  394
<211>  750
<212>  DNA
<213>  Leptinotarsa decemlineata

<400>  394
cgcccagcag tggtatcaac gcagagtacg cgggagacat tcaagtcttg tgatagtgca      60

ggcacggcag ttcaaataaa ctggtgcctt caatttattt atatatttat acttttttac     120

tagaaaccaa atactaacca atcaacatgt gtgacgaaga ggttgccgca ttagtcgtag     180

acaatggatc tggtatgtgc aaagctggat ttgctgggga tgatgccccc cgtgcagttt     240

tcccatccat tgttggtcgt ccaagacatc aaggagttat ggtaggaatg ggccaaaagg     300

actcgtatgt aggagatgaa gcccaaagca aaagaggtat ccttaccttg aaatacccca     360

ttgaacacgg tattgtcaca aactgggatg atatggagaa aatctggcac cataccttct     420

acaatgaact tcgagttgcc cccgaagagc accctgtttt gttgacagag gcaccattga     480

accccaaagc caacagggag aagatgaccc agatcatgtt tgaaaccttc aatacccccg     540

ccatgtacgt cgccatccaa gctgtattgt ctctgtatgc ttctggtcgt acaactggta     600

ttgtgctgga ttctggagat ggtgtttctc acacagtacc aatctatgaa ggttatgccc     660

ttcctcatgc catccttcgt ttggacttgg ctggtagaga cttgactgat taccttatga     720

aaattctgac tgaacgtggt tactctttca                                      750

<210>  395
<211>  204
<212>  PRT
<213>  Leptinotarsa decemlineata

<400>  395

Pro Ile Asn Met Cys Asp Glu Glu Val Ala Ala Leu Val Val Asp Asn
1               5                   10                  15

Gly Ser Gly Met Cys Lys Ala Gly Phe Ala Gly Asp Asp Ala Pro Arg
            20                  25                  30

Ala Val Phe Pro Ser Ile Val Gly Arg Pro Arg His Gln Gly Val Met
        35                  40                  45

Val Gly Met Gly Gln Lys Asp Ser Tyr Val Gly Asp Glu Ala Gln Ser
    50                  55                  60

Lys Arg Gly Ile Leu Thr Leu Lys Tyr Pro Ile Glu His Gly Ile Val
65                  70                  75                  80

Thr Asn Trp Asp Asp Met Glu Lys Ile Trp His His Thr Phe Tyr Asn
                85                  90                  95

Glu Leu Arg Val Ala Pro Glu Glu His Pro Val Leu Leu Thr Glu Ala
            100                 105                 110

Pro Leu Asn Pro Lys Ala Asn Arg Glu Lys Met Thr Gln Ile Met Phe
        115                 120                 125

Glu Thr Phe Asn Thr Pro Ala Met Tyr Val Ala Ile Gln Ala Val Leu
    130                 135                 140

Ser Leu Tyr Ala Ser Gly Arg Thr Thr Gly Ile Val Leu Asp Ser Gly
145                 150                 155                 160

Asp Gly Val Ser His Thr Val Pro Ile Tyr Glu Gly Tyr Ala Leu Pro
                165                 170                 175

His Ala Ile Leu Arg Leu Asp Leu Ala Gly Arg Asp Leu Thr Asp Tyr
            180                 185                 190

Leu Met Lys Ile Leu Thr Glu Arg Gly Tyr Ser Phe
        195                 200

<210>  396
<211>  44
<212>  DNA
<213>  Artificial

<220>
<223>  Primer

<400>  396
gcgtaatacg actcactata ggatgtgtga cgaagaggtt gccg                        44

<210>  397
<211>  24
<212>  DNA
<213>  Artificial

<220>
<223>  Primer

<400>  397
gtcaacaaaa cagggtgctc ttcg                                              24

<210>  398
<211>  22
<212>  DNA
<213>  Artificial

<220>
<223>  Primer

<400>  398
atgtgtgacg aagaggttgc cg                                                22


<210>  399
<211>  46
<212>  DNA
<213>  Artificial

<220>
<223>  Primer

<400>  399
gcgtaatacg actcactata gggtcaacaa aacagggtgc tcttcg                      46


<210>  400
<211>  320
<212>  DNA
<213>  Leptinotarsa decemlineata

<400>  400
atgtgtgacg aagaggttgc cgcattagtc gtagacaatg gatctggtat gtgcaaagct       60

ggatttgctg gggatgatgc cccccgtgca gttttcccat ccattgttgg tcgtccaaga      120

catcaaggag ttatggtagg aatgggccaa aaggactcgt atgtaggaga tgaagcccaa      180

agcaaaagag gtatccttac cttgaaatac cccattgaac acggtattgt cacaaactgg      240

gatgatatgg agaaaatctg gcaccatacc ttctacaatg aacttcgagt tgcccccgaa      300

gagcaccctg ttttgttgac                                                  320


<210>  401
<211>  1152
<212>  DNA
<213>  Artificial

<220>
<223>  Hairpin

<400>  401
tgacgacctt gagttggttt ctgaagttga actcggcagc cttcttctgg agcttggcga       60

atttgttttc gtacttggaa acctttttca aggtaggttt gacgaattta ccacggaggt      120

cgttgacctg gctgttgagg tcggcgatct caaggtcacg tctctccact tcgaattcga      180

tgtcaatttt actcctctcc agagcgtcaa ttcgcttatg gtagtctgtg cagagtttct      240

tcaaggttgc ttcattggcg ttgtcgacgt cggcaatttg cccgcagcgc tcctcaatcg      300

ttcgcctcct ctcagctgct ttgcgttcct gctccttctt cagttcctca gcggcttttt      360

tcctcagcag gagtcggagt ttcttcttcc tttccggggt catgaaaccc ttcttggctt      420

tcttcgcctt agaggcttcc tccatcctct tgcgcacttc agcgcgcttc ctctcgattt      480

cggcctgttt gtctagaagg taagtgtaca cactacattt tcatgaacat tattgcgacc 540

gttgagattc tcattgtttg gtgattgatt atctaaagta gaagcatgaa tagatataac 600

ataaactagt aactaatggg ttagttatgg gtatacttca tgcttttctc tcaggctcga 660

gcaaacaggc cgaaatcgag aggaagcgcg ctgaagtgcg caagaggatg gaggaagcct 720

ctaaggcgaa gaaagccaag aagggtttca tgaccccgga aaggaagaag aaactccgac 780

tcctgctgag gaaaaaagcc gctgaggaac tgaagaagga gcaggaacgc aaagcagctg 840

agaggaggcg aacgattgag gagcgctgcg ggcaaattgc cgacgtcgac aacgccaatg 900

aagcaacctt gaagaaactc tgcacagact accataagcg aattgacgct ctggagagga 960

gtaaaattga catcgaattc gaagtggaga gacgtgacct tgagatcgcc gacctcaaca 1020

gccaggtcaa cgacctccgt ggtaaattcg tcaaacctac cttgaaaaag gtttccaagt 1080

acgaaaacaa attcgccaag ctccagaaga aggctgccga gttcaacttc agaaaccaac 1140

tcaaggtcgt ca 1152

<210> 402
<211> 1192
<212> DNA
<213> Artificial

<220>
<223> Hairpin

<400> 402
ctcttgcttc ttggtggcga tcctctcctc gcgcctcttc ttcgcctcct tgaccttgag 60

acgtctcgcc tctgcctggt ccttcaacat ctttgatctc gccttttcag ccttcttctt 120

gtgaatgaag tccatcagta ccctcttgtt tttgaagacg ttacctttgg ctttcatgta 180

aaggtcgtgg tacatttgcc tatcgatctt cttggcttct ctgtattttt taaggagccg 240

tcgcaggact ctcattctgt tgacccacag gaccttcaca ggcattctgg cgttggcggt 300

acccttcctc ttaccgaagc cacagtgacg acccttccgt ctggcttctg tgtttttacg 360

gacgcgggct ctggagtgga cagccacagg ctttttgatg atcaaaccat ccttgatcag 420

cttacggatg ttttgcctag agttggtgtt ggcgatttcg ttgatttcat tagggtccaa 480

ccacactttc ttcttgccgc atctcatcac ctctagaagg taagtgtaca cactacattt 540

tcatgaacat tattgcgacc gttgagattc tcattgtttg gtgattgatt atctaaagta 600

gaagcatgaa tagatataac ataaactagt aactaatggg ttagttatgg gtatacttca 660

tgcttttctc tcaggctcga gggtgatgag atgcggcaag aagaaagtgt ggttggaccc 720

taatgaaatc aacgaaatcg ccaacaccaa ctctaggcaa aacatccgta agctgatcaa 780

ggatggtttg atcatcaaaa agcctgtggc tgtccactcc agagcccgcg tccgtaaaaa 840

cacagaagcc agacggaagg gtcgtcactg tggcttcggt aagaggaagg gtaccgccaa 900

cgccagaatg cctgtgaagg tcctgtgggt caacagaatg agagtcctgc gacggctcct 960

taaaaaatac agagaagcca agaagatcga taggcaaatg taccacgacc tttacatgaa 1020

agccaaaggt aacgtcttca aaaacaagag ggtactgatg gacttcattc acaagaagaa 1080

ggctgaaaag gcgagatcaa agatgttgaa ggaccaggca gaggcgagac gtctcaaggt 1140

caaggaggcg aagaagaggc gcgaggagag gatcgccacc aagaagcaag ag 1192

<210> 403
<211> 792
<212> DNA
<213> Artificial

<220>
<223> Hairpin

<400> 403
actctgtctg gcttttggct gtgacgcaca gttcatagag ataaccttca cccgaatatg 60

ccttgcgagg tcgcaaaatc ggcgaaattc catacctgtt caccgacgac ggcgctggat 120

caattccaca gttttcgcga tccagactga atgcccacag gccgtcgagt tttttgattt 180

cagatacgta cacttttccc ggcaataaca tacggcgtga catcggcttc aaatggcgta 240

tagccgccct gatgctccat cacactttgc cgtaatgagt gaccgcatcg aaacgcagca 300

cgatacgctg gtctagaagg taagtgtaca cactacattt tcatgaacat tattgcgacc 360

gttgagattc tcattgtttg gtgattgatt atctaaagta gaagcatgaa tagatataac 420

ataaactagt aactaatggg ttagttatgg gtatacttca tgcttttctc tcaggctcga 480

gccagcgtat cgtgctgcgt ttcgatgcgg tcactcatta cggcaaagtg tgatggagca 540

tcagggcggc tatacgccat ttgaagccga tgtcacgccg tatgttattg ccgggaaaag 600

tgtacgtatc tgaaatcaaa aaactcgacg gcctgtgggc attcagtctg gatcgcgaaa 660

actgtggaat tgatccagcg ccgtcgtcgg tgaacaggta tggaatttcg ccgattttgc 720

gacctcgcaa ggcatattcg ggtgaaggtt atctctatga actgtgcgtc acagccaaaa 780

gccagacaga gt 792

<210> 404
<211> 839
<212> DNA
<213> leptinotarsa decemlineata

<400> 404
cagtcagcac cgagtccttg ttgactgctc acattttcca tcgtttctac cagaacaaca 60

gcaacaactt tcatcatggc ggacgacgag gaaaagagga ggaaacaagc ggaaattgaa 120

cgcaagaggg ctgaggtcag ggctcgcatg gaagaggcct ccaaggccaa aaaagccaag 180

aaaggtttca tgacccctga gaggaagaag aaacttaggt tattgctgag aaagaaagca 240

gcagaagaac tgaaaaaaga acaagaacgc aaagctgccg aaaggcgtat tattgaagag 300

agatgcggaa aaccaaaact cattgatgag gcaaatgaag agcaggtgag gaactattgc 360

aagttatatc acggtagaat agctaaactg gaggaccaga aatttgattt ggaatacctt 420

gtcaaaaaga aagacatgga gatcgccgaa ttgaacagtc aagtcaacga cctcaggggt 480

aaattcgtca aacccactct caagaaagta tccaaatacg agaacaaatt tgctaaactc 540

caaaagaaag cagcagaatt caatttccgt aatcaactga aagttgtaaa gaagaaggag 600

ttcaccctgg aggaggaaga caaagaaaag aagcccgatt ggtcgaagaa gggagacgaa 660

aagaaggtac aagaagtgga agcatgatct gtccctacaa tttaatattt cccttcgtcc 720

gtggaaattt tacaacttaa gatatattta ttttattcgc ttcttatgag actatgaaag 780

tgatgtctgc atgtatatta ttcgttttat gtatgtatta aaaaaagaac ttgattgaa 839

<210> 405
<211> 203
<212> PRT
<213> leptinotarsa decemlineata

<400> 405

Met Ala Asp Asp Glu Glu Lys Arg Arg Lys Gln Ala Glu Ile Glu Arg
1               5                   10                  15

Lys Arg Ala Glu Val Arg Ala Arg Met Glu Glu Ala Ser Lys Ala Lys
            20                  25                  30

Lys Ala Lys Lys Gly Phe Met Thr Pro Glu Arg Lys Lys Lys Leu Arg
        35                  40                  45

Leu Leu Leu Arg Lys Lys Ala Ala Glu Glu Leu Lys Lys Glu Gln Glu
    50                  55                  60

Arg Lys Ala Ala Glu Arg Arg Ile Ile Glu Glu Arg Cys Gly Lys Pro
65                  70                  75                  80

Lys Leu Ile Asp Glu Ala Asn Glu Glu Gln Val Arg Asn Tyr Cys Lys
                85                  90                  95

Leu Tyr His Gly Arg Ile Ala Lys Leu Glu Asp Gln Lys Phe Asp Leu
            100                 105                 110

Glu Tyr Leu Val Lys Lys Lys Asp Met Glu Ile Ala Glu Leu Asn Ser
        115                 120                 125

Gln Val Asn Asp Leu Arg Gly Lys Phe Val Lys Pro Thr Leu Lys Lys
    130                 135                 140

Val Ser Lys Tyr Glu Asn Lys Phe Ala Lys Leu Gln Lys Lys Ala Ala
145                 150                 155                 160

Glu Phe Asn Phe Arg Asn Gln Leu Lys Val Val Lys Lys Lys Glu Phe
                165                 170                 175

Thr Leu Glu Glu Glu Asp Lys Glu Lys Lys Pro Asp Trp Ser Lys Lys
            180                 185                 190

Gly Asp Glu Lys Lys Val Gln Glu Val Glu Ala
        195                 200